(19) Europäisches Patentamt
European Patent Office
Office européen des brevets

(11) **EP 4 450 506 A1**

(12) **EUROPEAN PATENT APPLICATION**
published in accordance with Art. 153(4) EPC

(43) Date of publication:
**23.10.2024 Bulletin 2024/43**

(21) Application number: **22906709.5**

(22) Date of filing: **16.12.2022**

(51) International Patent Classification (IPC):
***C07D 491/22*** (2006.01) ***C07D 495/22*** (2006.01)
***A61K 47/68*** (2017.01) ***A61P 35/00*** (2006.01)

(52) Cooperative Patent Classification (CPC):
**A61K 47/68; A61P 35/00; C07D 491/22; C07D 495/22**

(86) International application number:
**PCT/CN2022/139765**

(87) International publication number:
**WO 2023/109965 (22.06.2023 Gazette 2023/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.12.2021 CN 202111544686**

(71) Applicants:
- **Mabwell (Shanghai) Bioscience Co., Ltd.**
  **Shanghai 201210 (CN)**
- **Jiangsu Mabwell Health Pharmaceutical R&D Co., Ltd.**
  **China Medical City**
  **Taizhou, Jiangsu 225300 (CN)**

(72) Inventors:
- **ZHOU, Wei**
  **Taizhou, Jiangsu 225300 (CN)**
- **XU, Hui**
  **Taizhou, Jiangsu 225300 (CN)**
- **ZHU, Huikai**
  **Taizhou, Jiangsu 225300 (CN)**
- **WANG, Zhenzhen**
  **Taizhou, Jiangsu 225300 (CN)**
- **TAN, Xiaoding**
  **Taizhou, Jiangsu 225300 (CN)**

(74) Representative: **Schiweck Weinzierl Koch Patentanwälte Partnerschaft mbB**
**Ganghoferstraße 68 B**
**80339 München (DE)**

Remarks:
The complete document including Reference Table(s) and the Sequence Listing(s) can be downloaded from the EPO website

# (54) CAMPTOTHECIN COMPOUND AND CONJUGATE THEREOF

(57) Provided is a camptothecin compound, which is a compound represented by structural formula I or a pharmaceutically acceptable salt, stereoisomer, solvate or prodrug thereof. An antibody-drug conjugate comprising the camptothecin compound is also provided.

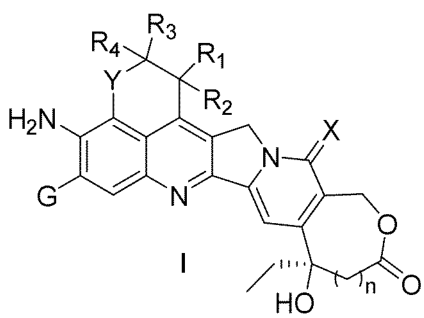

I

EP 4 450 506 A1

## Description

### CROSS-REFERENCE TO RELATED APPLICATION(S)

The present patent application claims the benefit of priority to Chinese Patent Application for Invention No. CN202111544686.7 filed on 16 December 2021, which is incorporated herein by reference in its entirety for all purposes.

### TECHNICAL FIELD

The invention belongs to the field of biotechnology, and more particularly relates to a camptothecin analogue having a novel structure and application thereof in the preparation of medicaments, especially antibody-drug conjugates.

### BACKGROUND OF THE INVENTION

DNA Topoisomerase is an essential enzyme widely existing in living bodies and is a general name for enzymes catalyzing mutual conversion between DNA topoisomers, which are mainly divided into two types, i.e., Topoisomerase I (Topo I) and Topoisomerase II (Topo II). Among them, Topoisomerase I has high expression in a variety of tumor cells, e.g., colon cancer cells, cervical cancer cells, and ovarian cancer cells, showing a content largely higher than its content in normal tissues or cells, and exhibits a greatly increased activity in S-phase tumor cells. Therefore, an activity inhibitor aiming at Topoisomerase I can selectively inhibit the DNA replication in the tumor cells in proliferative phase.

Researches have shown that Topoisomerase I is the main target of Camptothecin (CPT) and its analogues. Camptothecin is a cytotoxic quinoline alkaloid, and stabilizes the covalent complexes of Topoisomerase I and DNA strands that would be dissociated normally, forming ternary complexes. As ternary complexes form, CPT inhibits DNA cleavage/religation reactions initially mediated by Topoisomerase I, so exerts an anticancer effect by inhibiting DNA synthesis and leading to cell death.

In view of the above properties, Camptothecin and its analogues have become an important class of antitumor drugs; and have been used in antibody-drug conjugates as small molecule drugs. Antibody-drug conjugate (ADC) is a novel therapeutic for tumor treatment, which is generally composed of an antibody or antibody-like ligand, a small molecule drug, and a linker coupling the two together. Antibody-drug conjugate (ADC) combines the anti-tumor activity of the small molecule drug with the high selectivity and stability and good pharmacokinetic characteristics of the antibody or antibody-like ligand, and currently is an attentive hotspot in the field of tumor treatment.

So far ADCs prepared using Camptothecins (CPTs) at home and abroad mainly comprise:

1. Sacituzumab govitecan (IMMU-132), an antibody-drug conjugate targeting Trop-2 with DAR of 8 prepared through coupling topoisomerase I inhibitor SN38 with the anti-Trop-2 antibody SAC via CL2A-SN38 (a drug-containing linker).

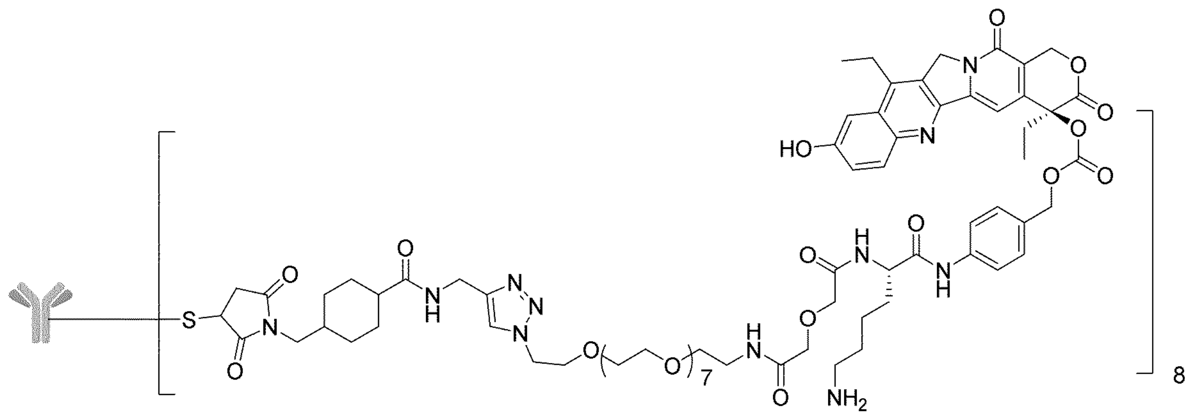

IMMU-132

2. Enhertu (DS-8201) developed by Daiichi Sankyo, an antibody-drug conjugate with DAR of 8 prepared through coupling Exatecan analogues with the anti-HER2 antibody Trastuzumab via MC-GGFG-Dxd (a drug-containing linker).

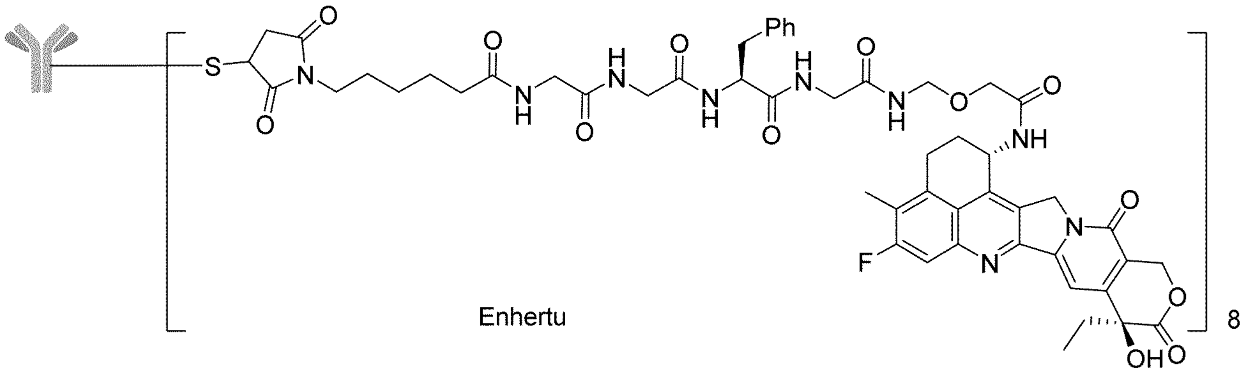

Enhertu

3. DS-1062 and DS-7300 in research by Daiichi Sankyo. DS-1062 is an antibody-drug conjugate with DAR of 4 prepared through coupling Exatecan analogues with the anti-Trop-2 antibody hTINAI via MC-GGFG-Dxd (a drug-containing linker) by means of stochastic conjugation. DS-7300 is an antibody-drug conjugate targeting B7H3 with DAR of 4 prepared through coupling MC-GGFG-Dxd (a drug-containing linker) with the anti-B7H3 antibody hM30 by means of stochastic conjugation.

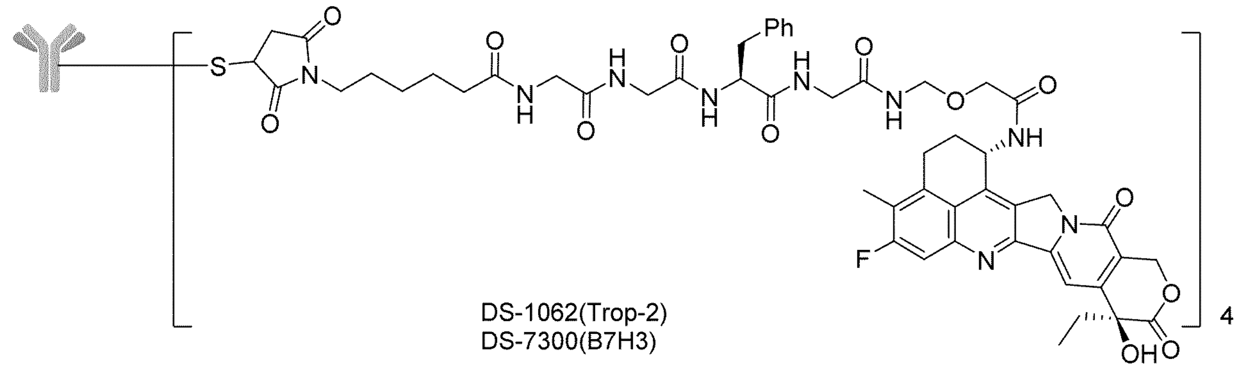

DS-1062(Trop-2)
DS-7300(B7H3)

The ADCs above show some therapeutic effects, while have certain problems. For example, due to the chemical instability of the carbonate linkage in IMMU-132, IMMU-132 exhibited a half-life in plasma of only about 12 hours, and caused problems such as increased side effects which made patients have diarrhea, fatigue, nausea, febrile neutropenia, leukopenia and other toxic reactions in varying degrees (US2014/0170063A1). As for DS-1062 and DS-7300, the stochastic conjugation manner results in a rather great heterogeneity of the ADC products.

Presently available types of ADCs containing camptothecins are still few, and disadvantages e.g. narrow range of target population, poor treatment effect of single medicines, strong toxic and side effects, and the like exist. Therefore, there is still a need to develop new camptothecins and corresponding ADCs to meet treatment needs.

## SUMMARY OF THE INVENTION

In view of the above problems, the object of the present disclosure is to provide a compound having a novel structure, which is a compound of camptothecins itself or a compound formed by linking a compound of camptothecins to a linker, and an antibody-drug conjugate prepared using the same.

In the context of the present disclosure, halogen refers to fluorine (F), chlorine (Cl), bromine (Br) or iodine (I).

In the context of the present disclosure, term "linker" and term "linker compound" are used interchangeably.

In the context of the present disclosure, term "drug-containing linker" refers to a compound resulting from covalent bonding of a drug (e.g., a small molecule drug such as a compound of camptothecins) to a linker directly or indirectly.

In the context of the present disclosure, in the case a group is substituted, it may be substituted by one or more substituents, and the number of substituents depends on the number of hydrogen atoms comprised by the group, and all hydrogen atoms are substitutable.

The present disclosure provides technical solutions as follows.

In the first aspect, the present disclosure provides a compound of camptothecins.

In a first aspect of the present disclosure, the compound of camptothecins is a compound represented by structural formula I or a pharmaceutically acceptable salt, stereoisomer, solvate or prodrug thereof:

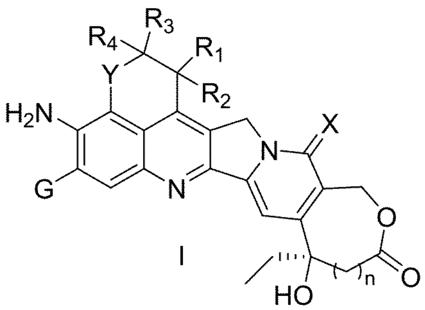

I

structural formula I.

In structural formula I, and if not otherwise stated in the context of the present disclosure, $R_1$ $R_2$, $R_3$, and $R_4$ independently of one another are selected from the group consisting of hydrogen, halogen, hydroxyl, C1-6 alkoxy, amino or substituted amino, C1-7 alkyl or substituted C1-7 alkyl, or any two of $R_1$, $R_2$, $R_3$, and $R_4$, together with the carbon atoms to which they are bonded, form a C3-6 cycloalkyl. When $R_1$, $R_2$, $R_3$, and $R_4$ independently of one another are C1-6 alkoxy, the C1-6 alkoxy includes linear or branched C1-6 alkoxy, preferably linear or branched C1-3 alkoxy, more preferably methoxy. When $R_1$, $R_2$, $R_3$, and $R_4$ independently of one another are substituted amino, the substituted amino is an amino substituted with one or more substituents selected from the group consisting of methyl and ethyl. When $R_1$, $R_2$, $R_3$, and $R_4$ independently of one another are Cl-7 alkyl or substituted C1-7 alkyl, the C1-7 alkyl or substituted C1-7 alkyl includes linear or branched C1-7 alkyl or substituted C1-7 alkyl, and the substituted C1-7 alkyl is a C1-7 alkyl substituted with one or more substituents selected from the group consisting of cyclopropyl and cyclobutyl; or, the linear or branched C1-7 alkyl or substituted C1-7 alkyl is preferably C1-3 alkyl or substituted C1-3 alkyl, e.g., methyl, and halomethyl (preferably trifluoromethyl).

In structural formula I, and if not otherwise stated in the context of the present disclosure, G is hydrogen, halogen, methyl or methoxy. Preferably, G is hydrogen, fluorine or chlorine.

In structural formula I, and if not otherwise stated in the context of the present disclosure, Y is oxygen, sulfur, sulfone, sulfoxide, methylene, or substituted methylene. In the substituted methylene, either one hydrogen of methylene may be substituted, or both hydrogens of methylene may be substituted, with a substituent which can be benzyl or alkyl; and when the substituent is alkyl, the alkyl and $R_3$ and/or $R_4$, together with the carbon atoms to which they are bonded, may form a C3-6 fused or spiro ring structure. When Y is a substituted methylene, the substituted methylene is preferably a methylene substituted with alkyl, more preferably a linear or branched C1-4 alkyl. Preferably, Y is oxygen, sulfur, sulfone, or sulfoxide; or, preferably, Y is oxygen, sulfur or methylene.

In structural formula I, and if not otherwise stated in the context of the present disclosure, X is oxygen or sulfur.

In structural formula I, and if not otherwise stated in the context of the present disclosure, n = 0 or 1.

In structural formula I, in the case $R_1$, $R_2$, $R_3$, and $R_4$ are hydrogen simultaneously, X is oxygen and n = 0, G cannot be hydrogen or fluorine when Y is methylene; and G cannot be hydrogen when Y is oxygen or sulfur.

Preferably, $R_1$, $R_2$, $R_3$, and $R_4$ independently of one another are hydrogen, halogen (e.g., fluorine), C1-7 alkyl or substituted C1-7 alkyl, or any two of $R_1$, $R_2$, $R_3$, and $R_4$, together with the carbon atoms to which they are bonded, form a C3-6 cycloalkyl (e.g., C3-5 cycloalkyl). Further, $R_1$ and $R_2$ may be the same; and/or, $R_3$ and $R_4$ may be the same.

Preferably, Y is a methylene substituted with alkyl, and the alkyl and $R_3$ and/or $R_4$, together with the carbon atoms to which they are bonded, may form a C3-6 fused or spiro ring structure.

Preferably, X may be oxygen.

Preferably, X is oxygen, G is hydrogen, halogen (e.g. fluorine or chlorine), methyl or methoxy, and Y and $R_1$, $R_2$, $R_3$, and $R_4$ are defined as above.

Preferably, X is oxygen, G is hydrogen, Y is methylene or substituted methylene, oxygen or sulfur, and $R_1$, $R_2$, $R_3$, and $R_4$ are defined as above.

Preferably, X is oxygen, G is fluorine, Y is methylene or substituted methylene, oxygen or sulfur, and $R_1$, $R_2$, $R_3$, and $R_4$ are defined as above.

Preferably, X is oxygen, G is chlorine, Y is methylene or substituted methylene, oxygen or sulfur, and $R_1$, $R_2$, $R_3$, and $R_4$ are defined as above.

Preferably, X is oxygen, G is methyl, Y is methylene or substituted methylene, oxygen or sulfur, and $R_1$, $R_2$, $R_3$, and $R_4$ are defined as above.

Preferably, X is oxygen, G is methoxy, Y is methylene or substituted methylene, oxygen or sulfur, and $R_1$, $R_2$, $R_3$, and $R_4$ are defined as above.

Preferably, X is oxygen, G is hydrogen, Y is oxygen, sulfone or sulfoxide, and $R_1$, $R_2$, $R_3$, and $R_4$ are defined

as above.

Preferably, X is oxygen, G is hydrogen, Y is sulfone or sulfoxide, and $R_1$, $R_2$, $R_3$, and $R_4$ are defined as above.

Preferably, the compound represented by structural formula I provided by the present disclosure is further a compound represented by structural formula IA:

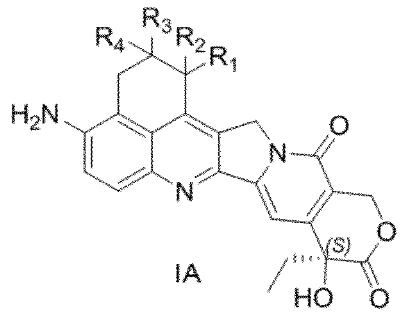

structural formula IA.

In structural formula IA, groups $R_1$, $R_2$, $R_3$, and $R_4$ have the same meaning as defined above for the groups $R_1$, $R_2$, $R_3$, and $R_4$ in structural formula I, but $R_1$, $R_2$, $R_3$, and $R_4$ are not hydrogen simultaneously.

Alternatively, in the first aspect of the present disclosure, the compound of camptothecins is a compound represented by structural formula II or a pharmaceutically acceptable salt, stereoisomer, solvate or prodrug thereof:

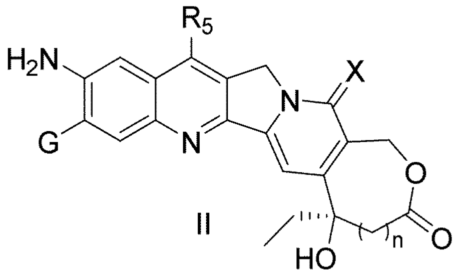

structural formula II.

In structural formula II, and if not otherwise stated in the context of the present disclosure, $R_5$ is C1-5 alkyl or C1-5 alkyl substituted with one or more substituents, C3-6 cycloalkyl or C3-6 cycloalkyl substituted with one or more substituents, phenyl or substituted phenyl. When $R_5$ is C1-5 alkyl or substituted C1-5 alkyl, the C1-5 alkyl includes linear or branched C1-5 alkyl. Further, $R_5$ is C1-4 linear alkyl. When $R_5$ is substituted C1-5 alkyl or substituted C3-6 cycloalkyl, the substituted C1-5 alkyl or substituted C3-6 cycloalkyl is C1-5 alkyl or C3-6 cycloalkyl substituted with substituent(s) selected from the group consisting of halogen, hydroxyl, methoxy, trifluoromethyl, amino or substituted amino, methanesulfonyl and C3-6 cycloalkyl; and among the substituent(s), the substituted amino is an amino substituted with one or more substituents selected from the group consisting of methyl and ethyl. When $R_5$ is substituted phenyl, the substituted phenyl is a phenyl substituted with substituent(s) selected from the group consisting of alkyl (e.g., C1-6 alkyl, preferably C1-3 alkyl) and halogen.

In structural formula II, and if not otherwise stated in the context of the present disclosure, G is hydrogen, halogen (e.g., fluorine), methyl, or methoxy. Preferably, G is hydrogen, fluorine or chlorine.

In structural formula II, X is oxygen or sulfur.

In structural formula II, n = 0 or 1.

In structural formula II, when X is oxygen, G is hydrogen, and n = 0, $R_5$ cannot be n-butyl.

Preferably, the compound represented by structural formula II provided by the present disclosure is further a compound represented by structural formula IIA:

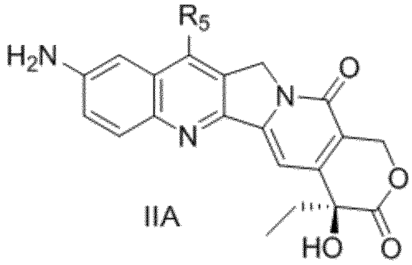

IIA

structural formula IIA.

In structural formula IIA, group $R_5$ has the same meaning as defined above for the group $R_5$ in structural formula II, but $R_5$ cannot be n-butyl.

According to particular embodiments of the present disclosure, in the first aspect of the present disclosure, the compound has one of the following structures:

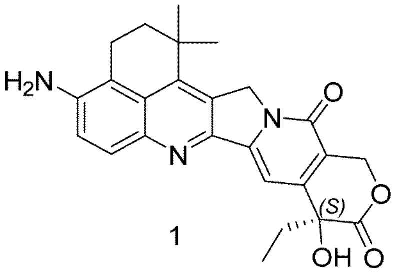
1

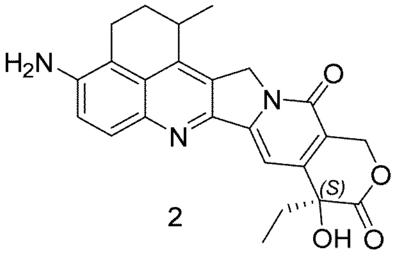
2

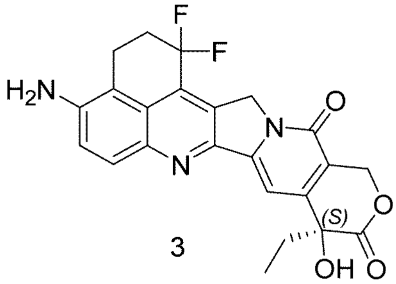
3

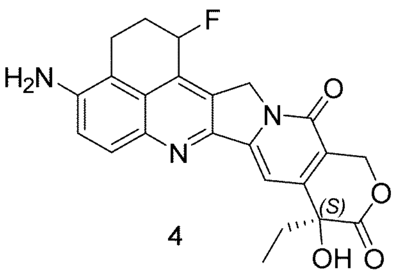
4

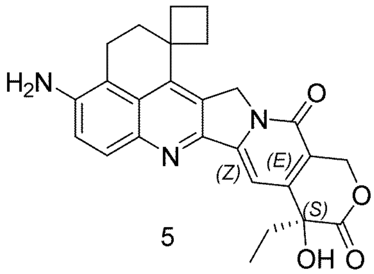
5

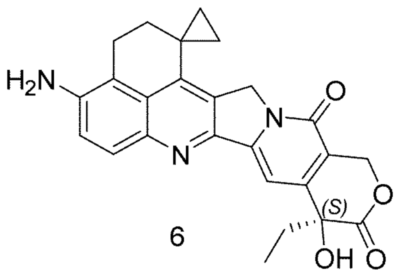
6

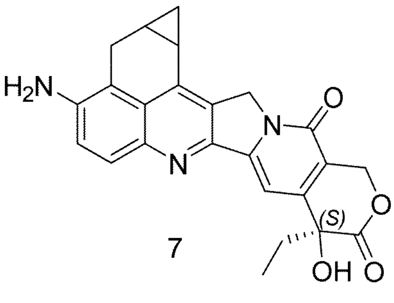
7

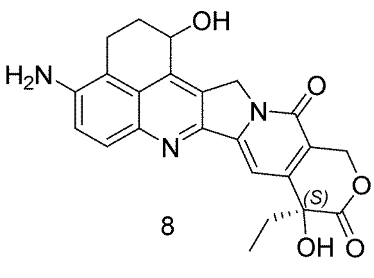
8

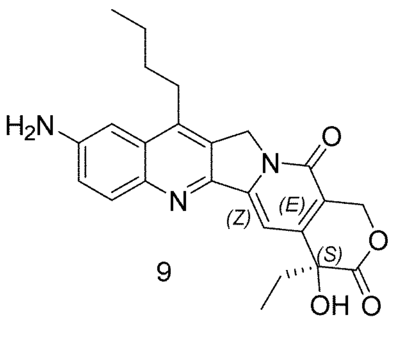
9

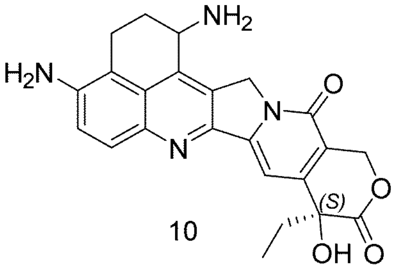
10

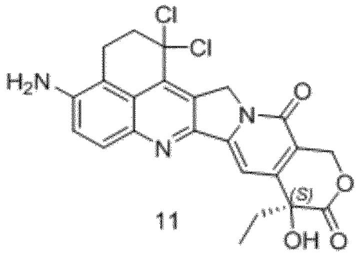
11

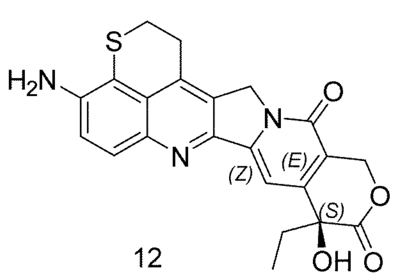
12

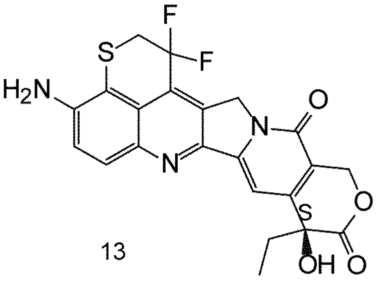
13

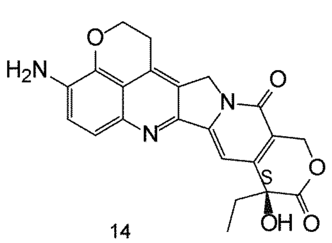
14

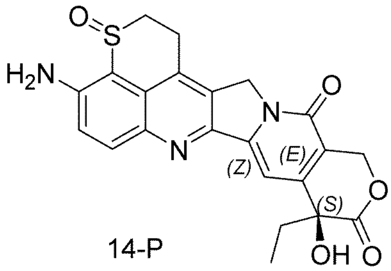
14-P

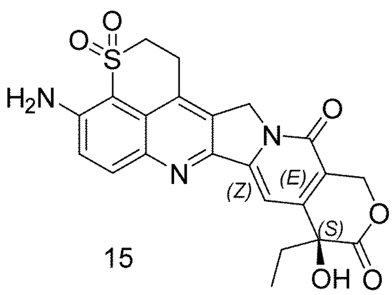
15

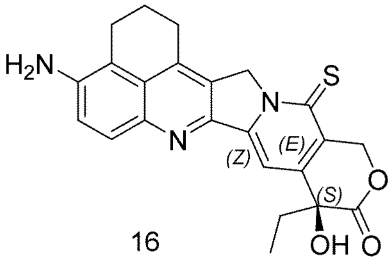
16

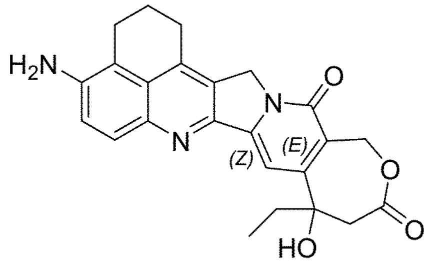
17

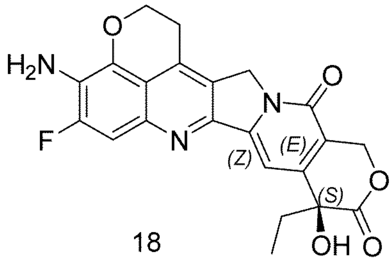
18

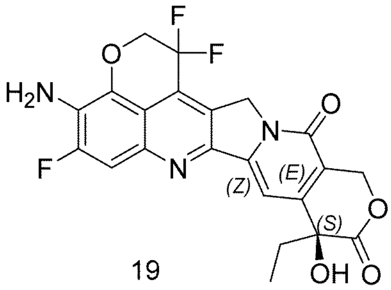
19

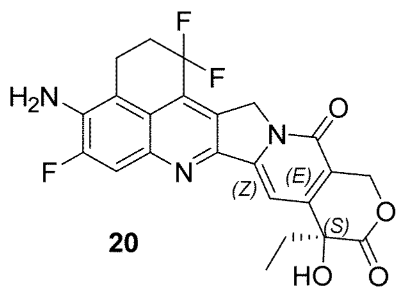
**20**

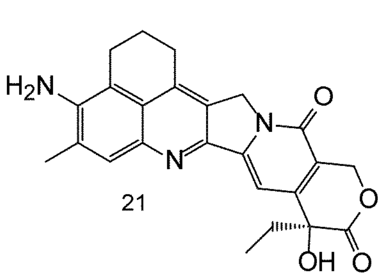
21

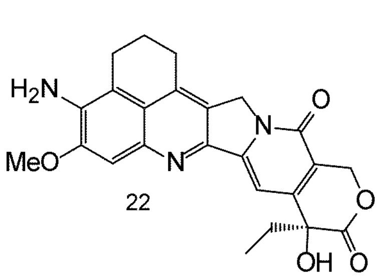
22

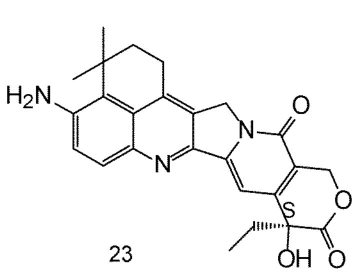
23

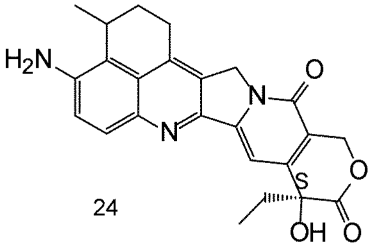
24

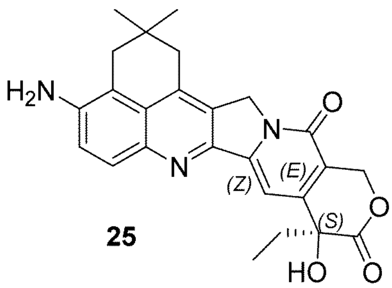
**25**

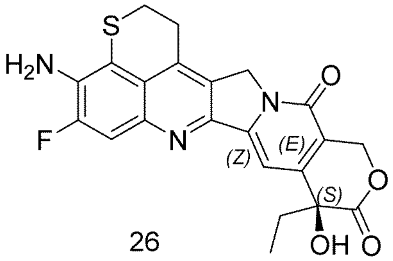
26

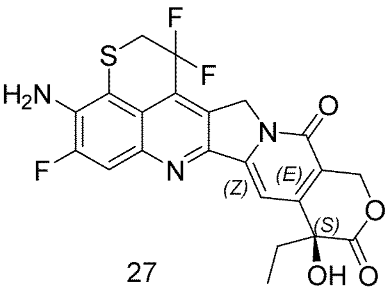
27

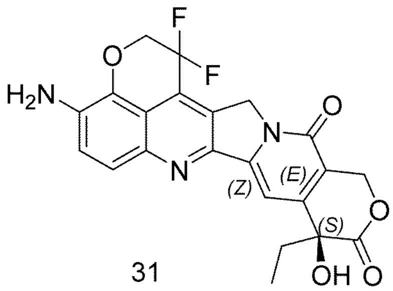
31

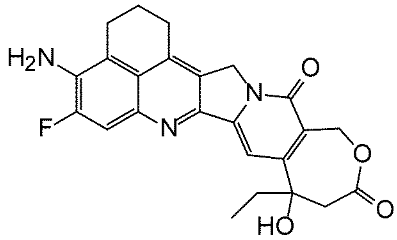
32

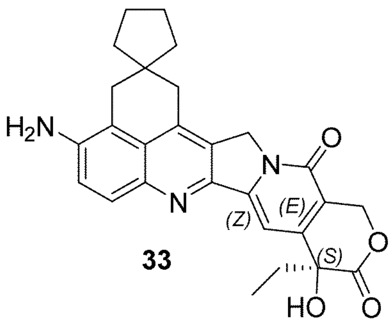
**33**

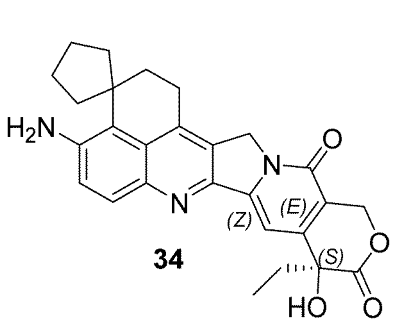
**34**

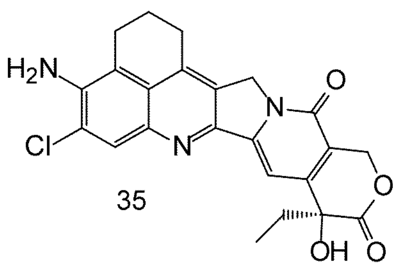
35

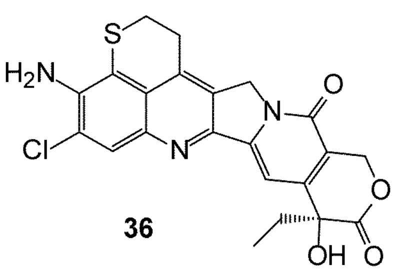
**36**

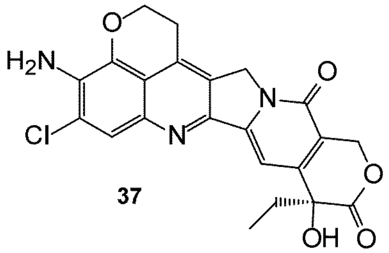
**37**

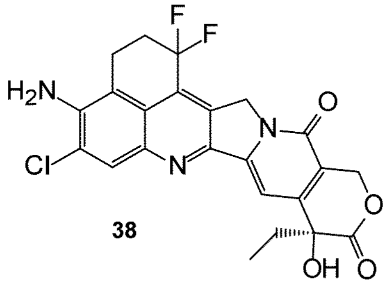
**38**

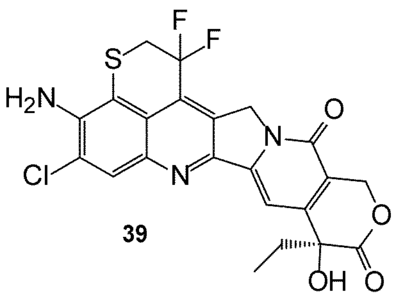
**39**

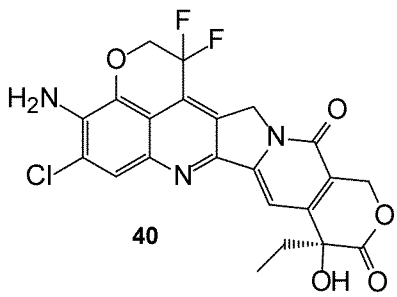
**40**

.

In a second aspect, the present disclosure provides a drug-containing linker having a structure represented by general formula "L-A-CPT", in which L means a linker used in an antibody-drug conjugate (ADC), A means a peptide

group of one or more amino acids, and CPT is a compound of camptothecins.

In the second aspect of the present disclosure, the drug-containing linker having the structure represented by the general formula "L-A-CPT" is a compound represented by structural formula III or a pharmaceutically acceptable salt, stereoisomer, solvate or prodrug thereof:

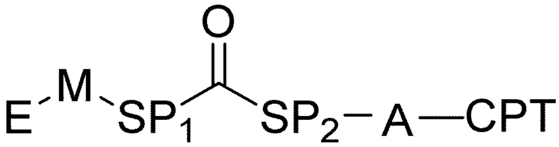

III

structural formula III.

In structural formula III, and if not otherwise stated in the context of the present disclosure, E is selected from the group consisting of the following groups, in which ∿ means the bonding site to M:

E-1: 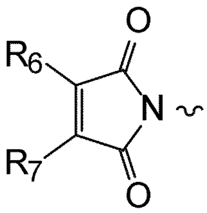 ; E-2: 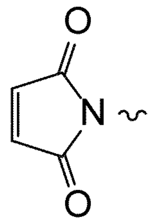 ; E-3: 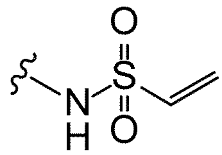 ;

E-4: 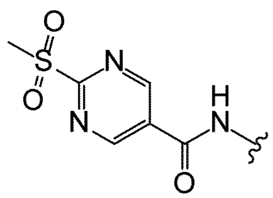 ; E-5: 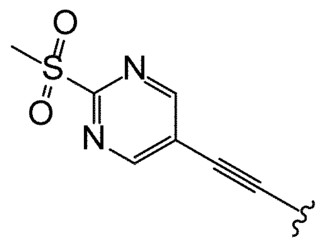 ; and E-6: 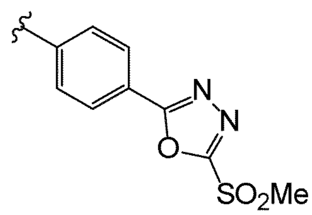 .

In structural formula III, and if not otherwise stated in the context of the present disclosure, M is phenylene or phenylene substituted with one or more substituents, or a chemical bond; and in the substituted phenylene, the phenylene is substituted with substituent(s) selected from the group consisting of alkyl (e.g., C1-C6 alkyl, preferably C1-C4 alkyl), haloalkyl (e.g., C1-C6 haloalkyl, preferably C1-C4 haloalkyl, e.g., trifluoromethyl), alkoxy (e.g., C1-C6 alkoxy, preferably C1-C4 alkoxy, preferably methoxy), halogen, ester, amide, and cyano; and preferably, M is halogen-substituted phenylene.

In structural formula III, and if not otherwise stated in the context of the present disclosure, $SP_1$ is selected from the group consisting of C1-8 alkylene, C1-8 cycloalkylene, or C1-21 (preferably C1-16, more preferably C1-11) linear heteroalkylene comprising 1-11 (preferably 1-6) heteroatoms selected from the group consisting of N, O, and S, in which the C1-8 alkylene, Cl-8 cycloalkylene, and C1-21 linear heteroalkylene independently of one another are optionally substituted with one or more substituents selected from the group consisting of hydroxyl, amino, sulfonic acid group, and cyano.

In structural formula III, and if not otherwise stated in the context of the present disclosure, $SP_2$ is selected from

the group consisting of -NH(CH2CH2O)aCH2CH2CO-, -NH(CH2CH2O)aCH2CO-, -S(CH2)aCO-, or a chemical bond, in which a is an integer in the range of 1 to 20, preferably an integer in the range of 1 to 10, more preferably an integer in the range of 1 to 6.

In structural formula III, and if not otherwise stated in the context of the present disclosure, A means a peptide group of 2 to 4 amino acids. When A means a peptide group of 2 amino acids, it may be *NH*-Phe-Lys-*CO*, *NH*-Val-Ala-*CO*, *NH*-Val-Lys-*CO*, *NH*-Ala-Lys-*CO*, *NH*-Val-Cit-*CO*, *NH*-Phe-Cit-*CO*, *NH*-Leu-Cit-*CO*, *NH*-Phe-Arg-*CO*, or *NH*-Gly-Val-*CO*, preferably *NH*-Phe-Lys-*CO*, *NH*-Val-Ala-*CO,* or *NH*-Val-Cit-*CO*. When A means a peptide group of 3 amino acids, it may be *NH*-Glu-Val-Ala-*CO*, *NH*-Glu-Val-Cit-*CO*, or *NH*-Ala-Ala-Ala-*CO*, preferably *NH*-Glu-Val-Ala-*CO* or *NH*-Ala-Ala-Ala-*CO*. When A means a peptide group of 4 amino acids, it may be *NH*-Gly-Gly-Phe-Gly-*CO* or *NH*-Gly-Phe-Gly-Gly-*CO*, preferably *NH*-Gly-Gly-Phe-Gly-*CO*. Preferably, A is *NH*-Val-Ala-*CO*, *NH*-Gly-Gly-Phe-Gly-*CO* or *NH*-Ala-Ala-Ala-*CO*.

In structural formula III, and if not otherwise stated in the context of the present disclosure, CPT is a compound of camptothecins.

When group E is E1, structural formula III provided by the present disclosure is further structural formula IIIA:

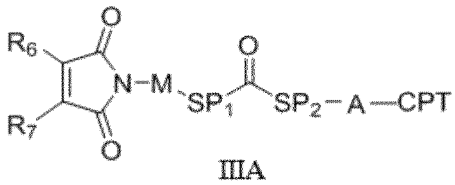

IIIA

structural formula IIIA.

In structural formula IIIA, and if not otherwise stated in the context of the present disclosure, $R_6$ and $R_7$ independently of one another are hydrogen, halogen or Ar'S in which Ar' is phenyl or phenyl substituted with one or more substituents; and in the substituted phenyl, the phenyl is substituted with substituent(s) selected from the group consisting of alkyl (e.g., C1-C6 alkyl, preferably C1-C4 alkyl), alkoxy (e.g., C1-C6 alkoxy, preferably C1-C4 alkoxy, preferably methoxy), halogen, ester, amide, and cyano. Preferably, Ar' is phenyl, phenyl substituted with 4-formylmethylamine

(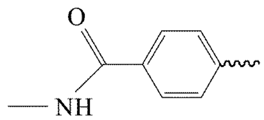)

or phenyl substituted with 4-formylmorpholine

(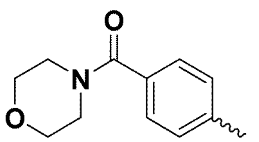).

For example, in structural formula III and structural formula IIIA, CPT is a compound represented by structural formula I or structural formula IA or a pharmaceutically acceptable salt, stereoisomer, solvate or prodrug thereof, as well as one of the corresponding specific compounds, all of which are provided in the first aspect of the present disclosure above.

When CPT is a compound represented by structural formula I or a pharmaceutically acceptable salt, stereoisomer, solvate or prodrug thereof which is provided in the first aspect of the present disclosure above:

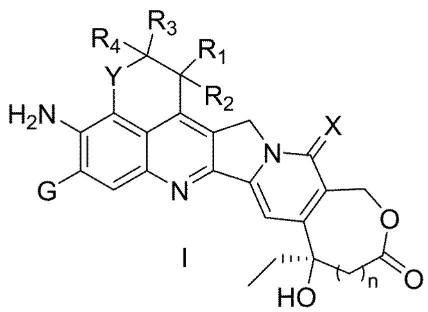

structural formula I,

in structural formula I, groups G, X, Y, $R_1$, $R_2$, $R_3$, $R_4$, and n have the same meaning as defined above in the first aspect for the groups G, X, Y, $R_1$, $R_2$, $R_3$, $R_4$, and n in structural formula I.

When CPT is a compound represented by structural formula IA or a pharmaceutically acceptable salt, stereoisomer, solvate or prodrug thereof which is provided in the first aspect of the present disclosure above:

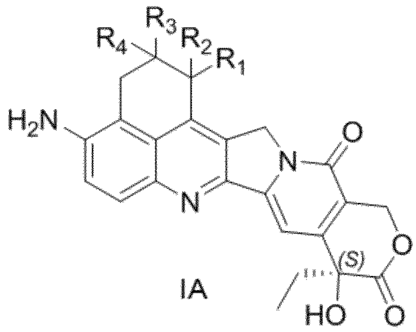

structural formula IA,

in structural formula IA, groups $R_1$, $R_2$, $R_3$, and $R_4$ have the same meaning as defined above in the first aspect for the groups $R_1$, $R_2$, $R_3$, and $R_4$ in structural formula IA, but $R_1$, $R_2$, $R_3$, and $R_4$ may be hydrogen simultaneously.

In particular, when "CPT" in the compound represented by structural formula IIIA is a compound represented by structural formula IA or a pharmaceutically acceptable salt, stereoisomer, solvate or prodrug thereof, $R_6$ and $R_7$ may or may not be hydrogen simultaneously. Similarly, $R_1$, $R_2$, $R_3$, and $R_4$ in structural formula IA may or may not be hydrogen simultaneously. According to particular embodiments of the present disclosure, in the case $R_6$ and $R_7$ are hydrogen simultaneously, $R_1$, $R_2$, $R_3$, and $R_4$ in structural formula IA cannot be hydrogen simultaneously; and in the case $R_6$ and $R_7$ are not hydrogen simultaneously, $R_1$, $R_2$, $R_3$, and $R_4$ in structural formula IA may or may not be hydrogen simultaneously.

In structural formula III and structural formula IIIA, the compound represented by structural formula I or structural formula IA is bonded to the carboxyl of A through an amide bond via its amino (respectively shown in structural formula I or structural formula IA), namely an amide bond is formed between the amino of the compound represented by structural formula I or structural formula IA and the carboxyl of A in structural formula III or IIIA.

For another example, in structural formula III and structural formula IIIA, CPT is a compound represented by structural formula II or structural formula IIA or a pharmaceutically acceptable salt, stereoisomer, solvate or prodrug thereof, as well as one of the corresponding specific compounds, all of which are provided in the first aspect of the present disclosure above.

When CPT is a compound represented by structural formula II or a pharmaceutically acceptable salt, stereoisomer, solvate or prodrug thereof which is provided in the first aspect of the present disclosure above:

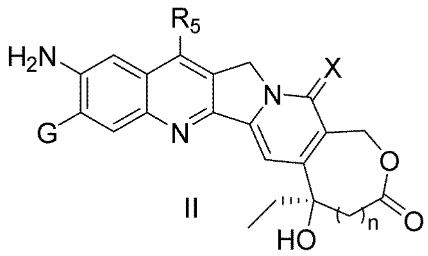

structural formula II,

in structural formula II, groups G, $R_5$, X, and n have the same meaning as defined above in the first aspect for the groups G, $R_5$, X, and n in structural formula II.

When CPT is a compound represented by structural formula IIA or a pharmaceutically acceptable salt, stereoisomer, solvate or prodrug thereof which is provided in the first aspect of the present disclosure above:

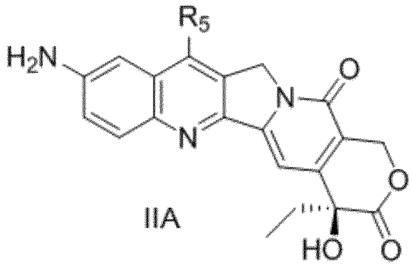

structural formula IIA,

in structural formula IIA, group $R_5$ has the same meaning as defined above in the first aspect for the group $R_5$ in structural formula IIA, but group $R_5$ may be n-butyl.

In structural formula III and structural formula IIIA, the compound represented by structural formula II or structural formula IIA is bonded to the carboxyl of A through an amide bond via its amino (respectively shown in structural formula II or structural formula IIA), namely an amide bond is formed between the amino of the compound represented by structural formula II or structural formula IIA and the carboxyl of A in structural formula III or IIIA.

Each of the compounds 1, 2, 3, 4, 5, 6, 7, 8, 9, 10, 11, 12, 13, 14, 14-P, 15, 16, 17, 18, 19, 20, 21, 22, 23, 24, 25, 26, 27, 31, 32, 33, 34, 35, 36, 37, 38, 39, and 40 or a pharmaceutically acceptable salt, stereoisomer, solvate or prodrug thereof provided above can all be used as "CPT" in structural formula III and is bonded to the carboxyl of A in structural formula III or IIIA through an amide bond via its amino.

For another example, in structural formula III and structural formula IIIA, CPT can be an Exatecan derivative represented by structural formula IV or a pharmaceutically acceptable salt, stereoisomer, solvate or prodrug thereof:

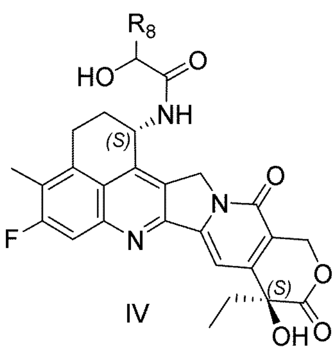

structural formula IV.

In structural formula IV, and if not otherwise stated in the context of the present disclosure, $R_8$ is hydrogen, trifluoromethyl, C1-5 alkyl or C1-5 alkyl substituted with one or more substituents, C3-6 cycloalkyl, or C3-6 cycloalkyl

substituted with one or more substituents, or halogen.

When $R_8$ is substituted C1-5 alkyl or substituted C3-6 cycloalkyl, the C1-5 alkyl or C3-6 cycloalkyl is substituted with substituent(s) selected from the group consisting of halogen, hydroxyl, methoxy, trifluoromethyl, amino or substituted amino, methanesulfonyl and C3-6 cycloalkyl; and among the substituents, the substituted amino is an amino substituted with one or more substituents selected from the group consisting of methyl and ethyl.

In particular, when "CPT" in the compound represented by structural formula IIIA is a compound represented by structural formula IV or a pharmaceutically acceptable salt, stereoisomer, solvate or prodrug thereof, $R_6$ and $R_7$ may or may not be hydrogen simultaneously. Similarly, $R_8$ in structural formula IV may or may not be hydrogen. According to particular embodiments of the present disclosure, in the case $R_6$ and $R_7$ are hydrogen simultaneously, $R_8$ may or may not be hydrogen.

In structural formula III and structural formula IIIA, the compound represented by structural formula IV is bonded to the carboxyl of A through a self-releasing structure via its hydroxyl which is bonded to the same carbon as $R_8$ (shown in structural formula IV), and the self-releasing structure is, e.g.

( 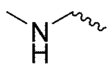 or 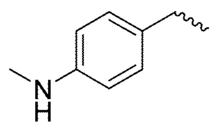 ),

in which the solid line indicates the bonding site to the carboxyl group of A in structural formula III or structural formula IIIA, and  means the bonding site to the hydroxyl in the structural formula IV.

In particular, regarding the compound represented by structural formula IIIA, when $R_6$ and $R_7$ are Ar'S, M is preferably phenylene or substituted phenylene, and at the meantime, $SP_1$ is C1-21 (preferably Cl-16, more preferably C1-11) linear heteroalkylene containing 1-11 (preferably 1-6) heteroatoms selected from the group consisting of N, O, and S.

Preferably, the compound represented by structural formula III or structural formula IIIA provided by the present disclosure is further a compound represented by structural formula V:

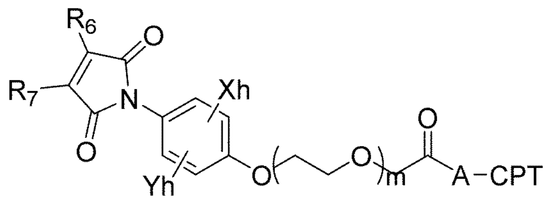

V

structural formula V.

In structural formula V, $R_6$ and $R_7$ independently of one another are Ar'S in which Ar' is phenyl or phenyl substituted with one or more substituents; and in the substituted phenyl, the phenyl is substituted with substituent(s) selected from the group consisting of alkyl (e.g., C1-C6 alkyl, preferably C1-C4 alkyl), alkoxy (e.g., C1-C6 alkoxy, preferably C1-C4 alkoxy, preferably methoxy), halogen, ester, amide, and cyano. Preferably, Ar' is phenyl, phenyl substituted with 4-formylmethylamine

( 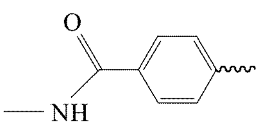 )

or phenyl substituted with 4-formylmorpholine

( 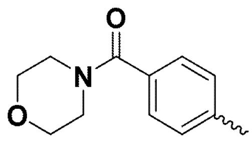 ).

In structural formula V, and if not otherwise stated in the context of the present disclosure, Xh and Yh independently of one another are hydrogen, halogen, haloalkyl (e.g., C1-C6 haloalkyl, preferably C1-C4 haloalkyl, e.g., trifluoromethyl), or alkoxy (e.g., C1-C6 alkoxy, preferably C1-C4 alkoxy, e.g., methoxy).

In structural formula V, and if not otherwise stated in the context of the present disclosure, m is any integer in the range of 1 to 10, preferably 1 to 5, more preferably 3 to 5.

In structural formula V, A means a peptide group of 2-4 amino acids, as defined above.

In structural formula V, the meaning of CPT and the bonding relationship thereof in structural formula V are the same as those of CPT in structural formula III and structural formula IIIA as defined above.

Preferably, the compound represented by structural formula V provided by the present disclosure is further a compound represented by structural formula V-A:

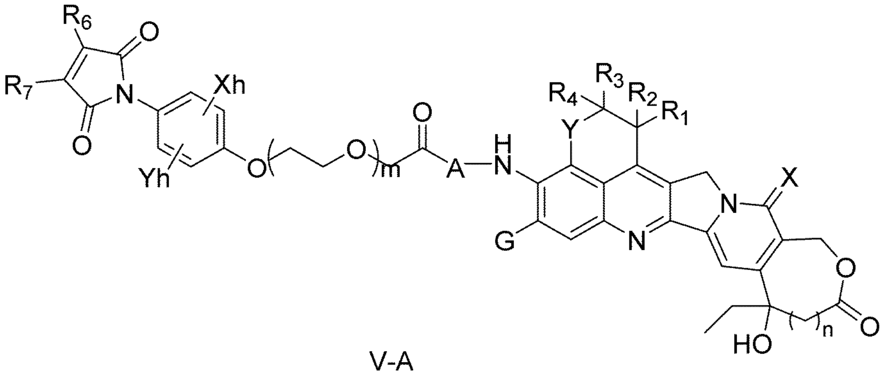

V-A

structural formula V-A.

In structural formula V-A, groups A, G, Y, $R_1$, $R_2$, $R_3$, $R_4$, X, and n have the same meaning as defined above for the groups A, G, Y, $R_1$, $R_2$, $R_3$, $R_4$, X, and n in structural formula III or structural formula IIIA.

Preferably, in structural formula V-A, G is hydrogen, fluorine or chlorine.

Preferably, in structural formula V-A, Y is methylene, sulfur or oxygen.

In structural formula V-A, and if not otherwise stated in the context of the present disclosure, "-A-NH-" means an amide bond is formed between the amino and the carboxyl of A.

Preferably, the compound represented by structural formula V-A provided by the present disclosure is further a compound represented by structural formula V-A-1:

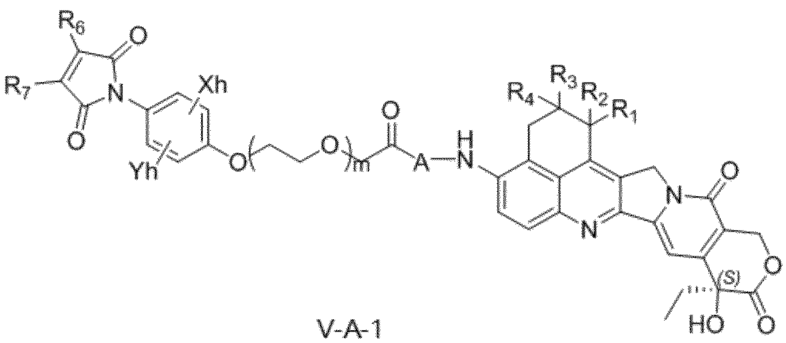

V-A-1

structural formula V-A-1.

Alternatively, the compound represented by structural formula V provided by the present disclosure is further

a compound represented by structural formula V-B:

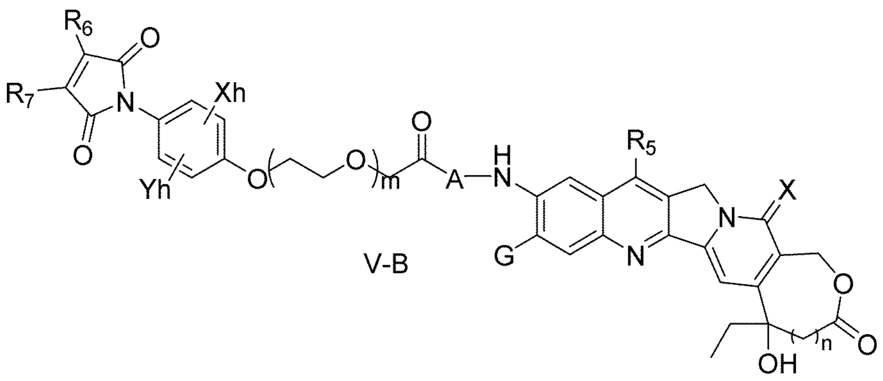

V-B

structural formula V-B.

In structural formula V-B, groups A, G, $R_5$, X, and n have the same meaning as defined above for the groups A, G, $R_5$, X, and n in structural formula III or structural formula IIIA.

In structural formula V-B, and if not otherwise stated in the context of the present disclosure, "-A-NH-" means an amide bond is formed between the amino and the carboxyl of A.

Preferably, the compound represented by structural formula V-B provided by the present disclosure is further a compound represented by structural formula V-B-1:

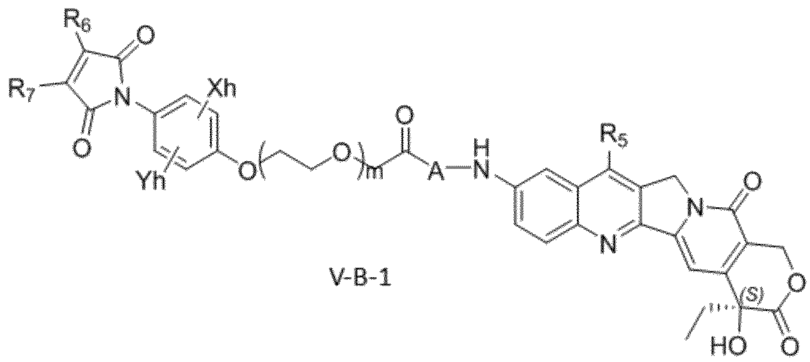

V-B-1

structural formula V-B-1.

Alternatively, the compound represented by structural formula V provided by the present disclosure is further a compound represented by structural formula V-C:

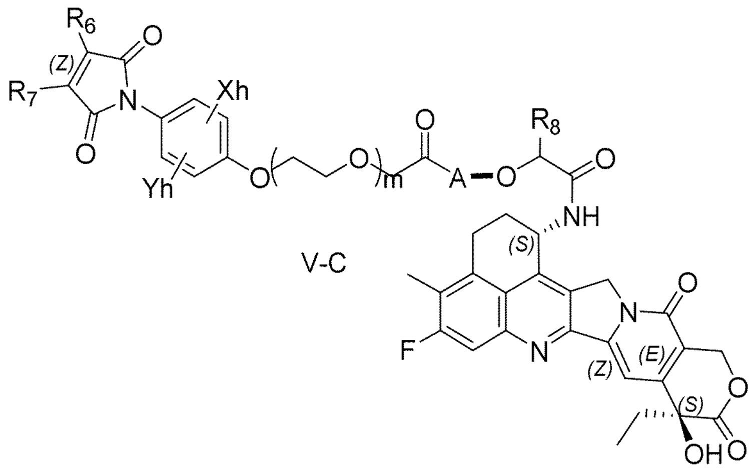

structural formula V-C.

In structural formula V-C, groups A, and $R_8$ have the same meaning as defined above for the groups A, and $R_8$ in structural formula III or structural formula IIIV.

In structural formula V-C, and if not otherwise stated in the context of the present disclosure, "-A-NH-" means an amide bond is formed between the amino and the carboxyl of A.

In structural formula V-C, and if not otherwise stated in the context of the present disclosure, "-" is the same as the self-releasing structure above when CPT in structure formula III or structure formula IIIV is represented by the structure formula IV

According to particular embodiments of the present disclosure, the compound represented by structural formula V provided by the present disclosure is further a compound represented by structural formula VI:

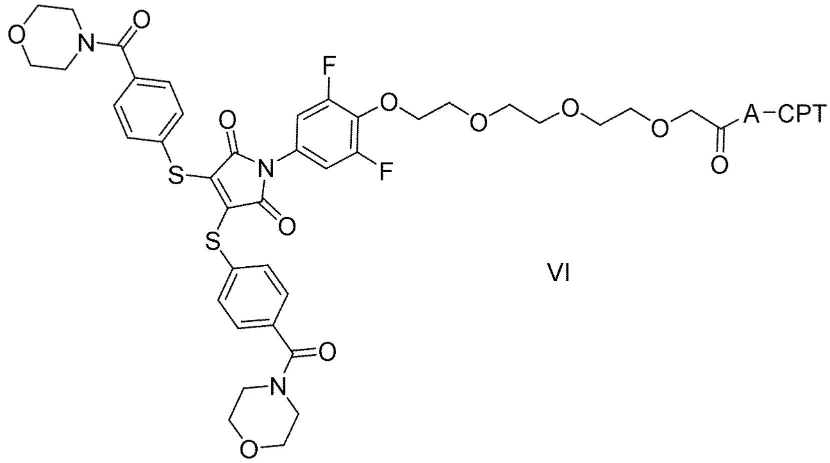

structural formula VI.

In structural formula VI, A means a peptide group of 2-4 amino acids, as defined above.

In structural formula VI, CPT is a compound of camptothecins. The meaning of CPT and the bonding relationship thereof in structural formula VI are the same as those of CPT in structural formula III and structural formula IIIA as defined in above.

Preferably, the compound represented by structural formula VI provided by the present disclosure is further a compound represented by structural formula VI-A:

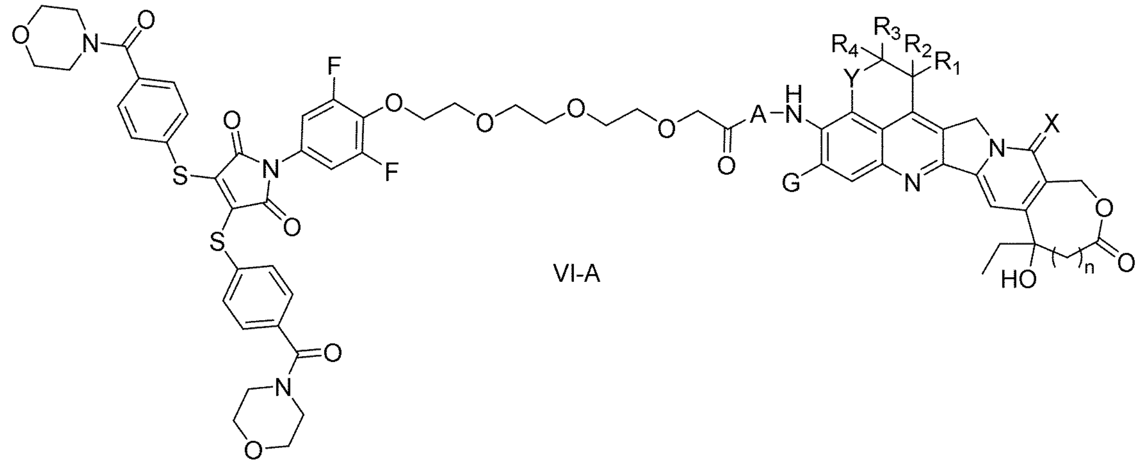

VI-A

structural formula VI-A.

In structural formula VI-A, groups A, G, Y, $R_1$, $R_2$, $R_3$, $R_4$, X, and n have the same meaning as defined above for the groups A, G, Y, $R_1$, $R_2$, $R_3$, $R_4$, X, and n in structural formula III or structural formula IIIA.

Preferably, in structural formula VI-A, G is hydrogen, fluorine or chlorine.

Preferably, in structural formula VI-A, Y is methylene, sulfur or oxygen.

In structural formula VI-A, and if not otherwise stated in the context of the present disclosure, "-A-NH-" means an amide bond is formed between the amino and the carboxyl of A.

Preferably, the compound represented by structural formula VI-A provided by the present disclosure is further a compound represented by structural formula VI-A-1:

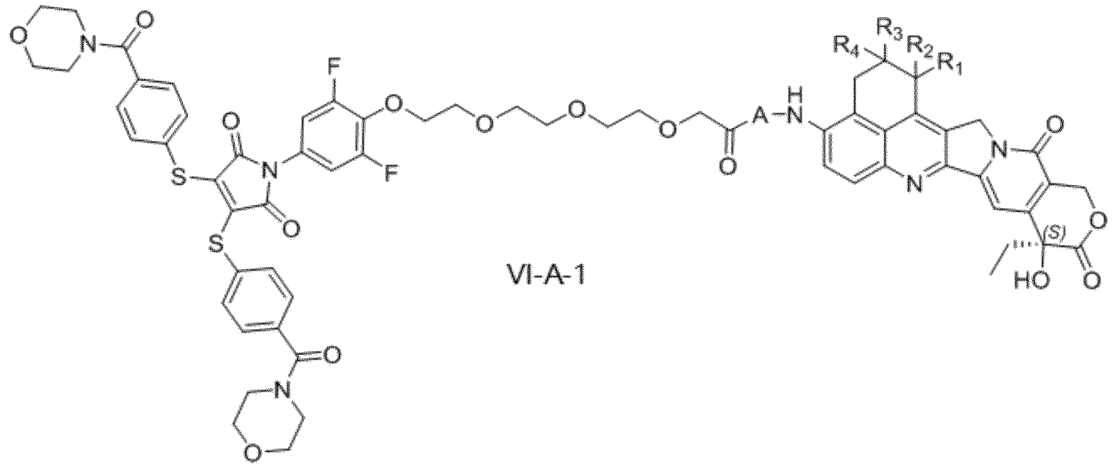

VI-A-1

structural formula VI-A-1.

Alternatively, the compound represented by structural formula VI is further a compound represented by structural formula VI-B:

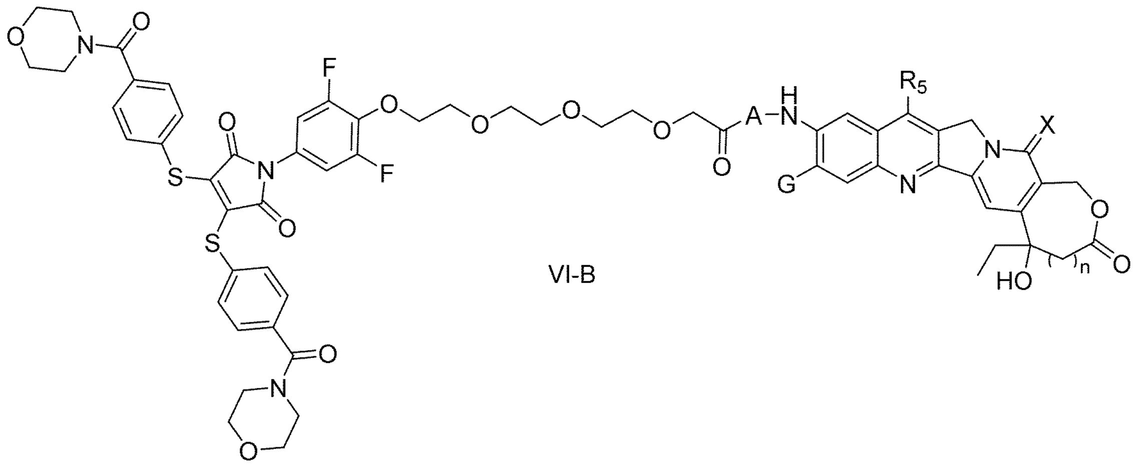

VI-B

structural formula VI-B.

In structural formula VI-B, groups A, G, $R_5$, X, and n have the same meaning as defined above for the groups A, G, $R_5$, X, and n in structural formula III or structural formula IIIA.

In structural formula VI-B, and if not otherwise stated in the context of the present disclosure, "-A-NH-" means an amide bond is formed between the amino of CPT and the carboxyl of A.

More preferably, the compound represented by structural formula VI-B is further a compound represented by structural formula VI-B-1:

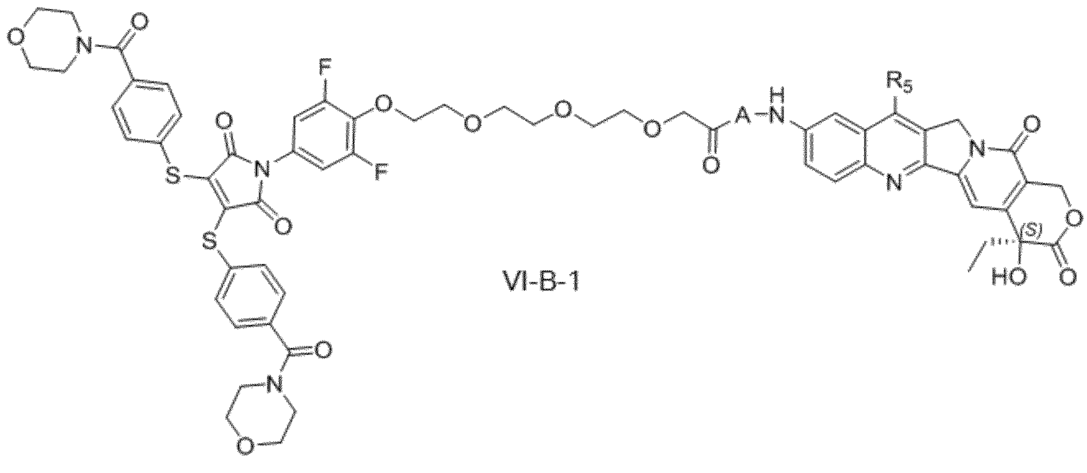

VI-B-1

structural formula VI-B-1.

Alternatively, the compound represented by structural formula VI provided by the present disclosure is further a compound represented by structural formula VI-C:

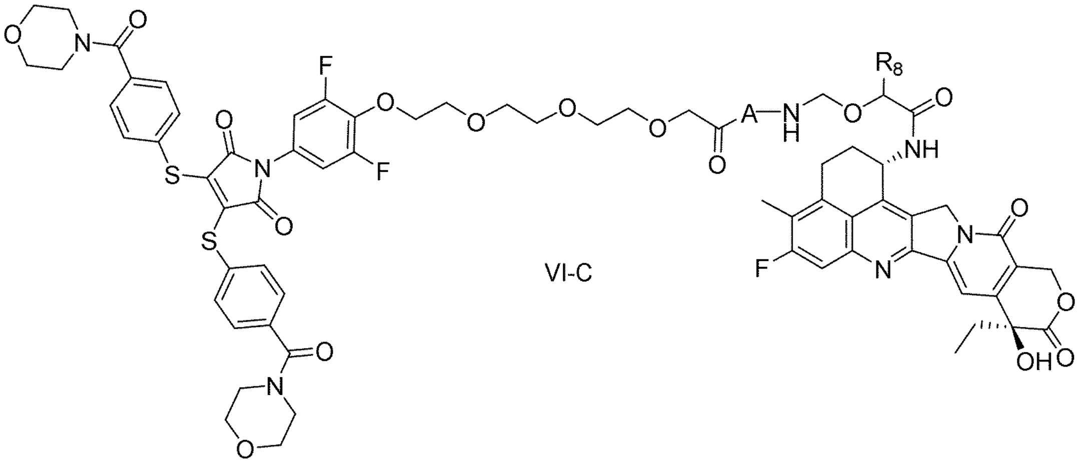

VI-C

structural formula VI-C.

In structural formula VI-C, groups A, and $R_8$ have the same meaning as defined above for the groups A, and $R_8$ in structural formula III or structural formula IIIA.

In structural formula VI-C, and if not otherwise stated in the context of the present disclosure, "-A-NH-" means an amide bond is formed between the amino and the carboxyl of A.

When the self-releasing structure in structural formula V-C is

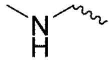

,

the structural formula V-C may be further structural formula VI-C.

According to particular embodiments of the present disclosure, in the second aspect of the present disclosure, the compound has one of the following structures:

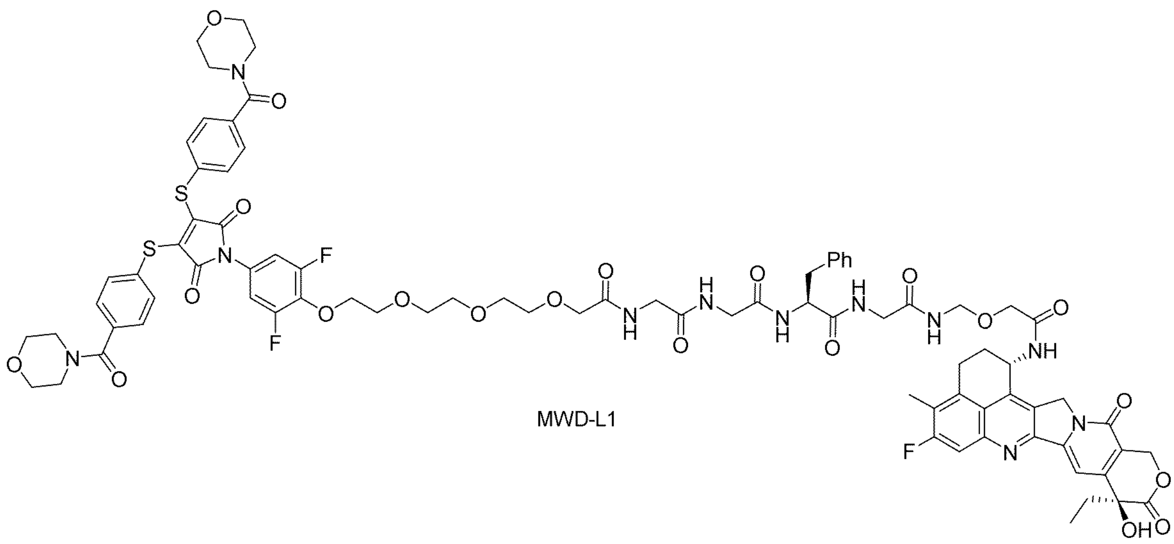

MWD-L1

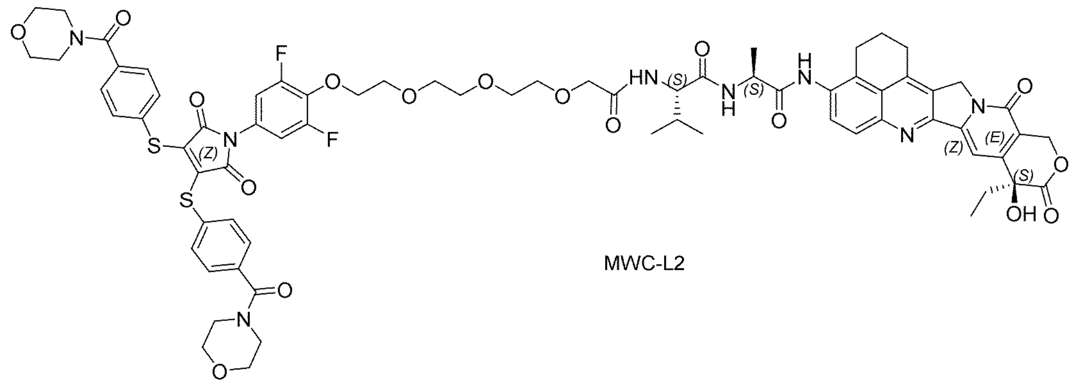

MWC-L2

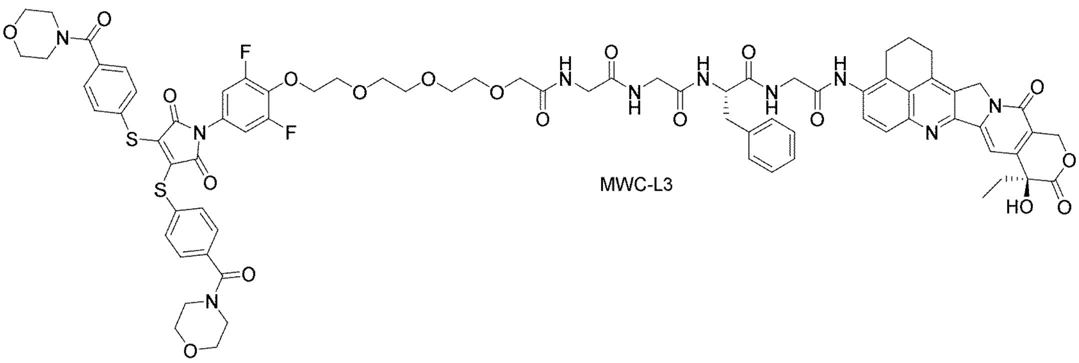

MWC-L3

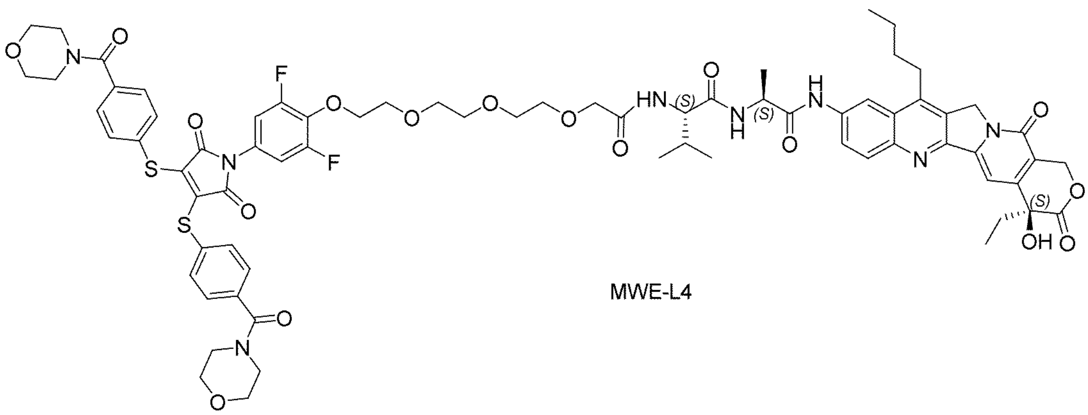

MWE-L4

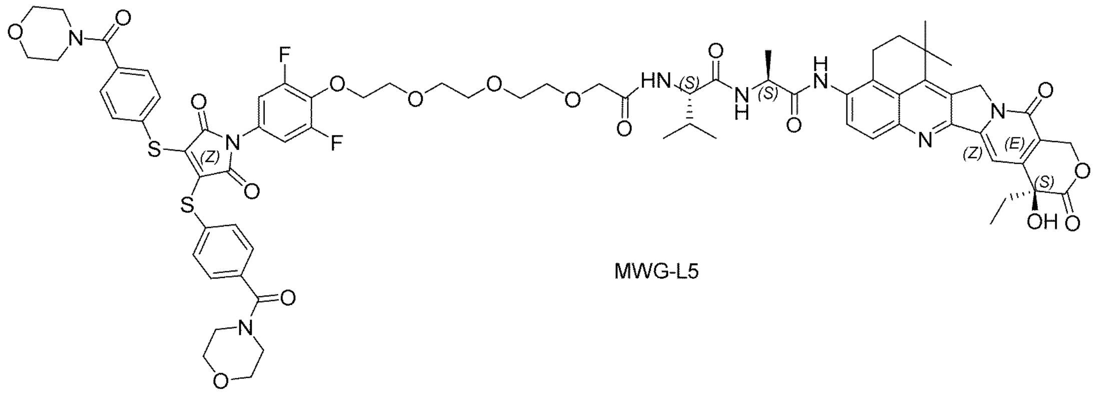

MWG-L5

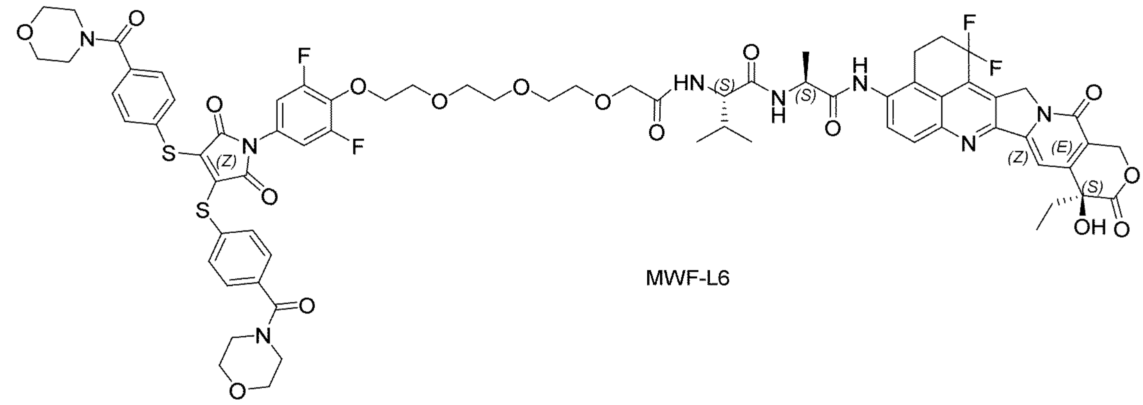

MWF-L6

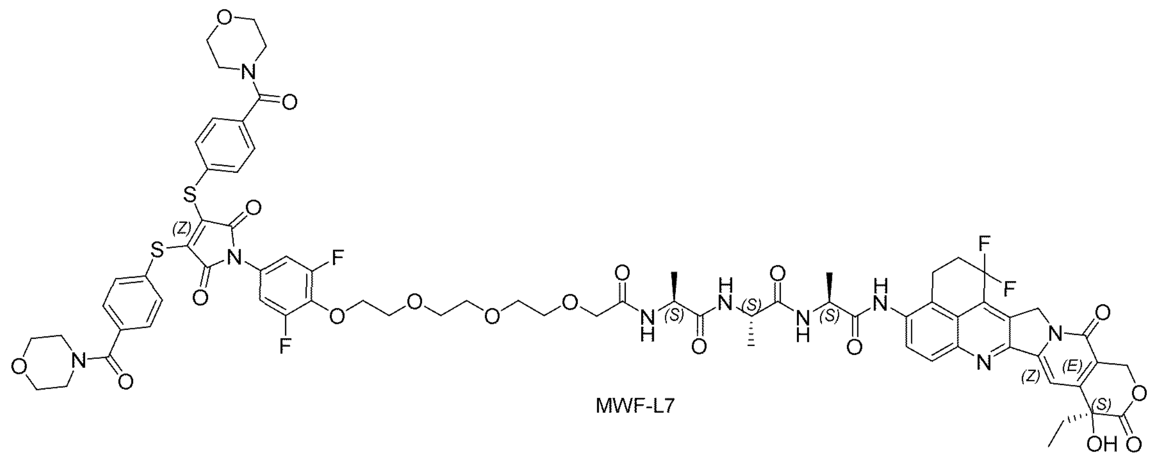

MWF-L7

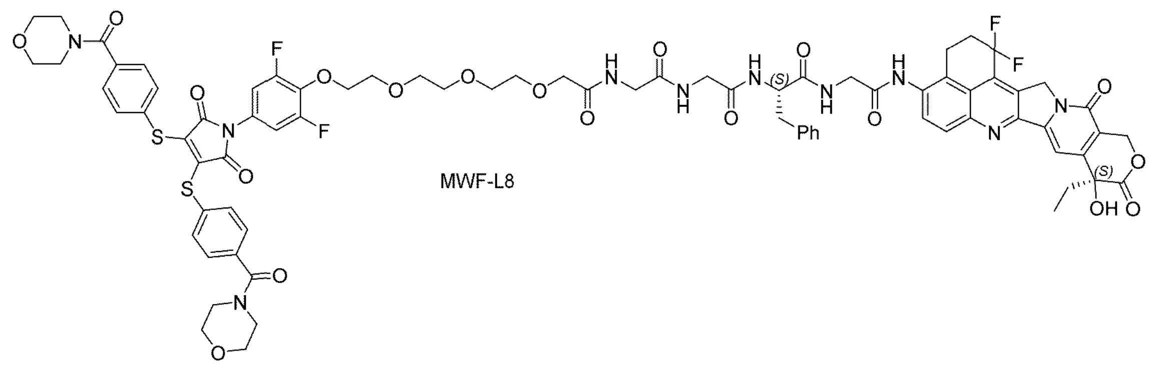

MWF-L8

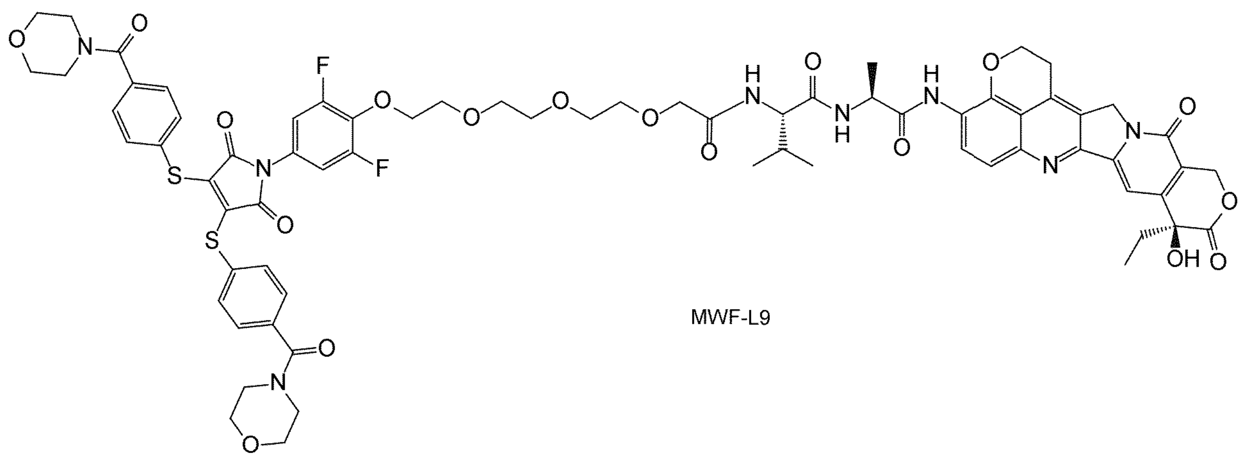

MWF-L9

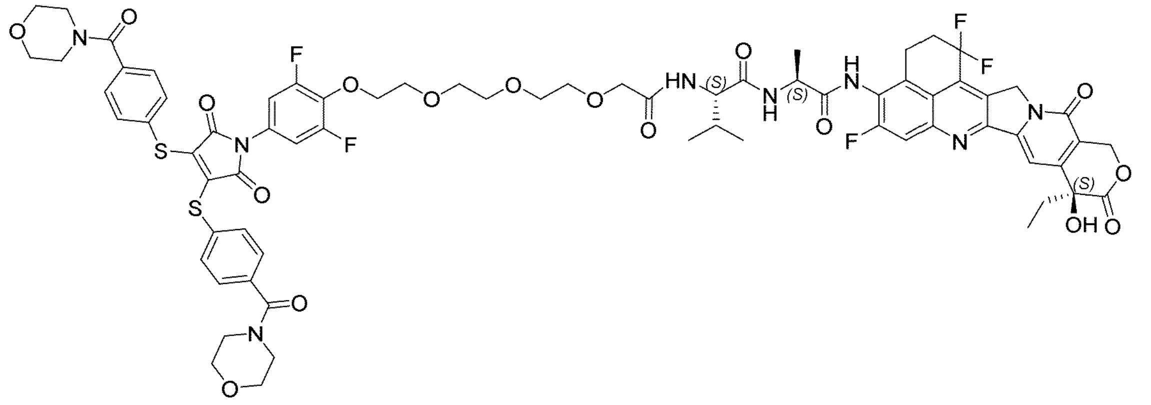

MWF-L10

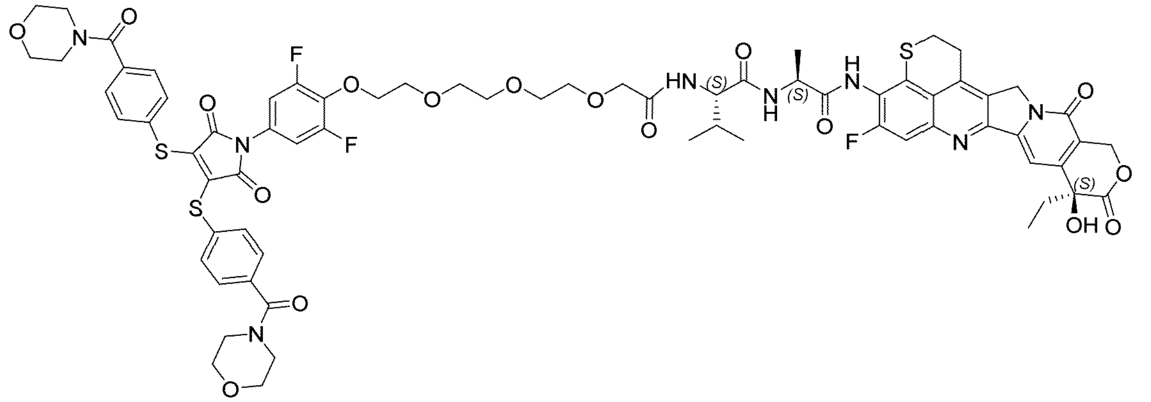

MWF-L11

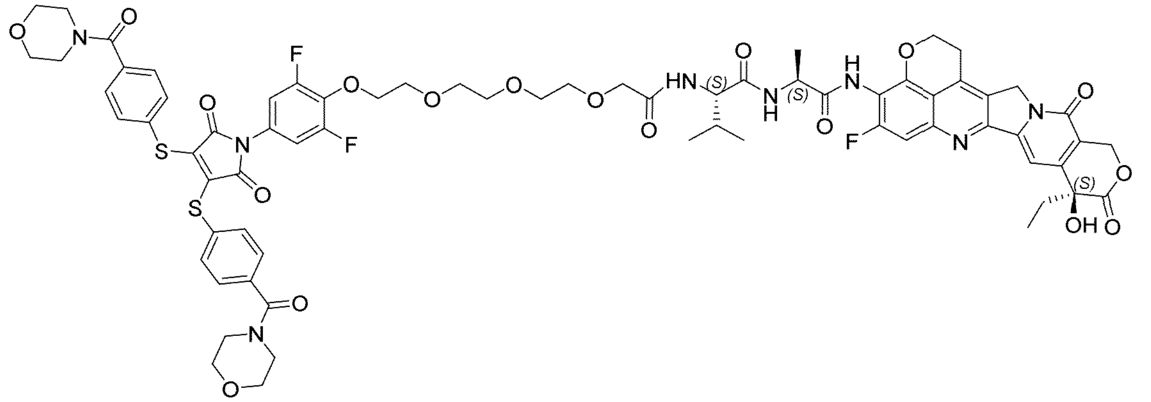

MWF-L12

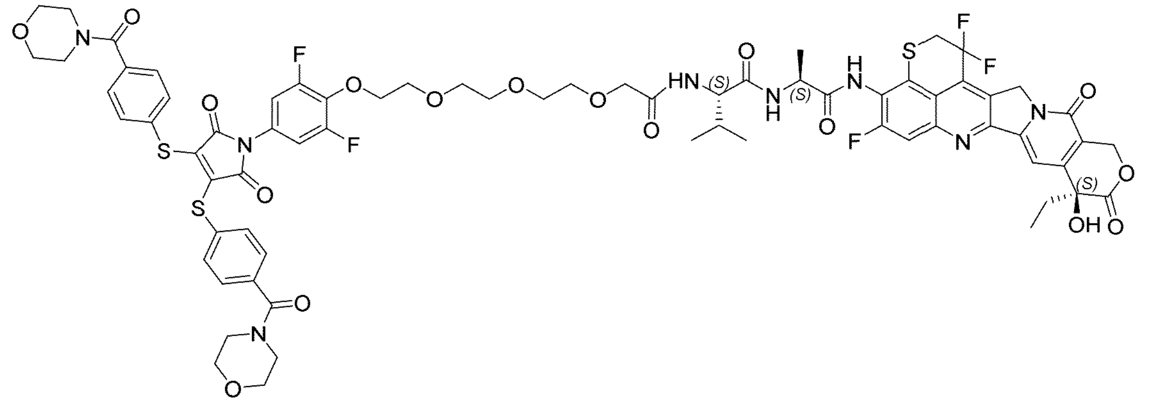

MWF-L13

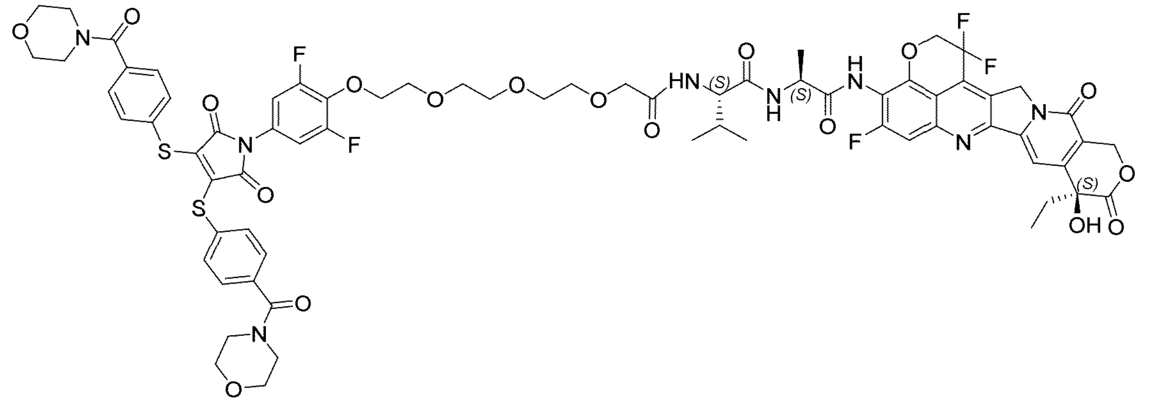

MWF-L14

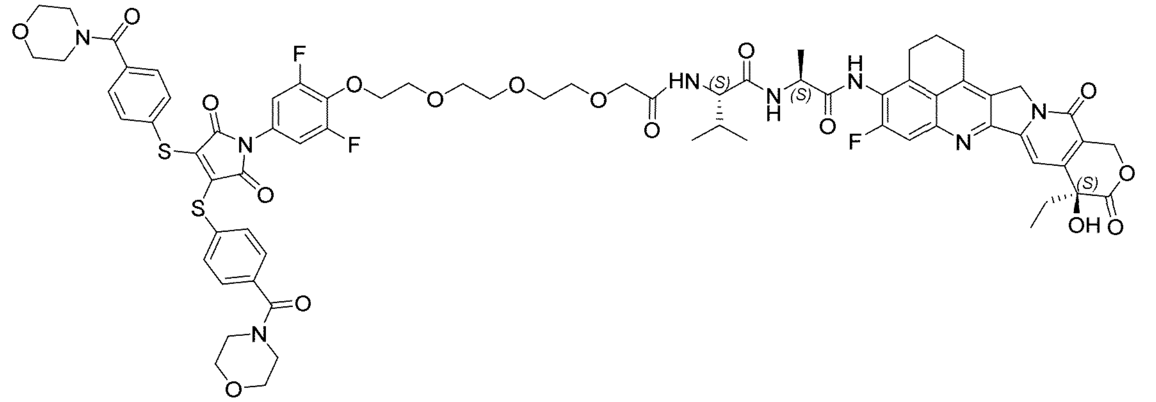

MWF-L15

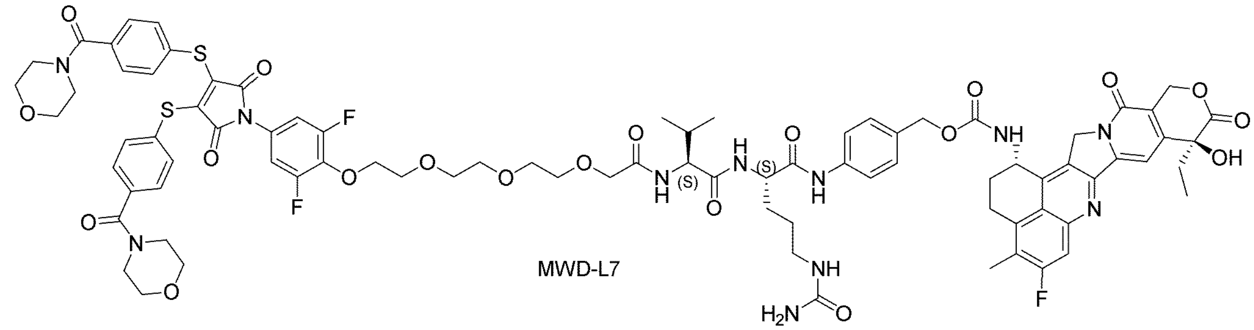

MWD-L7

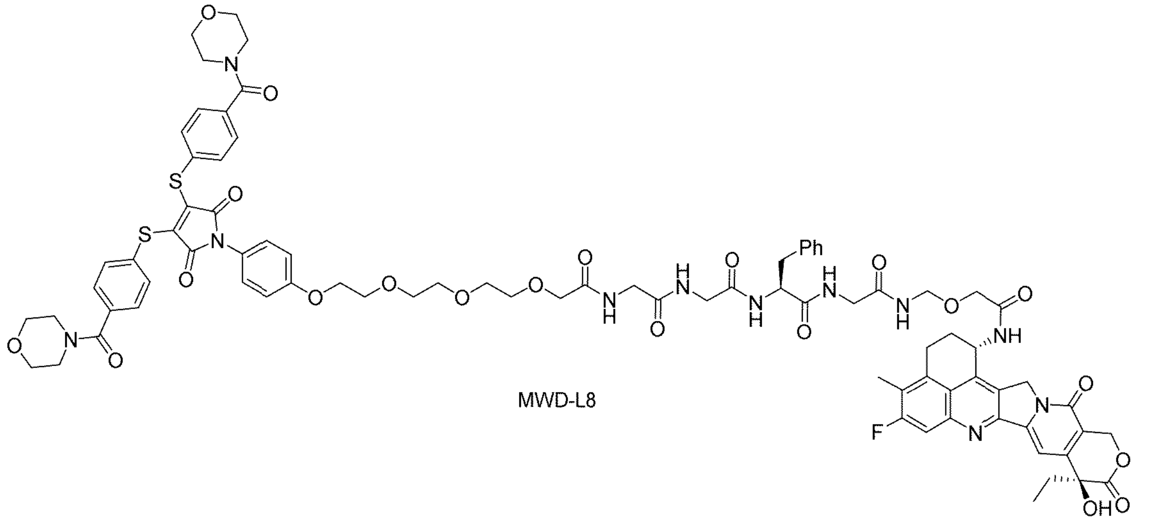

MWD-L8

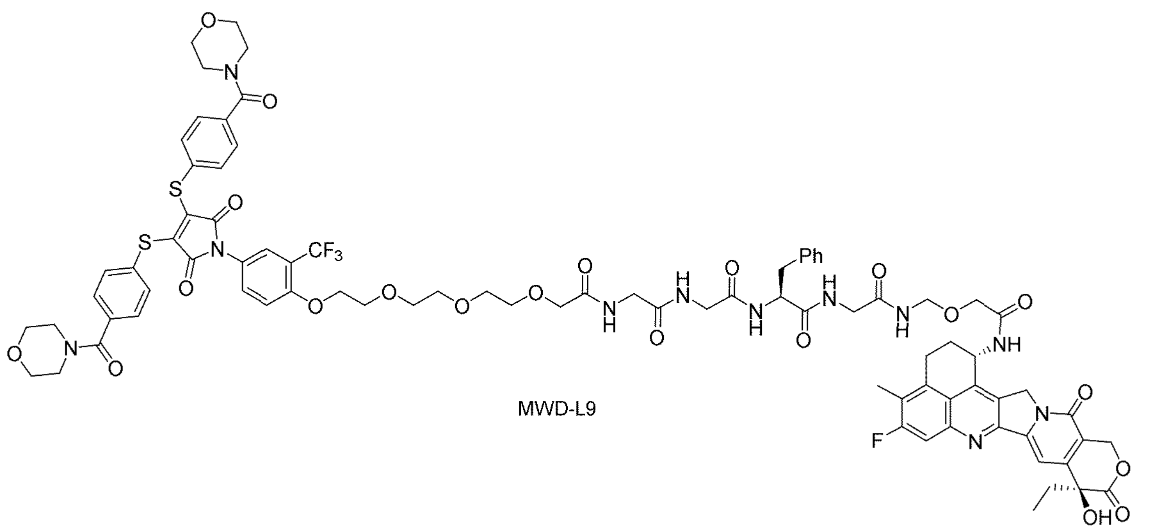

MWD-L9

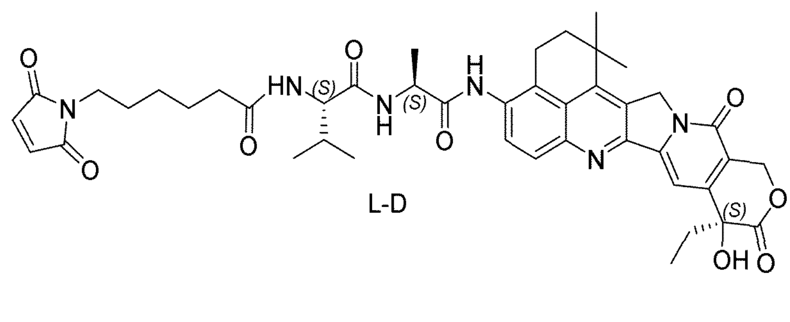

L-D

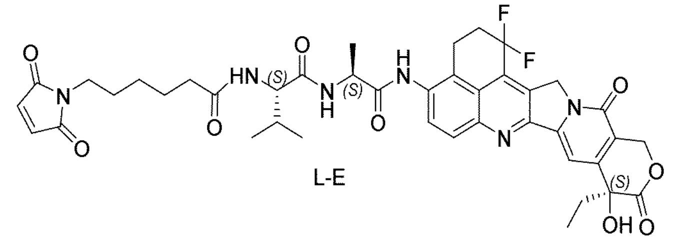

L-E

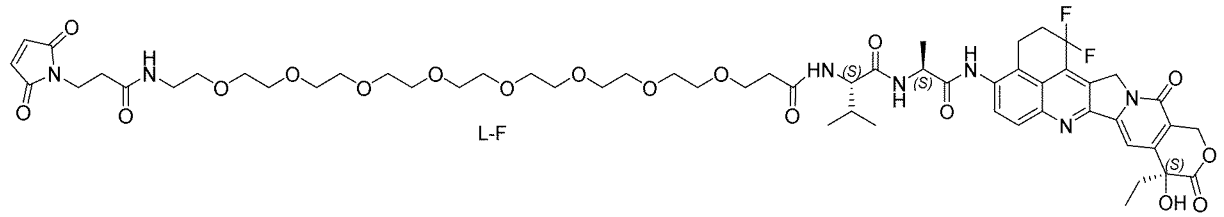

L-F

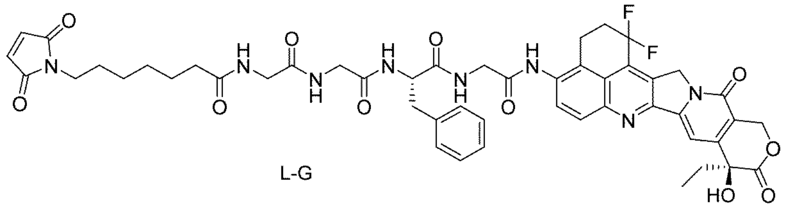

L-G

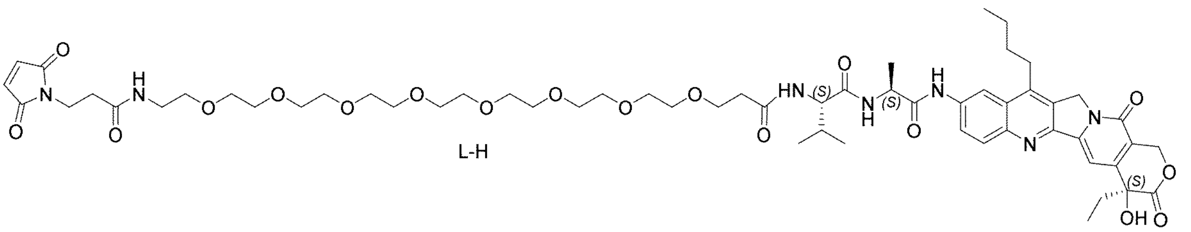

L-H

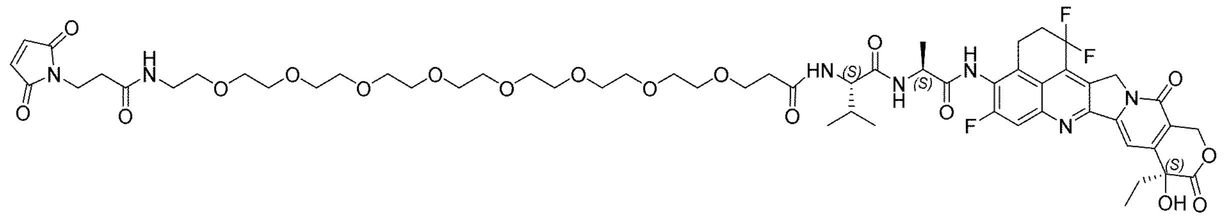

L-I

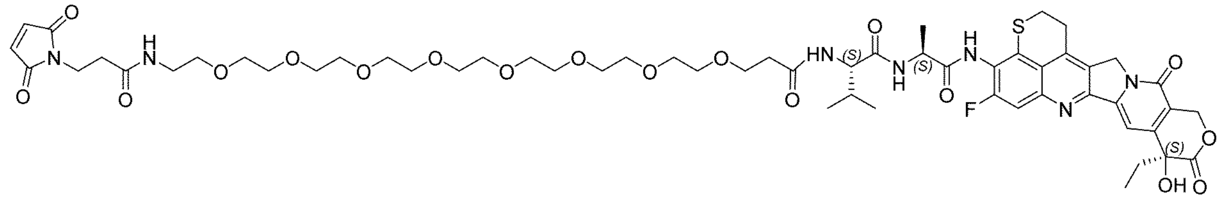

L-J

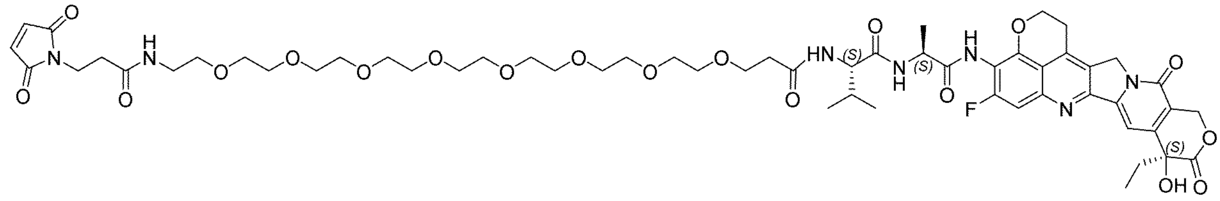

L-K

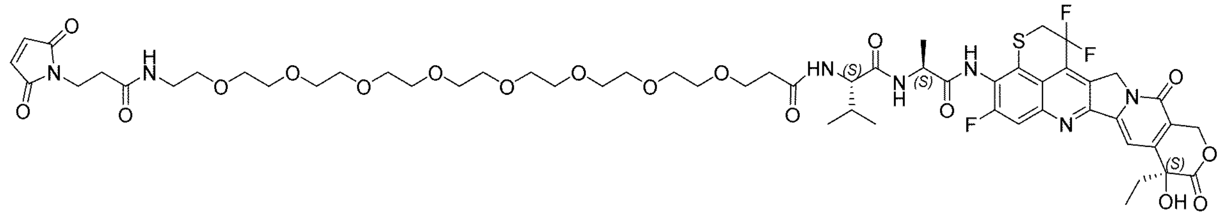

L-L

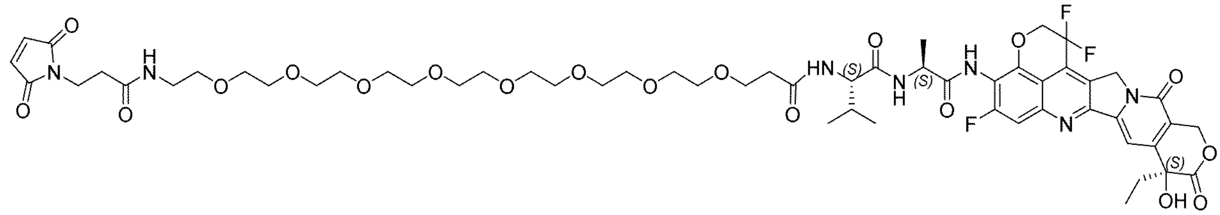

L-M

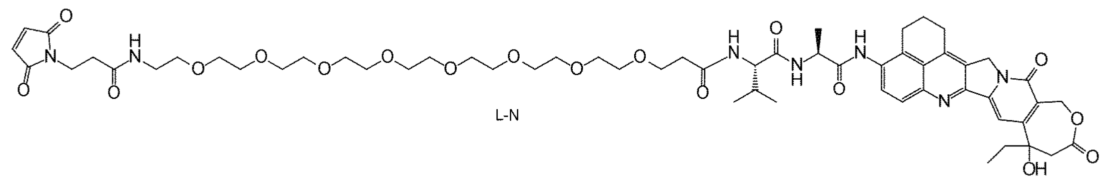

L-N

L-N

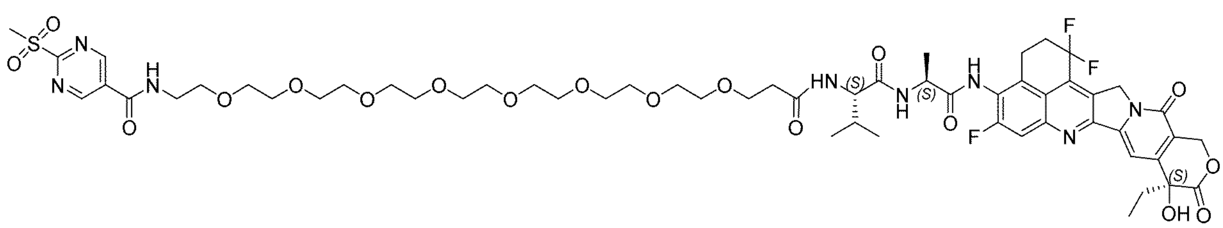

MWS-L1

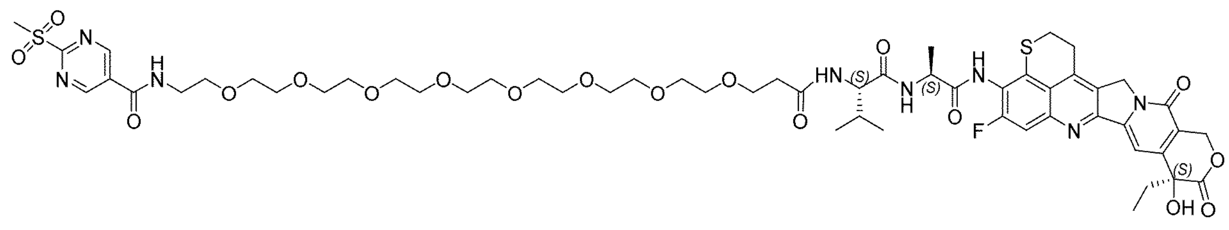

MWS-L2

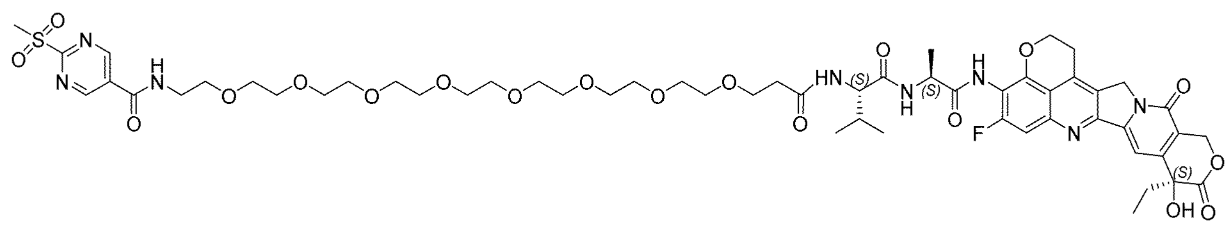

MWS-L3

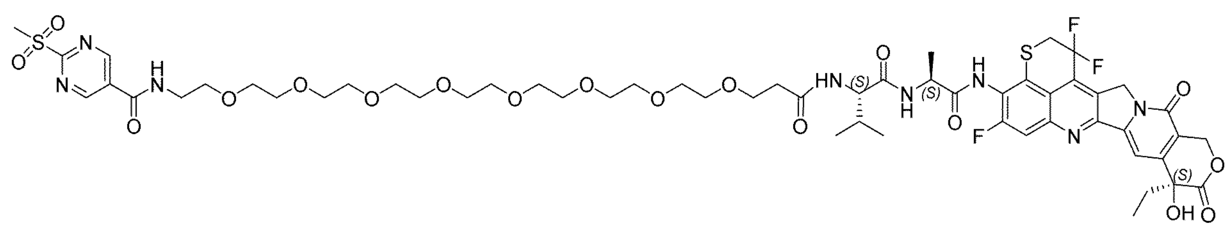

MWS-L4

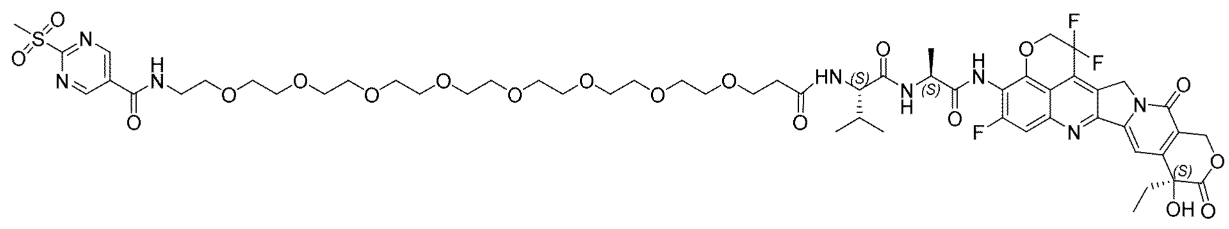

MWS-L5

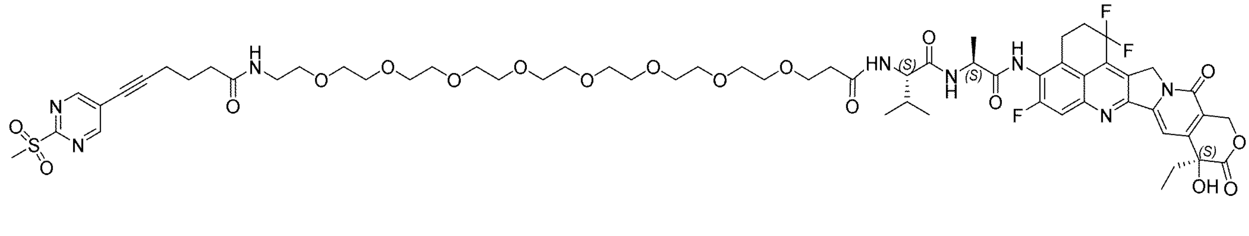

MWS-L6

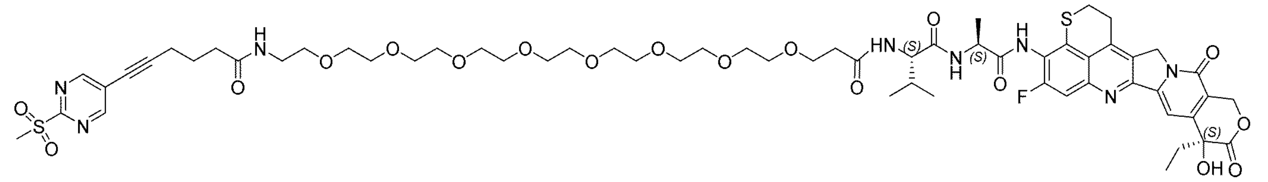

MWS-L7

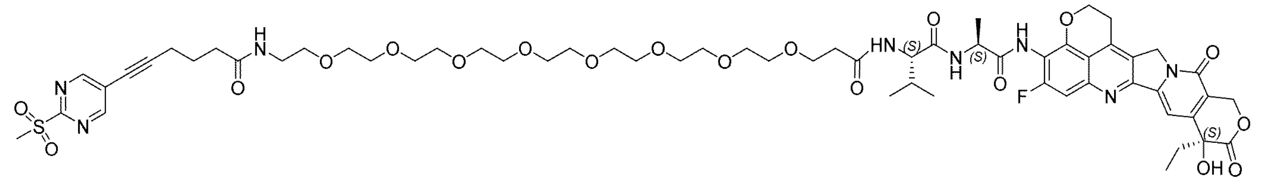

MWS-L8

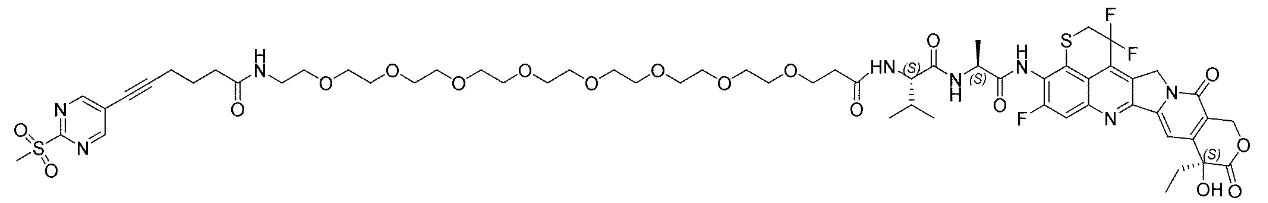

MWS-L9

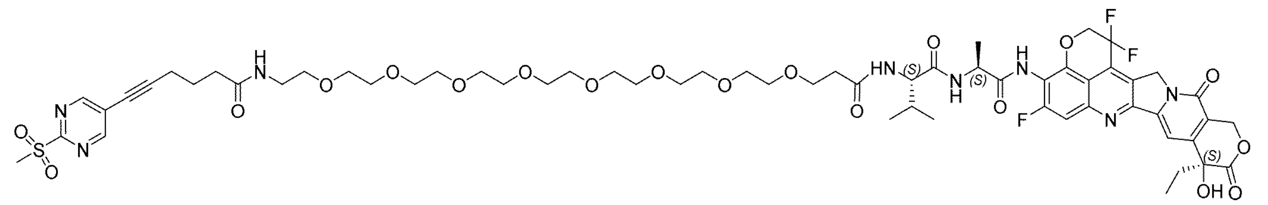

MWS-L10.

In a third aspect, the present disclosure provides an antibody-drug conjugate prepared using a compound represented by structural formula I, I-A, II, II-A, III, III-A, V, V-A, V-A-1, V-B, V-B-1, V-C, VI, VI-A, VI-A-1, VI-B, VI-B-1, or VI-C, or a pharmaceutically acceptable salt, stereoisomer, solvate or prodrug thereof and an antibody or fragment thereof.

In the third aspect of the present disclosure, the antibody or fragment thereof targets a tumor-associated antigen which is, e.g., HER2, B7H3, HER3, CD19, CD20, CD22, CD30, CD33, CD37, CD45, CD56, CD66e, CD70, CD74, CD73, CD79b, CD138, CD147, CD223, EpCAM, Mucin 1, STEAP1, GPNMB, FGF2, FOLR1, EGFR, EGFRvIII, Tissue factor, c-MET, FGFR, Nectin 4, AGS-16, Guanylyl cyclase C, Mesothelin, SLC44A4, PSMA, EphA2, AGS-5, GPC-3, c-KIT, RoR1, PD-L1, CD27L, 5T4, Mucin16, NaPi2b, STEAP, SLITRK6, ETBR, BCMA, Trop-2, CEACAM5, SC-16, SLC39A6, Delta-like protein 3, or Claudin 18.2.

Preferably, the antibody-drug conjugate has a structure represented by general formula

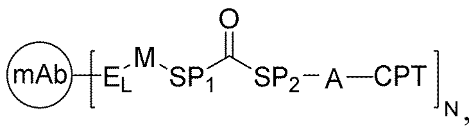

in which mAb means an antibody or fragment thereof, and groups M, $SP_1$, $SP_2$, A and CPT have the same meaning as defined above for the groups M, $SP_1$, $SP_2$, A and CPT in structural formula III or structural formula IIIA. N is in the range of 1 to 10, preferably 1 to 8 (e.g., 1 to 5), more preferably 3 to 8.

In the general formula, $E_L$ is selected from the group consisting of following groups, in which

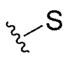

means bonding to the cysteine in mAb,  means bonding to M: $E_L$-1a and/or $E_L$-1b:

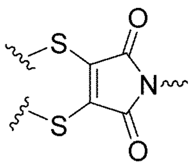 and/or 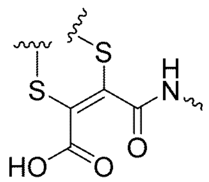 ; $E_L$-2: 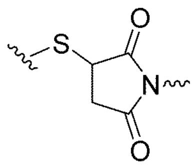 ; $E_L$-3:

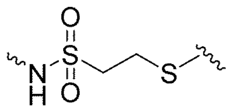 ; $E_L$-4: 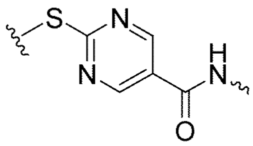 ; $E_L$-5: 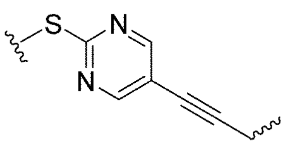 ; $E_L$-6:

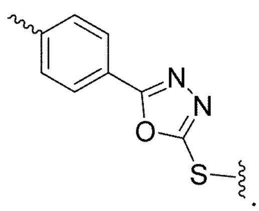

In the general formula, mAb may be an antibody or fragment thereof of IgG type targeting any of the tumor-associated antigens described above, preferably an antibody or fragment thereof of IgG 1 subtype.

Preferably, when the compound represented by structural formula VI is conjugated to an antibody or fragment thereof, the antibody-drug conjugate has a structure represented by general formula VII or general formula VIII as follows:

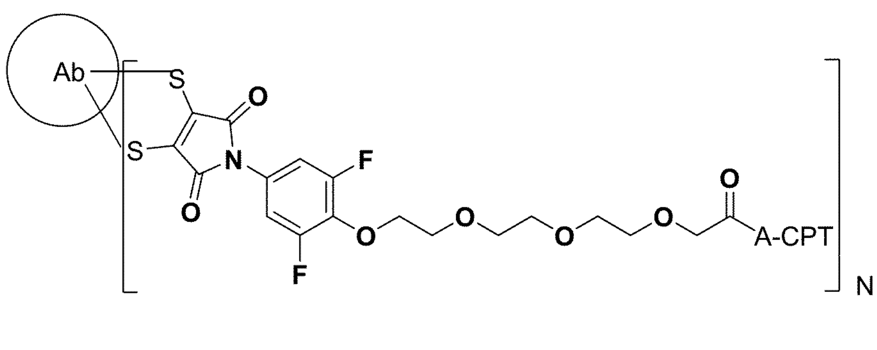

general formula VII

and/or

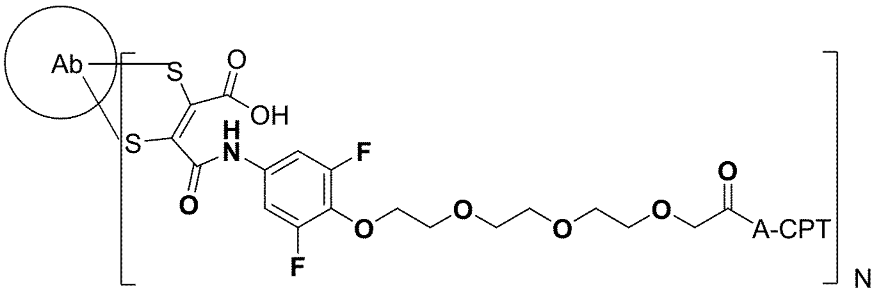

general formula VIII.

In general formula VII and general formula VIII, N is 1 to 10, preferably 1 to 8 (e.g., 1 to 5), more preferably 3 to 8.

In general formula VII and general formula VIII, Ab corresponds to the mAb in general formula

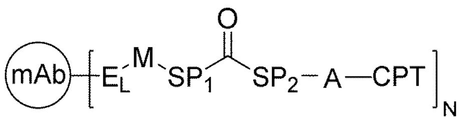 above.

In general formula VII and general formula VIII, groups A and CPT have the same meaning as defined above in the second aspect for the groups A and CPT in structural formula III or structural formula IIIA.

The antibody-drug conjugate represented by general formula VII or VIII provided by the present disclosure in the third aspect may produce prodrug metabolites in cells, e.g., tumor cells.

In a fourth aspect, the present disclosure provides a compound represented by structural formula IX:

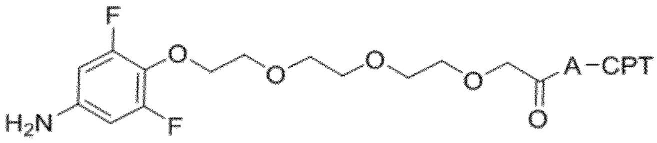

structural formula IX.

In structural formula IX, groups A and CPT have the same meaning as defined above in the second aspect for the groups A and CPT in structural formula III or structural formula IIIA.

Specifically, the prodrug metabolite of the antibody-drug conjugate prepared using the compound represented by structural formula VI-A is a compound represented by structural formula IX-A:

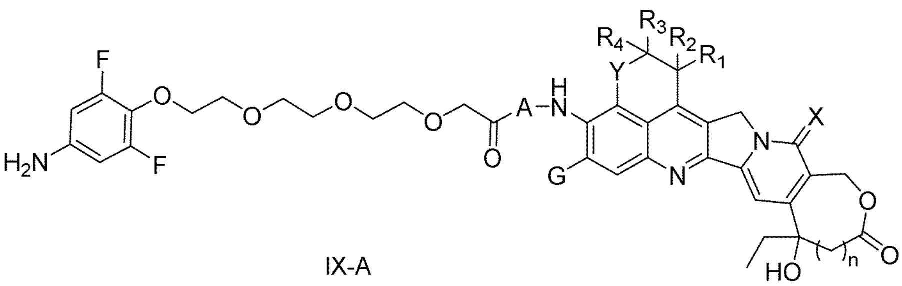

IX-A

structural formula IX-A.

In structural formula IX-A, groups A, G, Y, $R_1$, $R_2$, $R_3$, $R_4$, X, and n have the same meaning as defined above in the second aspect for the groups A, G, Y, $R_1$, $R_2$, $R_3$, $R_4$, X, and n in structural formula III or structural formula IIIA.

In structural formula IX-A, and if not otherwise stated in the context of the present disclosure, "-A-NH-" means

an amide bond is formed between the amino and the carboxyl of A.

Preferably, the compound represented by structural formula IX-A provided by the present disclosure is further a compound represented by structural formula IX-A-1:

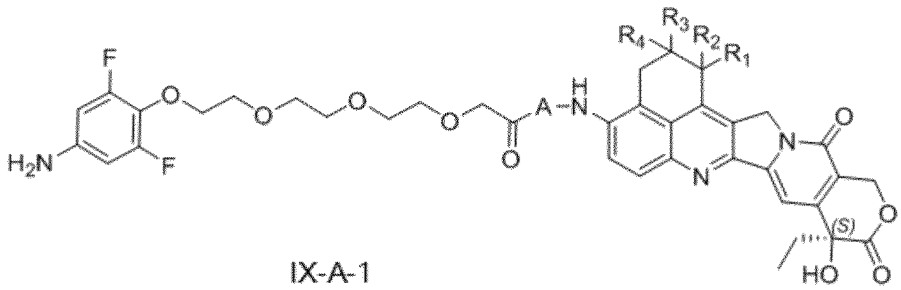

IX-A-1

structural formula IX-A-1.

The prodrug metabolite of the antibody-drug conjugate prepared using the compound represented by structural formula VI-B is a compound represented by structural formula IX-B:

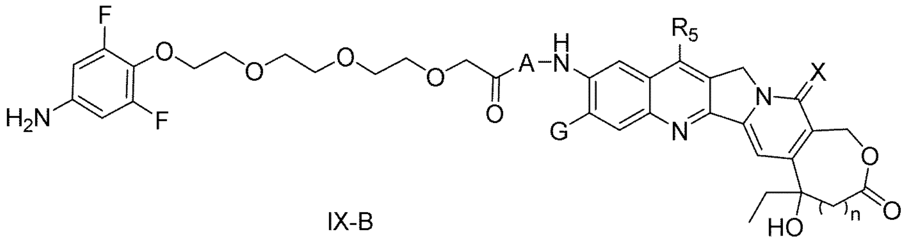

IX-B

structural formula IX-B.

In structural formula IX-B, groups A, G, $R_5$, X, and n have the same meaning as defined above in the second aspect for the groups A, G, $R_5$, X, and n in structural formula III or structural formula IIIA.

In structural formula IX-B, and if not otherwise stated in the context of the present disclosure, "-A-NH-" means an amide bond is formed between the amino and the carboxyl of A.

The prodrug metabolite of the antibody-drug conjugate prepared using the compound represented by structural formula VI-C is a compound represented by structural formula IX-C:

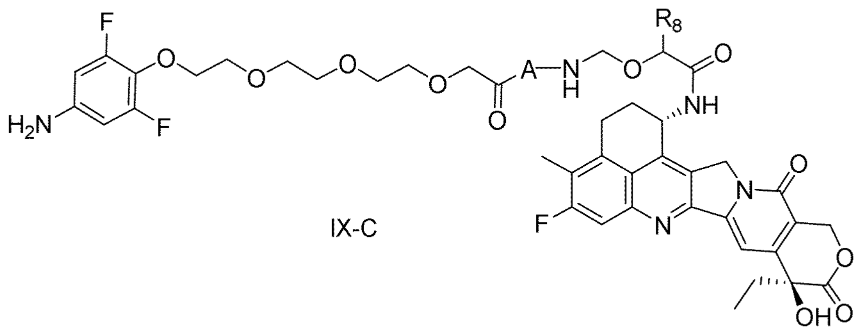

IX-C

structural formula IX-C.

In structural formula IX-C, groups A and $R_8$ have the same meaning as defined above in the second aspect for the groups A and $R_8$ in structural formula III or structural formula IIIA.

In structural formula IX-C, and if not otherwise stated in the context of the present disclosure, "-A-NH-" means an amide bond is formed between the amino and the carboxyl of A.

In a fifth aspect, the present disclosure provides use of the compound or a pharmaceutically acceptable salt, stereoisomer, solvate or prodrug thereof or the antibody-drug conjugate according to the invention in the manufacture of a medicament for the treatment of a tumor.

Preferably, the tumor is a cancer. Preferably, the tumor is positive for a tumor-associated antigen which is, e.g., HER2, B7H3, HER3, CD19, CD20, CD22, CD30, CD33, CD37, CD45, CD56, CD66e, CD70, CD74, CD73, CD79b, CD138, CD147, CD223, EpCAM, Mucin 1, STEAP1, GPNMB, FGF2, FOLR1, EGFR, EGFRvIII, Tissue factor, c-MET, FGFR, Nectin 4, AGS-16, Guanylyl cyclase C, Mesothelin, SLC44A4, PSMA, EphA2, AGS-5, GPC-3, c-KIT, RoR1, PD-L1, CD27L, 5T4, Mucin16, NaPi2b, STEAP, SLITRK6, ETBR, BCMA, Trop-2, CEACAM5, SC-16, SLC39A6, Delta-like protein 3, or Claudin 18.2, or is associated with the high expression of the tumor-associated antigen.

Preferably, the tumor is colorectal cancer, bladder cancer, breast cancer, pancreatic cancer, liver cancer, ovarian cancer, endometrial cancer, fallopian tube cancer, gastric cancer, prostate cancer, small cell lung cancer, non-small cell lung cancer, esophageal squamous cell carcinoma, head and neck squamous cell carcinoma, melanoma, leukemia, lymphoma, glioma, or glioblastoma.

In a sixth aspect, the present disclosure provides a method for treating a tumor with the compound or a pharmaceutically acceptable salt, stereoisomer, solvate or prodrug thereof or the antibody-drug conjugate according to the invention, comprising administering to a subject in need thereof the compound or a pharmaceutically acceptable salt, stereoisomer, solvate or prodrug thereof or the antibody-drug conjugate.

Preferably, the tumor is a cancer. Preferably, the tumor is positive for a tumor-associated antigen which is, e.g., HER2, B7H3, HER3, CD19, CD20, CD22, CD30, CD33, CD37, CD45, CD56, CD66e, CD70, CD74, CD73, CD79b, CD138, CD147, CD223, EpCAM, Mucin 1, STEAP1, GPNMB, FGF2, FOLR1, EGFR, EGFRvIII, Tissue factor, c-MET, FGFR, Nectin 4, AGS-16, Guanylyl cyclase C, Mesothelin, SLC44A4, PSMA, EphA2, AGS-5, GPC-3, c-KIT, RoR1, PD-L1, CD27L, 5T4, Mucin16, NaPi2b, STEAP, SLITRK6, ETBR, BCMA, Trop-2, CEACAM5, SC-16, SLC39A6, Delta-like protein 3, or Claudin 18.2, or is associated with the high expression of the tumor-associated antigen.

Preferably, the tumor is colorectal cancer, bladder cancer, breast cancer, pancreatic cancer, liver cancer, ovarian cancer, endometrial cancer, fallopian tube cancer, gastric cancer, prostate cancer, small cell lung cancer, non-small cell lung cancer, esophageal squamous cell carcinoma, head and neck squamous cell carcinoma, melanoma, leukemia, lymphoma, glioma, or glioblastoma.

Preferably, the subject is a mammal, preferably a primate, more preferably a human.

## BRIEF DESCRIPTION OF THE DRAWINGS

Embodiments of the invention are described in detail below with reference to the attached figures, in which:

FIG. 1: Analysis Results of antibody-drug conjugates according to the invention via Hydrophobic Interaction Chromatography (HIC).

FIG. 2: Study results of drug potency of an antibody-drug conjugate according to the invention in a mouse model of pancreatic cancer.

FIG. 3: Study results of drug potency of an antibody-drug conjugate according to the invention in a mouse model of bladder cancer.

FIG. 4: Study results of drug potency of an antibody-drug conjugate according to the invention in a mouse model of lung cancer.

FIG. 5: Study results of bystander effect of an antibody-drug conjugate according to the invention (mean ± SD, n = 2).

FIG. 6: Time-effect curves of internalization of an antibody-drug conjugate and an antibody according to the invention (mean ± SD, n = 3).

FIG. 7: Study results of the induction of apoptosis in tumor cells by an antibody-drug conjugate according to the invention.

FIG. 8: Dose-response curves of the binding to tumor cells of an antibody-drug conjugate and an antibody according to the invention.

## DETAILED DESCRIPTION OF PREFERRED EMBODIMENTS

The invention is illustrated below with reference to specific examples. It will be understood by those skilled in the art that these examples are merely illustrative of the invention and do not limit the scope of the invention in any way.

Experimental procedures in the following examples are all conventional ones, unless otherwise specified. Raw materials and reagents used in the following examples are all commercially available products, unless otherwise specified.

### Group 1 of Examples Synthesis of camptothecins

Universal method for synthesizing camptothecins:

The compound of camptothecins according to the invention can be obtained by Friedlander reaction with a compound represented by formula A and a tricyclic compound (cyclic compound CDE), and the general reaction is shown as follows:

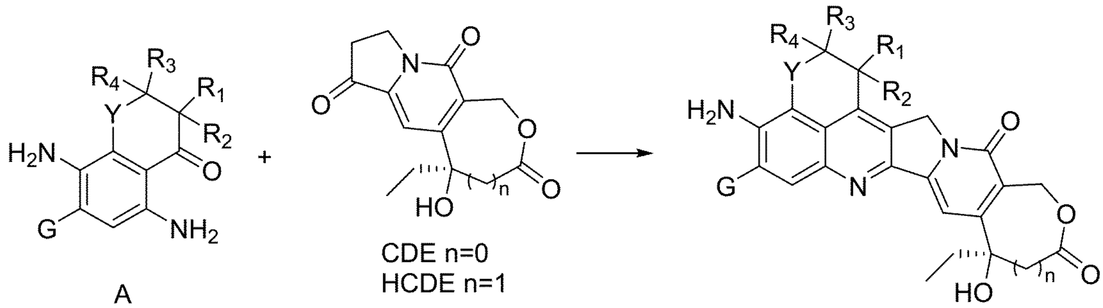

A

CDE n=0
HCDE n=1

In the reaction, the tricyclic compound CDE can be purchased from MCE (MedChemExpress):

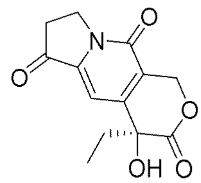

**CDE** .

The tricyclic compound HCDE can be obtained according to the method described in Bioorganic & Medicinal Chemistry, 2010, vol. 18, # 9, p. 3140-3146:

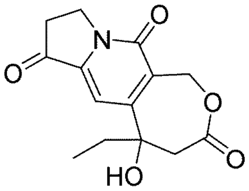

HCDE .

### Group 1.1 of Examples Synthesis of the compound represented by formula A

#### Example 1.1.1 Synthesis of Compound A1

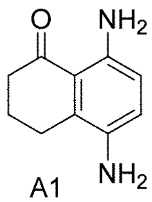

A1

Method 1: Compound A1 was synthesized according to the method described in the patent publication WO2020200880A1, and synthetic route is shown as follows:

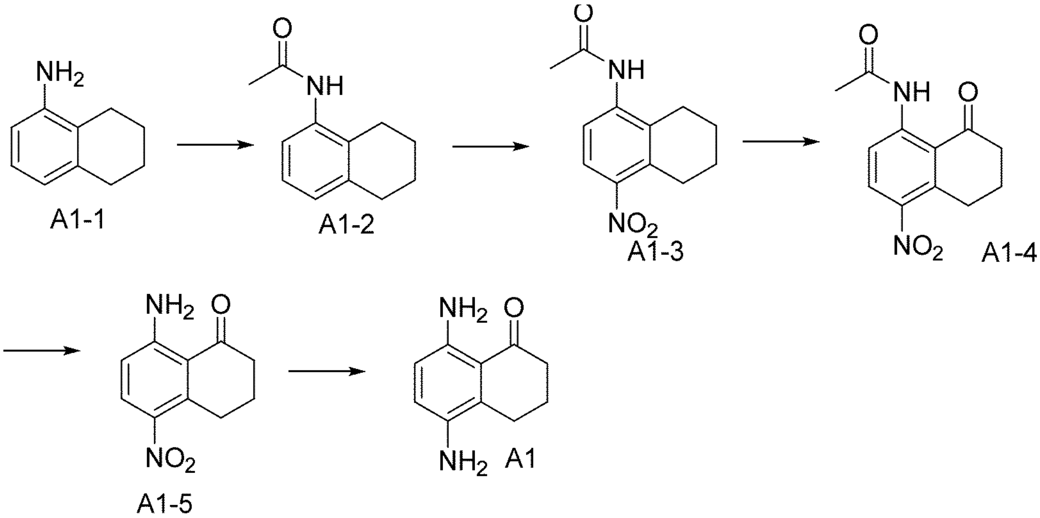


Method 2: Compound A1 was synthesized by a palladium-catalyzed coupling reaction with a zinc reagent, and synthetic route is shown as follows:

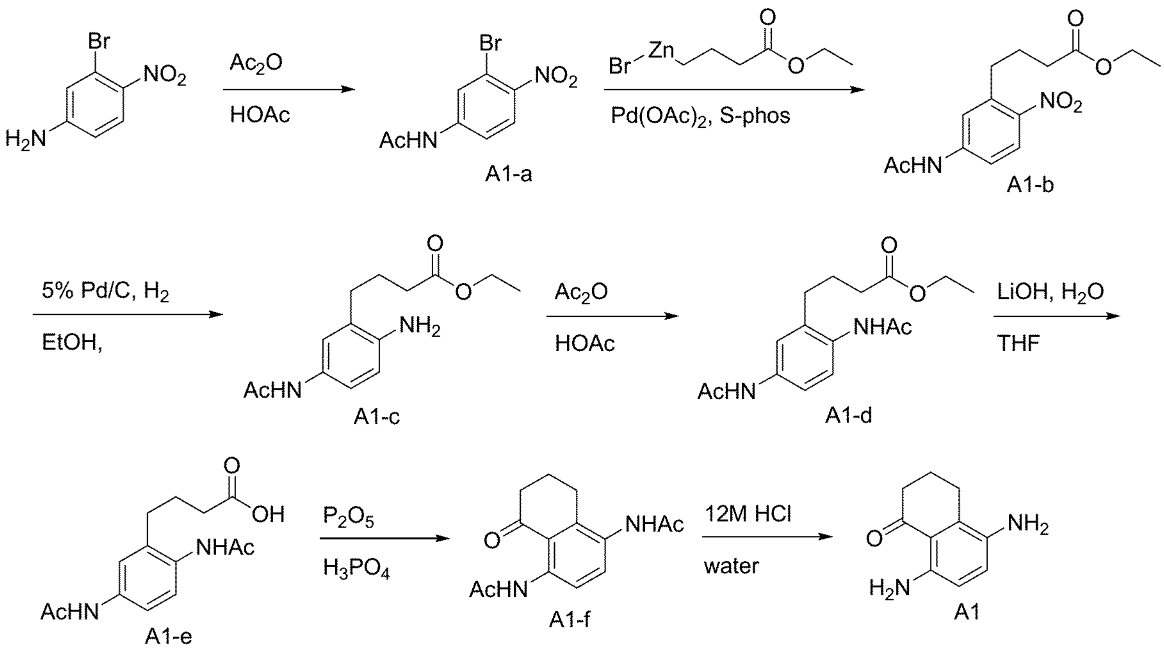


(1) Acetylation:

3-bromo-4-nitroaniline (25.00 g, 0.12 mol) and 200 mL of acetic acid were added into a 500 mL flask, into which 50 mL of acetic anhydride was then slowly added dropwise. Those starting compounds reacted at room temperature

for 16 hours. After the reaction completed as confirmed with TLC detection, reaction liquid was filtered to obtain filtrate, which was in turn concentrated under reduced pressure to remove acetic acid. Filter cakes obtained were pooled and slurried with 200 mL of MTBE, and then the slurry was filtered, and 27.6 g of dried yellow solid (intermediate A1-a) was obtained with a yield of 92%. LC-MS (ESI): m/z 259 (M+H)+.

(2) Preparation of 4-ethoxy-4-oxobutylzinc bromide

Activated zinc powder (19.0 g, 0.29 mol, 2.00 eq) was added into a 250 mL three-neck flask (equipped with a thermometer, reflux condenser tubes and rubber plugs). Then air in the flask was replaced with nitrogen, anhydrous DMF (145 mL) was added, and then the air in the flask was replaced with nitrogen again. Iodine (1.86 g, 0.015mol, 0.1 eq) was added at room temperature, and the color of the solution was observed to change from colorless to brownish red, to light yellow gradually, and to colorless finally (in 2-3 min). Next, ethyl 4-bromobutyrate (28.6 g, 0.15 mol, and 1.00 eq) was added into the flask which was then heated to 80 °C (inner temperature) for reaction for 4-5 hours. When the reaction completed as confirmed with TLC detection, reaction liquid was allowed to stand and cool to room temperature for later use. The supernatant obtained was light yellow and had a concentration of 1 mol/L.

(3) Coupling with the Zinc reagent

Anhydrous DMF (120 mL) and the intermediate A1-a (25.0 g, 1.00 eq) were added into a 500 mL reaction flask. Then air in the flask was replaced with nitrogen, palladium acetate (433 mg, 0.02 eq) was added, and then the air in the flask was replaced with nitrogen again. The mixture in the flask was stirred at room temperature for 10 min, then S-PHOS (1.6 g, 0.04 eq) was added, and then the air in the flask was replaced with nitrogen again. Next, the mixture in the flask was stirred for 20 min, 4-ethoxy-4-oxobutylzinc bromide (145 mL, 1.50 eq) prepared in the above step was added dropwise at room temperature (25-30 °C), and then the reaction was maintained at 25-30 °C for 16 hours. When the starting compounds reacted completely as confirmed with TLC detection (DCM: EA =5: 1), reaction liquid was cooled to room temperature, and ammonium chloride solution (15 mL) was added to the reaction liquid to quench the reaction. Then the reaction liquid was poured into 1 L of water, and 400 mL of ethyl acetate was added, to obtain separated phases. The phases were filtered through a buchner funnel, and then aqueous phase obtained was extracted with ethyl acetate (400 mL × 2); and organic phases then obtained were washed with water (500 mL) twice and with saturated sodium chloride solution (500 mL) once, then dried over anhydrous sodium sulfate, and concentrated under reduced pressure, and 37.0 g of reddish brown oily liquid (the intermediate A1-b) was obtained with a yield of 130%. The crude product was used in the next reaction without further purification. LC-MS (ESI): [M+1]+ = 295.

(4) Synthesis of the intermediate A1-c

The intermediate A1-b (37.0 g, 1.0 eq) and ethanol (1.5 L) were added into a 3 L three-neck reaction flask and the starting material was completely dissolved in the ethanol. The reaction system was cooled to 5 °C, 5% Pd/C (5.7 g, 1.0 eq) was added, and the temperature was raised to 25 °C in a hydrogen atmosphere (atmospheric pressure) for reaction for 16 hours. When the reaction completed as confirmed with TLC detection (DCM: EA =5: 1), the reaction system was filtered through celite, and organic phase obtained was concentrated to obtain 33.0 g of a crude product (the intermediate A1-c) with a yield of 130%. The crude product was used directly in the next reaction without further purification. LC-MS (ESI): [M+1]+ = 265.

(5) Synthesis of the intermediate A1-d

The intermediate Al-c (33.0 g, 1.00 eq) and 260 mL of acetic acid were added into a 1 L flask, into which 46 mL of acetic anhydride was then slowly added dropwise. The starting material compounds reacted at room temperature for 2 hours. When the starting compounds reacted completely as confirmed with TLC detection (DCM: MeOH =10: 1), reaction liquid was filtered to obtain filtrate, which was in turn concentrated under reduced pressure to remove acetic acid. 250 mL of water was added, and then aqueous phase obtained was extracted with ethyl acetate (400 mL × 2); and organic phases then obtained were washed with water (500 mL) twice and with saturated sodium chloride solution (500 mL) once, then dried over anhydrous sodium sulfate, and concentrated under reduced pressure, and 32 g of a crude product was obtained. The crude product was slurried with 400 mL of MTBE, then filtered, and 27.0 g of dried light yellow solid (the intermediate Al-d) was obtained with a yield of 91%. The total yield of the four steps was found to be 83.7%.

LC-MS ( ESI ): [M+1]+ =307.

1H NMR (400 MHz, DMSO) δ 9.87 (s, 1H), 9.19 (s, 1H), 7.41 (s, 1H), 7.37 (d, J = 8.7 Hz, 1H), 7.20 (d, J = 8.5 Hz, 1H), 4.06 (q, J = 7.0 Hz, 2H), 2.52 (d, J = 8.5 Hz, 2H), 2.29 (t, J = 7.3 Hz, 2H), 2.02 (s, 6H), 1.79 - 1.64 (m, 2H), 1.18

(t, J = 7.1 Hz, 3H).

(6) Synthesis of the intermediate A1-e

The intermediate A1-d (27.0 g, 88 mmol, 1.0 eq), water (100 mL) and tetrahydrofuran (200 mL) were added into a 500 mL three-neck reaction flask and the starting material was completely dissolved. Lithium hydroxide monohydrate (18.5 g, 441 mmol, 5.0 eq) was added at room temperature and the reaction system was maintained at room temperature for reaction for 3 hours. When the reaction completed as confirmed with TLC detection, a majority of tetrahydrofuran was distilled off under reduced pressure, and 300 mL of water were added to residue obtained. Aqueous phase obtained was extracted with EA (2 × 100 mL), and then organic phases obtained were discarded and aqueous phases obtained were pooled and placed in an ice bath and adjusted to pH 4 with the addition of 6 N hydrochloric acid. Solid precipitated and was filtered, and 19.1 g of white solid (the intermediate A1-e) was obtained with a yield of 78%. 1H NMR (400 MHz, DMSO) δ 12.09 (s, 1H), 9.86 (s, 1H), 9.18 (s, 1H), 7.38 (d, J = 15.5 Hz, 2H), 7.23 (d, J = 8.5 Hz, 1H), 2.51 (d, J = 8.1 Hz, 2H), 2.23 (t, J = 7.1 Hz, 2H), 2.02 (s, 6H), 1.76 - 1.61 (m, 2H)

(7) Synthesis of Compound A1

40 mL of polyphosphoric acid was added to a 250 mL reaction flask and heated to 90 °C, and then the intermediate A1-e (5.0 g, 17.97 mmol) was added in several parts. The internal temperature was maintained at 95-100 °C for reaction for 5 hours. When the reaction completed as confirmed with TLC detection, heating source was removed, and the reaction system was allowed to cool to 50-60 °C. Next, 15 mL of 4 M HCl (aq) was added dropwise into the reaction system to quench the reaction (the temperature would raise to 100 °C). After that, 600 mL of 4 M NaOH aqueous solution was added dropwise to adjust the pH to 10. Aqueous phase obtained was extracted with ethyl acetate (50 mL x 3), and organic phases then obtained were pooled and washed with saturated sodium chloride solution (50 mL) once, then dried over anhydrous sodium sulfate, and concentrated under reduced pressure, and 3.75 g of yellow solid (the intermediate A1-f) was obtained with a yield of 80.5%. The solid was directly used in the next reaction without purification. LC-MS (ESI): [M+1]+ = 261.

The starting material intermediate A1-f (3.75 g) was suspended in 19% hydrochloric acid (30 mL) in a 250 mL three-neck reaction flask. The reaction system was heated to 90 °C (the inner temperature) for reaction for 3 hours. When the reaction completed as confirmed with TLC detection, the flask was placed in an ice-salt bath, cooling to below 5 °C. 4 M NaOH solution (45 mL, 30 eq) was added dropwise to adjust the pH to 10. Aqueous phase obtained was extracted with ethyl acetate (80 mL x 5), and organic phases then obtained were pooled and washed with saturated sodium chloride solution (50 mL) once, then dried over anhydrous sodium sulfate, concentrated under reduced pressure, and 2.45 g of a crude product was obtained. The crude product was purified by column chromatography using DCM as an eluent, and then 1.93 g of yellow solid (Compound A1) was obtained with a yield of 76%. The total yield of the two steps was found to be 61.2%.

LC-MS (ESI): [M+1]+ = 177.

1H NMR (400 MHz, DMSO) δ 6.76 (d, J = 8.7 Hz, 1H), 6.68 (s, 2H), 6.42 (d, J = 8.7 Hz, 1H), 4.17 (s, 2H), 2.55 (t, J = 5.9 Hz, 2H), 2.46 (t, J = 6.2 Hz, 2H), 2.00 - 1.80 (m, 2H)

Method 3: Compound A1 was synthesized by lactam hydrolysis, and synthetic route is shown as follows:

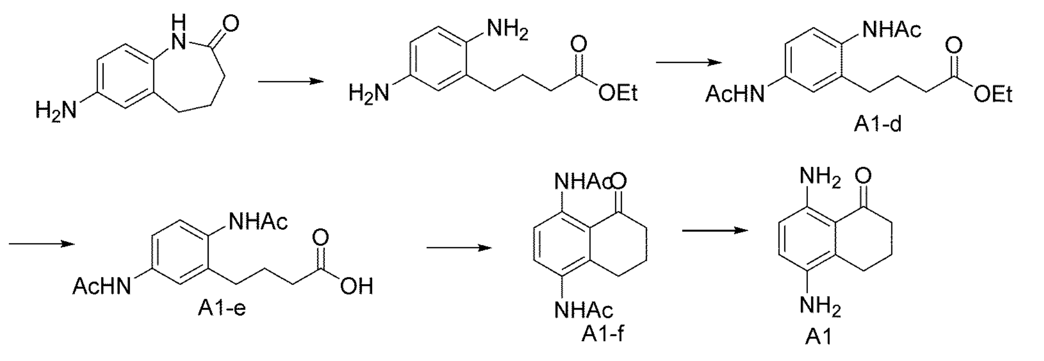

An aminolactam compound was prepared according to the method described in the patent publication CN106349233A.

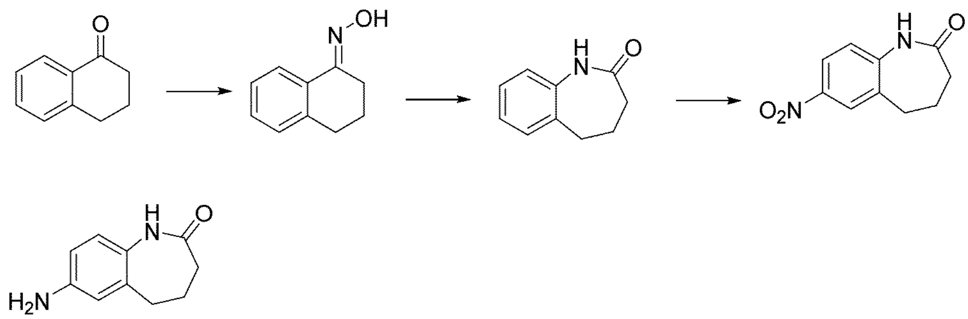

The aminolactam compound (17.6 g, 0.1 mmol), ethanol (250 mL), and 98% sulfuric acid (5 mL) were mixed in a 500 mL three-neck flask, and heated under reflux and reacted for 24 hours. Reaction liquid was sampled and detected to see whether the reaction completed. When the reaction completed, the reaction liquid was concentrated under reduced pressure to dryness. Dichloromethane (200 mL) and water (100 mL) were added into residue obtained, and the mixture obtained was placed in an ice bath, cooling to below 10 °C. 1 N sodium hydroxide aqueous solution was added to adjust the pH to 7-8, and then the mixture was stirred to obtain separated phases. Aqueous phase obtained was extracted with dichloromethane, and organic phases then obtained were pooled and washed with saturated saline water, then dried over anhydrous sodium sulfate, suctioned, and distilled under reduced pressure to remove the organic solvent and obtain 25 g of diaminoethyl ester intermediate crude product, which was directly used in the next reaction.

The diaminoethyl ester intermediate was dissolved in dichloromethane (200 mL), into which triethylamine (20.2 g, 0.2 mol, 2 eq) was added. The mixture was placed in an ice bath, cooling to below 10 °C, and acetic anhydride (25.5 g, 0.25 mol, 2.5 eq) was added dropwise. After that, the temperature was maintained for reaction for 1 hour. Reaction liquid was sampled and detected to see whether the reaction completed. When the reaction completed, the reaction liquid was poured into 1 N ice-cold hydrochloric acid, and stirred for 15 min. Separated phases were obtained, and aqueous phase obtained was extracted with dichloromethane (50 mL × 3); and organic phases then obtained were pooled and dried over anhydrous sodium sulfate, suctioned, and distilled under reduced pressure to remove the organic solvent and obtain a crude product. The crude product was slurried with 400 mL of MTBE, then filtered, dried, and 26.9 g of the intermediate A1-e was obtained as light yellow solid. The total yield of the two steps was found to be 87.8%. LC-MS (ESI): m/z 307 $(M+H)^+$.

Compound A1 was then prepared from intermediate A1-e according to Method 2.

**Example 1.1.2 Synthesis of Compound A2**

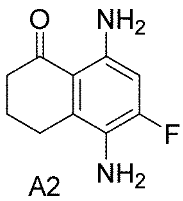

A2

Compound A2 was synthesized according to the method described in the patent publication WO2021148501, and synthetic route is shown as follows:

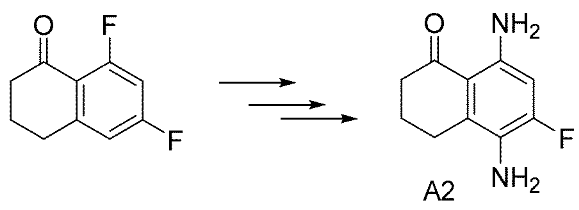

A2

**Example 1.1.3 Synthesis of Compound A3**

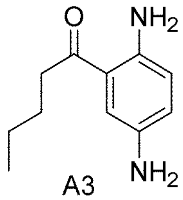

A3

Compound A3 was synthesized according to the method described in the patent publication US2004266803A.

**Example 1.1.4 Synthesis of Compound A4**

Compound A4 was synthesized according to a method similar to that described in Journal of Medicinal chemistry, 1998, 41(13), 2308-2318, and synthetic route is shown as follows:

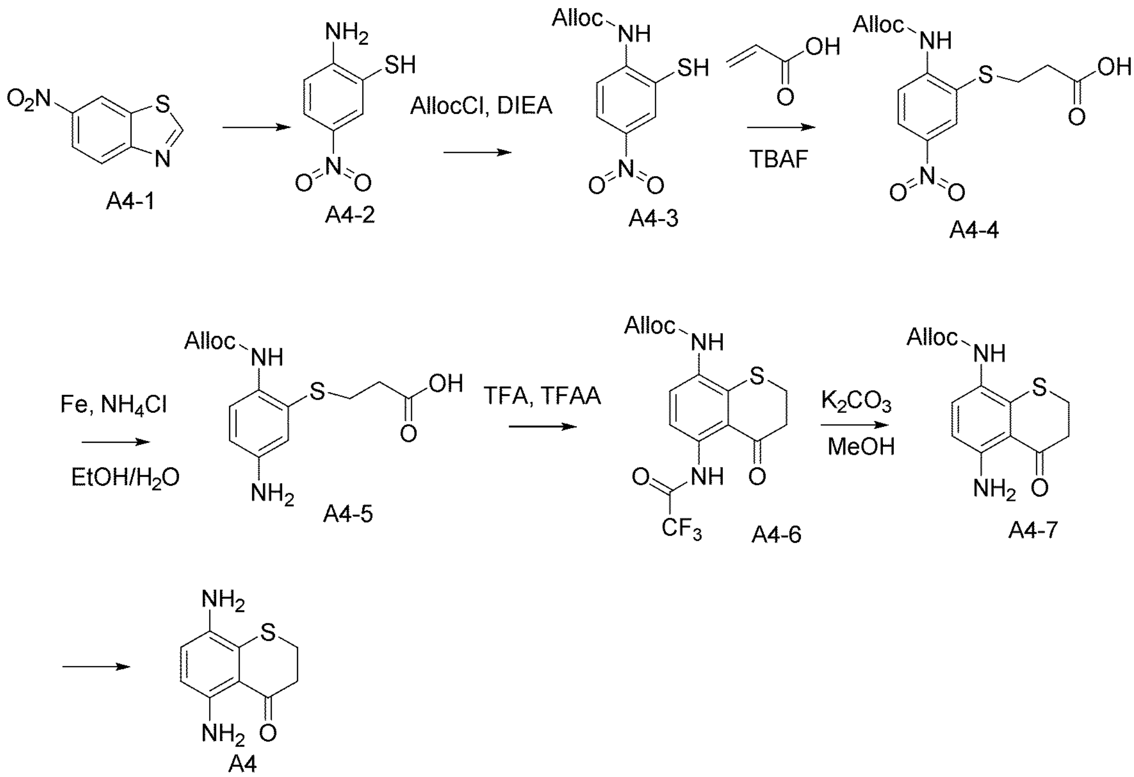


A4-1 A4-2 A4-3 A4-4 A4-5 A4-6 A4-7 A4

Step 1:

The intermediate A4-2 was prepared from the compound A4-1 according to the method described in the literature.

Step 2:

The intermediate A4-2 (3.4 g) was dissolved in dichloromethane at -5-5 °C, into which triethylamine (3.0 g) was added and AllocCl (2.8 g) was then added dropwise slowly. When the addition was finished, mixture obtained was stirred and reacted for 1-2 hours, and then the reaction was quenched by adding water. Reaction product obtained was washed with water once, with saturated saline once, dried over anhydrous sodium sulfate, concentrated to dryness, and 5.5 g of the intermediate A4-3 crude product was obtained. LC-MS (ESI): [M+1]+ =255.7.

Step 3:

The intermediate A4-3 (5.5 g) was added into TBAF (55 ml) and acrylic acid (110 ml) at normal temperature, and mixture obtained was heated to 50-55 °C, and stirred and reacted for 24 hours. Reaction liquid was directly concentrated to dryness, and purified by column chromatography using petroleum ether to a methanol/dichloromethane (1:50) mixture as eluents, and about 5.2 g of the intermediate A4-4 crude product was obtained. LC-MS (ESI): [M+1]+ = 327.4.

Step 4:

The intermediate A4-4 (5.0 g) was added into 100 ml of a mixed solution of ethanol/water (3: 1) at normal temperature, into which ammonium chloride (5.5 g) and iron powder (5.5 g) were added, and mixture obtained was heated under reflux and reacted for 1-2 hours. Reaction liquid was cooled to room temperature, filtered, and the solid obtained was washed with ethanol and concentrated to dryness, and about 3.8 g of the intermediate A4-5 crude product was obtained. LC-MS (ESI): [M+1]+ = 297.4.

Step 5:

The intermediate A4-5 (3.8 g) was added into trifluoroacetic acid (38 ml) at 20-30 °C, into which trifluoroacetic anhydride (38 ml) was added. Mixture obtained was stirred and reacted for 18-24 hours, and then the reaction product obtained was subjected to the following post-treatment: it was directly concentrated to dryness, and purified by column chromatography using a petroleum ether/ethyl acetate (1:0-4:1) mixture as an eluent, and 1.0 g of the intermediate A4-6 was obtained. LC-MS (ESI): [M+1]+ = 375.3.

Step 6:

The intermediate A4-6 (1.0 g) was added into methanol (20 ml) at normal temperature, and then potassium carbonate (2.0 g) and water (5 ml) were added. Mixture obtained was stirred and reacted for 1-2 hours. Water was added to dilute reaction liquid, which then was extracted with ethyl acetate. Organic phase obtained was washed with saturated saline, dried over anhydrous sodium sulfate, and concentrated to dryness, and a crude product was obtained. The crude product was purified by column chromatography using a petroleum ether/ethyl acetate (1:0-2:1) mixture as an eluent, and 0.65 g of the intermediate A4-7 was obtained. LC-MS (ESI): [M+1]+ = 279.3.

Step 7:

The intermediate A4-7 (500 mg) was dissolved in tetrahydrofuran (20 ml) at normal temperature, into which pyrrole (120 mg) and Tetrakis(triphenylphosphine)palladium (160 mg) were added under the protection of nitrogen. When the addition was finished, mixture obtained was stirred and reacted for 1-2 hours. Reaction liquid was directly concentrated to dryness, and purified by column chromatography using a dichloromethane/methanol (30:1) mixture as an eluent, and 30 mg of Compound A4 was obtained as brown solid. LC-MS (ESI): [M+1]+ = 195.4.

**Example 1.1.5 Synthesis of Compound A5**

Compound A5 was synthesized according to a method similar to that described in Journal of Medicinal chemistry, 1998, 41(13), 2308-2318, and synthetic route is shown as follows:

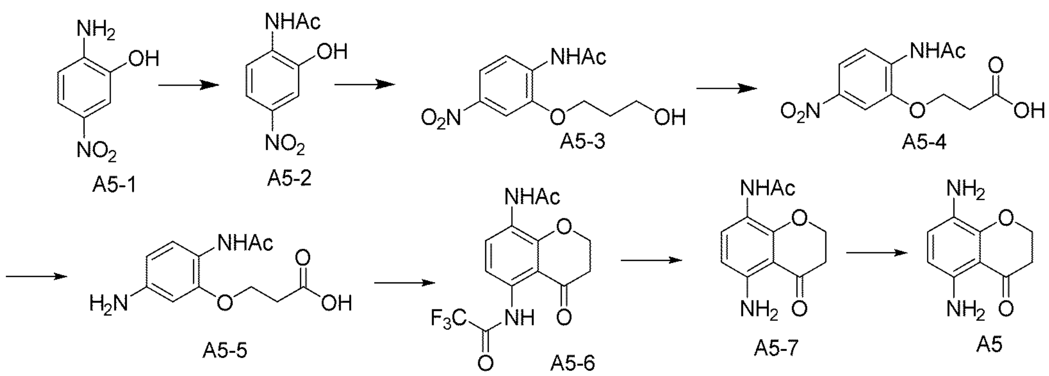

A5-1 A5-2 A5-3 A5-4 A5-5 A5-6 A5-7 A5

Step 1:

Compound A5-1 (25 g) was added into acetic acid (100 ml) at 20-30 °C, into which acetic anhydride (24.9 g) was added slowly. When the addition was finished, mixture obtained was stirred for 3-4 hours. Then reaction liquid was slowly placed into ice water, and stirred. Solid precipitated, and was filtered and collected, which was then washed with water, and dried in vacuum, and 31.0 g of the intermediate A5-2 was obtained as yellow solid. LC-MS (ESI): [M+1]+ = 197.2.

Step 2:

The intermediate A5-2 (29 g), potassium carbonate (40 g), potassium iodide (5 g) and bromopropanol (25 g) were mixed in DMF (300 ml) at 20-30 °C, and mixture obtained was heated to 100-110 °C and stirred for 3-4 hours. Then reaction liquid was cooled to room temperature, and placed in ice water for quenching, and then was extracted with 2 L of ethyl acetate for three times. Organic phases obtained were pooled, washed with saturated saline, dried over anhydrous sodium sulfate, and concentrated to dryness, and then was slurried with petroleum ether to obtain a solid. The solid was dried in vacuum, and 33.0 g of the intermediate A5-3 was obtained as yellow solid. LC-MS (ESI): [M+1] + = 255.3.

Step 3:

The intermediate A5-3 (23 g) was dissolved in acetonitrile (1 L) at 20-25 °C, into which a solution of sodium dihydrogen phosphate (0.67 mol, pH 6.7, 900 ml), TEMPO (5 g), a solution of sodium chlorite (52.0 g dissolved in 60 ml of water), and a solution of sodium hypochlorite (38 ml mixed with 38 ml of water) were added sequentially. When the addition was finished, reaction liquid obtained appeared dark brown, and was stirred for 30 minutes. Next, the reaction liquid was cooled to room temperature, adjusted to pH 2-3 with the addition of 2 N hydrochloric acid, and was extracted with ethyl acetate (1000 ml × 3). Ethyl acetate phases obtained were pooled, washed with saturated saline, dried over anhydrous sodium sulfate, filtered and concentrated to dryness, and then was slurried with petroleum ether, and 24.0 g of the intermediate A5-4 crude product was obtained. LC-MS (ESI): [M+1]+ = 269.2.

Step 4:

The intermediate A5-4 (25 g) and 5% palladium-on-charcoal (5 g) were mixed in methanol (500 ml) at normal temperature, and pressurized hydrogenation was carried out for 3-4 hours. The palladium-on-charcoal was filtered out, and solid obtained was washed with methanol; and the filtrate was concentrated and residue obtained was slurried with petroleum ether to obtain a crude product. The crude product was dried in vacuum, and 18.0 g of the intermediate A5-5 as yellow solid was obtained. LC-MS (ESI): [M+1]+ = 239.5.

Next, starting from the intermediate A5-5, the intermediate A5-7 was synthesized according to procedures similar to those described in step 5 and step 6 in the method for synthesizing Compound A4, and was then deprotected to remove acetyl group with 6 N hydrochloric acid, and Compound A5 was obtained as yellow solid. LC-MS (ESI): [M+1] +=179.2.

**Example 1.1.6 Synthesis of Compound A6**

Compound A6 was synthesized according to a method described in Journal of Medicinal chemistry, 1998, 41(13), 2308-2318, and synthetic route is shown as follows:

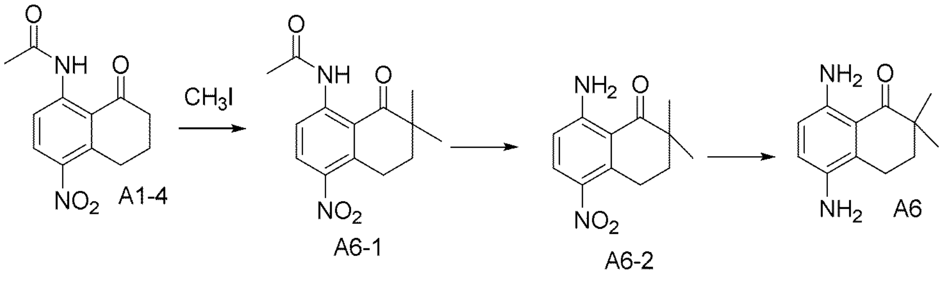


Compound A6 is yellow solid. LC-MS (ESI): [M+1]+ = 248.9.
$^1H$ NMR (400MHz , d6-DMSO): δ 12.289 (1H, s) , 8.666-8.648 (1H , d), 8.198-8.1 (1H , d), 3.138-3.108 (2H, t), 2.768-2.734 (2H, t), 2.219 (3H, s), 2.050-1.991 (2H, m)

Step 1:

The intermediate A1-4 (1.25 g, 5 mmol, 1 eq) was dissolved in 150 ml of tetrahydrofuran under the protection of nitrogen in a 250 mL three-neck reaction flask. The reaction system was cooled to -60 °C in a dry ice bath, and then LDA (2M in THF, 7.6 ml, 15.2 mmol, 3.04 eq) was added. When the addition was finished, mixture obtained was stirred at -60 °C for 30 minutes. MeI (1.45 g, 10.21 mmol, 2.04 eq) was added. When the addition was finished, the dry ice bath was removed and the reaction was allowed to warm naturally to room temperature and continued overnight. Next day, the reaction was quenched by the addition of 50 ml of aqueous ammonium chloride solution, and extracted with ethyl acetate (150 ml × 3). Organic phases obtained were pooled, washed with water once, and concentrated to dryness to obtain a crude product. The crude product was purified by column chromatography using a petroleum ether/ethyl acetate (1:0-5:1) mixture as an eluent, and 350 mg of Compound A6-1 was obtained as yellow solid with a yield of 28%.
$^1$H NMR($CDCl_3$): δ 12.49 (1H, s), 8.77 (1H, d), 8.03 (1H, d), 3.21 (2H, m), 2.22 (3H, s), 1.90 (2H, t), 1.20 (6H, s)

Step 2:

Compound A6-1 (280 mg) was mixed with 20 ml of 6 N hydrochloric acid in a 100 ml single-neck flask, and mixture obtained was then heated under reflux and reacted for 2 hours. The heating was stopped, and reaction liquid was allowed to cool to room temperature. Then the reaction liquid was adjusted to pH 7-8 with the addition of sodium bicarbonate, and extracted with dichloromethane (30 ml × 3). Organic phases obtained were pooled, and concentrated to dryness, and 275 mg of Compound A6-2 was obtained as brown gray solid. The crude product was used directly in the next reaction without purification.

Step 3:

Compound A6-2 (220 mg) was dissolved in acetic acid (25 ml) in a 50 ml three-neck flask, into which 1.3 g of iron powder was then added. After the addition was finished, mixture obtained was stirred for 1-2 hours at 80-85 °C, and when the reaction completed, reaction liquid was distilled under reduced pressure to remove acetic acid, and water (30 ml) was added into residue obtained. Mixture obtained was adjusted to pH 7-8 with the addition of sodium bicarbonate, and extracted with dichloromethane (30 ml × 3). Organic phases obtained were pooled, and concentrated to dryness, to obtain a crude product. The crude product was purified by column chromatography using a petroleum ether/ethyl acetate (1:0-2:1) mixture as an eluent, and 122 mg of Compound A6 was obtained as brown solid with a yield of 75%. LC-MS (ESI): [M+1]+ = 205.0.

**Example 1.1.7 Synthesis of Compound A7**

Synthetic route is shown as follows:

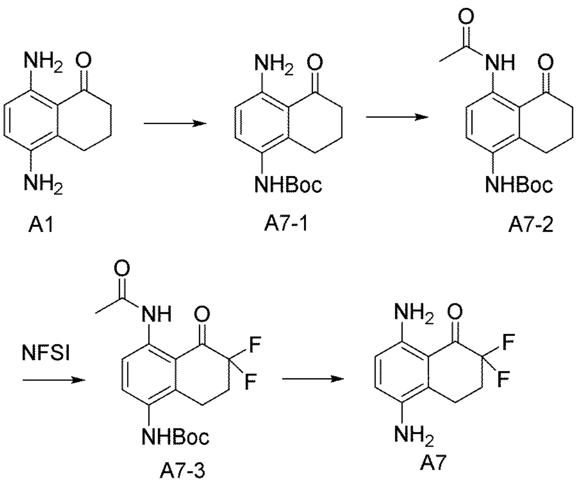

A1 A7-1 A7-2 A7-3 A7

Compound A1 (2.18 g, 124 mmol, 1 eq) was dissolved in tetrahydrofuran (100 ml) in a 250ml three-neck flask, into which Boc anhydride (8.2 g, 376mmol, 3 eq) was then added. Mixture obtained was stirred at 40-45 °C for 5 hours, and then reaction liquid was directly concentrated to dryness, and residue obtained was purified by column chromatography using a petroleum ether/ethyl acetate (1:0-1:3) mixture as an eluent, and 3.0 g of Compound A7-1 was obtained

as yellow solid with a yield of 90%.

1H NMR (400 MHz, CDCl3) δ 7.25 (s, 1H), 6.41 (t, J = 10.2 Hz, 3H), 5.90 (s, 1H), 2.73 (t, J = 6.2 Hz, 2H), 2.61 - 2.47 (m, 2H), 2.02 - 1.86 (m, 3H), 1.49 - 1.36 (m, 9H)

Compound A7-1 (3.0 g, 10.8 mmol, 1 eq) and DIPEA (3.5 g, 27.1 mmol, 1.5 eq) were mixed in dichloromethane (125 ml) in a 250 ml three-neck flask. Reaction liquid was cooled in an ice-salt bath to -5-0 °C, and acetyl chloride (1.3 g, 16.5mmol, 2 eq) was added dropwise. When the addition was finished, the ice-salt bath was removed, and the reaction liquid was allowed to warm naturally and then was stirred at room temperature and reacted for 4 hours. Then the reaction liquid was concentrated to dryness, and residue obtained was purified by column chromatography using a petroleum ether/ethyl acetate (1:0-1:3) mixture as an eluent, and 3.48 g of Compound A7-2 was obtained as light yellow solid with a yield of 100%.

1H NMR (400 MHz, CDCl3) δ 12.01 (s, 1H), 8.55 (d, J = 9.1 Hz, 1H), 7.57 (t, J = 36.2 Hz, 1H), 6.09 (s, 1H), 2.80 (t, J = 6.2 Hz, 2H), 2.65 - 2.58 (m, 2H), 2.15 (s, 3H), 2.07 - 1.98 (m, 2H), 1.44 (s, 9H)

Compound A7-2 (2.45 g, 7.7 mmol, 1 eq) was dissolved in tetrahydrofuran (200 ml) in a 500 ml three-neck flask under the protection of nitrogen. Reaction liquid was cooled in a dry ice-acetone bath to -70 °C - -60 °C, and KHMDS (1 M, 31 ml, 31.0 mmol, 4 eq) was added dropwise slowly. When the addition was finished, the reaction liquid was stirred at -70 °C - -60 °C for 10 minutes. Next, a solution of NFSI in tetrahydrofuran (NFSI (7.35 g, 23 mmol, 3 eq) was dissolved in tetrahydrofuran (70 ml)) was added dropwise, and when the addition was finished, mixture obtained was stirred at -70 °C - -60 °C for 10 minutes. Then reaction liquid was allowed to warm naturally to 20-30 °C and stirred for 3 hours. Saturated ammonium chloride aqueous solution (80 ml) was added to quench the reaction and reaction liquid was extracted with ethyl acetate (150 ml × 3). Organic phases obtained were pooled, washed with saturated saline, and concentrated to dryness to obtain a crude product. The crude product obtained was purified by column chromatography using a petroleum ether/ethyl acetate (1:0-1:3) mixture as an eluent, and 0.85 g of Compound A7-3 was obtained as yellow solid with a yield of 31.2%. LC-MS (ESI): [M+1]+ = 355.8

1H NMR (400 MHz, CDCl3) δ 11.49 (s, 1H), 8.64 (d, J = 9.2 Hz, 1H), 7.74 (d, J = 8.5 Hz, 1H), 6.06 (s, 1H), 3.01 (t, J = 6.4 Hz, 2H), 2.55 - 2.39 (m, 2H), 2.19 (s, 3H), 1.44 (s, 9H)

Compound A7-3 (0.85 g, 2.4 mmol) was mixed with 6 N hydrochloric acid (30 ml) in a 100 ml single-neck flask, and mixture obtained was heated under reflux for 4 hours. Then the heating was stopped, and when reaction liquid was allowed to cool to room temperature, it was adjusted to pH 7-8 with sodium bicarbonate, and extracted with dichloromethane (30 ml × 3). Organic phases obtained were pooled, washed with saturated saline, and concentrated to dryness, to obtain a crude product. The crude product obtained was purified by column chromatography using a petroleum ether/ethyl acetate (1:0-1:3) mixture as an eluent, and 0.25 g of Compound A7 was obtained as brown solid with a yield of 49.1%. LC-MS (ESI): [M+1]+ = 213.9.

**Example 1.1.8 Synthesis of Compound A8**

Synthetic route is shown as follows:

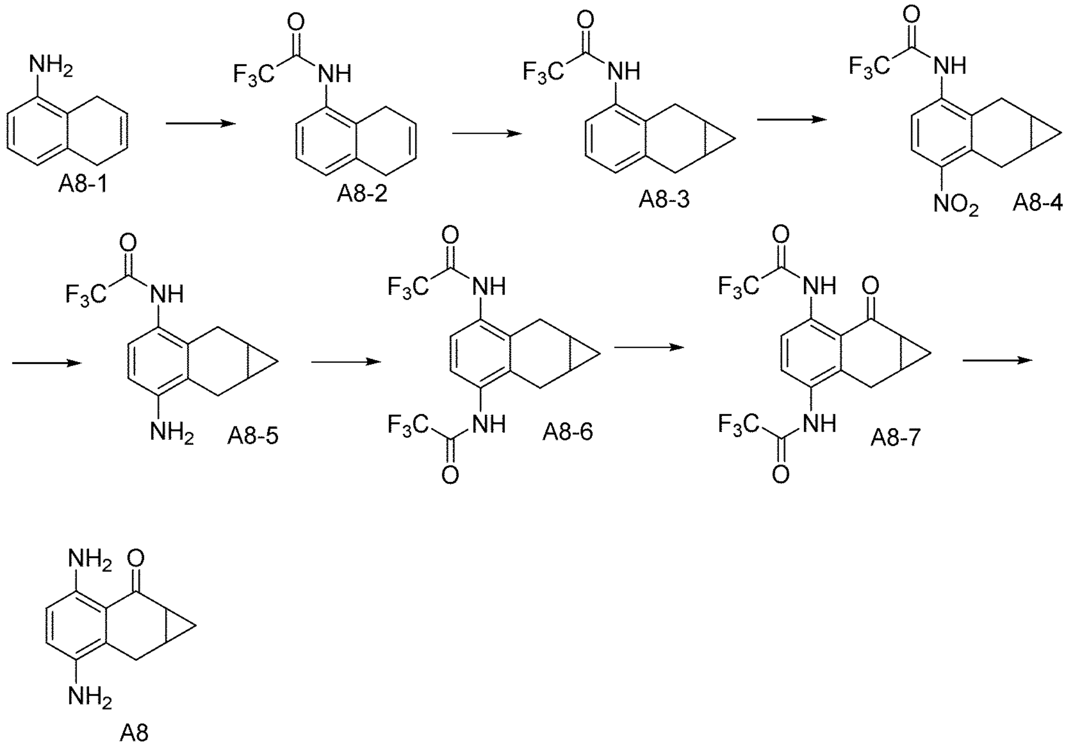

A8-1 A8-2 A8-3 A8-4 A8-5 A8-6 A8-7 A8

Compound A8-1 was synthesized according to the method described in Journal of Medicinal Chemistry, 1989, vol. 32, # 6, p.1217-1230. LC-MS: (ESI): [M+1]+ = 146.3.

Compound A8-1 (1.8 g) and DIEA (4.8 g) were added into DCM (50 ml), and cooled to 0 °C. Trifluoroacetic anhydride (4.8 g) was added thereto, and mixture obtained was warmed to room temperature and reacted for 1 hour. Water (20 ml) was added to separate phases, and organic phase was spun to dryness. Residue obtained was purified by column chromatography (EA: PE = 0-30%), and 2.7 g of the intermediate A8-2 was obtained as white solid. LC-MS: (ESI): [M+1]+ = 242.2.

$Zn(Et)_2$ (33.6 mL) was added into DCM (70 mL) and cooled to 0 °C. TFA (3.8 g) and $CH_2I_2$ (9.0 g) were added thereto, and mixture obtained was reacted for 15 minutes. A solution of Compound A8-2 (2.7 g) in DCM (30 ml) was added dropwise. When the addition was finished, mixture obtained was stirred at room temperature overnight. Water (30 ml) was added to separate phases, and organic phase was spun to dryness, and 2.7 g of the intermediate A8-3 was obtained as off white solid. LC-MS (ESI): [M+1]+ = 256.1.

The intermediate A8-3 (1.5 g) was added into DCM (100 ml) and acetic anhydride (30 ml), and cooled to 0 °C. 65% nitric acid (2.8 g) was added thereto, and mixture obtained was reacted overnight at room temperature. Water (50 ml) and DCM (100 ml) were added to separate phases, and organic phase was spun in an oil pump to dryness, and 1.6 g of the intermediate A8-4 crude product was obtained. The crude product was directly used in the next step. LC-MS (ESI): [M+1]+ = 301.2.

The intermediate A8-4 (1.6 g crude), iron powder (3.2 g), and ammonium chloride (6.4 g) were added to anhydrous ethanol (100 ml), and mixture obtained was warmed under reflux overnight. Reaction liquid was cooled, filtered, spun-dried, and residue obtained was purified by column chromatography (EA: PE = 0-50%), and 0.25 g of the intermediate A8-5 was obtained as brown oil. LC-MS (ESI): [M+1]+ = 271.3.

The intermediate A8-5 (250 mg) and DIEA (400 mg) were added into DCM (10 ml), and cooled to 0 °C. Trifluoroacetic anhydride (320 mg) was added thereto, and mixture obtained was reacted at room temperature for 1 hour. Reaction liquid was washed with water (5 ml), and organic phase was obtained, dried, filtered, and spun to dryness, and 340 mg of the intermediate A8-6 was obtained as yellow solid. LC-MS (ESI): [M+1]+ = 367.3.

The intermediate A8-6 (150 mg) and $CrO_3$ (400 mg) were added to acetic acid (8 ml) and acetic anhydride (4 ml), and mixture obtained was reacted at 30 to 40 °C for 3 hours. Reaction liquid was spun to dry the solvents, and then water (5 ml) was added and extracted with DCM (20 ml). Organic phase was obtained, dried, filtered, and spun to dryness, and 160 mg of the intermediate A8-7 crude product was obtained. The crude product was directly used in the next step. LC-MS (ESI): [M+1]+ = 381.3.

The intermediate A8-7 was deprotected with 6 N hydrochloric acid to remove the two trifluoroacetyl groups and then Compound A8 was obtained upon being subjected to purification by column chromatography. LC-MS (ESI): [M+1] + = 189.3.

Following compounds can be synthesized according to methods similar to those described above.

| | Structure | Mass spectrum | Synthetic Method |
|---|---|---|---|
| A9 | 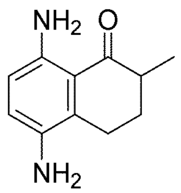 | $[M+1]^+$=191.2 | Synthesized according to a method similar to the method for synthesizing Compound A6, in which 1.5 eq of LDA and 1.2 eq of MeI were used. |
| A10 | 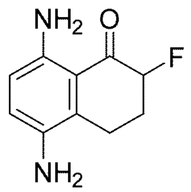 | $[M+1]^+$ =195.2 | Synthesized according to a method similar to the method for synthesizing Compound A7, in which 1.5 eq of LDA and 1.2 eq of NFSI were used. |
| A11 | 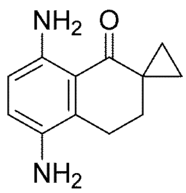 | $[M+1]^+$=203.2 | Synthesized according to a method similar to the method for synthesizing Compound A6, in which 1.2 eq of 1,2-diiodoethane was used, instead of MeI. |
| A12 | 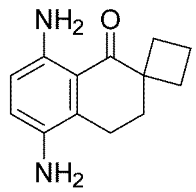 | $[M+1]^+$=217.2 | Synthesized according to a method similar to the method for synthesizing Compound A6, in which 1.2 eq of 1,3-dibromopropane, instead of MeI. |
| A13 | 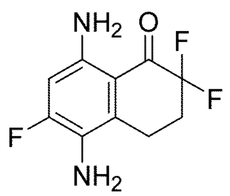 | $[M+1]^+$=231.2 | Synthesized according to a method similar to the method for synthesizing Compound A7, in which Compound A2 was used, instead of Compound A1. |
| A14 | 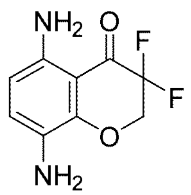 | $[M+1]^+$=215.2 | Synthesized according to a method similar to the method for synthesizing Compound A7, in which Compound A5 was used, instead of Compound A1. |
| A15 | 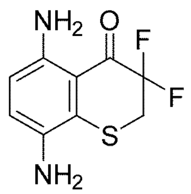 | $[M+1]^+$=231.2 | Synthesized according to a method similar to the method for synthesizing Compound A7, in which Compound A4 was used, instead of Compound A1. |

(continued)

| | Structure | Mass spectrum | Synthetic Method |
|---|---|---|---|
| A16 | 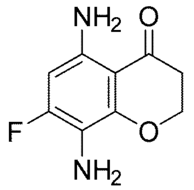 | $[M+1]^+$=197.2 | Synthesized according to a method similar to the method for synthesizing Compound A5, in which 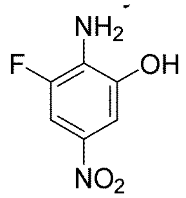 was used. |
| A17 | 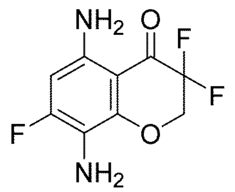 | $[M+1]^+$=233.2 | Synthesized according to a method similar to the method for synthesizing Compound A7, in which Compound A16 was used, instead of Compound A1. |
| A18 | 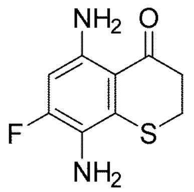 | $[M+1]^+$=213.2 | Synthesized according to a method similar to the method for synthesizing Compound A6, in which 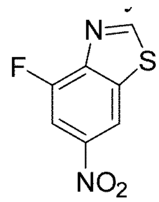 was used. |
| A19 | 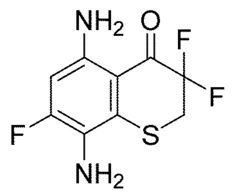 | $[M+1]^+$=249.3 | Synthesized according to a method similar to the method for synthesizing Compound A7, in which Compound A18 was used, instead of Compound A1. |
| A20 | 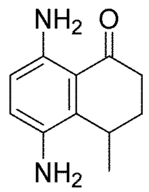 | $[M+1]^+$=191.2 | Synthesized according to Method 3 for synthesizing Compound A1, in which 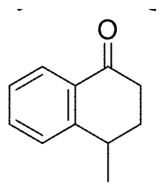 was used. |
| A21 | 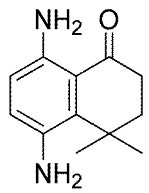 | $[M+1]^+$ =205.3 | Synthesized according to Method 3 for synthesizing Compound A1, in which 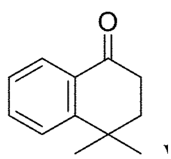 was used. |

(continued)

| | Structure | Mass spectrum | Synthetic Method |
|---|---|---|---|
| A22 | 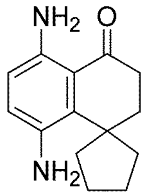 | $[M+1]^+$ =231.4 | Synthesized according to Method 3 for synthesizing Compound A1, in which 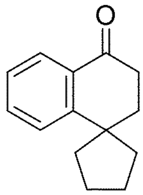 was used. |
| A23 | 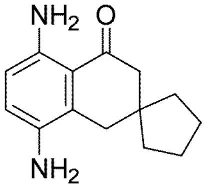 | $[M+1]^+$ =231.3 | Synthesized according to Method 3 for synthesizing Compound A1, in which 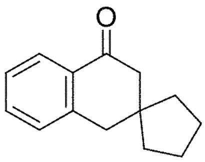 was used. |
| A24 | 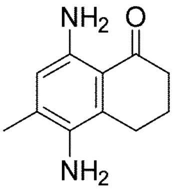 | $[M+1]^+$ = 191.2 | Synthesized according to Method 2 for synthesizing Compound A1, in which 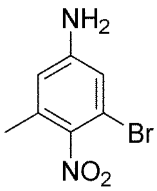 was used. |
| A25 | 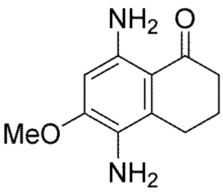 | $[M+1]^+$ =207.3 | Synthesized according to Method 2 for synthesizing Compound A1, in which 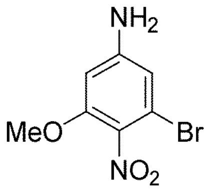 was used. |
| A26 | 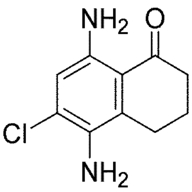 | $[M+1]^+$ =211.6 | Synthesized according to a method similar to the method for synthesizing Compound A2, in which 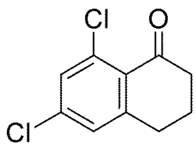 was used. |
| A27 | 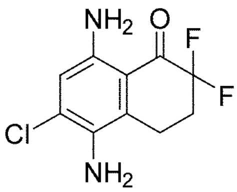 | $[M+1]^+$ =247.6 | Synthesized according to a method similar to the method for synthesizing Compound A7, in which Compound A26 was used, instead of Compound A1. |

(continued)

| | Structure | Mass spectrum | Synthetic Method |
|---|---|---|---|
| A28 | 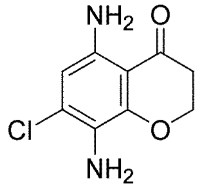 | $[M+1]^+$ =213.6 | Synthesized according to a method similar to the method for synthesizing Compound A5, in which 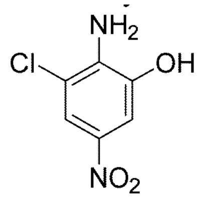 was used. |
| A29 | 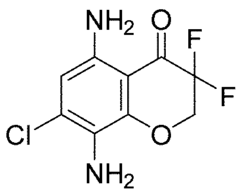 | $[M+1]^+$ =249.6 | Synthesized according to a method similar to the method for synthesizing Compound A7, in which Compound A28 was used, instead of Compound A1. |
| A30 | 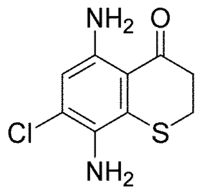 | $[M+1]^+$ =229.7 | Synthesized according to a method similar to the method for synthesizing Compound A6, in which 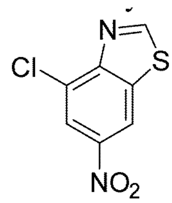 was used. |
| A31 | 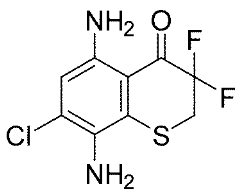 | $[M+1]^+$ =265.7 | Synthesized according to a method similar to the method for synthesizing Compound A7, in which Compound A30 was used, instead of Compound A1. |
| A32 | 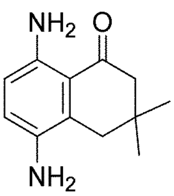 | $[M+1]^+$ =205.3 | Synthesized according to Method 3 for synthesizing Compound A1, in which 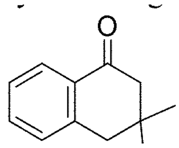 was used. |

**Group 1.2 of Examples Synthesis of camptothecins (Friedlander reaction)**

Control compound of camptothecins MWC-1 was synthesized as follows:

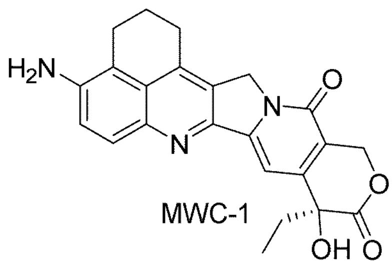

MWC-1

Compound A1 (176 mg, 1 eq), cyclic compound CDE (315 mg, 1.2 eq), and PPTS (301 mg, 1.2 eq) were mixed in toluene (50 ml) at normal temperature, and mixture obtained was reacted under reflux and water separation for 3-4 hours. Then reaction liquid was directly concentrated to dryness, and residue obtained was purified by column chromatography using petroleum ether to a methanol/dichloromethane (1:20) mixture as eluents, and 260 mg of compound MWC-1 was obtained as yellow solid. LC-MS (ESI): [M+1]+ = 404.

**Example 1.2.1** Synthesis of Camptothecin Compound 1

Synthetic route is shown as follows:

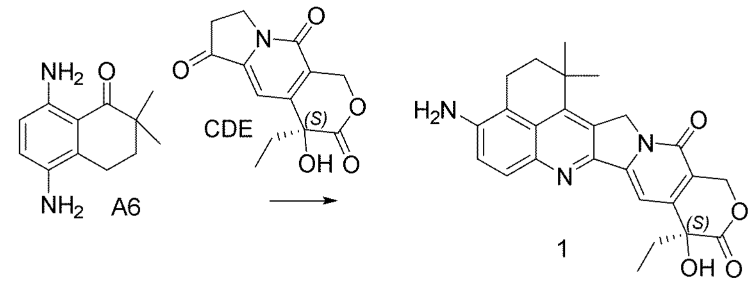

Compound A6 (120 mg, 0.59 mmol, 1 eq), tricyclic compound CDE (180 mg, 0.68 mmol, 1.2 eq), and PPTS (35 mg, 0.14 mmol, 0.25 eq) were mixed in 15 ml in a 50 ml single-neck reaction flask under the protection of nitrogen, and then heated under reflux and reacted for 3-4 hours. Reaction liquid was distilled under reduced pressure to distill off a majority of acetic acid, and residue obtained was purified by column chromatography using a dichloromethane/methanol (15:1) mixture as an eluent, and 80 mg of a crude product was obtained. The crude product was further separated and purified by preparative liquid phase chromatography, and 3 mg of Camptothecin Compound 1 was obtained as yellow solid.

LC-MS (ESI): [M+1]+ =432.4.

**Example 1.2.2** Synthesis of Camptothecin Compound 2 (2A and 2B)

Synthetic route is shown as follows:

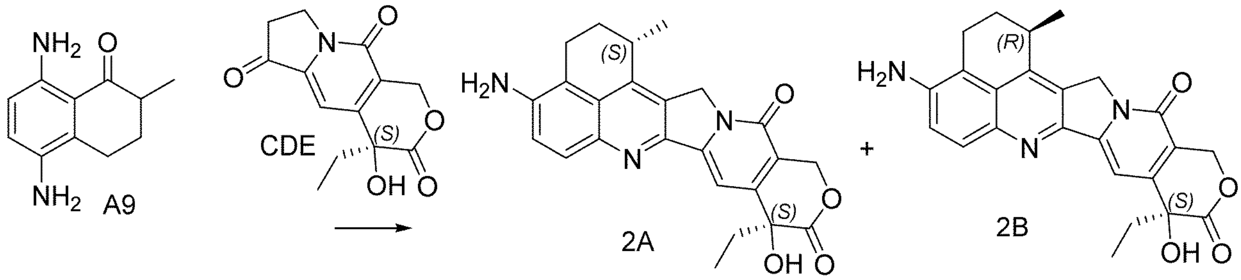

According to a method similar to that described in Example 1.2.1, in which Compound A9 was used instead of Compound A6, Camptothecin Compound 2A (having a short retention time) and Camptothecin Compound 2B (having a long retention time) were obtained upon separation and purification by preparative liquid phase chromatography. Camptothecin Compound 2A was obtained as yellow solid. LC-MS (ESI): [M+1]+ =418.3. Camptothecin Compound 2B was obtained as yellow solid. LC-MS (ESI): [M+1]+ =418.4.

**Example 1.2.3** Synthesis of Camptothecin Compound 3

Synthetic route is shown as follows:

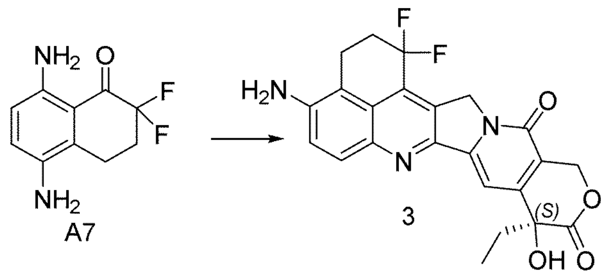

A7

3

According to a method similar to that described in Example 1.2.1 in which Compound A7 was used instead of Compound A6 and toluene was used as the solvent instead of acetic acid, Camptothecin Compound 3 was prepared and obtained as earthy yellow solid. LC-MS (ESI): [M+1]+ =440.8.

1H NMR (400 MHz, Acetone) $\delta$ 7.89 (d, J = 9.1 Hz, 1H), 7.48 (d, J = 9.1 Hz, 1H), 7.37 (s, 1H), 7.31 (d, J = 8.9 Hz, 0H), 5.59 - 5.26 (m, 5H), 3.11 - 3.05 (m, 4H), 2.73 - 2.59 (m, 3H), 1.98 (dt, J = 14.0, 7.0 Hz, 1H), 1.05 - 0.98 (m, 3H)
1H NMR (400 MHz, DMSO) $\delta$ 7.87 (d, J = 9.1 Hz, 0H), 7.41 (d, J = 9.1 Hz, 0H), 7.21 (s, 0H), 6.50 (s, 0H), 6.15 (s, 1H), 5.36 (d, J = 46.6 Hz, 1H), 2.93 (t, J = 6.5 Hz, 1H), 2.66 - 2.54 (m, 1H), 1.97 - 1.68 (m, 3H), 1.24 (s, 1H), 0.88 (t, J = 7.3 Hz, 3H)

**Example 1.2.4** Synthesis of Camptothecin Compound 4 (4A and 4B)

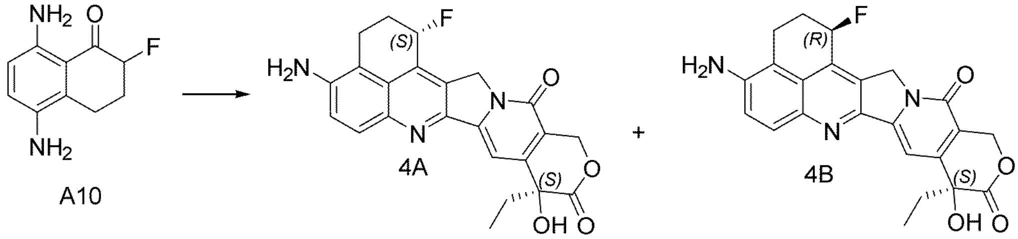

A10

4A

+

4B

According to a method similar to that described in Example 1.2.1, in which Compound A10 was used instead of Compound A6, Camptothecin Compound 4A (having a short retention time) and Camptothecin Compound 4B (having a long retention time) were obtained upon separation and purification by preparative liquid phase chromatography. Compound 4A was obtained as earthy yellow solid. LC-MS (ESI): [M+1]+ =422.3. Camptothecin Compound 4B was obtained as earthy yellow solid. LC-MS (ESI): [M+1]+ =422.3.

**Example 1.2.5** Synthesis of Camptothecin Compound 5

Synthetic route is shown as follows:

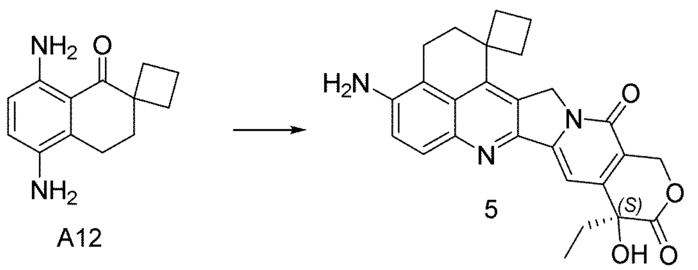

A12

5

According to a method similar to that described in Example 1.2.1, in which Compound A12 was used instead of Compound A6, Camptothecin Compound 5 was prepared and obtained as yellow solid. LC-MS (ESI): [M+1]+ =444.3.

**Example 1.2.6** Synthesis of Camptothecin Compound 6

Synthetic route is shown as follows:

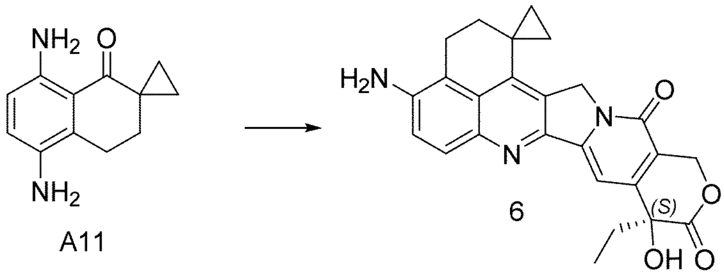

A11

6

According to a method similar to that described in Example 1.2.1, in which Compound A11 was used instead of Compound A6, Camptothecin Compound 6 was prepared and obtained as yellow solid. LC-MS (ESI): [M+1]+ =430.5.

**Example 1.2.7** Synthesis of Camptothecin Compound 7

Synthetic route is shown as follows:

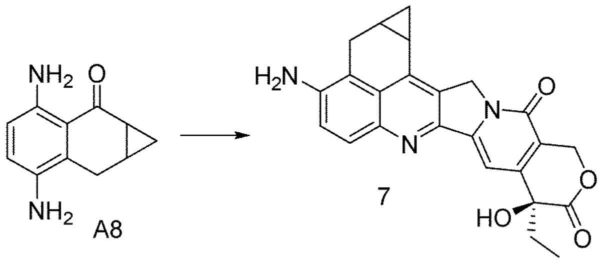

A8

7

According to a method similar to that described in Example 1.2.1, in which Compound A8 was used instead of Compound A6, 0.7 mg of Camptothecin Compound 7 was prepared and obtained as yellow solid. LC-MS (ESI): [M+1] + =416.3.

**Example 1.2.8** Synthesis of Camptothecin Compound 9

Synthetic route is shown as follows:

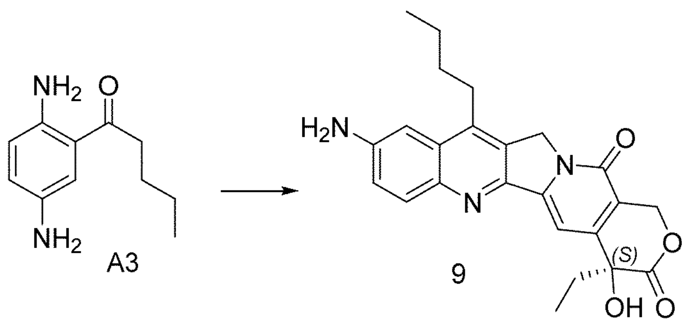

A3

9

According to a method similar to that described in Example 1.2.1, in which Compound A3 was used instead of Compound A6, Camptothecin Compound 9 was prepared and obtained as brownish red solid. LC-MS (ESI): [M+1]+ =420.3.

**Example 1.2.9** Synthesis of Camptothecin Compound 12

Compound 12 was synthesized according to a method similar to that described in Journal of Medicinal chemistry,

1998, 41(13), 2308-2318, and synthetic route is shown as follows:

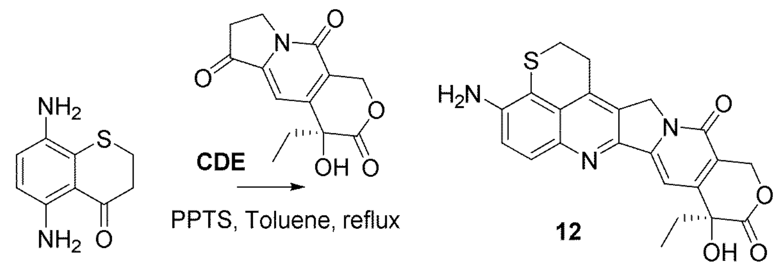


A4

12

According to a method similar to that described in Example 1.2.1, in which Compound A4 was used instead of Compound A6, and toluene was used as the solvent instead of acetic acid, 30 mg of Camptothecin Compound 12 was synthesized and obtained as brown solid. LC-MS (ESI): [M+1]+ =422.5.

**Example 1.2.10** Synthesis of Camptothecin Compound 13

Synthetic route is shown as follows:

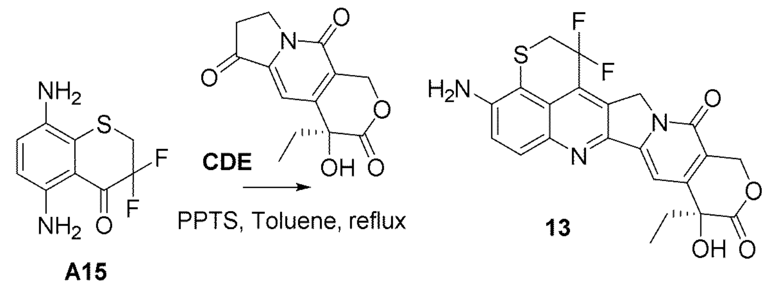


A15

13

According to a method similar to that described in Example 1.2.1, in which Compound A15 was used instead of Compound A6 and toluene was used as the solvent, Compound 13 was synthesized and obtained as brown solid. LC-MS (ESI): [M+1]+=458.4.

**Example 1.2.11** Synthesis of Camptothecin Compound 14

Synthetic route is shown as follows:

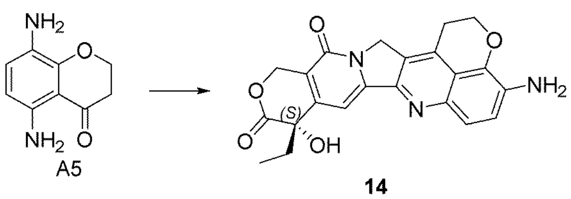

A5

14

According to a method similar to that described in Example 1.2.1, in which Compound A5 was used instead of Compound A6 and toluene was used as the solvent, Compound 14 was synthesized and obtained as yellow solid. LC-MS (ESI): [M+1]+=406.1.

**Example 1.2.12** Synthesis of Camptothecin Compound 14-P

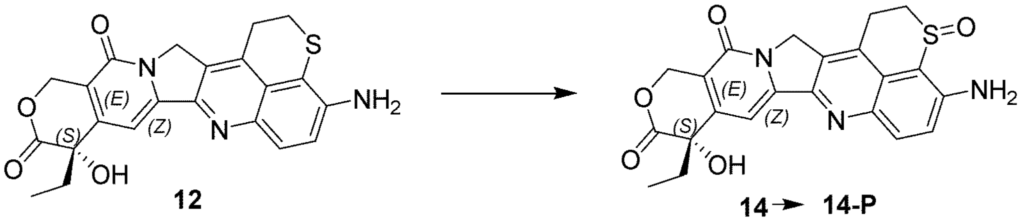

12

14 → 14-P

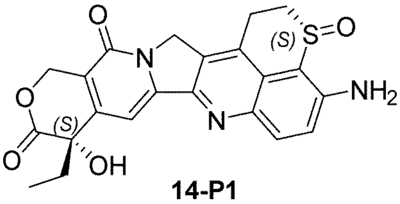

14-P1

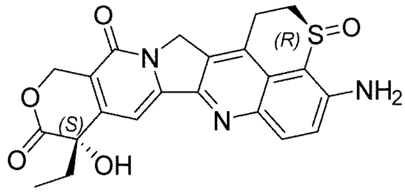

14-P2

Compound 12 (200 mg) was dissolved in a mixed solvent of acetic acid (24 ml) and water (3 ml) at normal temperature, and mixture obtained was cooled to 5 °C under the protection of nitrogen, into which 30% $H_2O_2$ (580 mg) was then added. Reaction liquid was stirred for 2 hours, concentrated to dryness, and residue obtained was subjected to preparative HPLC, and Compounds 14-P1 and 14-P2 were separated and obtained. Being lyophilized, 42 mg of Compound 14-P1 was obtained as brown solid; and 25 mg of Compound 14-P2 was obtained as brown solid. LC-MS (ESI): [M+1]+ =438.5.

<u>**Example 1.2.13**</u> Synthesis of Camptothecin Compound 15

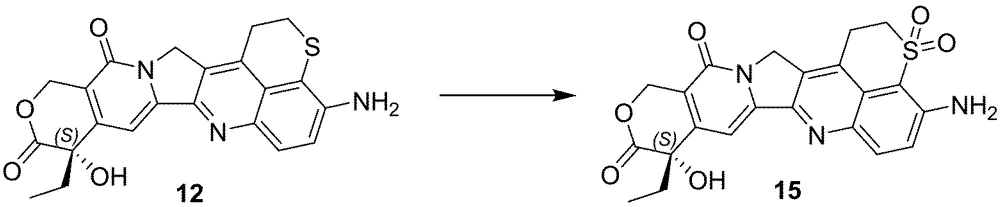

12

15

Compound 12 (100 mg) was dissolved in a mixed solvent of acetic acid (24 ml) and water (3 ml) at normal temperature, and mixture obtained was cooled to 5 °C under the protection of nitrogen, into which 30% $H_2O_2$ (750 mg) was then added. Reaction liquid was stirred for 2 hours, allowed to warm to room temperature 25 °C, and stirred overnight. After the reaction completed as confirmed with LCMS analysis, the reaction liquid was concentrated to dryness, and residue obtained was subjected to preparative HPLC, and Compound 15 was separated and collected. Being lyophilized, 8 mg of Compound 15 was obtained as brown solid. LC-MS (ESI): [M+1]+=454.4.

<u>**Example 1.2.14**</u> Synthesis of Camptothecin Compound 16

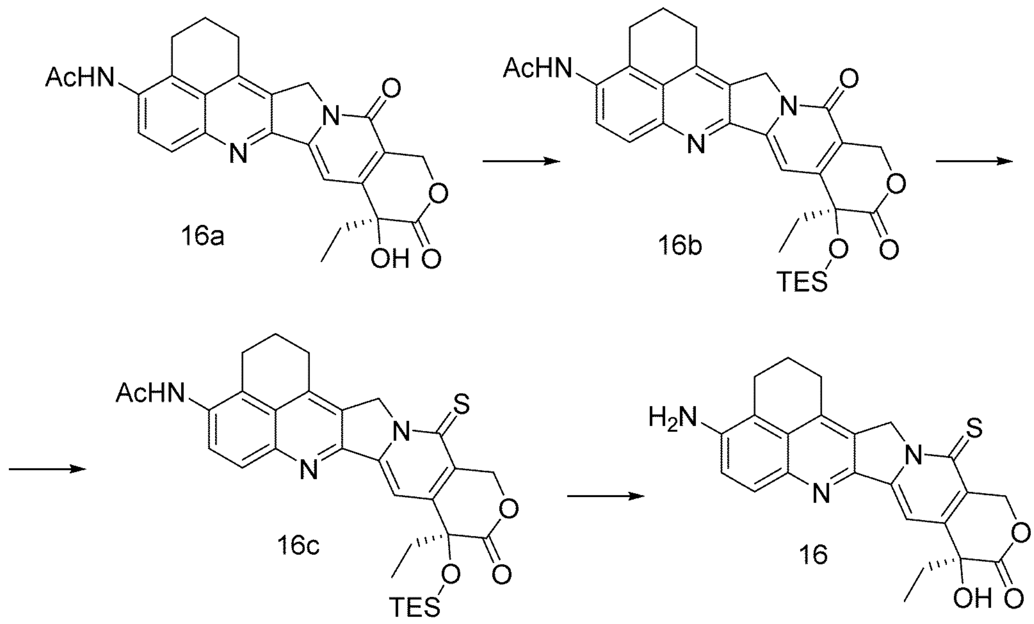

16a 16b 16c 16

The intermediate 16a was synthesized according to the method described in WO2020200880A1, and then the intermediate 16c having a structure of 16a-thiocamptothecin was prepared according to the method described in a literature (J. Med. Chem. 2008, 51, 3040-3044), and finally was de-protected to remove the acetyl group, and Compound 16 was prepared and obtained. LC-MS (ESI): [M+1]+=420.3.

**Example 1.2.15** Synthesis of Camptothecin Compound 17

Synthetic route is shown as follows:

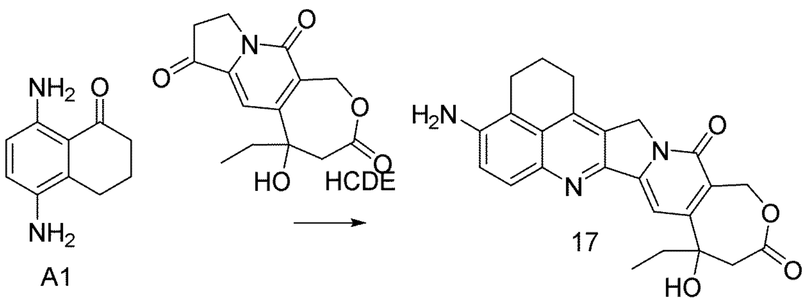

A1 HCDE 17

According to a method similar to that described in Example 1.2.1, in which Compound A1 was used instead of Compound A6, HCDE was used instead of CDE, and toluene was used as the solvent, homocamptothecin Compound 17 (a mixture of S, and R configurations) was obtained. LC-MS (ESI): [M+1]+=418.5.

**Example 1.2.16** Synthesis of Camptothecin Compound 18

Synthetic route is shown as follows:

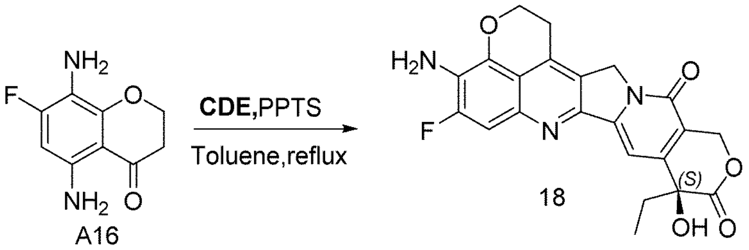


A16 18

According to a method similar to that described in Example 1.2.1, in which Compound A16 was used instead of Compound A6 and toluene was used as the solvent, Compound 18 was synthesized as light yellow solid. LC-MS (ESI): [M+1]+ =424.2.

**Example 1.2.17** Synthesis of Camptothecin Compound 19

Synthetic route is shown as follows:

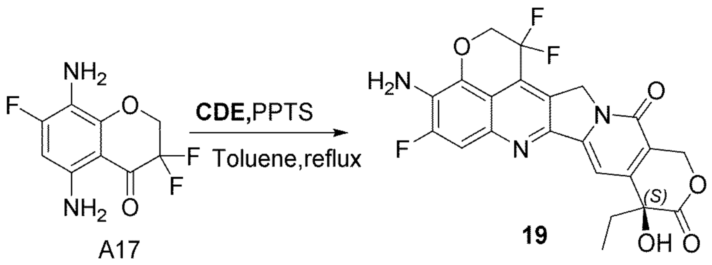


A17

**19**

According to a method similar to that described in Example 1.2.1, in which Compound A17 was used instead of Compound A6 and toluene was used as the solvent, Compound 19 was synthesized as yellow solid. LC-MS (ESI): [M+1]+ =460.5.

**Example 1.2.18** Synthesis of Camptothecin Compound 20

Synthetic route is shown as follows:

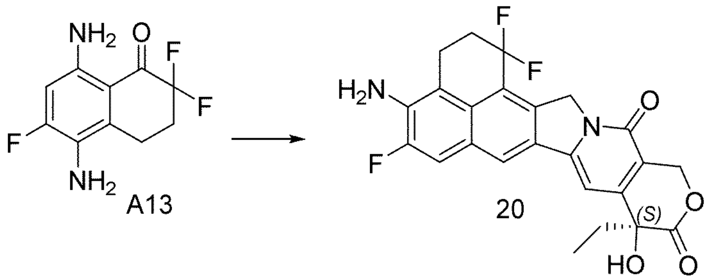

A13

20

According to a method similar to that described in Example 1.2.1, in which Compound A13 was used instead of Compound A6 and toluene was used as the solvent, Compound 20 was synthesized as yellow solid. LC-MS (ESI): [M+1]+ =458.2.

**Example 1.2.19** Synthesis of Camptothecin Compound 21

Synthetic route is shown as follows:

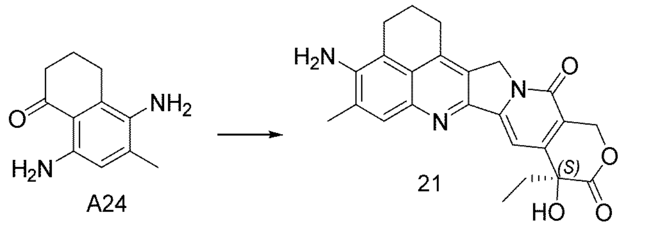

A24

21

According to a method similar to that described in Example 1.2.1, in which Compound A24 was used instead of Compound A6 and toluene was used as the solvent, Compound 21 was obtained as brownish red solid. LC-MS (ESI): [M+1]+ = 418.4.

**Example 1.2.20** Synthesis of Camptothecin Compound 22

Synthetic route is shown as follows:

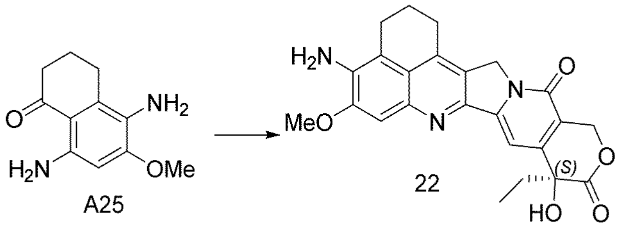

A25

22

According to a method similar to that described in Example 1.2.1, in which Compound A25 was used instead of Compound A6 and toluene was used as the solvent, Compound 22 was obtained as brownish red solid. LC-MS (ESI): [M+1]+= 434.4.

**Example 1.2.21** Synthesis of Camptothecin Compound 23

Synthetic route is shown as follows:

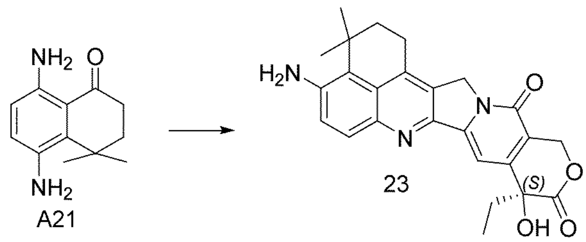

A21

23

According to a method similar to that described in Example 1.2.1, in which Compound A21 was used instead of Compound A6 and toluene was used as the solvent, Compound 23 was obtained as brownish red solid. LC-MS (ESI): [M+1]+ = 432.4.

**Example 1.2.22** Synthesis of Camptothecin Compound 24

Synthetic route is shown as follows:

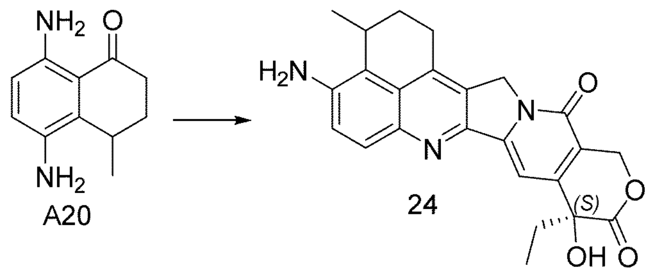

A20

24

According to a method similar to that described in Example 1.2.1, in which Compound A20 was used instead of Compound A6 and toluene was used as the solvent, Compound 24 (a pair of isomers) was obtained as brownish red solid. LC-MS (ESI): [M+1]+ = 418.4.

**Example 1.2.23** Synthesis of Camptothecin Compound 25

Synthetic route is shown as follows:

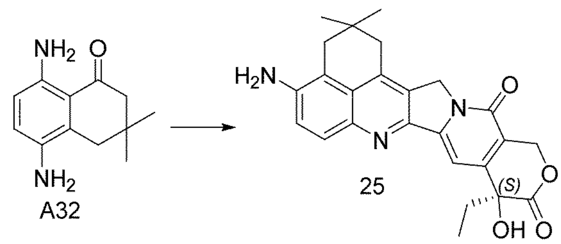

According to a method similar to that described in Example 1.2.1, in which Compound A32 was used instead of Compound A6 and toluene was used as the solvent, Compound 25 was obtained as brownish red solid. LC-MS (ESI): [M+1]+ = 432.4.

**Example 1.2.24** Synthesis of Camptothecin Compound 26

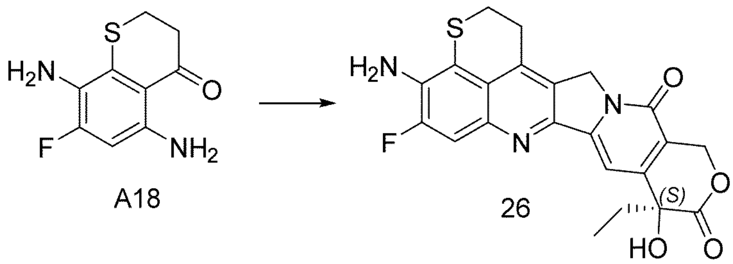

According to a method similar to that described in Example 1.2.1, in which Compound A18 was used instead of Compound A6 and toluene was used as the solvent, Compound 26 was obtained as brownish red solid. LC-MS (ESI): [M+1]+= 440.4.

**Example 1.2.25** Synthesis of Camptothecin Compound 27

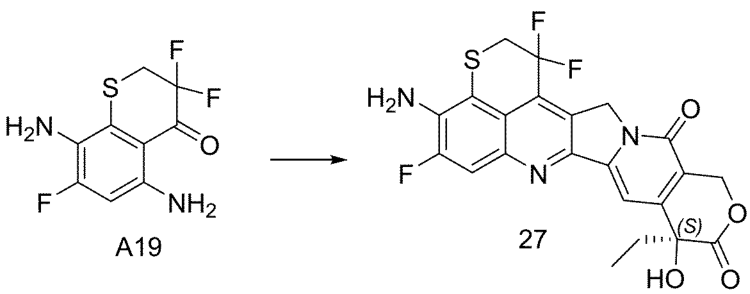

According to a method similar to that described in Example 1.2.1, in which Compound A19 was used instead of Compound A6 and toluene was used as the solvent, Compound 27 was obtained as brownish red solid. LC-MS (ESI): [M+1]+= 476.4.

**Example 1.2.26** Synthesis of Camptothecin Compound 31

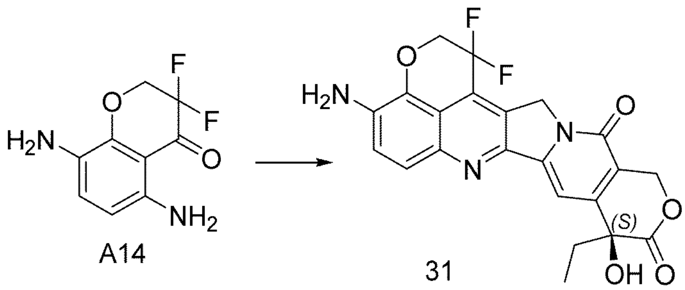

A14 31

According to a method similar to that described in Example 1.2.1, in which Compound A14 was used instead of Compound A6 and toluene was used as the solvent, Compound 31 was obtained as yellow solid. LC-MS (ESI): [M+1]+= 442.4.

**Example 1.2.27** Synthesis of Camptothecin Compound 32

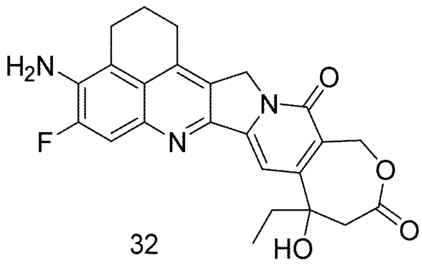

32

According to a method similar to that described in Example 1.2.1, in which Compound A2 was used instead of Compound A6, HCDE was used instead of CDE, and toluene was used as the solvent, Compound 32 was synthesized and obtained as yellow solid. LC-MS (ESI): [M+1]+ =436.4.

**Example 1.2.28** Synthesis of Camptothecin Compound 33

Synthetic route is shown as follows:

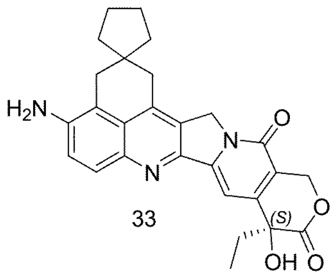

33

According to a method similar to that described in Example 1.2.1, in which Compound A23 was used instead of Compound A6, and toluene was used as the solvent, Compound 33 was synthesized as yellow solid. LC-MS (ESI): [M+1]+= 458.5.

**Example 1.2.29** Synthesis of Camptothecin Compound 34

Synthetic route is shown as follows:

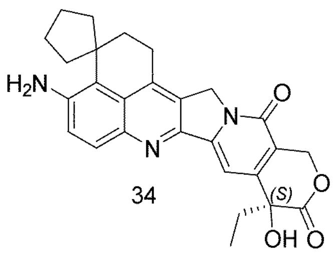

34

According to a method similar to that described in Example 1.2.1, in which Compound A22 was used instead of Compound A6, and toluene was used as the solvent, Compound 34 was synthesized as yellow solid. LC-MS (ESI): [M+1]+= 458.5.

<u>**Example 1.2.30**</u> Synthesis of Camptothecin Compound 35 and Compound 38

Synthetic route is shown as follows:

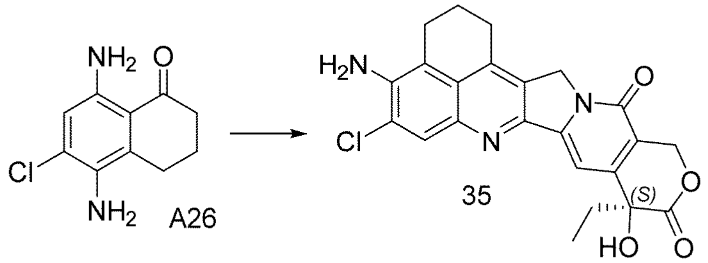

A26

35

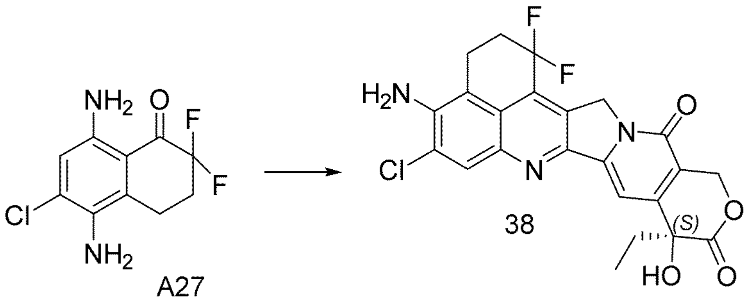

A27

38

According to a method similar to that described in Example 1.2.1, in which Compound A26 was used instead of Compound A6, and toluene was used as the solvent, Compound 35 was synthesized as brownish red solid. LC-MS (ESI): [M+1]+ = 438.7.

According to a method similar to that described in Example 1.2.1, in which Compound A27 was used instead of Compound A6, and toluene was used as the solvent, Compound 38 was synthesized and obtained as brownish red solid. LC-MS (ESI): [M+1]+= 474.7.

<u>**Example 1.2.31**</u> Synthesis of Camptothecin Compound 36 and Compound 39

Synthetic route is shown as follows:

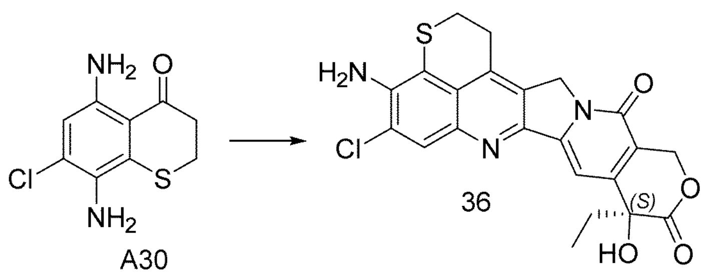

A30

36

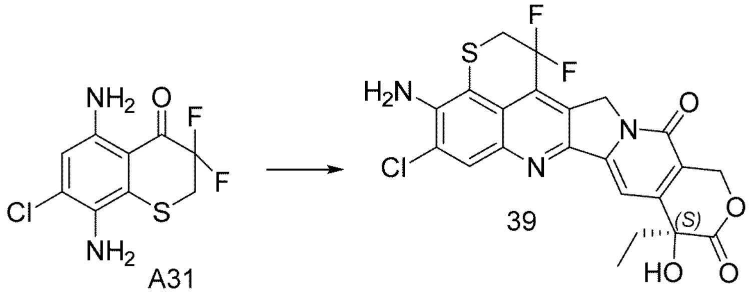

A31

39

According to a method similar to that described in Example 1.2.1, in which Compound A30 was used instead of Compound A6, and toluene was used as the solvent, Compound 36 was synthesized as brownish red solid. LC-MS (ESI): [M+1]+= 456.7.

According to a method similar to that described in Example 1.2.1, in which Compound A31 was used instead of Compound A6, and toluene was used as the solvent, Compound 39 was synthesized and obtained as brownish red solid. LC-MS (ESI): [M+1]+= 492.7.

**Example 1.2.32** Synthesis of Camptothecin Compound 37 and Compound 40

Synthetic route is shown as follows:

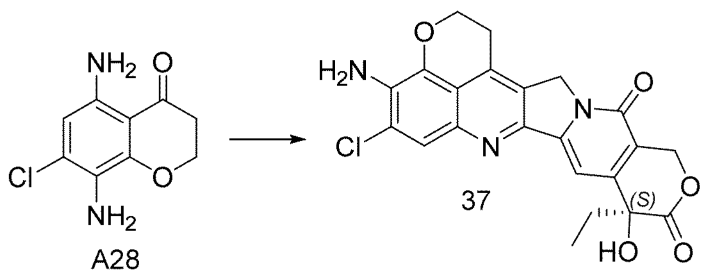

A28

37

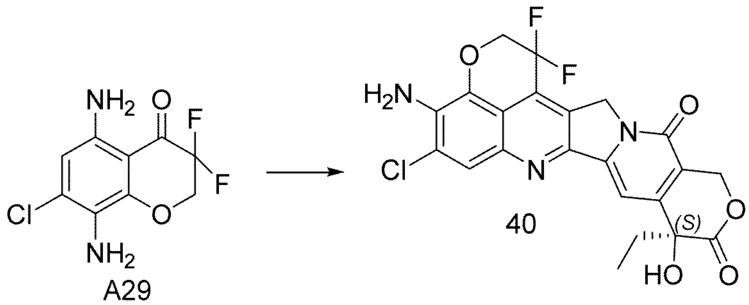

A29

40

According to a method similar to that described in Example 1.2.1, in which Compound A28 was used instead of Compound A6, and toluene was used as the solvent, Compound 37 was synthesized as brownish red solid. LC-MS (ESI): [M+1]+= 440.8.

According to a method similar to that described in Example 1.2.1, in which Compound A29 was used instead of Compound A6, and toluene was used as the solvent, Compound 40 was synthesized and obtained as brownish red solid. LC-MS (ESI): [M+1]+ = 476.7.

**Group 2 of Examples Synthesis of Drug-containing linkers**

**Example 2.1** Synthesis of Drug-containing linker MWD-L1

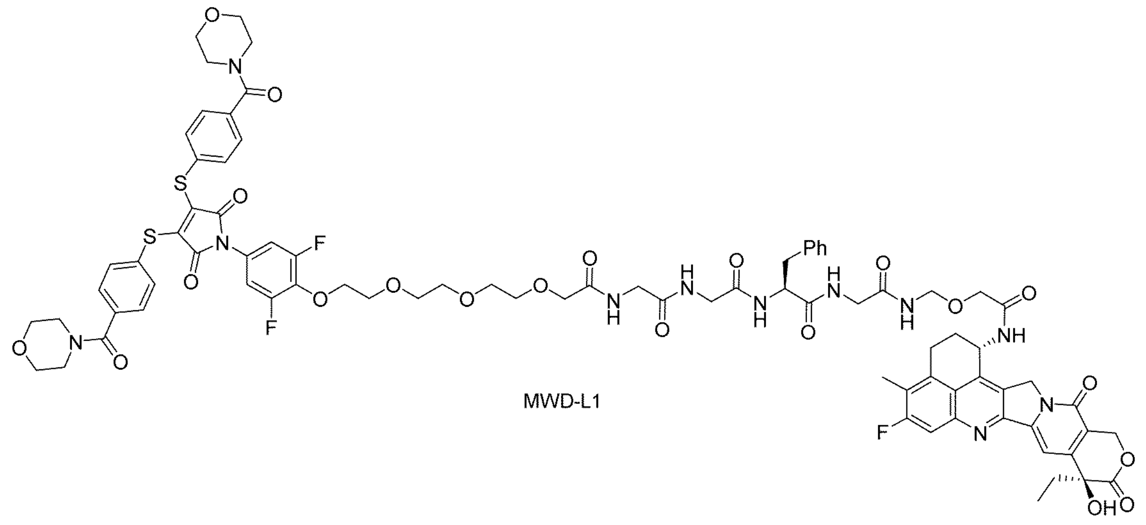

MWD-L1

Synthetic route is shown as follows:

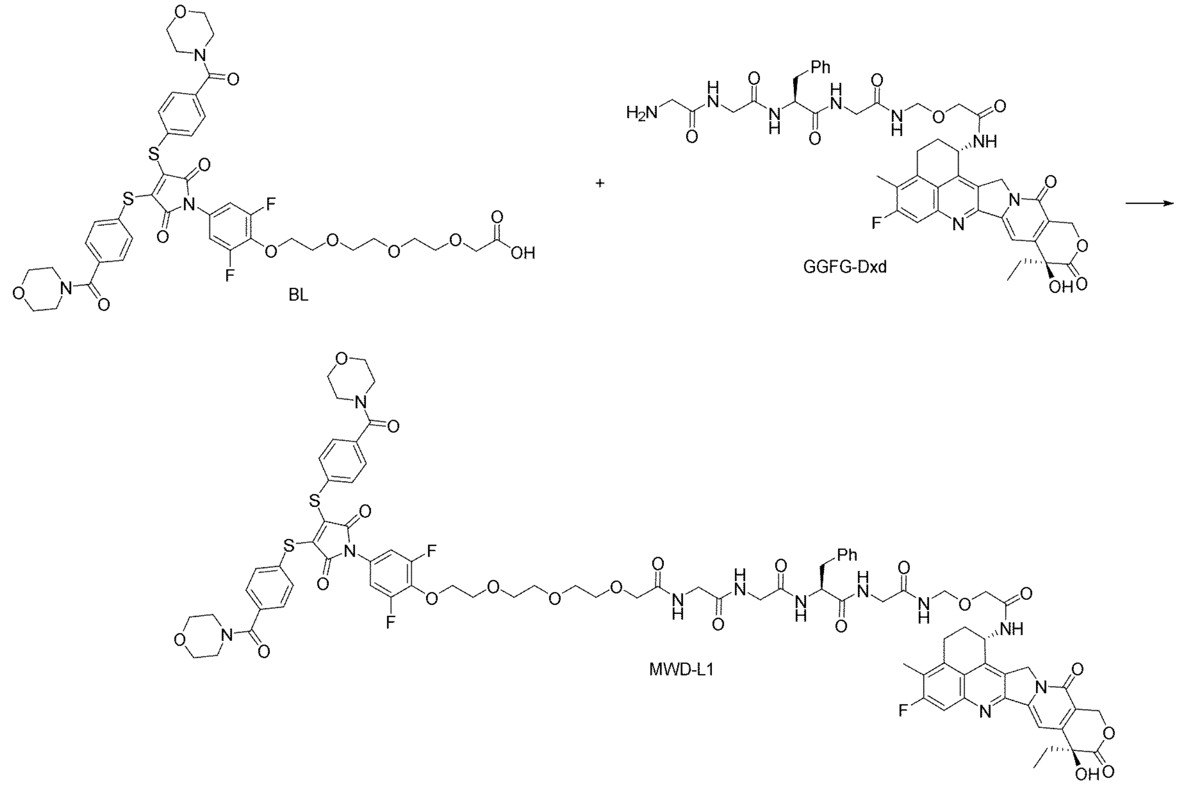

BL

GGFG-Dxd

MWD-L1

GGFG-Dxd was synthesized according to the method described in the patent publication US20190151328A1:

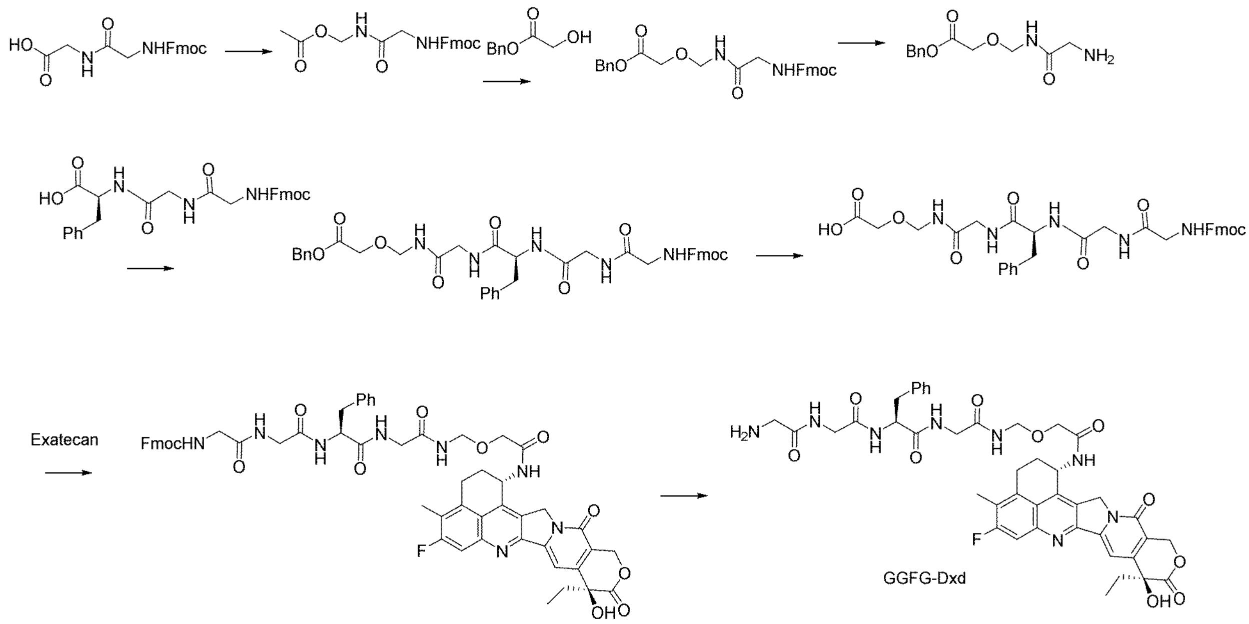

GGFG-Dxd

GGFG-Dxd was obtained as a light yellow solid. LC-MS (ESI): M+1= 841.

Linker compound BL was synthesized according to the method described in the patent publication WO2018/095422A1:

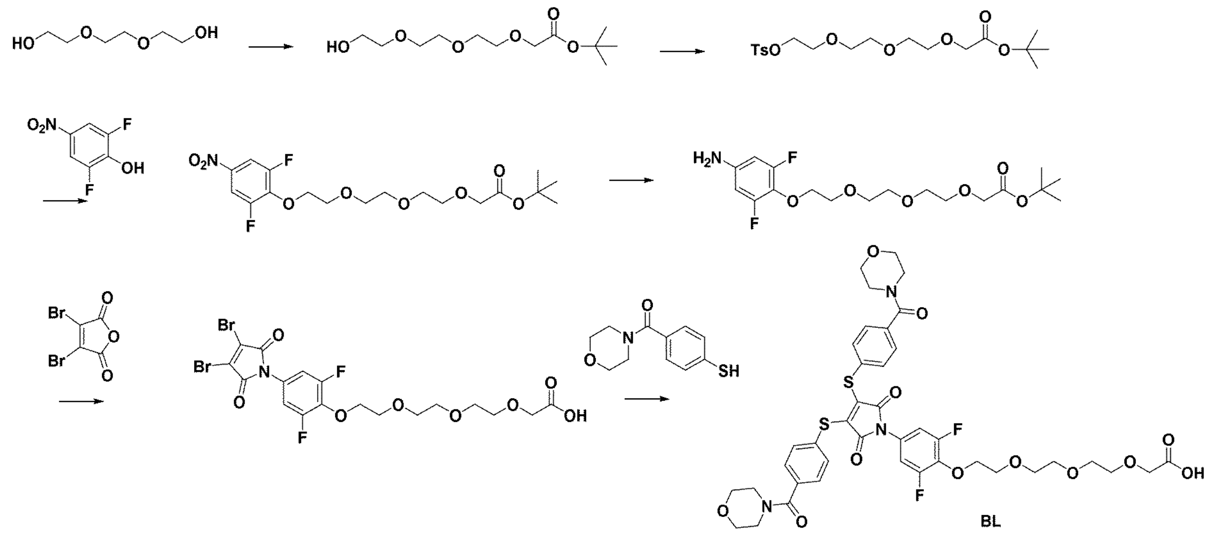
BL

Linker compound BL was obtained as yellow solid. LC-MS (ESI): M+1=858.

Linker compound BL (857 mg, 1 mmol), GGFG-Dxd (840 mg, 1mmol, 1 eq), DIPEA (323 mg, 2.5 mmol, 2.5 eq), and HATU (570 mg, 1.5 mmol, 1.5 eq) were dissolved in 30 ml of DCM, and stirred and reacted for 2 h. Reaction liquid was cooled to 5-10 °C, in which 1 N hydrochloric acid (20 ml) was added, and then was stirred for 0.5 h. Separated phases were obtained, and aqueous phase was extracted with DCM (30 ml × 2). Organic phases then obtained were pooled, washed with saturated brine, dried over anhydrous sodium sulfate, suctioned, and spun to dryness. Residue obtained was purified by column chromatography using a DCM/MeOH (50:1-10:1) mixture as an eluent, and 500 mg of Drug-containing linker MWD-L1 was obtained as yellow solid with a yield of 29.8%. LC-MS (ESI): M+1 = 1681, (M+1)/2=840.9.

__Example 2.2__ Synthesis of Drug-containing linker MWC-L2

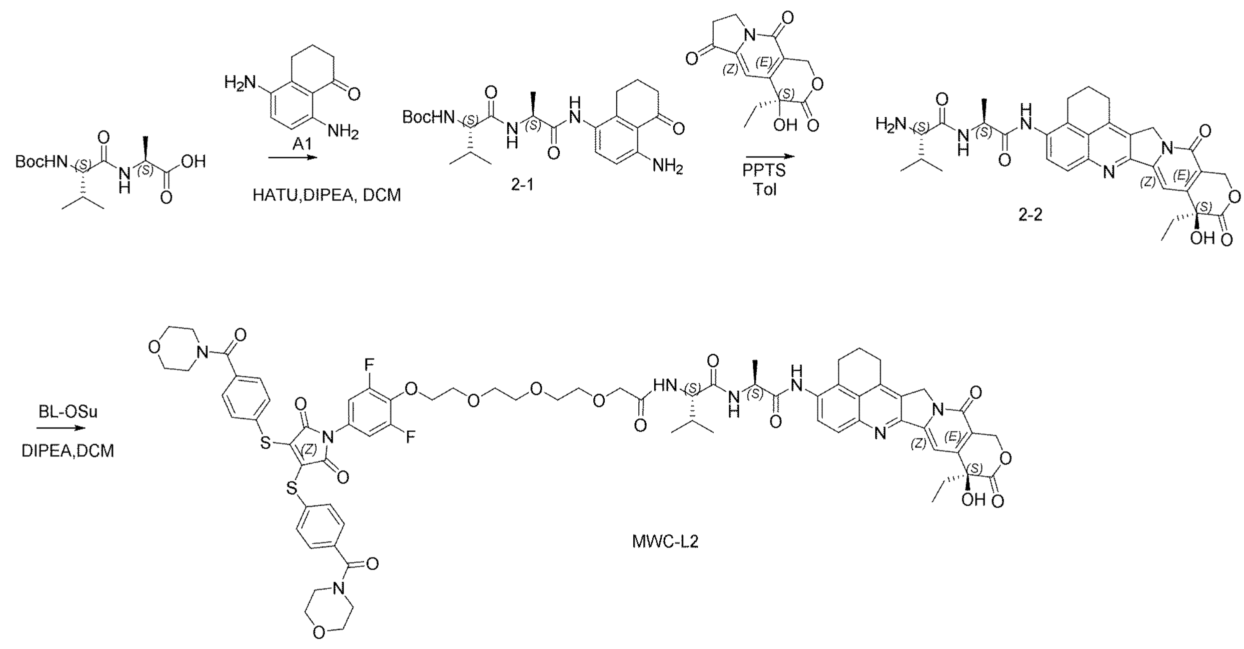


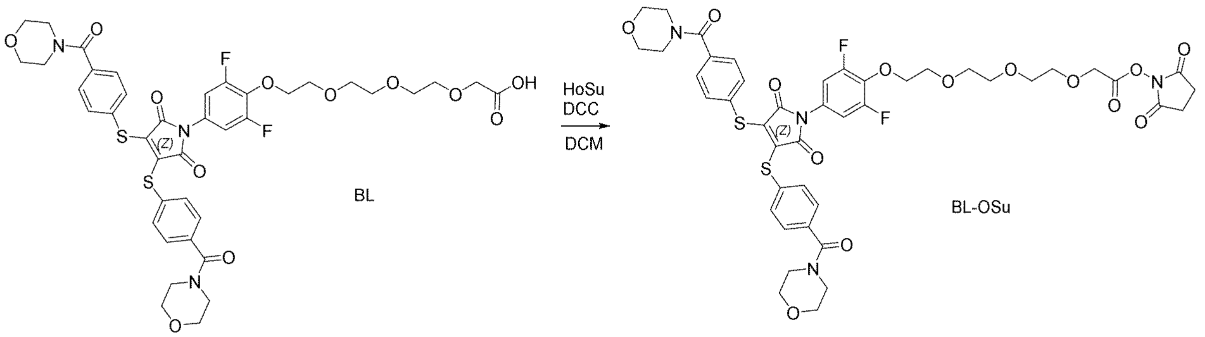


Step 1:

Boc-Val-Ala-OH (288 mg, 1 mmol), Compound A1 (176 mg, 1 mmol, 1 eq), DIPEA (322 mg, 2.5 mmol, 2.5 eq), and HATU (456 mg, 1.2 mmol, 1.2 eq) were dissolved in 30 ml of DCM, and stirred and reacted for 2 h. Reaction liquid was spun to dryness, and residue obtained was purified by column chromatography using a PE/EA (10:1-5:1) mixture as an eluent, and quantitatively 450 mg of the intermediate 2-1 was obtained as gray green solid.

Step 2:

The intermediate 2-1 (450 mg, 1 mmol), tricyclic compound CDE (263 mg, 1 mmol, 1 eq), and pyridinium p-toluenesulfonate (PPTS) (251 mg, 1 mmol, 1 eq) were suspended in 30 ml of toluene, and heated under reflux and reacted for 2 hours. The heating was stopped, and reaction liquid was allowed to cool and solid precipitates were collected, and 750 mg of the intermediate 2-2 crude product was obtained as brown solid. LC-MS (ESI): M+1 =574. The crude product was used directly in the next step without purification.

Step 3:

Linker compound BL (857 mg, 1 mmol), HoSu (138 mg, 1.2 mmol, 1.2 eq), and DCC (310 mg, 1.5 mmol, 1.5 eq) were dissolved in 30 ml of DCM, and stirred at room temperature for 3 h. Reaction liquid was suctioned, and filtrate obtained was a solution of A-Osu in DCM. The filtrate was added to a mixed solution of the crude intermediate 2-2, DIPEA (323 mg, 2.5 mmol, 2.5 eq), and DCM (30 ml), which was then stirred and reacted for 3 h. Then reaction liquid was cooled to below 10 °C and 1 N hydrochloric acid (20 ml) was added thereto, followed by stirring for 0.5 h. Separated

phases were obtained, and aqueous phase was extracted with DCM (30 ml × 2). Organic phases then obtained were pooled, washed with saturated brine, dried over anhydrous sodium sulfate, suctioned, and spun to dryness. Residue obtained was purified by column chromatography using a DCM/MeOH (50:1-10:1) mixture as an eluent, and 325 mg of Drug-containing linker MWC-L2 was obtained as orange solid with a yield of 23.0%. LC-MS (ESI): M+1 = 1414, (M+1)/2=707.

**Example 2.3** Synthesis of Drug-containing linker MWC-L3

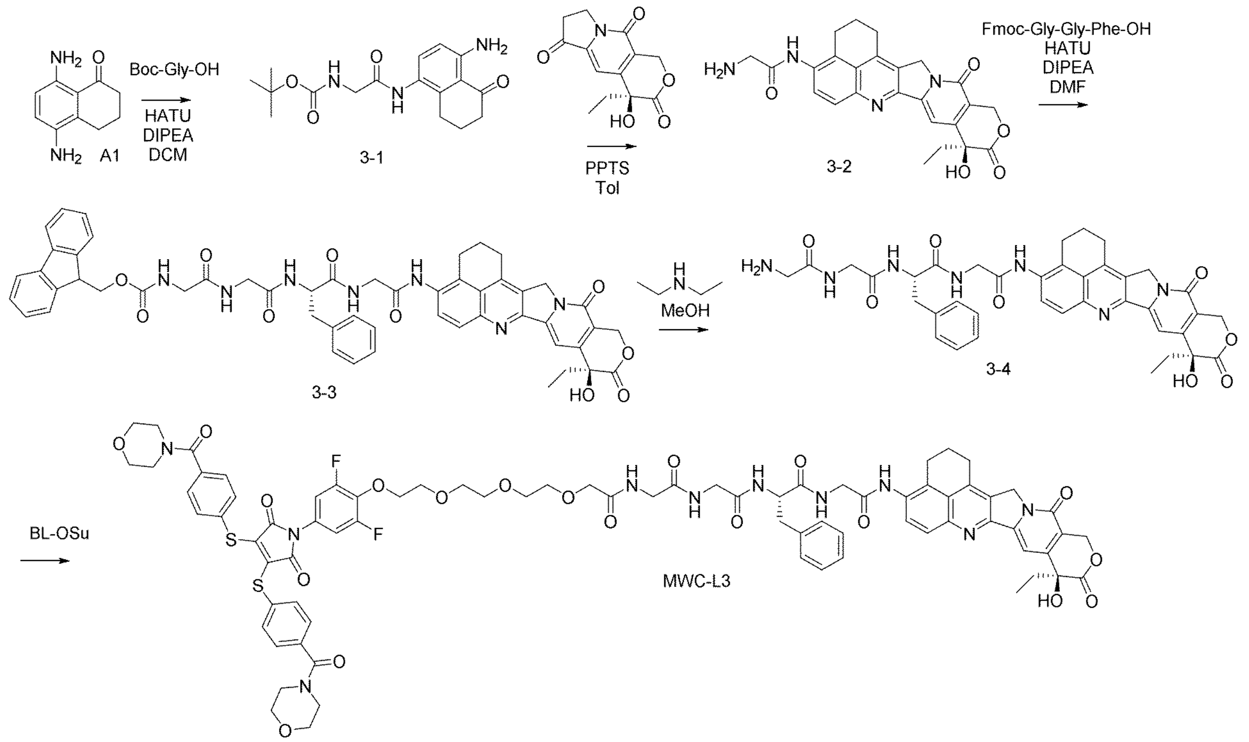


Step 1:

Boc-Gly-OH (175 mg, 1 mmol), Compound A1 (176 mg, 1 mmol, 1 eq), DIPEA (322 mg, 2.5 mmol, 2.5 eq), and HATU (456 mg, 1.2 mmol, 1.2 eq) were dissolved in 30 ml of DCM, and stirred and reacted for 2 h. Reaction liquid was spun to dryness, and residue obtained was purified by column chromatography using a PE/EA (10:1-5:1) mixture as an eluent, and quantitatively 335 mg of the intermediate 3-1 was obtained as light green solid.

Step 2:

The intermediate 3-1 (335 mg, 1 mmol), tricyclic compound CDE (263 mg, 1 mmol, 1 eq), and PPTS (251 mg, 1 mmol, 1 eq) were suspended in 30 ml of toluene, and heated under reflux and reacted for 2 hours. The heating was stopped, and reaction liquid was allowed to cool and solid precipitates were collected, and 560 mg of the intermediate 3-2 crude product was obtained as brown solid. LC-MS (ESI): M+1= 461. The crude product was used directly in the next step without purification.

Step 3:

Fmoc-Gly-Gly-Phe-OH (501 mg, 1 mmol), the crude intermediate 3-2 (560 mg), DIPEA (322 mg, 2.5 mmol, 2.5 eq), and HATU (456 mg, 1.2 mmol, 1.2 eq) were dissolved in 20 ml of DCM, and stirred and reacted for 3 h. 1 N hydrochloric acid (30 ml) was added thereto, followed by stirring for 0.5 h. Reaction liquid was extracted with DCM (30 ml × 2). Organic phases then obtained were pooled, washed with saturated brine, dried over anhydrous sodium sulfate, suctioned, and spun to dryness. Residue obtained was purified by column chromatography using a DCM/MeOH (50:1-10:1) mixture as an eluent, and 350 mg of the intermediate 3-3 was obtained as brown solid. LC-MS (ESI): M+1=945.

Step 4:

The intermediate 3-3 (350 mg, 0.37 mmol) was dissolved in methanol (20 ml), into which diethylamine (2 ml) was then added, and mixture obtained was stirred and reacted for 3 hours. Reaction liquid was spun to dryness, and the intermediate 3-4 crude product was obtained as brown sticky matter. LC-M (ESI): M+1=722. The crude product was used directly in the next step without purification.

Step 5:

Linker compound BL (387 mg, 0.37 mmol), HoSu (51 mg, 0.44 mmol, 1.2 eq), and DCC (114 mg, 0.56 mmol, 1.5 eq) were dissolved in 30 ml of DCM, and stirred at room temperature for 3 h. Reaction liquid was suctioned, and filtrate obtained was added to a mixed solution of the crude intermediate 3-4, DIPEA (120 mg, 0.93mmol, 2.5 eq), and DCM (30 ml), which was then stirred and reacted for 3 h. Then reaction liquid was cooled to below 10 °C and 1 N hydrochloric acid (20 ml) was added thereto, followed by stirring for 0.5 h. Separated phases were obtained, and aqueous phase was extracted with DCM (30 ml × 2). Organic phases then obtained were pooled, washed with saturated brine, dried over anhydrous sodium sulfate, suctioned, and spun to dryness. Residue obtained was purified by column chromatography using a DCM/MeOH (50:1-10:1) mixture as an eluent, and 120 mg of Drug-containing linker MWC-L3 was obtained as orange solid with a yield of 20.8%. LC-MS (ESI): M+1 = 1562, (M+1)/2=781.

**Example 2.4** Synthesis of Drug-containing linker MWE-L4

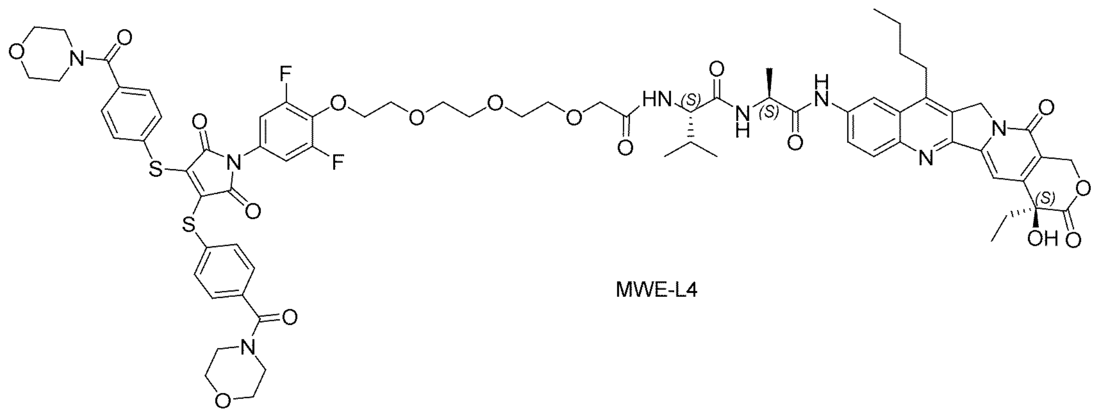

MWE-L4

According to the method described in Example 2.2, in which Compound A3 was used instead of Compound A1, MWE-L4 was synthesized and obtained as orange solid. LC-MS (ESI): M+1 =1430.

**Example 2.5** Synthesis of Drug-containing linker MWG-L5

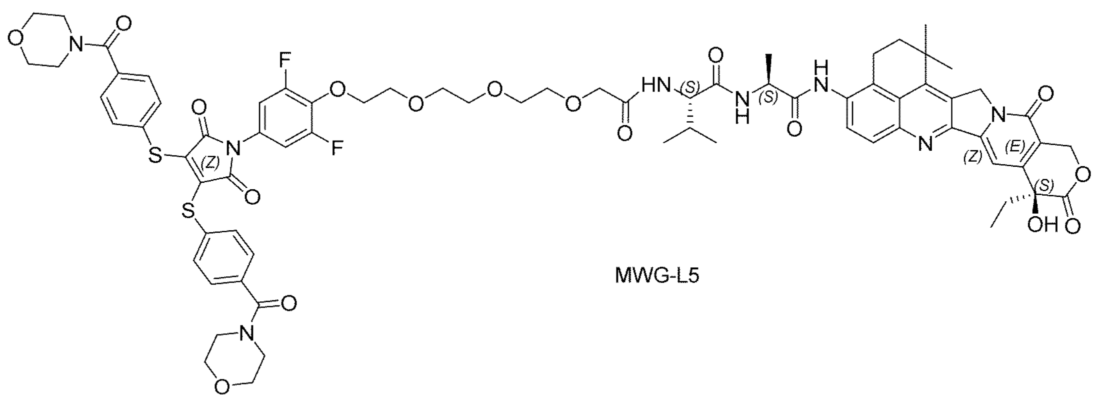

MWG-L5

According to the method described in Example 2.2, in which Compound A6 was used instead of Compound A1, MWG-L5 was synthesized and obtained as orange solid. LC-MS (ESI): M+1 =1442.

**Example 2.6** Synthesis of Drug-containing linker MWF-L6

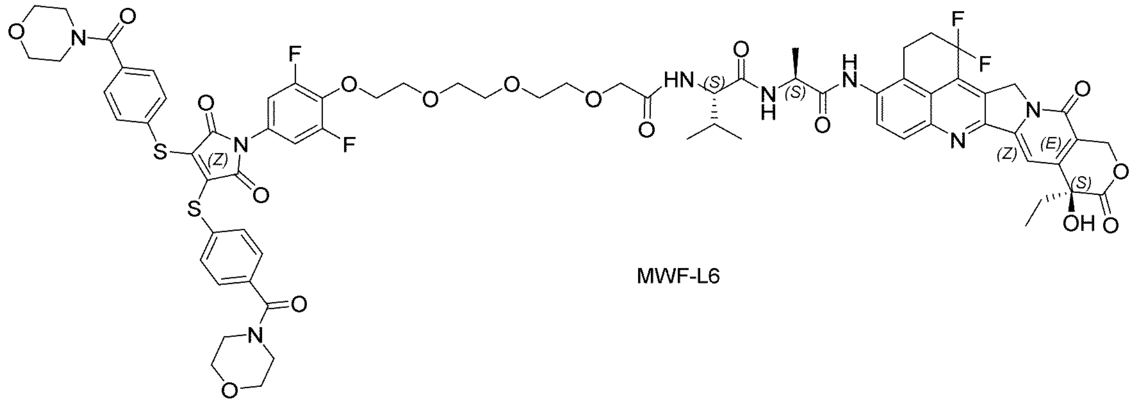

MWF-L6

According to the method described in Example 2.2, in which Compound A7 was used instead of Compound A1, MWF-L6 was synthesized and obtained as orange solid. LC-MS (ESI): M+1 =1450.

**Example 2.7** Synthesis of Drug-containing linker MWF-L7

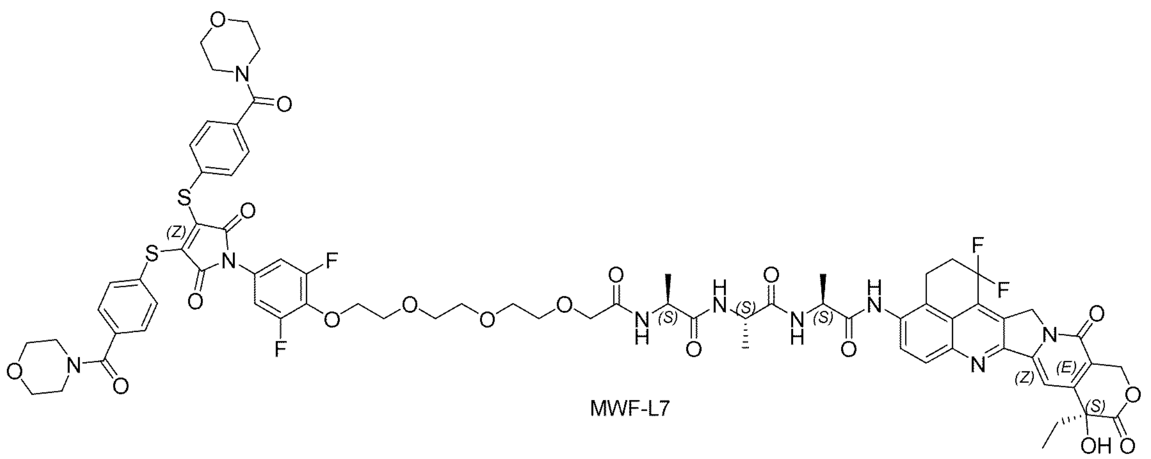

MWF-L7

According to the method described in Example 2.2, in which Compound A7 was used instead of Compound A1 and Alloc-Ala-Ala-Ala-OH was used instead of Alloc-Val-Ala-OH, MWF-L7 was synthesized and obtained as orange solid. LC-MS (ESI): M+1=1493.5.

**Example 2.8** Synthesis of Drug-containing linker MWF-L8

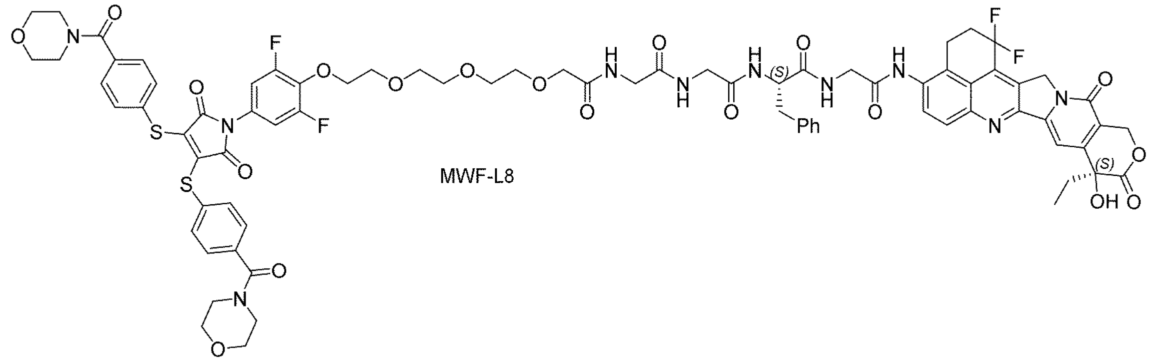

MWF-L8

According to the method described in Example 2.3, in which Compound A7 was used instead of Compound A1, MWF-L8 was synthesized and obtained as orange solid. LC-MS (ESI): M+1 =1598.4.

**Example 2.9** Synthesis of Drug-containing linker MWF-L9

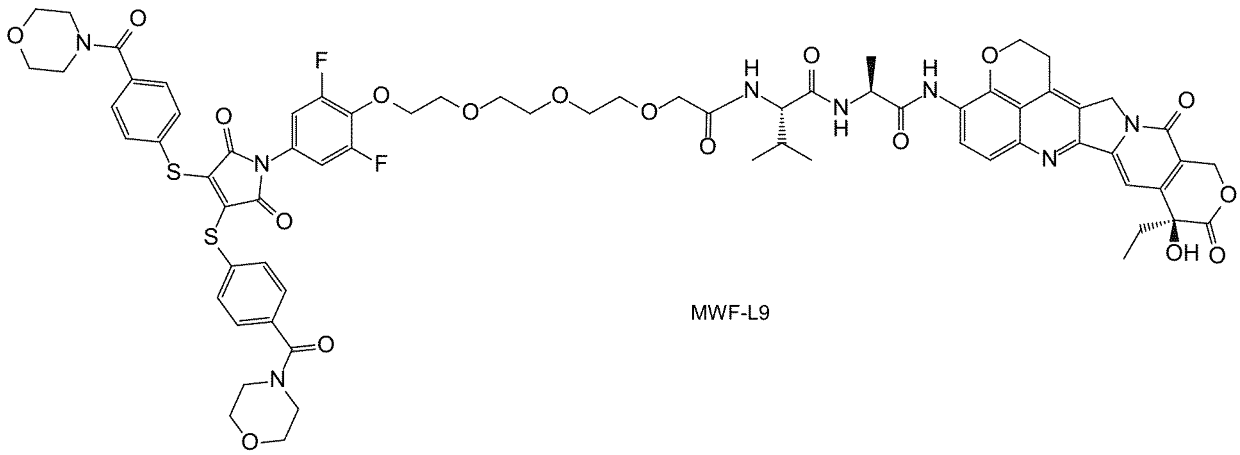

MWF-L9

According to the method described in Example 2.2, in which Compound A5 was used instead of Compound A1, MWF-L9 was synthesized and obtained as yellow solid. LC-MS (ESI): M+1 =1416.4

**Example 2.10** Synthesis of Drug-containing linker MWF-L10

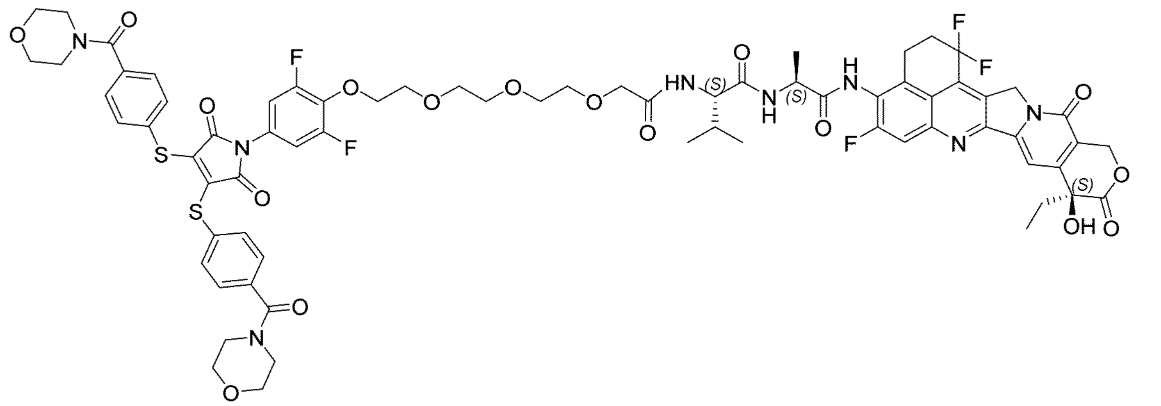

MWF-L10

According to the method described in Example 2.2, in which Compound A13 was used instead of Compound A1, MWF-L10 was synthesized and obtained as yellow solid. LC-MS (ESI): M+1 =1468.4.

**Example 2.11** Synthesis of Drug-containing linker MWF-L11

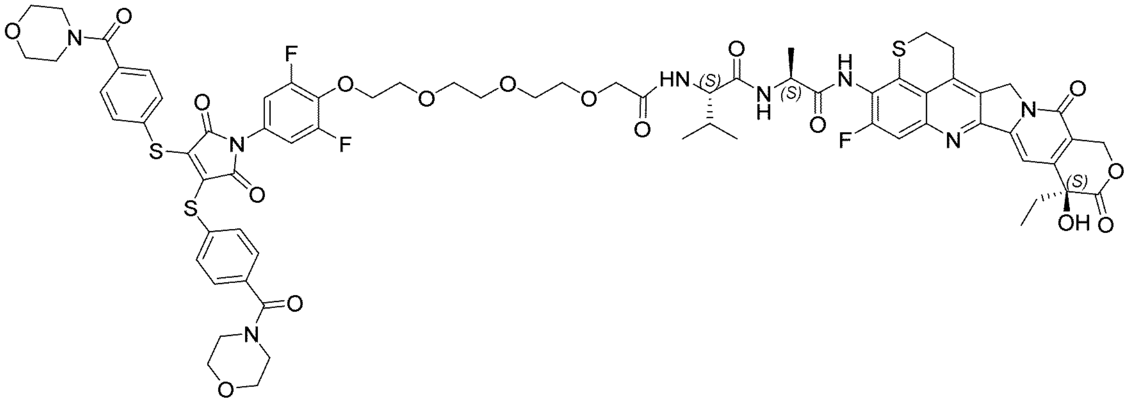

MWF-L11

According to the method described in Example 2.2, in which Compound A18 was used instead of Compound A1, MWF-L11 was synthesized and obtained as yellow solid. LC-MS (ESI): M+1 =1450.4.

**Example 2.12** Synthesis of Drug-containing linker MWF-L12

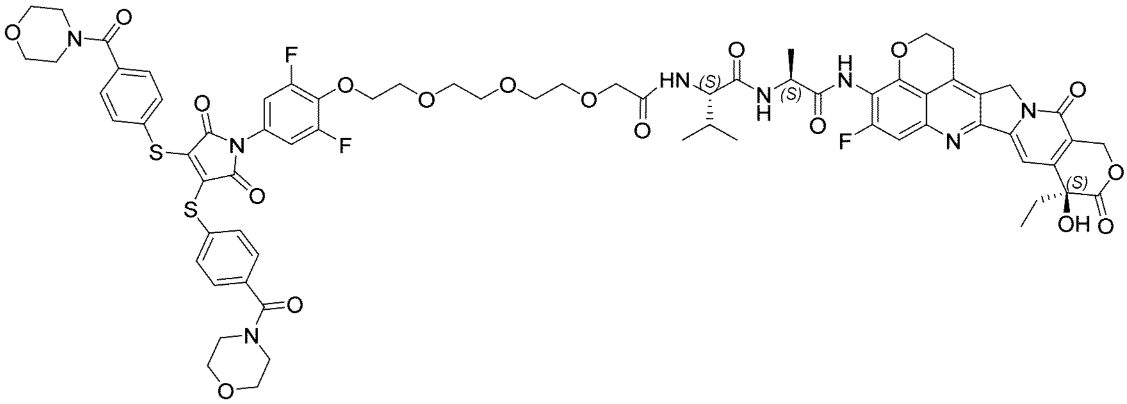

MWF-L12

According to the method described in Example 2.2, in which Compound A16 was used instead of Compound A1, MWF-L12 was synthesized and obtained as yellow solid. LC-MS (ESI): M+1 =1434.4.

**Example 2.13** Synthesis of Drug-containing linker MWF-L13

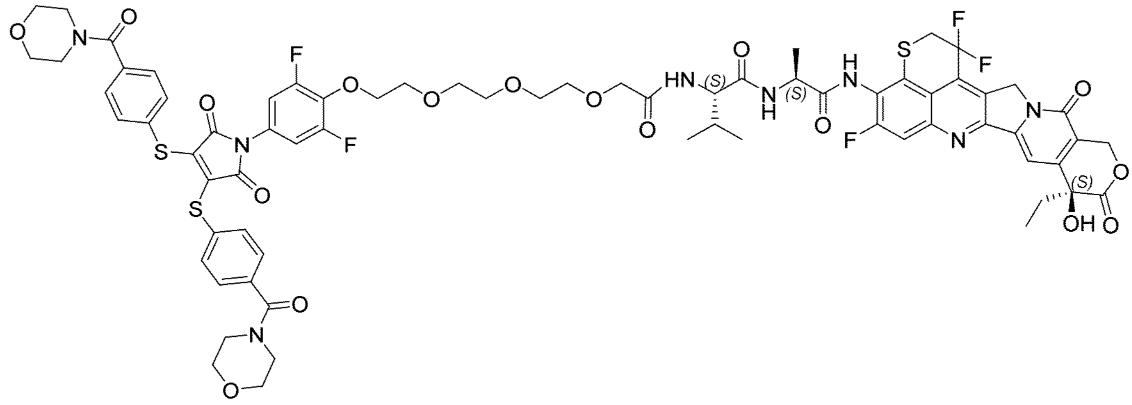

MWF-L13

According to the method described in Example 2.2, in which Compound A19 was used instead of Compound A1, MWF-L13 was synthesized and obtained as yellow solid. LC-MS (ESI): M+1 =1486.4.

**Example 2.14** Synthesis of Drug-containing linker MWF-L14

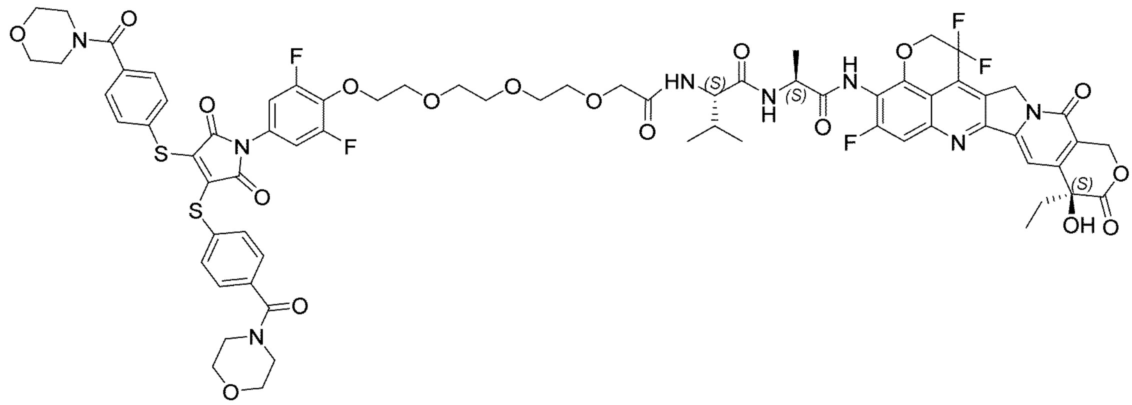

MWF-L14

According to the method described in Example 2.2, in which Compound A17 was used instead of Compound A1, MWF-L14 was synthesized and obtained as yellow solid. LC-MS (ESI): M+1 =1470.4.

**Example 2.15** Synthesis of Drug-containing linker MWF-L15

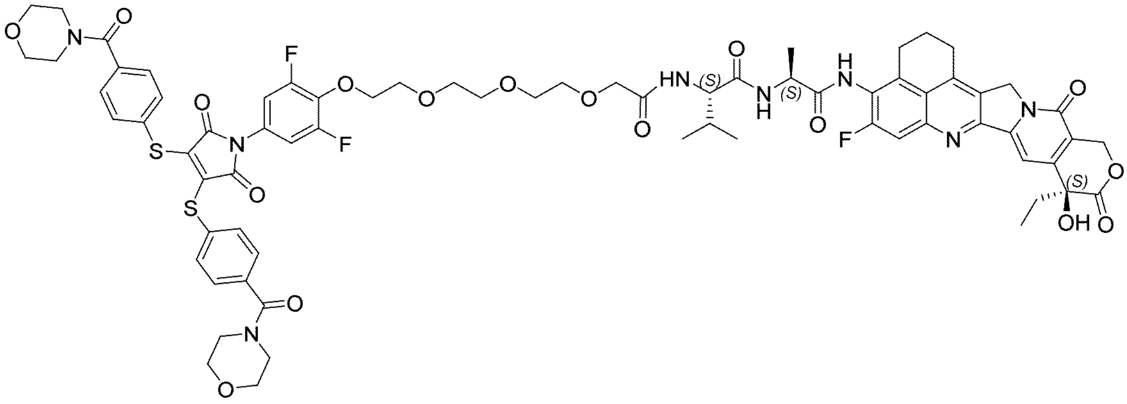

MWF-L15

According to the method described in Example 2.2, in which Compound A2 was used instead of Compound A1, MWF-L15 was synthesized and obtained as yellow solid. LC-MS (ESI): M+1 = 1432.4.

**Example 2.16** Synthesis of Drug-containing linker MWD-L7

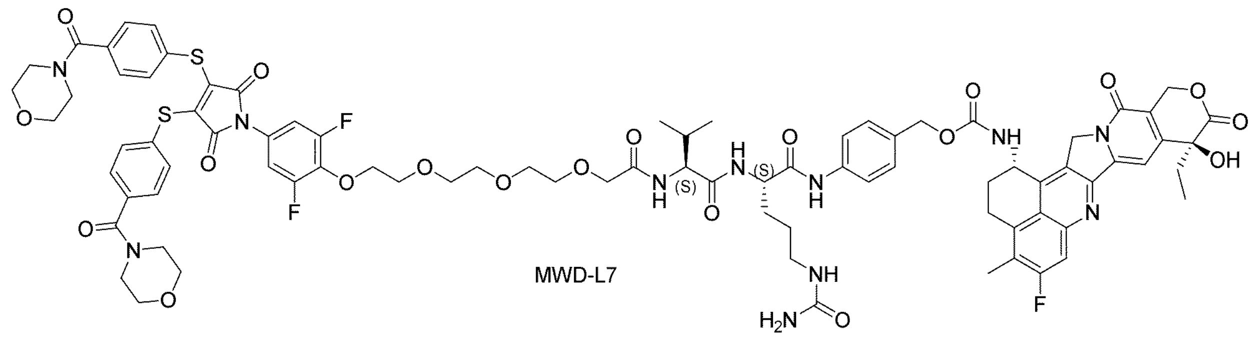

MWD-L7

Synthetic route is as follows:

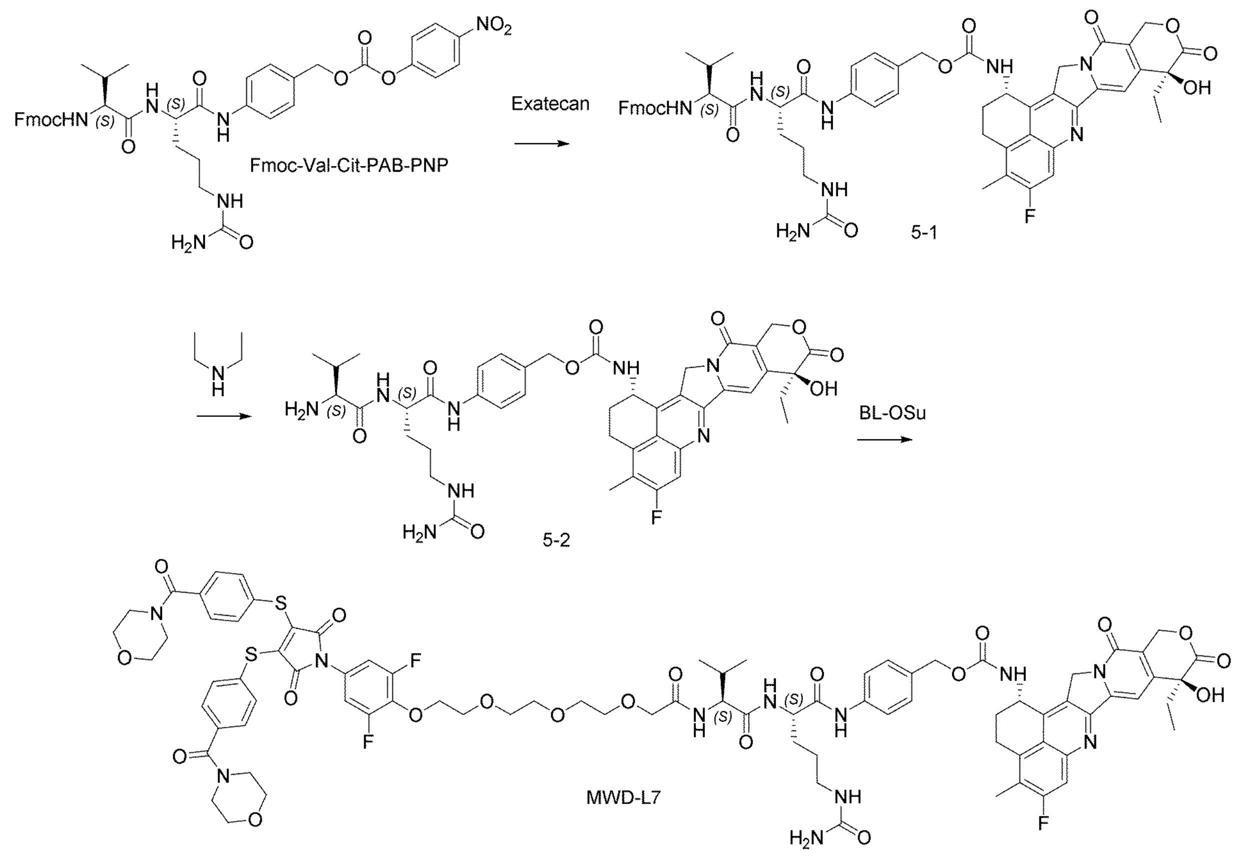


Fmoc-Val-Cit-PAB-PNP

5-1

5-2

MWD-L7

Step 1:

Fmoc-Val-Cit-PAB-PNP (153 mg, 0.2 mmol), Exatecan mesylate (106 mg, 0.2 mmol, 1 eq), and DIPEA (65 mg, 0.5mmol, 2.5 eq) were dissolved in 30 ml of DCM, and stirred and reacted for 2 h. Reaction liquid was spun to dryness, and residue obtained was purified by column chromatography using a DCM/MeOH (50:1-10:1) mixture as an eluent, and 180 mg of the intermediate 5-1 was obtained as yellow solid. LC-MS (ESI): M+1 =1064.

Step 2:

The intermediate 5-1 (180 mg, 0.17 mmol) was dissolved in methanol (20 ml), into which diethylamine (2 ml) was then added, and mixture obtained was stirred and reacted for 3 h. Reaction liquid was spun to dryness, and the intermediate 5-2 crude product was obtained as light yellow sticky matter. LC-M (ESI): M+1 =842. The crude product was used directly in the next step without purification.

Step 3:

Linker compound BL (146 mg, 0.17 mmol), HoSu (25 mg, 0.21 mmol, 1.2 eq), and DCC (54 mg, 0.26 mmol, 1.5 eq) were dissolved in 20 ml of DCM, and stirred at room temperature for 3 h. Reaction liquid was suctioned, and filtrate obtained was added to a mixed solution of the crude intermediate 5-1, DIPEA (56 mg, 0.43 mmol, 2.5 eq), and DCM (20 ml), which was then stirred and reacted for 3 h. Then reaction liquid was cooled to 5-10 °C and 1 N hydrochloric acid (20 ml) was added thereto, followed by stirring for 0.5 h. Separated phases were obtained, and aqueous phase was extracted with DCM (30 ml × 2). Organic phases then obtained were pooled, washed with saturated brine, dried over anhydrous sodium sulfate, suctioned, and spun to dryness. Residue obtained was purified by column chromatography using a DCM/MeOH (50:1-10:1) mixture as an eluent, and 67 mg of Drug-containing linker MWD-L7 was obtained as yellow solid with a yield of 23.1%. LC-MS (ESI): M+1 =1682, (M+1)/2=841.

<u>**Example 2.17**</u> Synthesis of Drug-containing linker MWD-L8

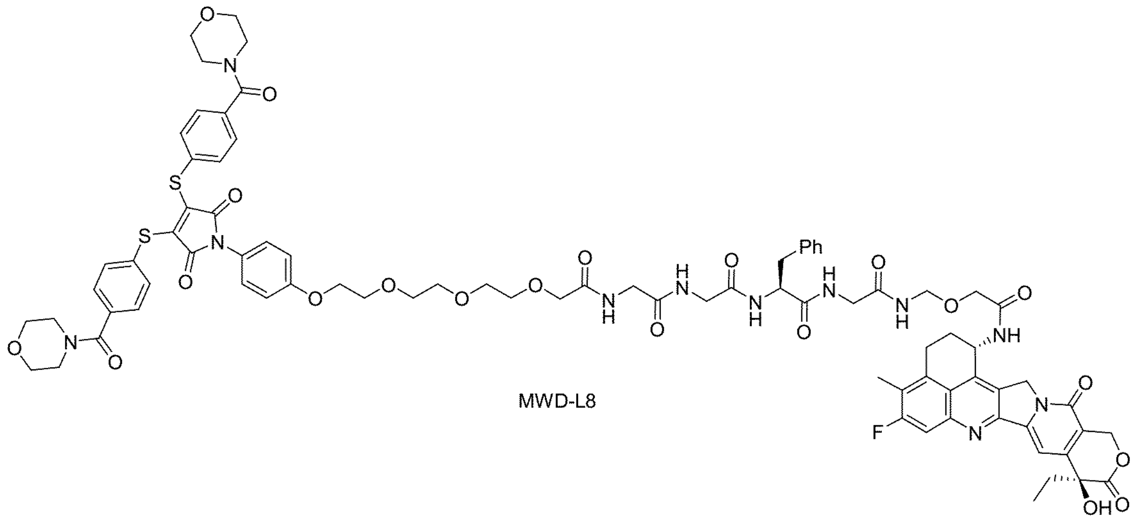

MWD-L8

According to a method similar to that described in Example 2.1, in which

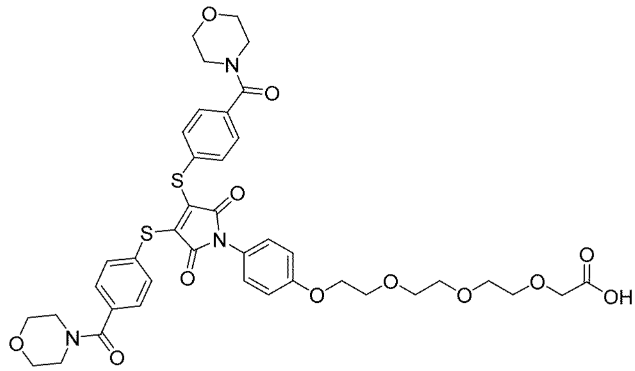

was used instead of Compound BL, MWD-L8 was synthesized and obtained as light yellow solid. LC-MS (ESI): M+1 =1645.7.

**Example 2.18** Synthesis of Drug-containing linker MWD-L9

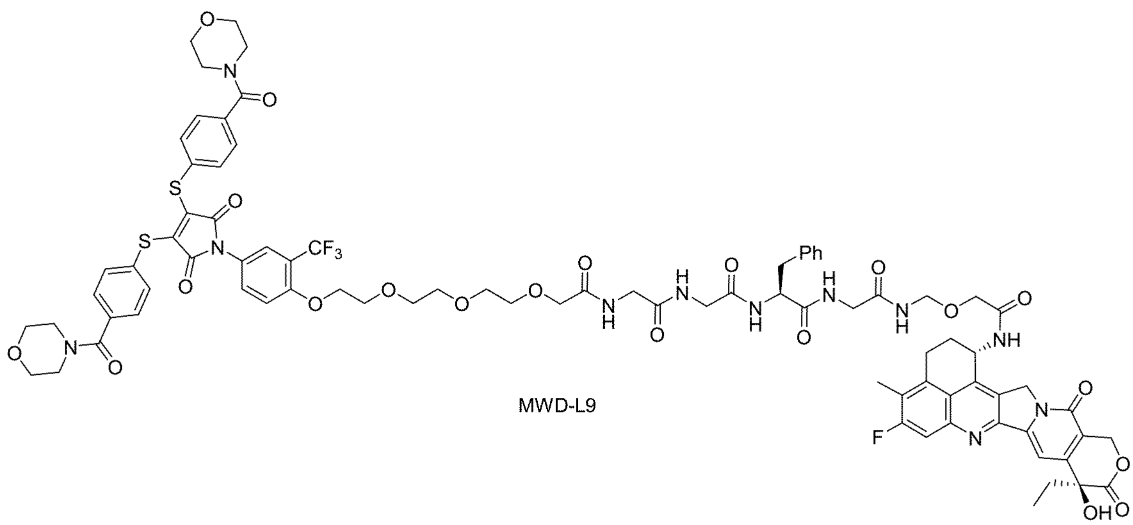

MWD-L9

According to a method similar to that described in Example 2.1, in which

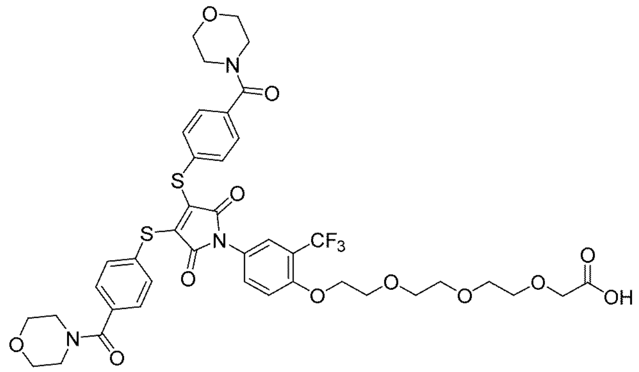

was used instead of Compound BL, MWD-L9 was synthesized and obtained as light yellow solid. LC-MS (ESI): M+1 =1713.7.

**Example 2.19** Synthesis of Drug-containing linker L-D

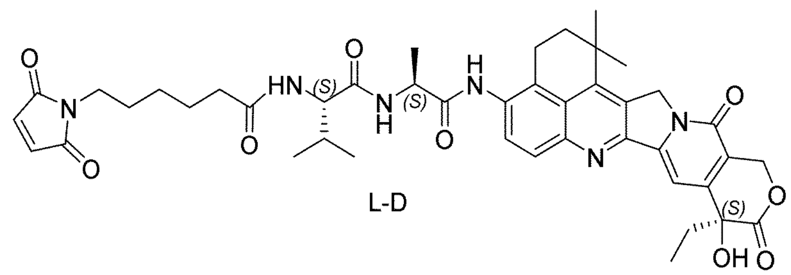

L-D

According to a method similar to that described in Example 2.5, in which MC-OSU was used instead of BL-OSU, L-D was synthesized and obtained as light yellow solid. LC-MS (ESI): M+1 =795.9.

**Example 2.20** Synthesis of Drug-containing linker L-E

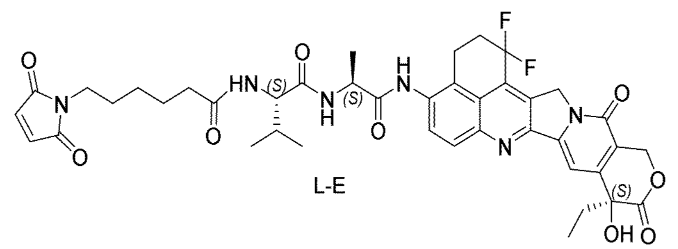

L-E

According to a method similar to that described in Example 2.2, in which MC-OSU was used instead of BL-OSU, L-E was synthesized and obtained as light yellow solid. LC-MS (ESI): M+1 =803.8.

**Example 2.21** Synthesis of Drug-containing linker L-F

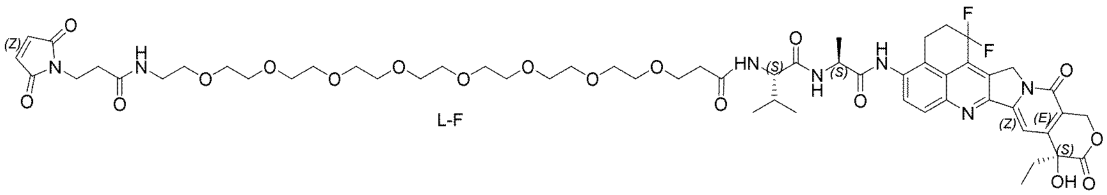

L-F

According to a method similar to that described in Example 2.2, in which A7 was used instead of A1 and MaL-PEG8-COOH was used instead of Compound BL, L-F was prepared and obtained as yellow solid. LC-MS (ESI): M+1= 1185.

**Example 2.22** Synthesis of Drug-containing linker L-G

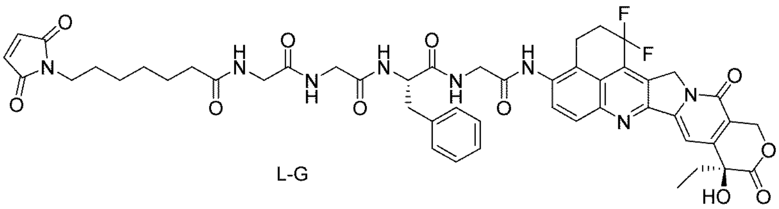

L-G

According to a method similar to that described in Example 2.3, in which compound MC-OSu was used instead of compound BL-OSu, L-G was prepared and obtained as yellow solid. LC-MS (ESI): M+1=965.98.

**Example 2.23** Synthesis of Drug-containing linker L-H

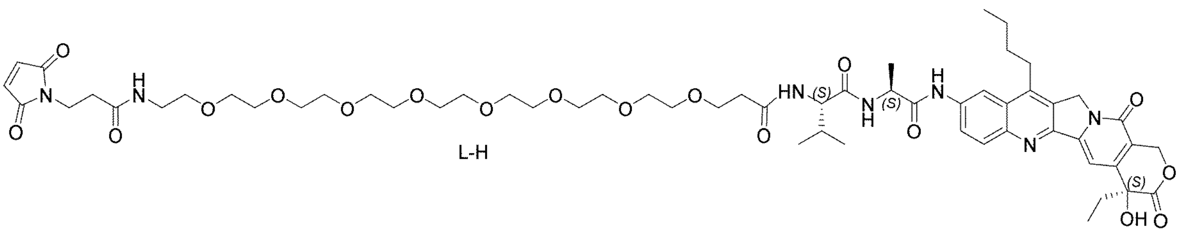

L-H

According to a method similar to that described in Example 2.4, in which MaL-PEG8-COOH was used instead of Compound BL, L-H was prepared and obtained as light yellow solid. LC-MS (ESI): M+1=1165.3.

**Example 2.24** Synthesis of Drug-containing linker L-I

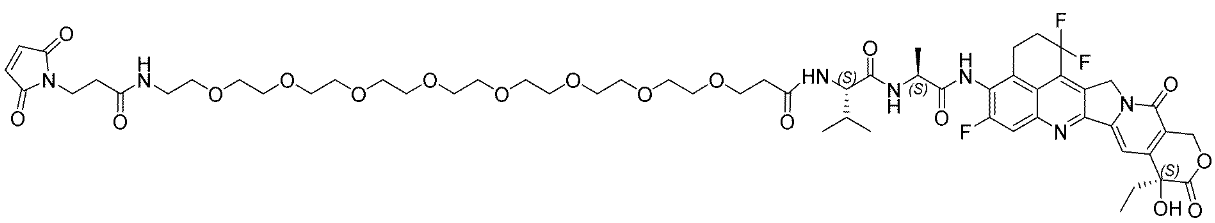

L-I

According to a method similar to that described in Example 2.2, in which A13 was used instead of A1, L-I was

prepared and obtained as yellow solid. LC-MS (ESI): M+1 =1203.4.

**Example 2.25** Synthesis of Drug-containing linker L-J

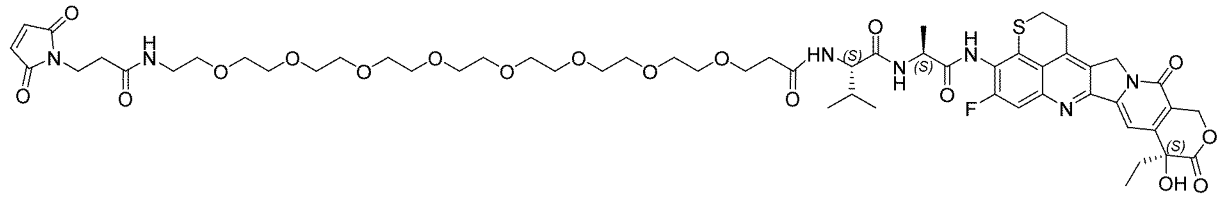

L-J

According to a method similar to that described in Example 2.2, in which A18 was used instead of A1, L-J was prepared and obtained as yellow solid. LC-MS (ESI): M+1 =1185.4.

**Example 2.26** Synthesis of Drug-containing linker L-K

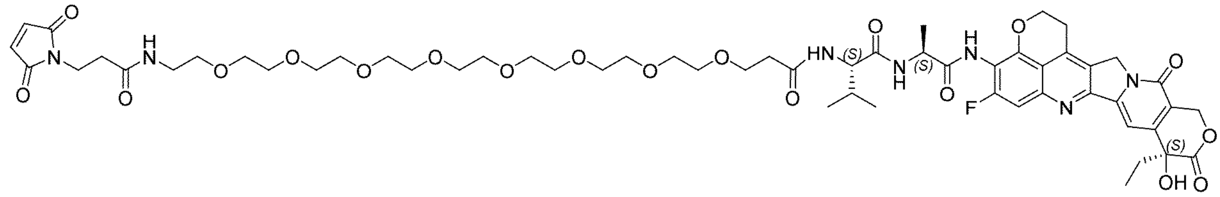

L-K

According to a method similar to that described in Example 2.2, in which A16 was used instead of A1, L-K was prepared and obtained as yellow solid. LC-MS (ESI): M+1 =1169.3.

**Example 2.27** Synthesis of Drug-containing linker L-L

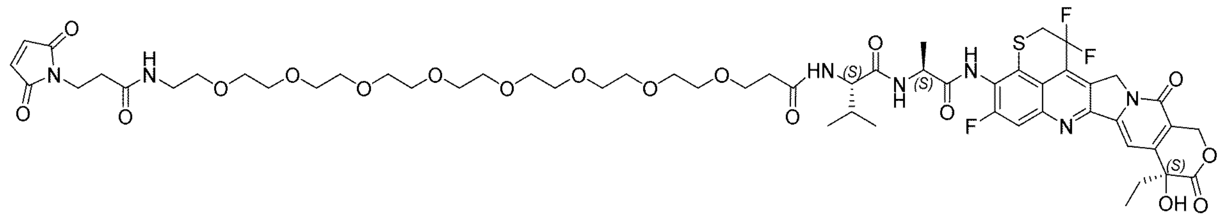

L-L

According to a method similar to that described in Example 2.2, in which A19 was used instead of A1, L-L was prepared and obtained as yellow solid. LC-MS (ESI): M+1 =1221.4.

**Example 2.28** Synthesis of Drug-containing linker L-M

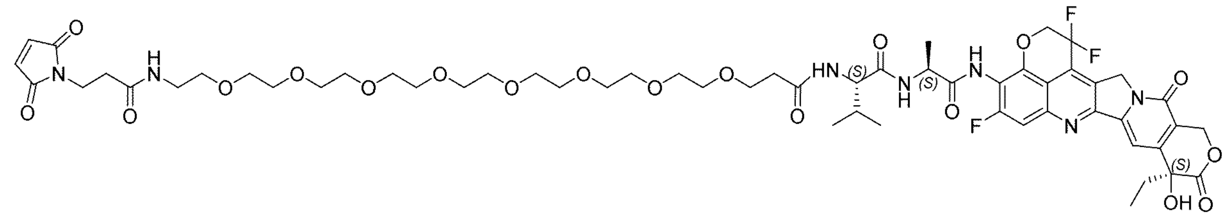

L-M

According to a method similar to that described in Example 2.2, in which A17 was used instead of A1, L-M was prepared and obtained as yellow solid. LC-MS (ESI): M+1 =1205.4.

**Example 2.29** Synthesis of Drug-containing linker L-N

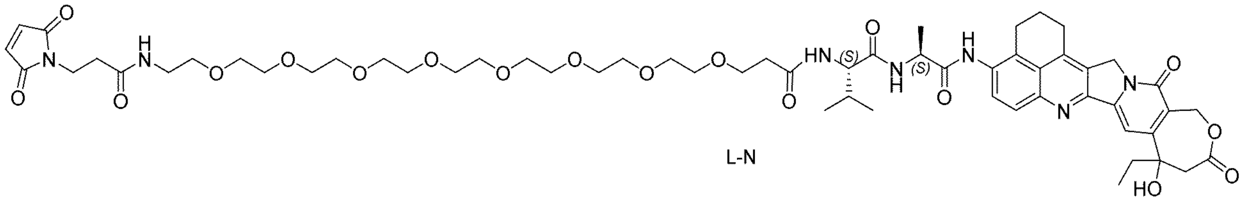

L-N

According to a method similar to that described in Example 2.2, in which the intermediate HCDE was used instead of the intermediate CDE and MaL-PEG8-COOH was used instead of Compound BL, L-N was prepared and obtained as yellow solid. LC-MS (ESI): M+1= 1163.2 (mixture of a pair of diastereoisomers).

**Example 2.30** Synthesis of Drug-containing linker MWS-L1

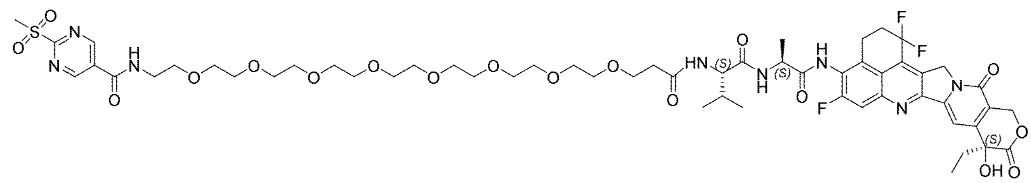

MWS-L1

According to a method similar to that described in Example 2.2, in which A13 was used instead of A1 and

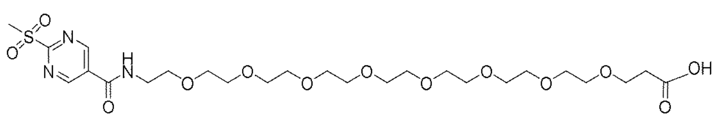

was used instead of Linker compound BL, MWS-L1 was prepared and obtained as yellow solid. LC-MS (ESI): M+1= 1236.3.

**Example 2.31** Synthesis of Drug-containing linker MWS-L2

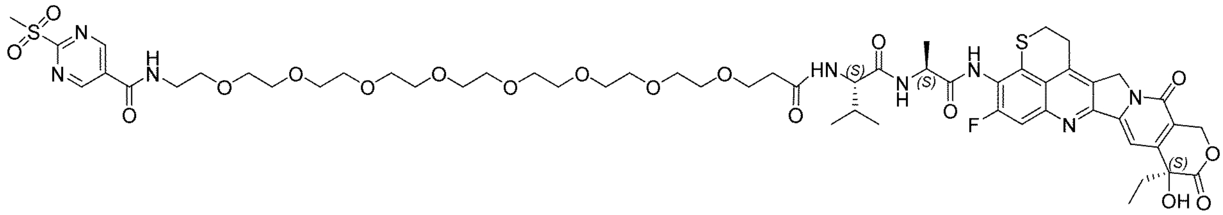

MWS-L2

According to a method similar to that described in Example 2.2, in which A18 was used instead of A1, MWS-L2 was prepared and obtained as yellow solid. LC-MS (ESI): M+1 =1218.4.

**Example 2.32** Synthesis of Drug-containing linker MWS-L3

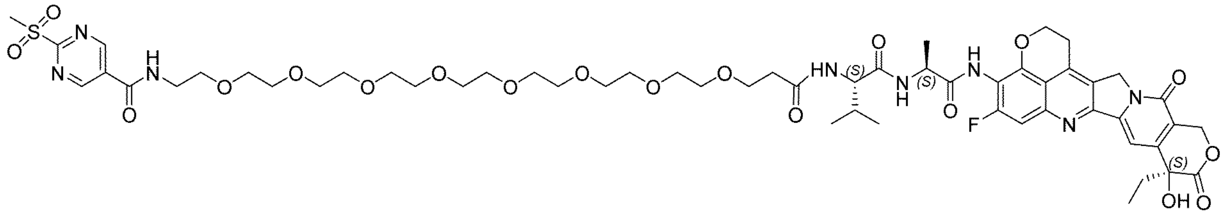

MWS-L3

According to a method similar to that described in Example 2.2, in which A16 was used instead of A1, MWS-L3 was prepared and obtained as yellow solid. LC-MS (ESI): M+1 =1202.4.

**Example 2.33** Synthesis of Drug-containing linker MWS-L4

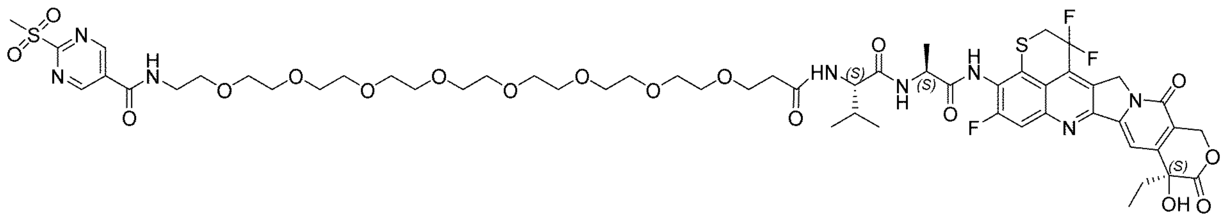

MWS-L4

According to a method similar to that described in Example 2.2, in which A19 was used instead of A1, MWS-L4 was prepared and obtained as yellow solid. LC-MS (ESI): M+1 =1254.4.

**Example 2.34** Synthesis of Drug-containing linker MWS-L5

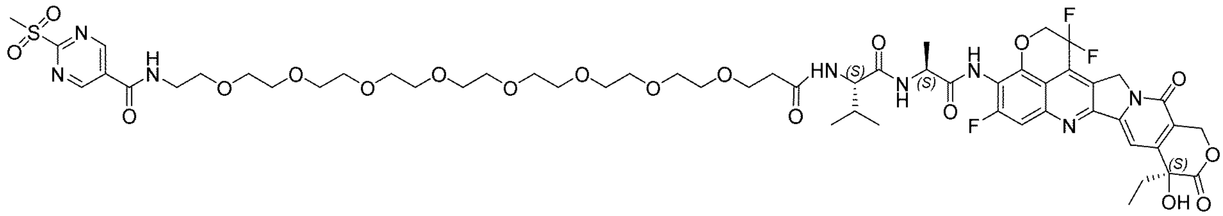

MWS-L5

According to a method similar to that described in Example 2.2, in which A17 was used instead of A1, MWS-L5 was prepared and obtained as yellow solid. LC-MS (ESI): M+1 =1238.4.

**Example 2.35** Synthesis of Drug-containing linker MWS-L6

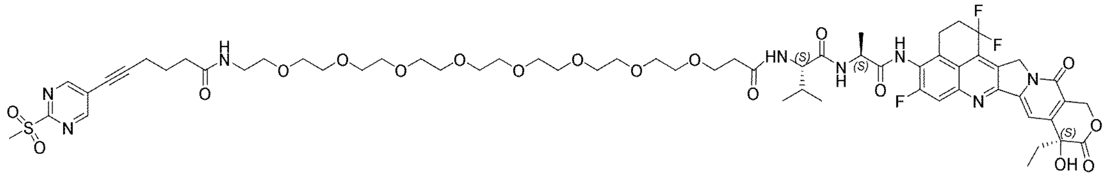

MWS-L6

According to a method similar to that described in Example 2.11, in which

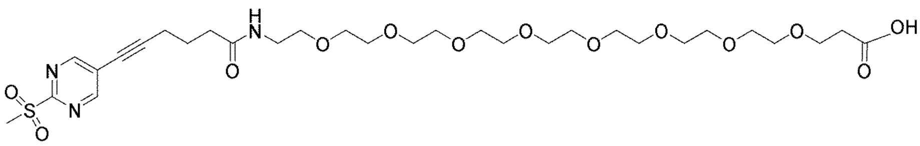

was used instead of Mal-PEG8-COOH, MWS-L6 was prepared and obtained as yellow solid. LC-MS (ESI): M+1= 1302.4.

**Example 2.36** Synthesis of Drug-containing linker MWS-L7

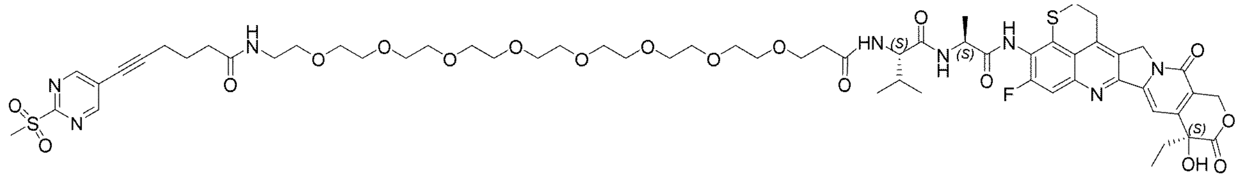

MWS-L7

According to a method similar to that described in Example 2.2, in which A18 was used instead of A1, MWS-L7 was prepared and obtained as yellow solid. LC-MS (ESI): M+1 =1284.4.

**Example 2.37** Synthesis of Drug-containing linker MWS-L8

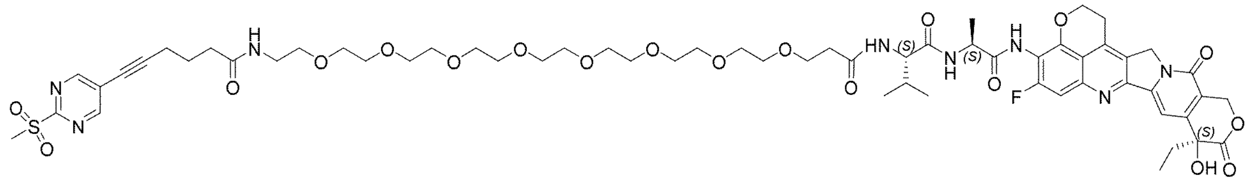

MWS-L8

According to a method similar to that described in Example 2.2, in which A16 was used instead of A1, MWS-L8 was prepared and obtained as yellow solid. LC-MS (ESI): M+1 =1268.4.

**Example 2.38** Synthesis of Drug-containing linker MWS-L9

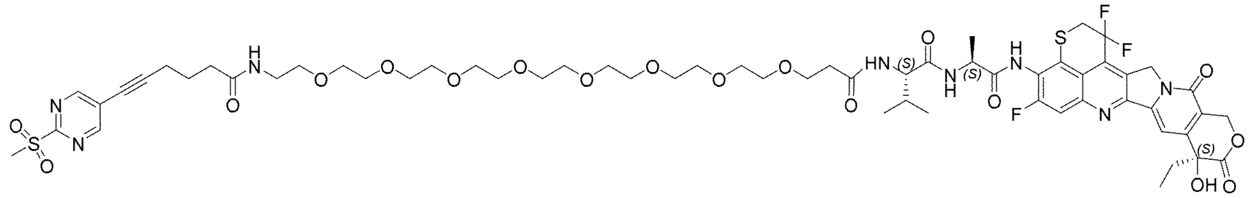

MWS-L9

According to a method similar to that described in Example 2.2, in which A19 was used instead of A1, MWS-L9 was prepared and obtained as yellow solid. LC-MS (ESI): M+1 =1320.4.

**Example 2.39** Synthesis of Drug-containing linker MWS-L10

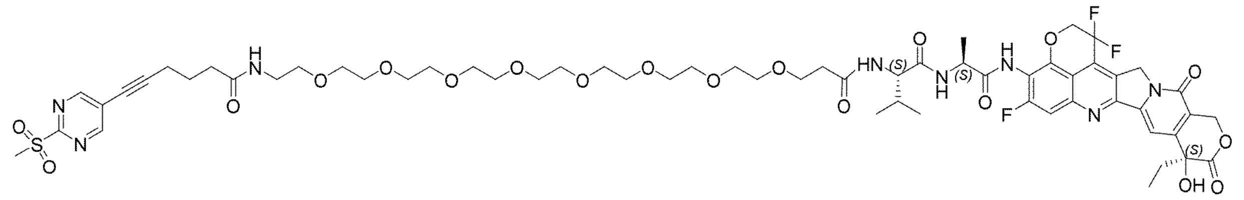

MWS-L10

According to a method similar to that described in Example 2.2, in which A17 was used instead of A1, MWS-L10 was prepared and obtained as yellow solid. LC-MS (ESI): M+1 =1304.4.

**Group 3 of Examples Synthesis of comparative Drug-containing linkers**

**Example 3.1** Synthesis of Drug-containing linkers L-A, L-B, and L-C

3.1.1 Synthesis of Drug-containing linkers for stochastic conjugating

Drug-containing linker MC-GGFG-Dxd was synthesized according the method described in the patent publication US20190151328A1, and MC-GGFG-Dxd was obtained as light yellow solid. LC-MS (ESI): M+1 =1034, $^1$H NMR ($CDCl_3$).

Drug-containing linkers L-A and L-B were synthesized according the method described in the patent publication WO2020200880, and L-A was obtained as yellow solid, LC-MS (ESI): M+1 =767; and L-B was obtained as brown sticky

matter, LC-MS (ESI): M+1 =1149.

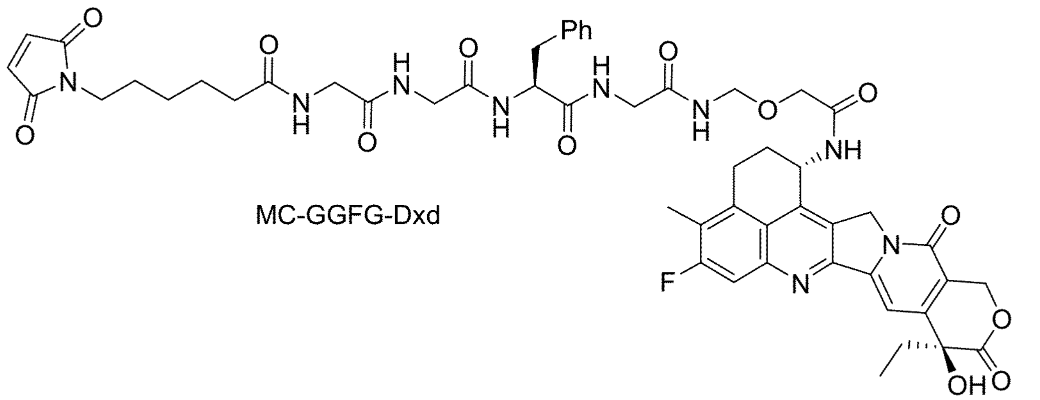

MC-GGFG-Dxd

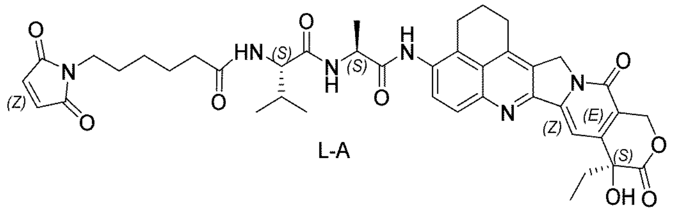

L-A

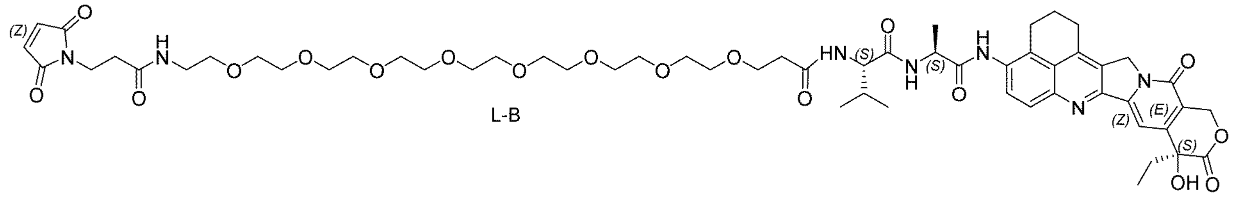

L-B

3.2.2 Synthesis of other Drug-containing linker L-C for ring bridging

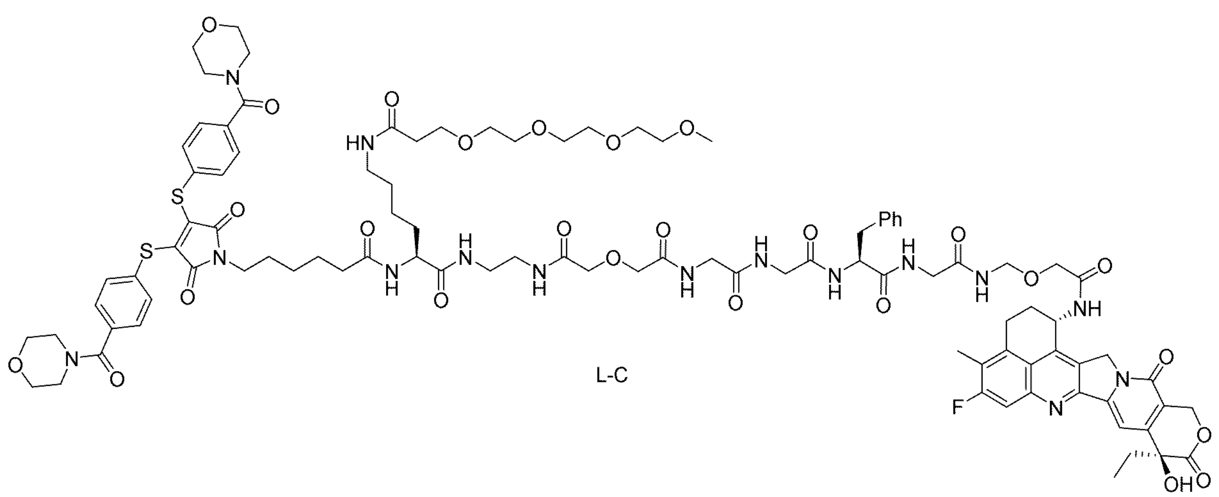

L-C

Synthetic route is as follows:

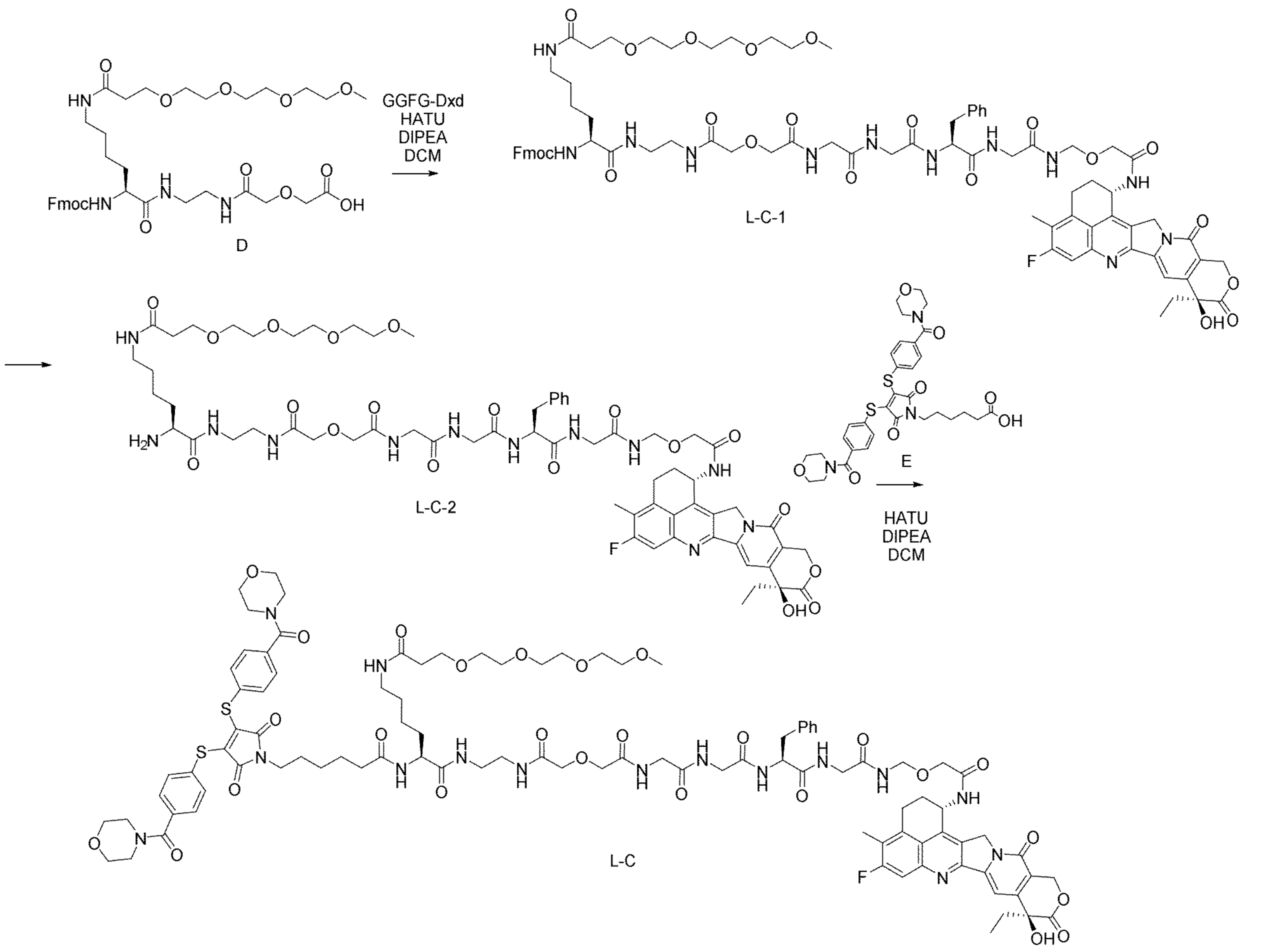


D

L-C-1

L-C-2

L-C

According to a method similar to that for Drug-containing linker L-1, linker L-C was synthesized as light yellow solid. LC-MS (ESI): M+1 =1982, (M+1)/2=991.

## <u>Group 4 of Examples</u> Preparation and physicochemical characterization of antibody-drug conjugates

Processes for preparing antibody-drug conjugates are as follows.

a. Site-specific conjugation process

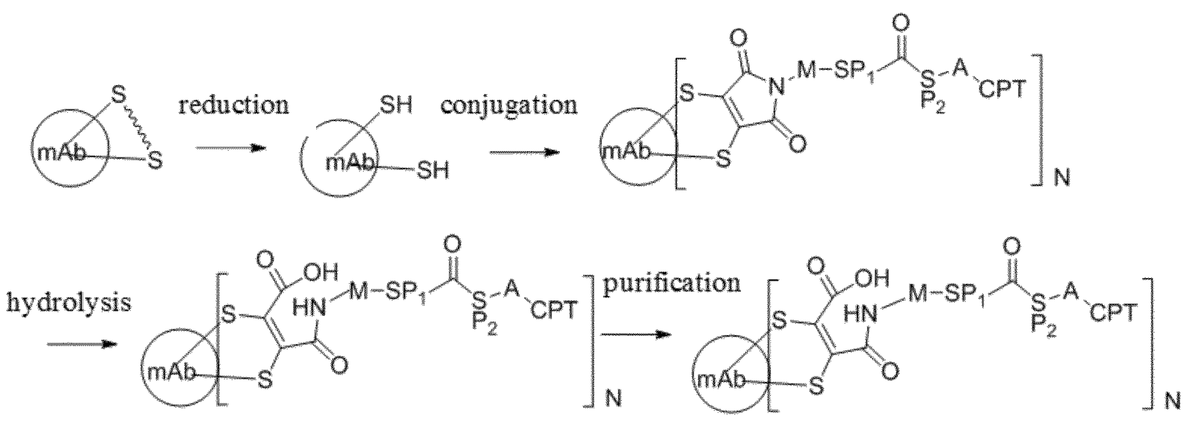


N=1~5

An antibody is reduced to break disulfide bonds, and the reduced antibody is conjugated with a linker to form a bridged antibody-drug conjugate, in which the maleimide ring is then opened via hydrolysis, and the obtained product is purified to provide an antibody-drug conjugate with DAR of 4.

b. Stochastic conjugation process

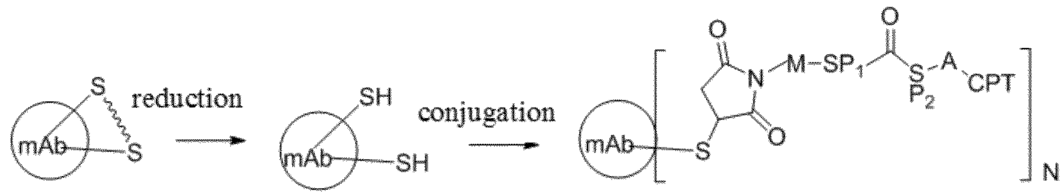


N=1~10

An antibody is reduced to break disulfide bonds, and then is conjugated with a linker to form a bridged antibody-drug conjugate.
Exemplary experiment methods used are as follows.

**4.1 Universal methods for preparing antibody-drug conjugates**

**a.** Universal preparation method for site-specific conjugation

Antibody reduction: A sample containing 120 mg of an antibody is displaced into a buffer comprising 50 mM sodium chloride and 50 mM sodium dihydrogen phosphate-disodium hydrogen phosphate, pH 7.0, with a NAP-25 chromatographic column packed with Sephadex G-25, in which buffer the antibody concentration is diluted to 10 mg/mL. 2.1 ml of an aqueous solution of TCEP (Sigma-Aldrich) at 10 mg/mL is added into 10 mL of the diluted antibody sample (100 mg in total) at an equivalent molar ratio of 1: 10 (antibody: TCEP). After incubation for 2 hours, the reaction solution is subjected to buffer exchange with a Sephadex G-25 chromatographic column, into a buffer comprising 50 mM NaCl and 50 mM sodium dihydrogen phosphate-disodium hydrogen phosphate, pH 6.5.
Conjugation of antibody and drug-containing linker and hydrolysis: the reduced antibody as above is diluted to 5 mg/mL, into which 0.38 mL of N,N-Dimethylacetamide (DMA) which accounts for 2% of the total reaction volume as a pre-solvent and a solution of a drug-containing linker in DMA at 10 mg/mL at an equivalent molar ratio of 1:5.5 (antibody: drug-containing linker) as a reaction solution are added sequentially. Mixture obtained is stirred at room temperature for 30 minutes, and then is subjected to buffer exchange into a buffer consisting of disodium hydrogen phosphate-sodium dihydrogen phosphate, pH 8.0, with a NAP-25 chromatographic column packed with Sephadex G-25, to remove excessive drug-containing linker. Solution obtained is heated in water bath at 37 °C for 3 hours.
Purification of antibody-drug conjugate: a sample of the solution as above is concentrated using an AMICOM ultrafiltration centrifuge tube, to a concentration of about 15 mg/mL. A buffer consisting of 50 mM disodium hydrogen phosphate-sodium dihydrogen phosphate and 3 M ammonium phosphate is added to achieve a conductivity of 100 ms/cm. Then the solution is applied to a hydrophobic column packed with TOYOPEAL Butyl-650M (purchased from TOSOH), using phase A: a buffer consisting of 50 mM disodium hydrogen phosphate-sodium dihydrogen phosphate and 0.6 M ammonium sulfate; phase B: a buffer consisting of 50 mM disodium hydrogen phosphate-sodium dihydrogen phosphate. 8 column-volume gradient elution with phase B from 0% to 100% is conducted and main peak is collected.
The final sample obtained is subjected to buffer exchange into a buffer consisting of 50 mM disodium hydrogen phosphate-sodium dihydrogen phosphate, pH 7.4 using an AMICOM ultrafiltration centrifuge tube, which is then filtered through a 0.22 μm filter (Sartorius stedim Ministart).

**b.** Universal preparation method for stochastic conjugation

Stochastically-conjugated antibody-drug conjugates are prepared and obtained according to the preparation method described in the patent publication CN105849126A.

**4.2 Universal methods for analyzing antibody-drug conjugates**

**a.** Ultraviolet spectrophotometry used to determine drug-to-antibody ratio (UV-DAR method) and concentration

The concentration of an antibody-drug conjugate can be obtained by measuring the UV absorbance at 280 nm and the absorption wavelengths characteristic of small molecules and calculating as follows.

**a1.** Determination of the drug-to-antibody ratio (DAR) values of antibody-drug conjugates

It is known from a literature [Clin Cancer Res. 2004 Oct 15; 10(20): 7063-70] that, DAR $\text{(drug-to-antibody ratio)} = (\varepsilon_{280}^{Ab} - A_{280} / A_z \times \varepsilon_z^{Ab}) / (A_{280} / A_z \times \varepsilon_z^{D} - \varepsilon_{280}^{D})$, in which $\varepsilon_{280}^{Ab}$ is the molar absorption coefficient of an antibody at 280 nm, $A_{280}$ is the UV absorbance of an antibody-drug conjugate at 280 nm, $A_Z$ is the UV absorbance of the antibody-drug conjugate at Z nm which is an absorption wavelength characteristic of the drug-containing linker in the antibody-drug conjugate, $\varepsilon_z^{Ab}$ is the molar absorption coefficient of the antibody at Z nm which is an absorption wavelength characteristic of the drug-containing linker, $\varepsilon_z^{D}$ is the molar absorption coefficient of the drug-containing linker at Z nm, and $\varepsilon_{280}^{D}$ is the molar absorption coefficient of the drug-containing linker at 280 nm, and in which:

| Drug-containing linker | $\varepsilon_Z^{D}$ | $\varepsilon_{280}^{D}$ |
|---|---|---|
| MWD-L1 | 20146(Z=370nm) | 16171 |
| MWD-L7 | 20146(Z=370nm) | 16171 |
| MWD-L8 | 20146(Z=370nm) | 16171 |
| MWD-L9 | 20146(Z=370nm) | 16171 |
| MWC-L2 | 20196(Z=370nm) | 18643 |
| MWC-L3 | 23501(Z=360nm) | 21039 |
| MWF-L6 | 16901(Z=385nm) | 18108 |
| MWF-L7 | 16901(Z=385nm) | 18108 |
| MWF-L8 | 16901(Z=385nm) | 18108 |
| L-J | 13738 | 18001 |
| MC-GGFG-DXD | 20217(Z=370nm) | 5178 |
| L-A | 21430(Z=370nm) | 7670 |
| L-B | 9635(Z=255nm) | 5534 |

**a2.** Determination of the concentration of antibody-drug conjugates

Since the total absorbance at a certain wavelength of a system is equal to the sum of the absorbances of all the absorbent chemical species present in the system (additive nature of the absorbance), it is assumed that if the molar absorption coefficients of the antibody and the drug-containing linker contained do not change prior to and after the conjugation of the antibody with the drug-containing linker, the concentration of the antibody-drug conjugate follows the relationship: $A_{280} = \varepsilon_{280}^{ADC} \times C_{ADC} \times L = (\varepsilon_{280}^{D} \times DAR + \varepsilon_{280}^{Ab})\, C_{ADC} \times L$.

Thus, the molar concentration (mol/L) of the antibody-drug conjugate $C_{ADC} = A_{280} / (\varepsilon_{280}^{Ab} + \varepsilon_{280}^{D} \times DAR)$. Therefore, the concentration (g/L) of the antibody-drug conjugate $C_{ADC} = A_{280} / (\varepsilon_{280}^{Ab} + \varepsilon_{280}^{D} \times$

$DAR) \times MW_{ADC} = A_{280} \ / \ (\varepsilon_{280}^{Ab} + \varepsilon_{280}^{D} \times DAR) \times (MW_{Ab} + MW_{D} \times DAR)$, in which $MW_{ADC}$ is the molecular weight of the antibody-drug conjugate, $MW_{Ab}$ is the molecular weight of the antibody, and $MW_D$ is the molecular weight of the drug-containing linker; and the protein concentration can be obtained by putting the DAR value into the equation.

**b.** Hydrophobic chromatography

**b1.** Hydrophobic chromatography (HIC-HPLC) used to determine the DAR values of antibody-drug conjugates

Sample preparation: a sample is diluted to 2.0 mg/mL with mobile phase B, and then is centrifuged at 12000 rpm for 10 min; and supernatant obtained is taken for HPLC analysis.
Chromatographic column: Sepax Proteomix HIC Butyl-NP5, 5 μm, 4.6 mm × 35 mm;
Mobile phase A: 1.5 M $(NH4)_2SO_4$ + 25 mM PB, pH 7.0;
Mobile phase B: 25 mM PB + 20% IPA, pH 7.0;
Flow rate: 0.6 mL/min;
Detection wavelength: 280 nm;
Column temperature: 30 °C;
Loading volume: 10 μL;
Chromatographic gradient used for HIC analysis:

| Time (min) | Mobile phase A (%) | Mobile phase B (%) |
|---|---|---|
| 0 | 100 | 0 |
| 10 | 80 | 20 |
| 20 | 40 | 60 |
| 25 | 0 | 100 |
| 25.1 | 100 | 0 |
| 35 | 100 | 0 |

Equation for DAR calculation:

DAR = Σ (weighted peak area) / 100, i.e., DAR = (D0 peak area ratio × 0 + D1 peak area ratio × 1 + D2 peak area ratio × 2 + D3 peak area ratio × 3 + D4 peak area ratio × 4 + D5 peak area ratio × 5 + D6 peak area ratio × 6 + D7 peak area ratio × 7 + D8 peak area ratio × 8) / 100.

**b2.** Hydrophobic chromatography (HIC-HPLC) used to determine the DAR values of antibody-drug conjugates

Sample preparation: a sample is diluted to 2.0 mg/mL with mobile phase B, and then is centrifuged at 12000 rpm for 10 min; and supernatant obtained is taken for HPLC analysis.
Chromatographic column: TSKgel Butyl-NPR, 2.5 μm, 4.6 mm × 100 mm;
Mobile phase A: 1.2 M $(NH4)_2SO_4$ + 25 mM PB, pH 7.0;
Mobile phase B: 25 mM PB + 20% IPA, pH 7.0;
Flow rate: 0.6 mL/min;
Detection wavelength: 280 nm;
Column temperature: 30 °C;
Loading volume: 10 μL;
Chromatographic gradient used for HIC analysis:

| Time (min) | Mobile phase A (%) | Mobile phase B (%) |
|---|---|---|
| 0 | 100 | 0 |
| 1 | 100 | 0 |

(continued)

| Time (min) | Mobile phase A (%) | Mobile phase B (%) |
|---|---|---|
| 20 | 0 | 100 |
| 20.1 | 100 | 0 |
| 30 | 100 | 0 |

Equation for DAR calculation: the same as in **b1.**

**b3.** Hydrophobic chromatography (HIC-HPLC) used to determine the DAR values of antibody-drug conjugates

Sample preparation: a sample is diluted to 2.0 mg/mL with mobile phase B, and then is centrifuged at 12000 rpm for 10 min; and supernatant obtained is taken for HPLC analysis.
Chromatographic column: Sepax Proteomix HIC Butyl-NP5, 5 μm, 4.6 mm × 35 mm;
Mobile phase A: 2.5 M $(NH4)_2SO_4$ + 125 mM PB, pH 7.0;
Mobile phase B: 125 mM PB, pH 7.0;
Mobile phase C: IPA;
Mobile phase D: $H_2O$;
Flow rate: 0.5 mL/min;
Detection wavelength: 280 nm;
Column temperature: 30 °C;
Loading volume: 10 μL;
Chromatographic gradient used for HIC analysis:

| Time (min) | Mobile phase A (%) | Mobile phase B (%) | Mobile phase C (%) | Mobile phase D (%) |
|---|---|---|---|---|
| 0 | 80 | 0 | 0 | 20 |
| 20 | 5 | 45 | 3 | 47 |
| 22 | 0 | 50 | 3 | 47 |
| 22.1 | 80 | 0 | 0 | 20 |
| 30 | 80 | 0 | 0 | 20 |

Equation for DAR calculation: the same as in **b1.**

c. Mass spectrometry (LC-MS) used to determine the DAR values

Sample treatment: an appropriate amount of a sample is placed in an ultrafiltration tube, and is subjected to buffer exchange into a buffer consisting of 50 mM $NH_4HCO_3$ (pH 7.1). Upon supplementing the buffer, the obtained sample is subjected to ultrafiltration centrifugation (13000 g × 5 min). 8 μL of PNGase F enzyme is added into the sample after buffer exchange, which is in turn incubated at 37 °C for 5 h for desugarization. After the incubation, the sample is centrifuged at 12000 rpm for 5 min, and supernatant obtained is added into a sample vial as a test sample for later testing.

Chromatographic column: PolyLC ® PolyHYDROXYETHYLA Column, 300 Å, 5 μm, 2.1 mm × 200 mm;
Mobile phase: 50 mM ammonium acetate, pH 7.0;
Running time: 10 min;
Flow rate: 0.1 mL/min;
Loading volume: 2 μL;
Column temperature: 25 °C;
Detection wavelength: 280 nm;
Ionization mode: ESI positive;
Drying gas temperature: 325 °C;
Drying gas flow rate: 8 L/min;
Atomizer pressure: 20 psig;

Sheath gas temperature: 325 °C;
Sheath gas flow rate: 12 L/min;
Scanning setting: 900-8000 m/z.

**d.** Size exclusion chromatography (SEC-HPLC) used to determine the molecular size heterogeneity

Sample treatment: a sample is diluted to 1.0 mg/mL with a mobile phase and then is centrifuged at 12000 rpm for 10 min; and supernatant obtained is taken for analysis.

Chromatographic column: TOSOH, TSKgel G3000SWXL, 5 μm, 7.8 mm × 300 mm;
Mobile phase: 100 mM PB +200 mM arginine hydrochloride, 5% isopropanol (pH 6.8);
Flow rate: 0.6 mL/min;
Detection wavelength: 280 nm;
Column temperature: 30 °C;
Loading volume: 20 uL;
Elution time: 20 min;
Elution gradient: isocratic elution.

e. Non-Reducing Capillary Electrophoresis-Sodium Dodecyl Sulfate (NR-CE-SDS) to determine the purity

The determination is conducted according to the method "Determination of molecular size variants of monoclonal antibodies" described in General rule 3127 of Part IV of Chinese Pharmacopoeia.

**f.** Reverse Phase High Performance Liquid Chromatography (RP-HPLC) used to determine the drug-antibody ratios (DAR values) of antibody-drug conjugates

Analysis is conducted according to the method described in the patent publication US10227417B2.
Equation for DAR calculation:

DAR = 2 × (Σ (weighted peak area of light chain) +Σ (weighted peak area of heavy chain)) / 100, i.e., DAR = 2× (L0 peak area ratio ×0 + L1 peak area ratio ×1 + H0 peak area ratio ×0 + H1 peak area ratio ×1 + H2 peak area ratio ×2 + H3 peak area ratio ×3) / 100.

**g.** Reverse Phase High Performance Liquid Chromatography (RP-HPLC) used to determine the drug-antibody ratios (DAR values) of antibody-drug conjugates

Sample pretreatment: a sample is treated according to the method described in the patent publication US10227417B2.
HPLC apparatus: Waters Acquity Arc;
Mobile phase A: 0.1% TFA + ACN;
Mobile phase B: 0.1% TFA + $H_2O$;
Analytic column: Waters; BioResolve; 2.1 × 100 mm; 2.7 μm; 450 Å; PN 0024168;
Loading volume: 5 μL;
Flow rate: 0.25 mL/min;
Column temperature: 80 °C;
Detector: PDA detector;
Detection wavelength: 280 nm.
Chromatographic gradient used:

| Time (min) | Mobile phase A (%) | Mobile phase B (%) |
|---|---|---|
| 0 | 25 | 75 |
| 12.5 | 36 | 64 |
| 20 | 45 | 55 |

(continued)

| Time (min) | Mobile phase A (%) | Mobile phase B (%) |
|---|---|---|
| 25 | 45 | 55 |
| 25.1 | 25 | 75 |
| 35 | 25 | 75 |

**Example 4.1** Preparation and characterization of antibody-drug conjugates targeting Trop-2

Anti-Trop-2 antibodies hTINA1 (prepared by reference to the sequences of Datopotamab provided in WHO Drug Information), and h23-12 (each 100 mg) were conjugated with the Drug-containing linkers MWD-L1, MWC-L2, MWC-L3, MWF-L6, MWD-L7, MWD-L8, MWD-L9 and MWF-L8 respectively, according to the methods described in the present **Group 4 of Examples,** and bridged ADCs 1a, 1b, 1c, 1d, 1j, 1k, 11, 1m, 1n, 1o, and 1p were obtained. An intermediate sample obtained during the preparation of ADC 1d which had not been subjected to hydrophobic chromatography was stored and named as intermediate sample 1j.

Anti-Trop-2 antibodies hTINA1 and h23-12 (each 100 mg) were conjugated with the Drug-containing linkers MC-GGFG-Dxd, L-A, L-B, L-J, MWS-L7, L-I, and MWS-L6 respectively, according to the method described in the patent publication CN105849126B, and stochastically conjugated ADCs 1e, 1f, 1g, 1h, 1i, 1q, 1r, 1s, 1t and 1u were obtained.

DAR values, concentration, and purity of the bridged ADCs (1a-1d, 1j, and 1k-1p) were determined using the ultraviolet spectrophotometry described in section **4.2 a,** hydrophobic chromatography described in section **4.2 b,** and size exclusion chromatography described in section **4.2 d,** in the present **Group 4 of Examples.**

DAR values of ADCs in which Drug-containing linker MC-GGFG-Dxd was conjugated (le, 1h, 1q, 1r, 1s, 1t and 1u) were determined using the RP-HPLC described in section **4.2 f** in the present **Group 4 of Examples;** DAR values of ADCs in which Drug-containing linker L-A or L-B was conjugated (1f, 1g, and 1i) were determined using the RP-HPLC described in section **4.2 g** in the present **Group 4 of Examples;** and concentration and purity of the non-bridged ADCs (1c-1i) were determined using the ultraviolet spectrophotometry described in section **4.2 a** and size exclusion chromatography described in section **4.2 d** in the present **Group 4 of Examples.**

Results are provided in Table 1.

Table 1. Characterization results of antibody-drug conjugates targeting Trop-2

| ADC # | Name | Antibody | Drug-containing linker | DAR | Concentration (mg/ml) | SEC |
|---|---|---|---|---|---|---|
| 1a | Trop2-ADC-1 | h23-12 | MWD-L1 | 4.0 | 4.6 | 99.0% |
| 1b | Trop2-ADC-2 | h23-12 | MWC-L2 | 4.0 | 3.8 | 99.2% |
| 1c | Trop2-ADC-3 | h23-12 | MWC-L3 | 4.0 | 12.3 | 97.2% |
| 1d | Trop2-ADC-4 | hTINA1 | MWD-L1 | 4.0 | 5.7 | 99.3% |
| 1e | Trop2-ADC-5 | h23-12 | MC-GGFG-Dxd | 3.8 | 4.5 | 99.9% |
| 1f | Trop2-ADC-6 | h23-12 | L-B | 3.7 | 6.1 | 88.1% |
| 1g | Trop2-ADC-7 | h23-12 | L-A | 4.1 | 7.3 | 97.7% |
| 1h | Trop2-ADC-8 | hTINA1 | MC-GGFG-Dxd | 4.2 | 9.5 | 99.2% |
| 1i | Trop2-ADC-9 | hTINA1 | L-B | 4.2 | 7.7 | 93.8% |
| 1j | Trop2-ADC-4’ | hTINA1 | MWD-L1 | Not recorded | 8.3 | Not recorded |
| 1k | Trop2-ADC-10 | h23-12 | MWD-L7 | 4.0 | 7.7 | 98.9% |
| 1l | Trop2-ADC-11 | h23-12 | MWD-L8 | 4.0 | 6.3 | 99.5% |
| 1m | Trop2-ADC-12 | h23-12 | MWD-L9 | 4.0 | 7.2 | 99.1% |
| 1n | Trop2-ADC-13 | hTINA1 | MWF-L6 | 4.0 | 7.1 | 98.1% |
| 1o | Trop2-ADC-14 | h23-12 | MWF-L6 | 4.1 | 4.3 | 99.6% |
| 1p | Trop2-ADC-15 | h23-12 | MWF-L8 | 3.3 | 4.7 | Not recorded |
| 1q | Trop2-ADC-16 | h23-12 | L-J | 3.9 | 5.1 | Not recorded |

(continued)

| ADC # | Name | Antibody | Drug-containing linker | DAR | Concentration (mg/ml) | SEC |
|---|---|---|---|---|---|---|
| 1r | Trop2-ADC-17 | h23-12 | MWS-L7 | 4.4 | about 7.3 | Not recorded |
| 1s | Trop2-ADC-18 | h23-12 | L-J | 8.0 | 4.4 | Not recorded |
| 1t | Trop2-ADC-19 | h23-12 | L-I | 3.0 | about 2.0 | 99.1% |
| 1u | Trop2-ADC-20 | h23-12 | MWS-L6 | 3.0 | about 2.5 | 99.1% |

(in the present disclosure, h23-12 is a monoclonal antibody having the following sequences:

Heavy chain variable region:

QVQLVQSGAEVKKPGASVKVSCKASGYTFTSYWMHWVRQAPGQGLEWMGEITPSDNYGSYNQKFKGRVTITRDTSTSTAYMELSSLRSEDTAVYYCARGHGNYVSFDYWGQGTLVTVSS (SEQ ID NO: 7)

Heavy chain CDR1 : SYWMH (SEQ ID NO: 1)
Heavy chain CDR2 : EITPSDNYGSYNQKFKG (SEQ ID NO: 2)
Heavy chain CDR3 : GHGNYVSFDY (SEQ ID NO: 3)
Light chain variable region:

DIQMTQSPSSLSASVGDRVTITCRASQDISNYLNWYQQKPGKAPKLLIYYTSRLESGVPSRFSGSGSGTDFTLTISSLQPEDFATYFCQQGYTLPPYTFGQGTKLEIK (SEQ ID NO: 8)

Light chain CDR1: RASQDISNYLN (SEQ ID NO: 4)
Light chain CDR2: YTSRLES (SEQ ID NO: 5)
Light chain CDR3: QQGYTLPPYT (SEQ ID NO: 6)
Heavy chain constant region:

ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK (SEQ ID NO: 9)

Light chain constant region:

RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO: 10)

**Example 4.2** Preparation of antibody-drug conjugates targeting HER-2

Anti-HER-2 antibody Trastuzumab (CAS: 180288-69-1, purchased from Shanghai Roche Pharmaceutical Co.,

Ltd.) (100 mg) was conjugated with the Drug-containing linkers MWD-L1, MWD-L2, MWE-L4, MWF-L6, MWD-L8, MWD-L9, L-C, and MWC-L3 respectively, according to the methods described in the present **Group 4 of Examples,** and bridged ADCs 2a, 2b, 2c, 2h, 2i, 2d, and 21 were obtained.

Anti-HER-2 antibody Trastuzumab (100 mg) were conjugated with the Drug-containing linkers MC-GGFG-DXD, L-A, and L-B respectively, according to the method described in the patent publication CN105829346B, and stochastically conjugated ADCs 2e, 2f, 2g, 2k, and 2m were obtained.

DAR values, concentration, and purity of the bridged ADCs (2a-2d, 2h-2j, and 21) were determined using the ultraviolet spectrophotometry described in section **4.2 a,** hydrophobic chromatography described in section **4.2 b,** and size exclusion chromatography described in section **4.2 d,** in the present **Group 4 of Examples,**

DAR values of ADCs in which Drug-containing linker MC-GGFG-Dxd was conjugated were determined using the RP-HPLC described in section **4.2 f** in the present **Group 4 of Examples;** DAR values of ADCs in which Drug-containing linker L-A or L-B was conjugated (2e, and 2b) were determined using the RP-HPLC described in section **4.2 g** in the present **Group 4 of Examples;** and DAR values and concentration, and purity of the non-bridged ADCs (2e-2g, 2k, and 2m) were determined using the ultraviolet spectrophotometry described in section **4.2 a,** and size exclusion chromatography described in section **4.2 d,** in the present **Group 4 of Examples.**

Results are provided in Table 2.

Table 2. Characterization results of antibody-drug conjugates targeting HER-2

| ADC # | Name | Antibody | Drug-containing linker | DAR | Concentration (mg/ml) | SEC |
|---|---|---|---|---|---|---|
| 2a | Her2-ADC-1 | Trastuzumab | MWC-L1 | 3.9 | 4.6 | 98.0% |
| 2b | Her2-ADC-2 | Trastuzumab | MWC-L2 | 4.0 | 3.8 | 99.2% |
| 2c | Her2-ADC-3 | Trastuzumab | MWE-L4 | 4.0 | 3.7 | 97.5% |
| 2d | Her2-ADC-4 | Trastuzumab | L-C | 3.9 | 3.8 | 97.72 |
| 2e | Her2-ADC-5 | Trastuzumab | L-A | 4.3 | 4.8 | 99.0% |
| 2f | Her2-ADC-6 | Trastuzumab | L-B | 4.1 | 6.2 | 98.6% |
| 2g | Her2-ADC-7 | Trastuzumab | MC-GGFG-Dxd | 4.0 | 6.6 | 99.8% |
| 2h | Her2-ADC-8 | Trastuzumab | MWD-L8 | 4.0 | 7.5 | 98.9% |
| 2i | Her2-ADC-9 | Trastuzumab | MWD-L9 | 4.0 | 7.8 | 99.5% |
| 2j | Her2-ADC-10 | Trastuzumab | MWF-L6 | 4.0 | 7.2 | 98.7% |
| 2k | Her2-ADC-11 | Trastuzumab | MC-GGFG-Dxd | 8.0 | 5.3 | 99.2% |
| 21 | Her2-ADC-12 | Trastuzumab | MWC-L3 | 3.5 | 16.7 | 99.5% |
| 2m | Her2-ADC-13 | Trastuzumab | L-B | 8.0 | 1.8 | 95.0% |

**Example 4.3** Structure characterization of compounds

ADCs targeting Trop-2 prepared in Example 4.1 (1a, 1h, and 1j) and ADCs targeting HER-2 prepared in Example 4.2 (2b, and 2e) were subjected to analysis by hydrophobic chromatography described in section **4.2 b** in the present **Group 4 of Examples,** and heterogeneity of the ADCs prepared by different conjugation methods was compared.

ADCs are shown in Table 3.

Table 3. Antibody-drug conjugates and characterization methods

| ADC # | Name | Antibody | Drug-containing linker | Hydrophobic chromatography (HIC-HPLC) |
|---|---|---|---|---|
| 1d | Trop2-ADC-4 | hTINA1 | MWC-L1 | **4.2 b3** |
| 1h | Trop2-ADC-8 | hTINA1 | MC-GGFG-Dxd | **4.2 b2** |
| 1j | Trop2-ADC-4' | hTINA1 | MWC-L1 | **4.2 b3** |
| 2b | Her2-ADC-2 | Trastuzumab | MWC-L2 | **4.2 b3** |
| 2e | Her2-ADC-5 | Trastuzumab | L-A | **4.2 b2** |

It is known from the comparison between Trop2-ADC-4', Trop2-ADC-4, and Trop2-ADC-8, Trop2-ADC-4 and Trop2-ADC-4' prepared using Drug-containing linker MWD-L1 had a more excellent drug homogeneity, compared with the existing ADC (Trop2-ADC-8) prepared using Drug-containing linker MC-GGFG-Dxd. Results are provided in FIG. 1.
It is known from the comparison between Her2-ADC-2 and Her2-ADC-5, ADC Her2-ADC-2 prepared using Drug-containing linker MWC-L2 had a better drug homogeneity than Her2-ADC-5 prepared using the existing Drug-containing linker L-A.

**Group 5 of Examples Evaluation of activities**

**Example 5.1** Comparative study of the activity of camptothecins

KB oral epithelial cancer cells (purchased from: ATCC) were cultured in DMEM medium supplemented with 10% FBS; NCI-N87 human gastric cancer cells (purchased from: ATCC) were cultured in RPMI1640 medium supplemented with 10% FBS; HT29 human colon cancer cells (purchased from: ATCC) were cultured in RPMI1640 medium supplemented with 10% FBS; and BxPC-3 human pancreatic adenocarcinoma cells (purchased from: ATCC) were cultured in RPMI1640 medium supplemented with 10% FBS.
Using corresponding media described above and in cell plates, the density of KB cells was adjusted to $2\times10^4$/mL; the density of NCI-N87 cells was adjusted to $5\times10^4$/mL; the density of HT29 cells was adjusted to $5\times10^4$/mL, and the cells were plated onto 96-well transparent flat bottom plates, at 100 μL/well, and cultured overnight; and the density of BxPC-3 cells was adjusted to $5\times10^4$/mL.
Samples to be detected were diluted to 20 μM with corresponding media, then 5-fold gradient dilution was performed, and 9 concentrations were obtained. A well having cells but with no any sample added was set as the well showing maximum cell growth; and a well having no cells was set as the well of medium background. The diluted samples were added into the cell plates, at 100 μL/well, which were then incubated in a $CO_2$ incubator for 96 hours. After the incubation, CCK-8 (purchased from: DOJINDO LABORATORIES) prepared beforehand was added into the plates, at 20 μL/well, and after further 3 hours incubation in the $CO_2$ incubator, OD values were read on a microplate reader.
Data obtained was fitted to a four-parameter curve using software Prism7, and equation for calculating maximum killing is: maximum killing (%) = 1 - (OD450 value of the well showing maximum killing - OD450 value of the well of medium background) / (OD450 value of the well showing maximum cell growth - OD450 value of the cell of medium background) × 100%. Results are shown in Table 4.

Table 4. Results of comparative study of the activity of camptothecins

| Cell strain | Sample name | EC50 (nM) | Maximum Killing (%) | Plate no. | Killing days |
|---|---|---|---|---|---|
| HT29 | MWC-1 | 9.894 | 89 | P1 | 4 days |
| | Compound 1 | 43.43 | 91.9 | | |
| | Dxd | 132.8 | 85.4 | | |
| | MWC-1 | 8.678 | 89.9 | P2 | |
| | Compound 3 | 6.045 | 90.6 | | |
| | Dxd | 126.6 | 87.4 | | |
| KB | MWC-1 | 14.83 | 98.6 | P1 | 4 days |
| | Compound 1 | 52.31 | 97.6 | | |
| | Dxd | 135.8 | 94.7 | | |
| | MWC-1 | 15.67 | 98.6 | P2 | |
| | Compound 3 | 7.467 | 95.6 | | |
| | Dxd | 138.5 | 94.9 | | |
| NCI-N87 | MWC-1 | 4.444 | 93.4 | P1 | |
| | Compound 1 | 21.93 | 92.1 | | |
| | Dxd | 45.33 | 92.8 | | |

(continued)

| Cell strain | Sample name | EC50 (nM) | Maximum Killing (%) | Plate no. | Killing days |
|---|---|---|---|---|---|
| | MWC-1 | 5.217 | 93.3 | P2 | 4 days |
| | Compound 3 | 3.371 | 92.1 | | |
| | Dxd | 43.71 | 92.7 | | |
| | Compound 3 | 0.706 | 90.9 | P3 | |
| | Compound 7 | 3.312 | 87.9 | | |
| | Compound 3 | 10.750 | 92.9 | P4 | |
| | Compound 12 | 8.426 | 94.9 | | |
| | Compound 3 | 6.219 | 87.7 | P5 | |
| | Compound 14-P1 | 503.40 | 42.5 | | |
| | Compound 27 | 1.746 | 90.6 | | |
| | Compound 3 | 7.849 | 88.4 | P6 | |
| | Compound 14-P2 | 2001.00 | 68.4 | | |
| | Compound 3 | 10.31 | 92.0 | P7 | |
| | Compound 15 | 178.4 | 83.6 | | |
| | Compound 3 | 14.510 | 90.2 | P8 | |
| | Compound 18 | 1.574 | 92.9 | | |
| | Compound 26 | 1.021 | 93.4 | | |
| | Compound 3 | 2.340 | 93.1 | P9 | |
| | Compound 20 | 0.059 | 93.2 | | |
| | Compound 3 | 4.503 | 88.3 | P10 | |
| | Compound 31 | 8.593 | 90.7 | | |
| | Dxd | 11.930 | 94.6 | P11 | |
| | Compound 17 | 7.429 | 87.6 | | |
| BxPC-3 | Compound 3 | 2.809 | 98.0 | P1 | 4 days |
| | Compound 7 | 11.36 | 94.4 | | |
| | Compound 3 | 3.531 | 96.6 | P2 | |
| | Compound 12 | 2.148 | 98.5 | | |
| | Compound 3 | 4.197 | 94.7 | P3 | |

(continued)

| Cell strain | Sample name | EC50 (nM) | Maximum Killing (%) | Plate no. | Killing days |
|---|---|---|---|---|---|
| | Compound 14-P1 | 2.17E+19 | 38.8 | | |
| | Compound 27 | 1.524 | 97.7 | | |
| | Compound 3 | 2.39 | 93.1 | P4 | |
| | Compound 14-P2 | 1.96E+32 | 43.3 | | |
| | Compound 3 | 3.234 | 97.7 | P5 | |
| | Compound 15 | 79.640 | 87.9 | | |
| | Compound 3 | 5.950 | 93.9 | P6 | |
| | Compound 18 | 1.451 | 99.6 | | |
| | Compound 26 | 1.111 | 99.1 | | |
| | Compound 3 | 3.004 | 99.3 | P7 | |
| | Compound 20 | 0.683 | 99.2 | | |
| | Compound 3 | 5.815 | 93.7 | P8 | |
| | Compound 31 | 4.423 | 93.7 | | |
| | Compound 3 | 3.234 | 97.7 | P9 | |
| | Compound 15 | 79.640 | 87.9 | | |
| | Compound 13 | 1.405 | 98.9 | | |
| | Dxd | 24.310 | 91.8 | P10 | |
| | Compound 19 | 1.402 | 97.4 | | |
| | Compound 3 | 2.388 | 95.9 | | |
| | Compound 3 | 5.815 | 93.7 | P11 | |
| | Compound 14 | 6.456 | 84.7 | | |
| | Compound 31 | 4.423 | 93.7 | | |

**Comparative study was performed on antibody-drug conjugates with different drug-containing linkers conjugated, and is provided below.**

**Example 5.2** Comparative study of the activity of ADCs targeting HER-2 with different drug-containing linkers conjugated

N87 cell line having high expression of HER-2 (purchased from: ATCC) was taken from liquid nitrogen, and recovered. Then the density of the cells was adjusted to $5\times10^4$/mL with complete medium, and the cells were added to cell culture plates (96-well plates were used, and sterile PBS or sterile purified water was added at 200 μL/well into rows A and H, and columns 1 and 12 of the plates) at 100 μL/well, and cultured overnight.

Samples of ADCs targeting HER-2 prepared above were diluted respectively to 50 μg/ml using complete medium, followed by 4-fold gradient dilution, and 9 concentrations and concentration zero were obtained. The diluted samples were added into the cell culture plates, and three duplicate wells were set for each of the diluted samples; and a negative control well (cell + medium) and a blank control well (no cells, medium only) were set in column 11 of the plates. Next, the plates were placed in a cell incubator and the cells were incubated for 120 h or 168 h (see Table below for details). After the incubation, MTS was added at 40 μL/well to the plates which were then placed back into the 37 °C incubator and allowed for reacting for 2-4 h. Then the cell plates were taken out, and OD values at 490 nm were read on a microplate reader.

Results are shown in Table 5.

Table 5. Results of the cell killing effect in vitro of ADCs targeting HER-2

| Plate no. | Sample name | Antibody | Drug-containing linker | EC50 (ng/ml) | Maximum cell killing effect (%) |
|---|---|---|---|---|---|
| Plate 1 (120 h) (NCI-N87: 5000 cells/ well) | Her2-ADC-7 | Trastuzumab | MC-GGFG-Dxd | 50.32 | 80.8 |
| | Her2-ADC-5 | Trastuzumab | L-A | 54.33 | 71.1 |
| | Her2-ADC-4 | Trastuzumab | L-C | 43.21 | 72.8 |
| Plate 2 (120 h) (NCI-N87: 5000 cells/ well) | Her2-ADC-7 | Trastuzumab | MC-GGFG-Dxd | 60.95 | 82.6 |
| | Her2-ADC-5 | Trastuzumab | L-A | 61.98 | 72.0 |
| | Her2-ADC-1 | Trastuzumab | MWD-L1 | 37.64 | 87.5 |

The results above show that the drug potency of the ADC prepared using Drug-containing linker MWD-L1 was stronger than that of the ADCs prepared using existing Drug-containing linkers MC-GGFG-Dxd and L-A; and the drug potency of using L-C home-made for bridging conjugation was inferior to that of using the Drug-containing linkers MWD-L1 and MC-GGFG-Dxd.

In addition, a further comparison was made between the ADC prepared using Drug-containing linker MWC-L2 and the ADCs prepared using L-B and MC-GGFG-Dxd, as described above. Results are shown in Table 6.

Table 6. Results of the cell killing effect in vitro of ADCs targeting HER-2

| Plate no. | Sample name | Antibody | Drug-containing linker | EC50 (ng/ml) | Maximum cell killing effect (%) |
|---|---|---|---|---|---|
| Plate 1 (168h) (NCI-N87: 5000 cells/ well) | Her2-ADC-7 | Trastuzumab | MC-GGFG-Dxd | 80.98 | 90.7 |
| | Her2-ADC-6 | Trastuzumab | L-B | NA | 71.1 |
| | Her2-ADC-2 | Trastuzumab | MWC-L2 | 36.61 | 89.5 |

The results above show that Drug-containing linker MWC-L2 had a certain advantage in cell killing in vitro as well, as compared with the existing Drug-containing linkers L-B and MC-GGFG-Dxd.

In addition, a further comparison was made between the Drug-containing linker MWD-L1 and the Drug-containing linkers MWD-L8 and MWD-L9, as described above. Results are shown in Table 7.

Table 7. Results of the cell killing effect in vitro of ADCs targeting HER-2

| Plate no. | Sample name | Antibody | Drug-contai ning linker | EC50 (ng/ml) | Maximum cell killing effect (%) |
|---|---|---|---|---|---|
| Plate 1 (168h) (NCI-N87: | Her2-ADC-8 | Trastuzumab | MWD-L8 | 97.05 | 94.9 |
| | Her2-ADC-9 | Trastuzumab | MWD-L9 | 110.90 | 69.5 |
| 5000 cells/well) | Her2-ADC-1 | Trastuzumab | MWD-L1 | 52.31 | 84.9 |

The results above show that the drug potency of the ADC prepared using Drug-containing linker MWD-L1 (in which phenylene is substituted) was more prominent, as compared with that of the ADCs prepared using Drug-containing linkers MWD-L8 (in which phenylene is not substituted) and MWD-L9 (in which phenylene is substituted with trifluoromethy).

<u>**Example 5.3**</u> Comparative study of the activity of ADCs targeting Trop-2 with different drug-containing linkers conjugated

BxPC3 cell line having high expression of Trop-2 (purchased from: ATCC) was taken from liquid nitrogen, and recovered. Then the density of the cells was adjusted to $5\times10^4$/mL with complete medium, and the cells were added to cell culture plates (96-well plates were used, and sterile PBS or sterile purified water was added at 200 μL/well into rows A and H, and columns 1 and 12 of the plates) at 100 μL/well, and cultured overnight.

Samples of ADCs targeting Trop-2 prepared above were diluted respectively to 50 μg/ml using complete medium, followed by 4-fold gradient dilution, and 9 concentrations and concentration zero were obtained. The diluted

samples were added into the cell culture plates, and three duplicate wells were set for each of the diluted samples; and a negative control well (cell + medium) and a blank control well (no cells, medium only) were set in column 11 of the plates. Next, the plates were placed in a cell incubator and the cells were incubated for 120 h. After the incubation, MTS was added at 40 μL/well to the plates which were then placed back into the 37 °C incubator and allowed for reacting for 2-4 h. Then the cell plates were taken out, and OD values at 490 nm were read on a microplate reader.

Results are shown in Table 8.

Table 8. Results of the cell killing effect in vitro of ADCs targeting Trop-2

| Plate no. | Sample name | Antibody | Drug-containing linker | EC50 (ng/ml) | Maximum cell killing effect (%) |
|---|---|---|---|---|---|
| Plate 1 (120 h) (BXPC3: 5000cells/well) | Trop2-ADC-1 | h23-12 | MWD-L1 | 40.90 | 76.9 |
| | Trop2-ADC-2 | h23-12 | MWC-L2 | 41.51 | 77.9 |
| | Trop2-ADC-6 | h23-12 | L-B | 321.50 | 55.5 |
| Plate 1 (120 h) (BXPC3:5000 cells/well) | Trop2-ADC-1 | h23-12 | MWD-L1 | 51.94 | 76.2 |
| | Trop2-ADC-2 | h23-12 | MWC-L2 | 57.29 | 73.9 |
| | Trop2-ADC-7 | h23-12 | L-A | 247.70 | 56.2 |
| Plate 1 (120 h) (BXPC3:5000 cells/well) | Trop2-ADC-4 | hTINA1 | MWD-L1 | 54.84 | 77.6 |
| | Trop2-ADC-9 | hTINA1 | L-B | 210.90 | 53.9 |
| | Trop2-ADC-8 | hTINA1 | MC-GGFG-Dxd | 83.49 | 81.3 |
| Plate1 (120 h) (BXPC3:5000 cells/well) | Trop2-ADC-8 | hTINA1 | MC-GGFG-Dxd | 133.1 | 84.9 |
| | Trop2-ADC-15 | h23-12 | MWF-L8 | 80.17 | 81.6 |
| | Trop2-ADC-14 | h23-12 | MWF-L6 | 67.11 | 82.2 |
| Plate 1 (120 h) (BXPC3:5000 cells/well) | Trop2-ADC-14 | h23-12 | MWF-L6 | 71.87 | 84.6 |
| | Trop2-ADC-16 | h23-12 | L-J | 53.2 | 79.9 |
| | Trop2-ADC-17 | h23-12 | MWS-L7 | 45.7 | 83.2 |
| Plate 1 (120 h) (BXPC3:5000 cells/well) | Trop2-ADC-14 | h23-12 | MWF-L6 | 73.4 | 85.2 |
| | Trop2-ADC-18 | h23-12 | L-J | 32.4 | 88.7 |
| Plate 1 (120 h) (BXPC3: 5000 | Trop2-ADC-14 | h23-12 | MWF-L6 | 70.2 | 83.1 |
| | Trop2-ADC-19 | h23-12 | L-I | 30.4 | 88.9 |
| | Trop2-ADC-20 | h23-12 | MWS-L6 | 34.5 | 86.3 |
| cells/well) | | | | | |

The results above show that the ADC prepared using Drug-containing linker MWD-L1 exhibited a comparable drug potency to the ADC prepared using MWC-L2; and, the drug potency of the ADCs prepared using the drug-containing linkers according to the invention was superior to that of the ADCs prepared using existing linkers L-B, L-A, and MC-GGFG-Dxd.

In addition, a further comparison was made between Trop2-ADC-1, Trop2-ADC-10, and Trop2-ADC-6, as described above. Results are shown in Table 9.

Table 9. Results of the cell killing effect in vitro of ADCs targeting Trop-2

| Plate no. | Sample name | Antibody | Drug-containing linker | EC50 (ng/ml) | Maximum cell killing effect (%) |
|---|---|---|---|---|---|
| Plate 1 (120 h) (BXPC3: 5000 cells/well) | Trop2-ADC-1 | h23-12 | MWD-L1 | 72.61 | 79.4 |
| | Trop2-ADC-10 | h23-12 | MWD-L7 | 95.83 | 75.8 |
| | Trop2-ADC-6 | h23-12 | L-B | 553.50 | 50.2 |

It can be seen from the results of comparative study above, there was a significant difference in the activity of ADCs prepared using drug-containing linkers with different drug releasing structures, and the ADCs using MWD-L1 had the best drug potency.

In addition, a further comparison was made between Trop2-ADC-1, Trop2-ADC-9 and Trop2-ADC-8, as described above. Results are shown in Table 10.

Table 10. Results of the cell killing effect in vitro of ADCs targeting Trop-2

| Plate no. | Sample name | Antibody | Drug-containing linker | EC50 (ng/ml) | Maximum cell killing effect (%) |
|---|---|---|---|---|---|
| Plate 1 (120 h) (BXPC3: 5000 cells/well) | Trop2-ADC-1 | h23-12 | MWD-L1 | 43.67 | 93.3 |
| | Trop2-ADC-9 | hTINA1 | L-B | 126.50 | 80.9 |
| | Trop2-ADC-8 | hTINA1 | MC-GGFG-Dxd | 90.58 | 92.0 |

The results above show that the novel ADC Trop2-ADC-1 provided by the present invention had a certain advantage in activity, as compared with Trop2-ADC-8 and Trop 2-ADC-9.

**Example 5.4** Study of drug potency in vitro of different ADCs on tumors expressing antigens of low abundance and cells with low antigen expression in vicinity of tumor cells

Due to the heterogeneity of advanced tumors, tumor tissues have varied antigen expression. The bystander effect in the case of tumors expressing antigens of low abundance and cells with low antigen expression in vicinity of tumor cells usually affects the prognosis of patients with advanced tumors, and is an important index for drug evaluation. Therefore, tumors expressing antigens of low abundance and cells with low antigen expression in vicinity of tumor cells were evaluated respectively.

(1) Study of drug potency in vitro of different ADCs on tumors expressing antigens of low abundance

HS-746T cells were purchased from ATCC. The cells were cultured in RPMI 1640/IMEM (1:1) containing 10% Fetal Bovine Serum (FBS) with penicillin and streptomycin supplemented simultaneously, and cultured at 37 °C in air containing 5% $CO_2$ in an incubator.

After inoculation, the cells were treated for 168 hours respectively with 0.1 μg/mL and 1 μg/mL Trop2-ADC-1, Trop2-ADC-2, Trop2-ADC-5, Trop2-ADC-10, and Trop2-ADC-14, and then the cells were harvested and counted. Next, the cells were incubated with Dylight 488 NHS Ester labeled ADCs targeting Trop-2 described above at 4 °C for 1 hour in dark, and then centrifuged to remove supernatant, re-suspended in phosphate buffer (PBS, pH7.4), washed with PBS (pH7.4) three times, and the number of HS-746T cells was detected and calculated using a flow cytometer BD ACCURI C6 PLUS.

Table 11. Cell killing effect in vitro of ADCs targeting Trop-2 on HS-746T tumor cells expressing antigen of low abundance

| Compound | Concentration administered (μg/mL) | Linker | Species of active drug | Number of cells (single cells having low expression) | Inhibition (%) |
|---|---|---|---|---|---|
| Vechicle | NA | NA | NA | 353700 | NA |
| Trop2-ADC-1 | 0.1 | MWD-L1 | Exatecan compound | 189000 | 46.6 |
| Trop2-ADC-1 | 1 | MWD-L1 | Exatecan compound | 171000 | 51.7 |
| Trop2-ADC-2 | 0.1 | MWC-L2 | MWC-1 | 269100 | 23.9 |
| Trop2-ADC-2 | 1 | MWC-L2 | MWC-1 | 267300 | 24.4 |
| Trop2-ADC-5 | 0.1 | GGFG-DXD | Exatecan compound | 345600 | 2.3 |
| Trop2-ADC-5 | 1 | GGFG-DXD | Exatecan compound | 345600 | 2.3 |
| Trop2-ADC-10 | 0.1 | MWD-L7 | Exatecan compound | 347700 | 1.7 |

(continued)

| Compound | Concentration administered (μg/mL) | Linker | Species of active drug | Number of cells (single cells having low expression) | Inhibition (%) |
|---|---|---|---|---|---|
| Trop2-ADC-10 | 1 | MWD-L7 | Exatecan compound | 339600 | 4.0 |
| Trop2-ADC-14 | 0.1 | MWF-L6 | Compound 3 | 210600 | 40.5 |
| Trop2-ADC-14 | 1 | MWF-L6 | Compound 3 | 206100 | 41.7 |

It can be seen from the study above that, ADCs prepared using Drug-containing linkers MWD-L1, MWC-L2, and MWF-L6 all showed certain killing effect on the HS-746T tumor cells expressing antigen of low abundance. ADCs prepared via two conjugation manners using Drug-containing linkers GGFG-DXD and MWD-L7 showed very little killing effect in such a tumor expressing antigen of low abundance. In addition, drug-containing linkers containing Compound 3 had better killing effect than the small molecule MWC-1, which is possibly associated with a better cell membrane penetration capability of Compound 3 itself.

(2) Study of drug potency in vitro of different ADCs on cells with low antigen expression in vicinity of tumor cells

KPL-4 cells and MDA-MB-468 cells were purchased from ATCC. The cells were cultured in RPMI 1640/IMEM (1:1) containing 10% Fetal Bovine Serum (FBS) with supplemented penicillin and streptomycin simultaneously, and cultured at 37 °C in air containing 5% $CO_2$ in an incubator.

KPL-4 cells positive for HER2 and MDA-MB-468 cells negative for HER2 were inoculated together, or the HER2-negative MDA-MB-468 cells were inoculated separately, and then all the cells were treated with ADCs for 168 hours. Then the cells were harvested and incubated with Dylight 488 NHS Ester labeled anti-HER2 antibodies at 4 °C for 1 hour in dark. Next, the cells were centrifuged to remove supernatant, re-suspended in PBS (pH7.4), washed with PBS three times, and cell proportions of KPL-4 cells and MDA-MB-468 cells were detected and calculated using a flow cytometer BD ACCURI C6 PLUS, and the numbers of the two kinds of cells were calculated.

Table 12. Study of the bystander effect of ADCs targeting HER-2 on HER2-positive KPL-4 cells and HER2-negative MDA-MB-468 cells

| Compound | Concentration administered (μg/mL) | Linker | Number of cells | HER2+ high expression | | HER2 negative | |
|---|---|---|---|---|---|---|---|
| | | | | Number of cells | Inhibition (%) | Number of cells | Inhibition (%) |
| Vehicle | NA | NA | 1.77E+06 | 1309761 | 0 | 459035 | 0 |
| Her2-ADC-7 | 0.1 | GGFG-DXD | 8.73E+05 | 537626 | 59 | 334170 | 27 |
| Her2-ADC-2 | 0.1 | MWC-L2 | 4.61E+05 | 239732 | 82 | 220064 | 52 |
| Her2-ADC-10 | 0.1 | MWF-L6 | 3.04E+05 | 117335 | 91 | 185460 | 60 |
| Her2-ADC-13 | 0.1 | L-B | 9.04E+05 | 566466 | 57 | 336329 | 27 |
| Her2-ADC-7 | 1 | GGFG-DXD | 2.04E+05 | 58643 | 96 | 144152 | 69 |
| Her2-ADC-2 | 1 | MWC-L2 | 1.16E+05 | 43408 | 97 | 71387 | 84 |
| Her2-ADC-10 | 1 | MWF-L6 | 1.10E+05 | 56423 | 96 | 52373 | 89 |
| Her2-ADC-13 | 1 | L-B | 3.82E+05 | 116920 | 91 | 263875 | 43 |

It can be seen from the study above that, all the groups administered with high concentration of ADCs (1 μg/mL) showed a tumor-inhibiting effect on the cells negative for the antigen. However, a comparison between the cells positive and negative for the antigen in the groups administered with different concentrations of ADCs showed that, the bystander killing effect of MWF-L6 and MWC-L2 was better than that of the controls GGFG-DXD and L-B.

**Example 5.5** Study of killing effect in vivo of different drug-containing linkers

BxPc-3 human pancreatic cancer cells purchased from the cell bank in Chinese academy of sciences were used. BxPC-3 cells were cultured in 10-cm culture dishes for adherent culture, in RPMI 1640 containing 10% Fetal Bovine Serum with penicillin and streptomycin supplemented, at 37 °C in an incubator containing 5% $CO_2$. The cells were passaged 2-3 times in one week, and when in exponential growth phase, they were digested using trypsin, harvested, counted and inoculated.

A comparison study was performed between groups of different doses of antibody-drug conjugates Trop2-ADC-1, Trop2-ADC-2, Trop2-ADC-3, and Trop2-ADC-5 prepared using different drug-containing linkers (Table 13). Mice were administered with those ADCs via intravenous injection (IV), at an administration volume of 10 mL/kg; and the mice in the group of vehicle were administered with the same volume of vehicle (saline). Specific dosages and administration schedule are shown in Table 13. Tumor volumes were measured 2 times per week, and the mice were weighed and the data were recorded.

When the experiment was completed, the experiment achieved the end point, or the tumor volume achieved 2000 $mm^3$, the animals were sacrificed under $CO_2$ anesthesia and the tumors were dissected and photographed. Results are shown in Table 13 and FIG. 2.

Table 13. Therapeutic effect of Trop2-ADC-1, Trop2-ADC-2, Trop2-ADC-3, and Trop2-ADC-5 on subcutaneous tumor in nude mice transplanted with BxPc-3 human pancreatic cancer cells (TGI (%) was calculated according to tumor volumes)

| Group | Drug-containing linker | TV (D0) SEM | TV (D21) SEM | T/C (%) | TGI (%) | P value | PR | Number of animals (D0) | Number of animals (D21) |
|---|---|---|---|---|---|---|---|---|---|
| Vehicle | | 123.3±1.4 | 2107.7±177.7 | - | - | - | 0 | 10 | 10 |
| Trop2-ADC-1 3 mg/kg | MWD-L1 | 124±2.1 | 1006.7±208.9 | 44 | 56 | 0.002 | 0 | 6 | 6 |
| Trop2-ADC-1 10 mg/kg | MWD-L1 | 120.9±1.5 | 197.8±33.6 | 4 | 96 | 0.000 | 1 | 6 | 6 |
| Trop2-ADC-2 3 mg/kg | MWC-L2 | 124.9±2.1 | 141.6±15.9 | 1 | 99 | 0.000 | 3 | 6 | 6 |
| Trop2-ADC-2 10 mg/kg | MWC-L2 | 121±1.7 | 106.6±7.1 | -12 | 112 | 0.000 | 5 | 6 | 6 |
| Trop2-ADC-3 3 mg/kg | MWC-L3 | 124.4±2.3 | 244±74.5 | 6 | 94 | 0.000 | 3 | 6 | 6 |
| Trop2-ADC-3 10 mg/kg | MWC-L3 | 123.5±2.8 | 107.6±6.7 | -13 | 113 | 0.000 | 5 | 6 | 6 |
| Trop2-ADC-5 3 mg/kg | GGFG-Dxd | 123±2.1 | 1301±130.2 | 59 | 41 | 0.007 | 0 | 6 | 6 |
| Trop2-ADC-5 10 mg/kg | GGFG-Dxd | 123.9±1.4 | 751.2±228.4 | 32 | 68 | 0.000 | 0 | 6 | 6 |

The results above show that the activity in vivo of all the Drug-containing linkers MWD-L1, MWC-L2 and MWC-L3 for site-specific conjugation was superior to that of the control molecule GGFG-DXD. Meanwhile, although MWC-L2 and MWC-L3 adopt a di-peptide structure of VA and a tetra-peptide releasing structure of GGFG respectively, their drug potency in vivo was at a comparable level.

**Example 5.6** Study of drug potency in vivo of antibody-drug conjugates targeting Trop-2

HT1376 human bladder cancer cells purchased from the cell bank in Chinese academy of sciences were used. HT1376 cells were cultured in 10-cm culture dishes for adherent culture, in RPMI 1640 containing 10% Fetal Bovine Serum with penicillin and streptomycin supplemented, at 37 °C in an incubator containing 5% $CO_2$. The cells were passaged 2-3 times in one week, and when in exponential growth phase, they were digested using trypsin, harvested, counted and inoculated.

A comparison study was performed between groups of different doses of antibody-drug conjugate Trop2-ADC-14 which was taken as an example (Table 14). Mice were administered with the ADC via intravenous injection (IV), at an administration volume of 10 mL/kg; the mice in the group of vehicle were administered with the same volume of vehicle (saline). Specific dosages and administration schedule are shown in Table 14. Tumor volumes were measured 2 times per week, and the mice were weighed and the data were recorded.

When the experiment was completed, the experiment achieved the end point, or the tumor volume achieved 2000 $mm^3$, the animals were sacrificed under $CO_2$ anesthesia and the tumors were dissected and photographed. Results are shown in Table 14 and FIG. 3, in which the growth changes in tumor volume of mice in each group are shown.

Table 14. Therapeutic effect of Trop2-ADC-14 on subcutaneous tumor in nude mice transplanted with HT1376 human bladder cancer cells (TGI (%) was calculated according to tumor volumes)

| Group | Mean tumor volume ($mm^3$) | | | Mean tumor volume ($mm^3$) | | | TGI (%) | P value |
|---|---|---|---|---|---|---|---|---|
| | D0 | | SEM | D21 | | SEM | D21 | D21 |
| Saline | 124.97 | ± | 0.61 | 1053.31 | ± | 111.3 | - | - |
| Trop2-ADC-14 1mg/kg | 126.07 | ± | 1.35 | 601.9 | ± | 54.1 | 49 | 0.003 |
| Trop2-ADC-14 3mg/kg | 125.82 | ± | 2.55 | 130.38 | ± | 37.7 | 100 | <0.001 |
| Trop2-ADC-14 10mg/kg | 124.94 | ± | 2.02 | 82.6 | ± | 3.2 | 134 | <0.001 |
| DS-1062a 3mg/kg | 123.51 | ± | 1.81 | 609.3 | ± | 65.8 | 48 | 0.004 |
| Trodelvy™ 3mg/kg | 123.7 | ± | 1.44 | 735.3 | ± | 91.3 | 34 | 0.045 |
| Note: p value is derived by comparing to the group of vehicle; DS-1062a is commercially available and can also be prepared according to the method described in the patent publication CN105849126A. | | | | | | | | |

The results above show that compared with the group of vehicle, Trop2-ADC-14 exhibited an obvious tumor-inhibiting activity in the transplantation tumor model of HT1376 human bladder cancer; on another hand, the tumor-inhibiting activity of Trop2-ADC-14 was obviously superior to that of DS-1062 and Trodelvy™ at the same dose.

**Example 5.7** Study of drug potency in vivo of antibody-drug conjugates targeting Trop-2

Calu-3 lung cancer cells purchased from the cell bank in Chinese academy of sciences were used. Calu-3 cells were cultured in 10-cm culture dishes for adherent culture, in RPMI 1640 containing 10% Fetal Bovine Serum with penicillin and streptomycin supplemented, at 37 °C in an incubator containing 5% $CO_2$. The cells were passaged 2-3 times in one week, and when in exponential growth phase, they were digested using trypsin, harvested, counted and inoculated.

A comparison study was performed between groups of different doses of antibody-drug conjugate Trop2-ADC-14 which was taken as an example (Table 15). Mice were administered with the ADC via intravenous injection (IV), at an administration volume of 10 mL/kg; the mice in the group of vehicle were administered with the same volume of vehicle (saline). Specific dosages and administration schedule are shown in Table 15. Tumor volumes were measured 2 times per week, and the mice were weighed and the data were recorded.

When the experiment was completed, the experiment achieved the end point, or the tumor volume achieved 2000 $mm^3$, the animals were sacrificed under $CO_2$ anesthesia and the tumors were dissected and photographed. Results are shown in Table 15 and FIG. 4, in which the growth changes in tumor volume of mice in each group are shown.

Table 15. Therapeutic effect of Trop2-ADC-14 on subcutaneous tumor in nude mice transplanted with Calu-3 human lung cancer cells (TGI (%) was calculated according to tumor volumes)

| Group | Mean tumor volume ($mm^3$) | | | Mean tumor volume ($mm^3$) | | | TGI (%) | P value |
|---|---|---|---|---|---|---|---|---|
| | D0 | | SEM | D21 | | SEM | D21 | D21 |
| Saline | 121.7 | ± | 1.0 | 1316.6 | ± | 94.5 | - | - |
| Trop2-ADC-14 1mg/kg | 119.6 | ± | 1.3 | 268.6 | ± | 94.0 | 88 | <0.001 |
| Trop2-ADC-14 3mg/kg | 121.1 | ± | 1.0 | 105.4 | ± | 5.2 | 113 | <0.001 |
| Trop2-ADC-14 10mg/kg | 121.6 | ± | 1.4 | 96.7 | ± | 1.8 | 120 | <0.001 |
| DS-1062a 3mg/kg | 121.2 | ± | 0.9 | 751.7 | ± | 52.2 | 99 | <0.001 |
| Trodelvy™ 3mg/kg | 120.2 | ± | 1.3 | 136.6 | ± | 17.2 | 47 | <0.001 |
| Note: p value is derived by comparing to the group of vehicle. | | | | | | | | |

The results above show that compared with the group of vehicle, Trop2-ADC-14 exhibited an obvious tumor-inhibiting activity in the transplantation tumor model of Calu-3 human lung cancer; meanwhile, the tumor-inhibiting activity of Trop2-ADC-14 was superior to that of DS-1062 and Trodelvy™ at the same dose.

**Example 5.8** Study of the bystander effect of antibody-drug conjugates targeting Trop-2

BxPC-3 cells (purchased from ATCC) positive for Trop-2 and HT-29 cells (purchased from ATCC) negative for Trop-2 were inoculated together, or the Trop-2-negative HT-29 cells were inoculated separately, in 6-well plates. Treated with Trop2-ADC-14 (10, 30, and 100 ng/mL) or DS-1062a (100, and 300 ng/mL) for 144 hours, the cells were harvested and counted. Next, the cells were incubated with Dylight 488 NHS Ester labeled Trop2-ADC-14 on ice for 1 hour in dark, and then centrifuged to remove supernatant, re-suspended in PBS, washed with PBS three times, and cell proportions of BxPC-3 cells and HT-29 cells were detected and calculated using a flow cytometer BD ACCURI C6 PLUS, and the numbers of the two kinds of cells were calculated. Results are shown in FIG. 5.

The results show that Trop2-ADC-14 had strong bystander effect, killing the cells negative for Trop-2 as well when killing the cells positive for Trop-2; the bystander killing effect of Trop2-ADC-14 at 30 ng/mL was comparable to that of reference drug DS-1062a at 300 ng/mL (5A in FIG. 5), suggesting that the bystander effect of Trop2-ADC-14 is overall stronger than that of reference drug DS-1062a. Trop2-ADC-14 and DS-1062a both had no obvious effect on proliferation of the Trop-2-negative HT-29 cells separately cultured (5B in FIG. 5).

**Example 5.9** Study of the proliferation-inhibiting effect of antibody-drug conjugates targeting Trop-2 and camptothecins on different tumor cells

For cells growing by adherence, SRB assay was used to detect effect of antibody-drug conjugates or camptothecins on the proliferation of tumor cells in vitro cultured. A certain number of cells in exponential growth phase were inoculated in 96-well cell culture plates, and when the cells grew by adherence overnight, antibody drug conjugates or camptothecins of different concentrations were added into the cells. 144 hours later, the cells were fixed with trichloroacetic acid, and then stained with SRB (prepared in 1% glacial acetic acid at a concentration of 4 mg/mL). 10 mM Tris solution was added into each well to solubilize the bound SRB. OD values at 510 nm were read on a microplate reader.

Inhibition (%) = (OD value of control well - OD value of dosed well)/OD value of control well ×100%

For cells growing in suspension, MTT assay was used to detect effect of antibody-drug conjugates or camptothecins on the proliferation of tumor cells in vitro cultured. A certain number of cells in exponential growth phase were inoculated in 96-well cell culture plates, and when the cells grew in suspension overnight, antibody-drug conjugates or camptothecins of different concentrations were added into the cells. 144 hours later, MTT was added to each well of the plates, and the incubation was continued for 4 hours at 37 °C in a 5% $CO_2$ saturated humidity incubator. Next, 100 μL of SDS-isobutanol-HCl solution was added into each well of the plates, and OD values at 570 nm and 690 nm were read on a microplate reader.

Half maximal inhibitory concentration (IC50) was calculated from the inhibition (%) at those different concentrations using the software Graphpad Prism 8.0. The experiment was repeated once and the data was expressed as mean ± SD. Results are shown in Table 16.

Table 16. Results of the proliferation-inhibiting effect of ADCs targeting Trop-2 and camptothecins on different tumor cells

| | | IC50 (nM) | | | | | | |
|---|---|---|---|---|---|---|---|---|
| Cell strain | Pathologic type | Trop2-ADC-14 | | | Compound 3 | | | h23-12 |
| | | 1st experiment | 2nd experiment | Mean ± SD | 1st experiment | 2nd experiment | Mean ± SD | |
| MDA-MB-231/ TROP2 | Triple negative breast cancer | 0.05 | 0.11 | 0.08 ± | 0.1 | 0.1 | 0.06 ± 0.01 | >67.3 |
| | | | | 0.05 | | | | |
| BxPC-3 | Pancreatic cancer | 0.09 | 0.10 | 0.09 ± 0.01 | 0.2 | 0.2 | 0.17 ± 0.01 | >67.3 |
| HT1376 | Bladder cancer | 0.24 | 0.20 | 0.22 ± 0.03 | 1.0 | 0.9 | 0.98 ± 0.08 | >67.3 |
| NCI-N87 | Gastric cancer | 0.40 | 0.54 | 0.47 ± 0.10 | 0.4 | 0.5 | 0.47 ± 0.07 | >67.3 |
| Calu-3 | Lung cancer | 3.20 | 3.50 | 3.35 ±0.21 | 0.4 | 0.4 | 0.40 ± 0.02 | >67.3 |
| MDA-MB-468 | Triple negative breast cancer | 7.26 | 5.13 | 6.20 ± 1.51 | 0.2 | 0.2 | 0.16 ± 0.01 | >67.3 |
| MCF-7 | ER-positive breast cancer | >65.4 | >65.4 | >65.4 | 0.2 | 0.1 | 0.11 ± 0.08 | >67.3 |
| Colo 205 | Colon cancer | >65.4 | >65.4 | >65.4 | 0.2 | 0.2 | 0.21 ± 0.05 | >67.3 |
| BT-20 | Triple negative breast cancer | >65.4 | >65.4 | >65.4 | 0.5 | 0.5 | 0.53 ± 0.01 | >67.3 |
| BT-474 | HER2-positive breast cancer | >65.4 | >65.4 | >65.4 | 2.3 | 0.5 | 1.43 ± 1.25 | >67.3 |
| MDA-MB-231 | Triple negative breast cancer | >654 | >654 | >654 | 0.8 | 0.8 | 0.69 ± 0.15 | >673 |

(continued)

| | | IC50 (nM) | | | | | | |
|---|---|---|---|---|---|---|---|---|
| Cell strain | Pathologic type | Trop2-ADC-14 | | | Compound 3 | | | h23-12 |
| | | 1st experiment | 2nd experiment | Mean ± SD | 1st experiment | 2nd experiment | Mean ± SD | |
| HT-29 | Colon cancer | >654 | >654 | >654 | 0.4 | 0.4 | 0.40 ± 0.01 | >673 |

**Example 5.10** Study of the internalization function of antibody-drug conjugates targeting Trop-2

BxPC-3 cells (purchased from: ATCC) were inoculated into 6-well plates, and fluorescently labeled Trop2-ADC-14 and the mAb in Trop2-ADC-14 (i.e., antibody h23-12) each at 1 μg/mL were added thereto respectively. The cells were incubated at 37 °C for 3, 6, 12, 18, 24, and 48 hours respectively, and then were digested with trypsin. The Trop2-ADC-14 and the mAb h23-12 not internalized were washed away, and fluorescence intensity was measured with a flow cytometer (BD ACCURI C6 PLUS). The experiment was repeated once. Results are shown in FIG. 6.

It can be seen from the study that, upon incubated with BxPC-3 cells positive for Trop-2, fluorescence labeled Trop2-ADC-14 and h23-12 were internalized into the cells. The internalization was time-dependent, and the amount of internalized drugs increased over time, and Trop2-ADC-14 was internalized more at 24 hour than antibody h23-12.

**Example 5.11** Study of the apoptosis-inducing effect of antibody-drug conjugates targeting Trop-2 and camptothecins

BxPC-3 cells (purchased from: ATCC) were inoculated into 6-well plates, and then treated with Trop2-ADC-14 (0.020, 0.196, and 1.963 nM), DS-1062 (0.020, 0.196, and 1.963 nM), the mAb in Trop2-ADC-14 (i.e., antibody h23-12) (6.728 nM) and Compound 3 (0.1, 1, and 10 nM) for 120 hours. Next, the cells were harvested, and Annexin-V-FITC and PI were added thereto, and the cells were stained at room temperature for 15 minutes in dark, and finally were re-suspended in the added 300 μl of 1 × binding buffer. Apoptosis was detected with a flow cytometer (BD AccuriTMC6 Plus flow cytometer), for which 1 $\times 10^4$ cells were gated for each group. Experimental data was analyzed using software BD CSampler™ Plus C6. Results are shown in FIG. 7.

It can be seen from the study that Trop 2-ADC-14, even at 0.196 nM, obviously induced the degradation of protein pro-PARP which is apoptosis marker, induced the hydrolysis of pro-caspase 3 into active protein caspase 3 (cleared-caspase 3), and its apoptosis-inducing effect was concentration dependent. The apoptosis-inducing effect of Trop2-ADC-14 was obviously stronger than that of DS-1062a at the same concentration, which showed a non-apparent apoptosis-inducing effect at 0.196 nM. The small-molecule compound i.e., Compound 3 induced apoptosis of BxPC-3 cells in a concentration-dependent manner as well, while antibody h23-12 showed no significant apoptosis-inducing effect on BxPC-3 cells.

**Example 5.12** Study of the binding activity of antibody-drug conjugates targeting Trop-2 to cells positive for Trop-2

Different concentrations (1, 3, 10, 30, 100, 300, 1000, 3000, 10000, 30000, and 100000 ng/mL) of fluorescently labeled Trop2-ADC-14 and the mAb in Trop2-ADC-14 (i.e., antibody h23-12) were incubated with BxPC-3 cells (purchased from: ATCC) on ice for 1 hour in dark, and then the cells were centrifuged to remove supernatant, washed with PBS, re-suspended, and the fluorescence intensity of the drugs bound to the cells was measured with a flow cytometer (BD ACCURI C6 PLUS). The experiment was repeated once. Results are shown in Table 17 and FIG. 8.

Table 17. Binding of Trop2-ADC-14 and h23-12 to BxPC-3 cells assessed by EC50 (mean ± SD, n = 2)

| Drug | EC50 (ng/mL, mean ± SD) |
|---|---|
| Trop2-ADC-14 | 1296.0 ± 155.6 |
| Antibody h23-12 | 1137.5 ± 128.0 |

It can be seen from the study that, both Trop2-ADC-14 and antibody h23-12 exhibited concentration-dependent binding to the Trop-2-positive BxPC-3 tumor cells, and their EC50 values was 1296.0 ± 155.6 ng/mL and 1137.5 ± 128.0 ng/mL respectively, indicating that their binding capacity to cells positive for Trop-2 is at a comparable level. As

a contrast, both Trop2-ADC-14 and antibody h23-12 had no obvious binding to the Trop-2- negative HT-29 cells, indicating that the binding of Trop2-ADC-14 and antibody h23-12 to tumor cells is dependent on the expression level of Trop-2.

The above description of the embodiments of the present disclosure is not intended to limit the present disclosure, and those skilled in the art may make various changes and modifications to the present disclosure without departing from the spirit of the present disclosure, which should fall within the scope of the appended claims.

## Claims

1. A compound represented by structural formula I or a pharmaceutically acceptable salt, stereoisomer, solvate or prodrug thereof:

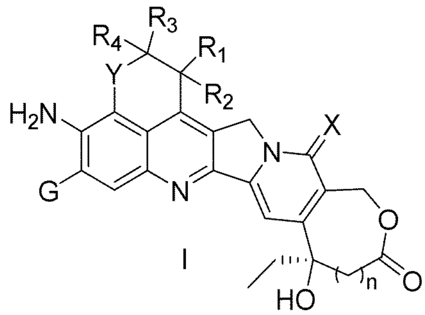

structural formula I,

in structural formula I, $R_1$, $R_2$, $R_3$, and $R_4$ independently of one another are selected from the group consisting of hydrogen, halogen, hydroxyl, C1-6 alkoxy, amino or substituted amino, C1-7 alkyl or substituted C1-7 alkyl, or any two of $R_1$, $R_2$, $R_3$, and $R_4$, together with the carbon atoms to which they are bonded, form a C3-6 cycloalkyl;
G is hydrogen, halogen, methyl or methoxy;
Y is oxygen, sulfur, sulfone, sulfoxide, methylene, or substituted methylene;
X is oxygen or sulfur;
n = 0 or 1.

2. The compound or a pharmaceutically acceptable salt, stereoisomer, solvate or prodrug thereof according to claim 1, characterized that, the compound is a compound represented by structural formula IA:

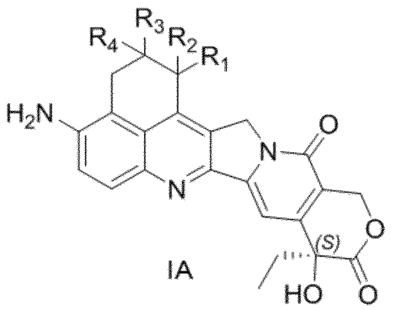

structural formula IA,

in structural formula IA, $R_1$, $R_2$, $R_3$, and $R_4$ are not hydrogen simultaneously.

3. A compound represented by structural formula II or a pharmaceutically acceptable salt, stereoisomer, solvate or prodrug thereof:

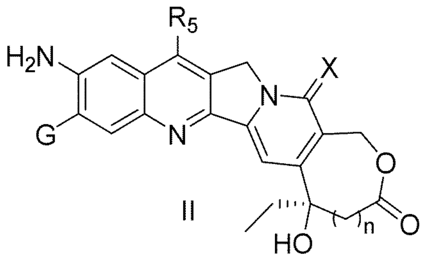

II

structural formula II,

in structural formula II, $R_5$ is C1-5 alkyl or C1-5 alkyl substituted with one or more substituents, C3-6 cycloalkyl, or C3-6 cycloalkyl substituted with one or more substituents, phenyl or substituted phenyl;
G is hydrogen, halogen, methyl, or methoxy;
X is oxygen or sulfur;
n = 0 or 1;
when X is oxygen, G is hydrogen, and n = 0, $R_5$ is not n-butyl.

4. The compound or a pharmaceutically acceptable salt, stereoisomer, solvate or prodrug thereof according to claim 3, characterized that, the compound is a compound represented by structural formula IIA:

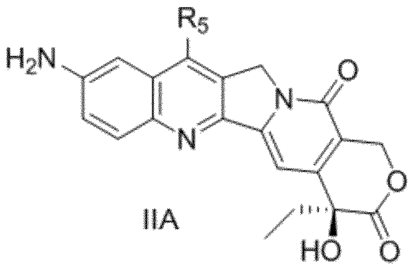

IIA

structural formula IIA,

in structural formula IIA, $R_5$ is not n-butyl.

5. The compound or a pharmaceutically acceptable salt, stereoisomer, solvate or prodrug thereof according to any one of claims 1 to 4, characterized that, the compound has one of the following structures:

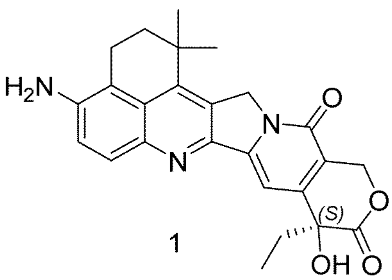

1

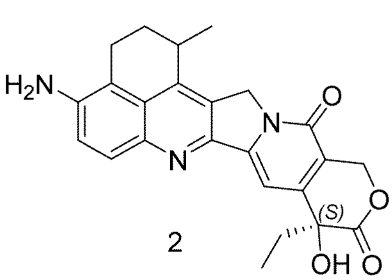

2

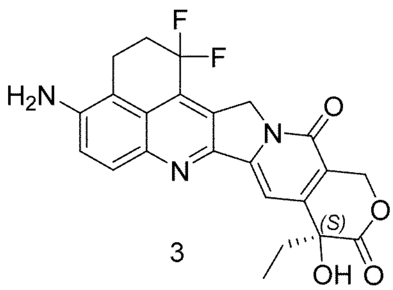

3

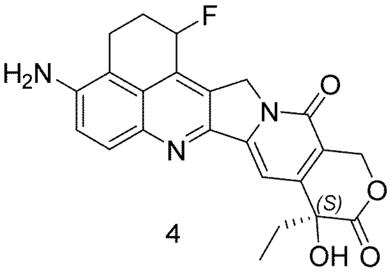
4

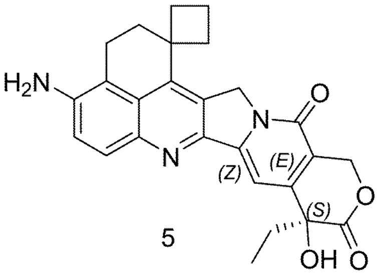
5

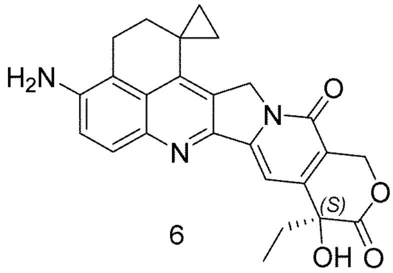
6

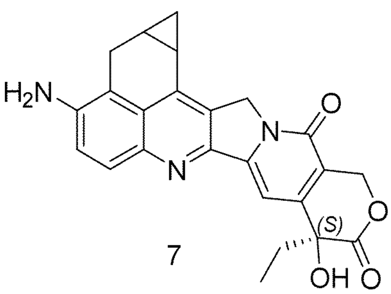
7

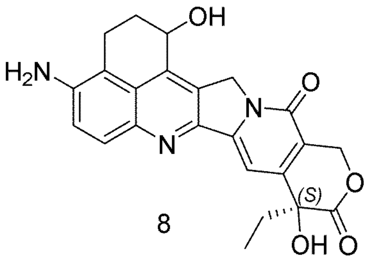
8

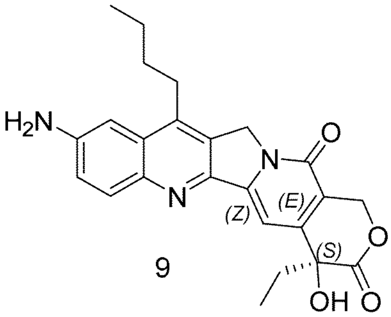
9

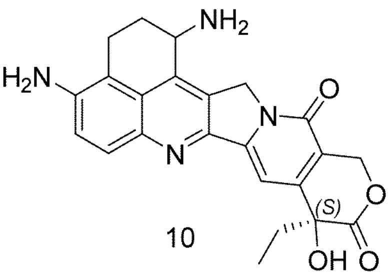
10

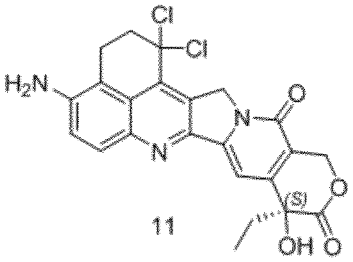
11

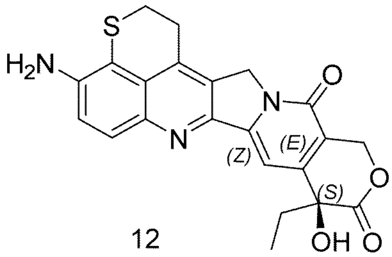
12

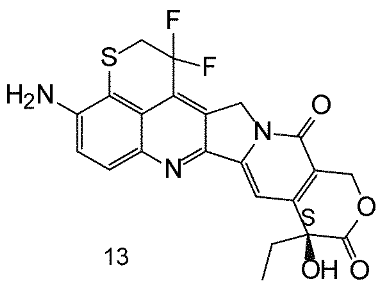
13

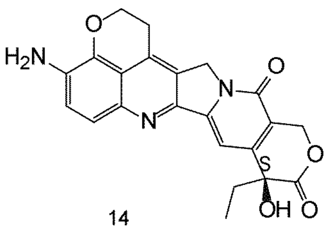
14

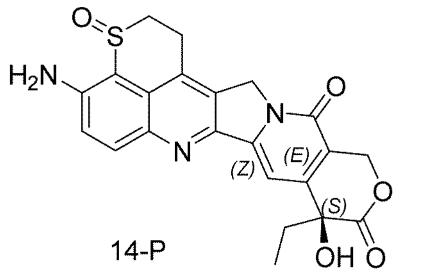
14-P

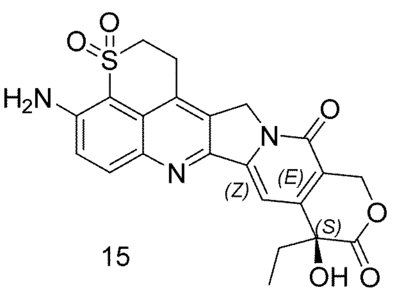
15

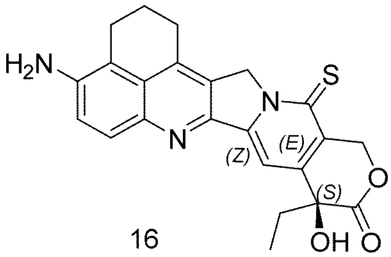
16

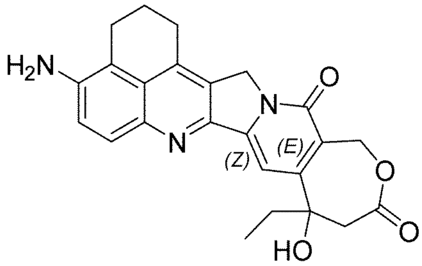
17

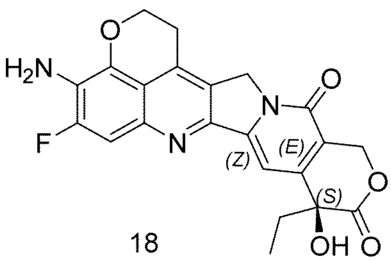
18

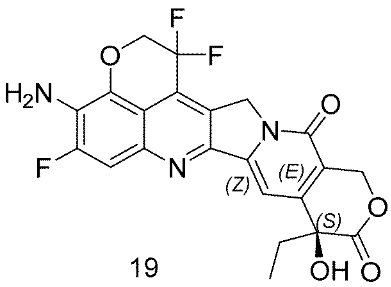
19

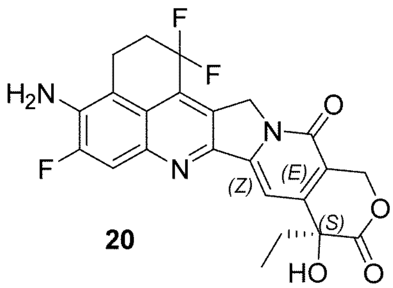
**20**

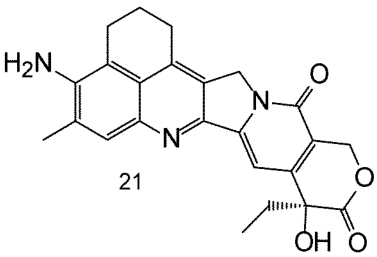
21

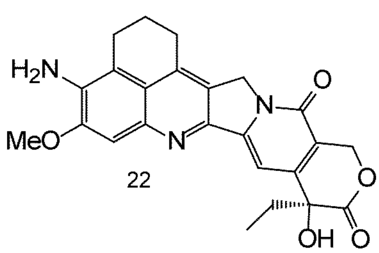
22

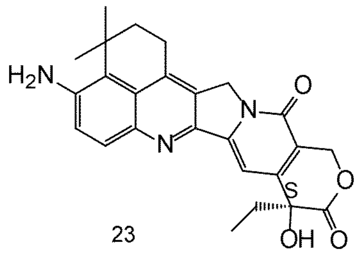
23

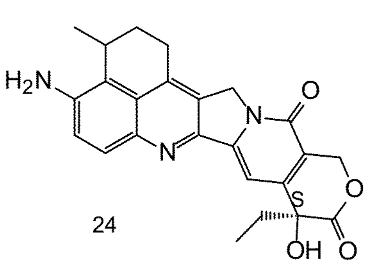
24

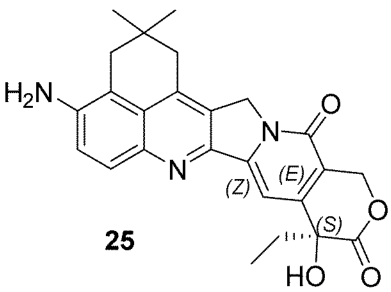
**25**

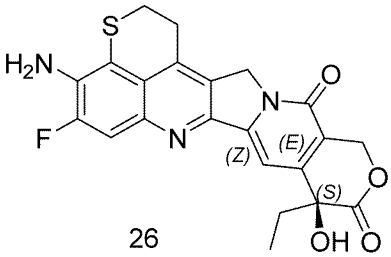
26

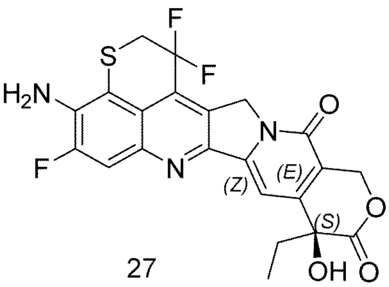
27

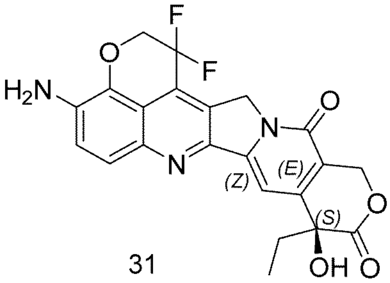
31

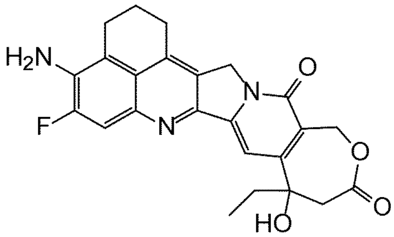
32

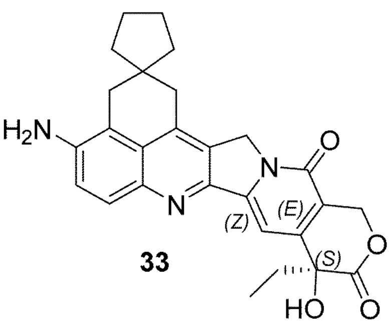
**33**

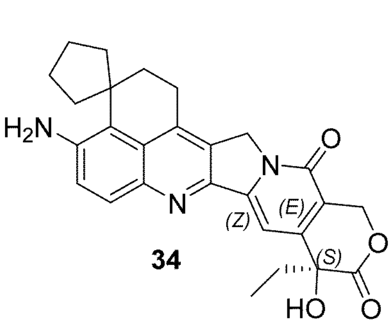
**34**

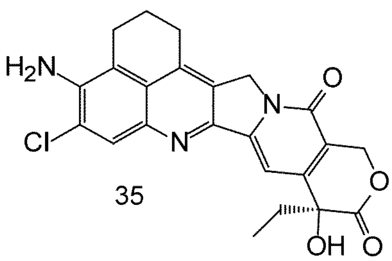
35

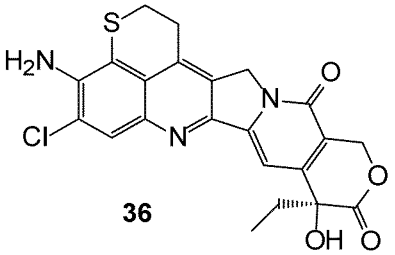

36

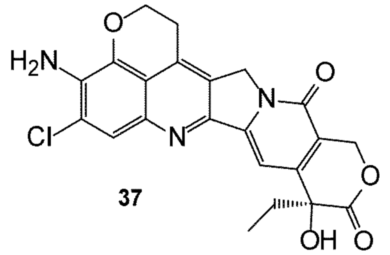

37

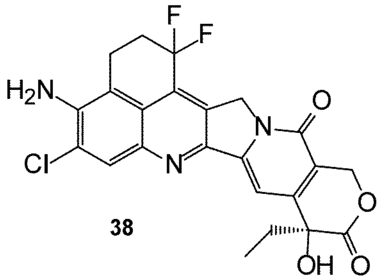

38

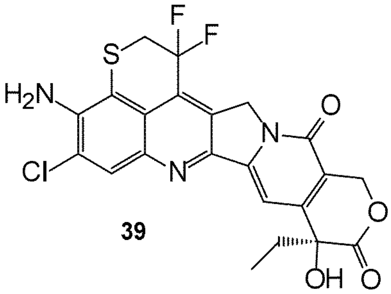

39

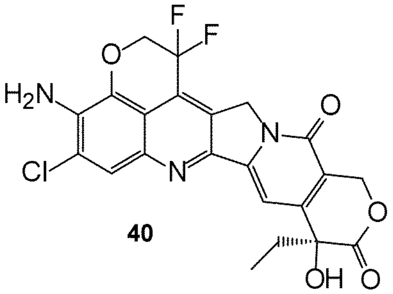

40

.

**6.** A compound represented by structural formula III or a pharmaceutically acceptable salt, stereoisomer, solvate or prodrug thereof:

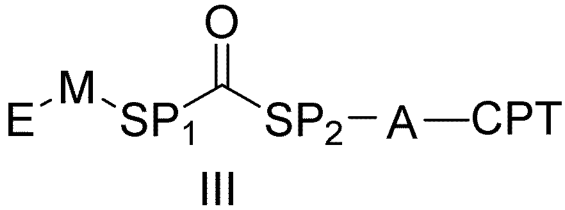

III

structural formula III,

in structural formula III, E is selected from the group consisting of the following groups, in which ~~~ means the bonding site to M:

E-1: 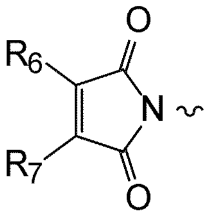 ; E-2: 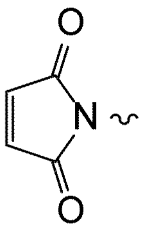 ; E-3: 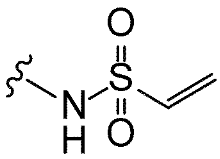 ;

E-4: 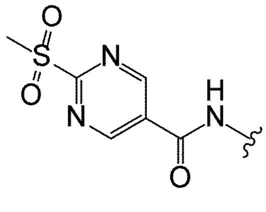 ; E-5: 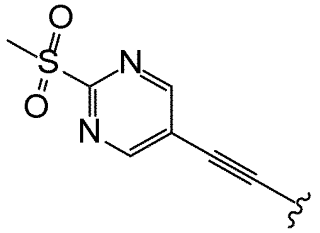 ; and E-6:

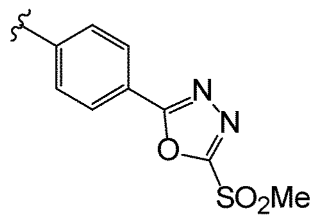

;

M is phenylene or phenylene substituted with one or more substituents, or a chemical bond; and in the substituted phenylene, the phenylene is substituted with substituent(s) selected from the group consisting of alkyl, haloalkyl, alkoxy, halogen, ester, amide, and cyano; and preferably, M is halogen-substituted phenylene;
$SP_1$ is selected from the group consisting of C1-8 alkylene, C1-8 cycloalkylene, or C1-21 linear heteroalkylene comprising 1-11 heteroatoms selected from the group consisting of N, O, and S, in which the C1-8 alkylene, Cl-8 cycloalkylene, and C1-21 linear heteroalkylene independently of one another are optionally substituted with one or more substituents selected from the group consisting of hydroxyl, amino, sulfonic acid group, and cyano;
$SP_2$ is selected from the group consisting of -NH(CH2CH2O)aCH2CH2CO-, -NH(CH2CH2O)aCH2CO-, -S(CH2)aCO-, or a chemical bond, in which a is an integer in the range of 1 to 20, preferably an integer in the range of 1 to 10, more preferably an integer in the range of 1 to 6;
A means a peptide group of 2 to 4 amino acids;
CPT is a compound of camptothecins.

**7.** The compound or a pharmaceutically acceptable salt, stereoisomer, solvate or prodrug thereof according to claim 6, characterized that, the compound is a compound represented by structural formula IIIA:

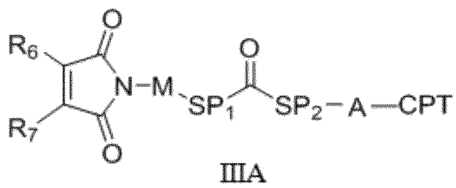

IIIA

structural formula IIIA,

in structural formula IIIA, $R_6$ and $R_7$ independently of one another are hydrogen, halogen or Ar'S in which Ar' is phenyl or phenyl substituted with one or more substituents; and in the substituted phenyl, the phenyl is substituted with substituent(s) selected from the group consisting of alkyl, alkoxy, halogen, ester, amide, and cyano;
preferably, Ar' is phenyl, phenyl substituted with 4-formylmethylamine

( 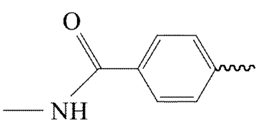 )

or phenyl substituted with 4-formylmorpholine

( 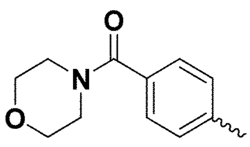 ).

**8.** The compound or a pharmaceutically acceptable salt, stereoisomer, solvate or prodrug thereof according to claim 6 or 7, characterized that, in structural formula III or structural formula IIIA, CPT is a compound represented by

structural formula I or a pharmaceutically acceptable salt, stereoisomer, solvate or prodrug thereof:

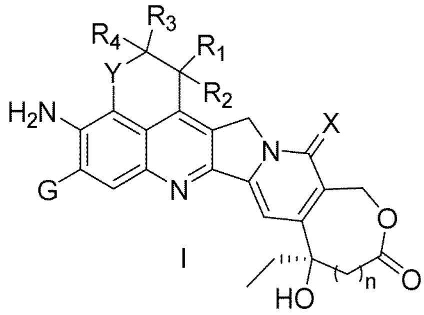

structural formula I,

in structural formula I, groups G, X, Y, $R_1$, $R_2$, $R_3$, $R_4$, and n have the same meaning as defined in claim 1 for the groups G, X, Y, $R_1$, $R_2$, $R_3$, $R_4$, and n;
preferably, CPT is a compound represented by structural formula IA or a pharmaceutically acceptable salt, stereoisomer, solvate or prodrug thereof:

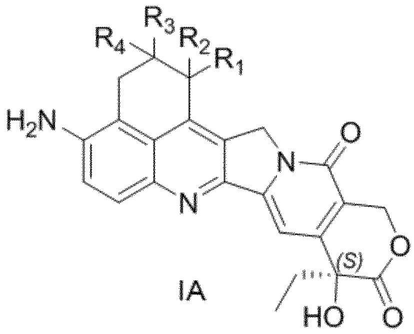

structural formula IA,

in structural formula IA, groups $R_1$, $R_2$, $R_3$, and $R_4$ have the same meaning as defined in claim 2 for the groups $R_1$, $R_2$, $R_3$, and $R_4$, but $R_1$, $R_2$, $R_3$, and $R_4$ can be hydrogen simultaneously;
in structural formula III or structural formula IIIA, the compound represented by structural formula I or structural formula IA is bonded to the carboxyl of A through an amide bond via its amino.

**9.** The compound or a pharmaceutically acceptable salt, stereoisomer, solvate or prodrug thereof according to claim 6 or 7, characterized that, in structural formula III or structural formula IIIA, CPT is a compound represented by structural formula II or a pharmaceutically acceptable salt, stereoisomer, solvate or prodrug thereof:

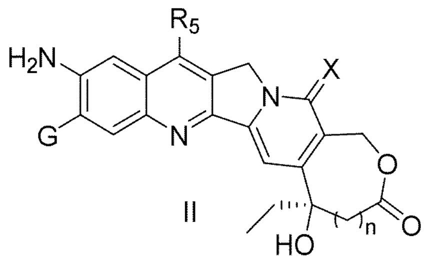

structural formula II,

in structural formula II, groups G, $R_5$, X, and n have the same meaning as defined in claim 3 for the groups G, $R_5$, X, and n;

preferably, CPT is a compound represented by structural formula IIA or a pharmaceutically acceptable salt, stereoisomer, solvate or prodrug thereof:

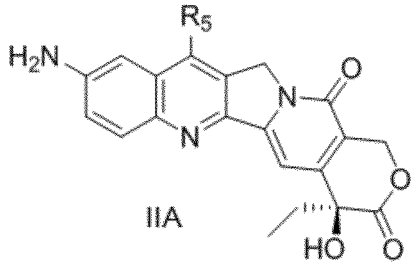

structural formula IIA,

in structural formula IIA, group $R_5$ has the same meaning as defined in claim 4 for group $R_5$, but group $R_5$ can be n-butyl.

**10.** The compound or a pharmaceutically acceptable salt, stereoisomer, solvate or prodrug thereof according to claim 6 or 7, characterized that, in structural formula III or structural formula IIIA, CPT is a an Exatecan derivative represented by structural formula IV or a pharmaceutically acceptable salt, stereoisomer, solvate or prodrug thereof:

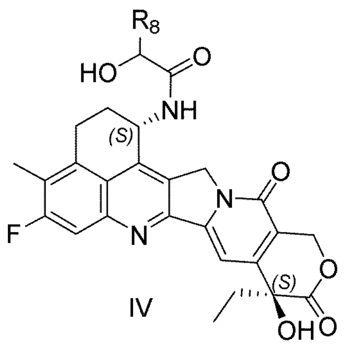

structural formula IV,

in structural formula IV, $R_8$ is hydrogen, trifluoromethyl, C1-5 alkyl or C1-5 alkyl substituted with one or more substituents, C3-6 cycloalkyl, or C3-6 cycloalkyl substituted with one or more substituents, or halogen;
in structural formula III or structural formula IIIA, the compound represented by structural formula IV is bonded to the carboxyl of A through a self-releasing structure via its hydroxyl which is bonded to the same carbon as $R_8$.

**11.** The compound or a pharmaceutically acceptable salt, stereoisomer, solvate or prodrug thereof according to any one of claims 6 to 10, characterized that, the compound represented by structural formula III or structural formula IIIA is further a compound represented by structural formula V:

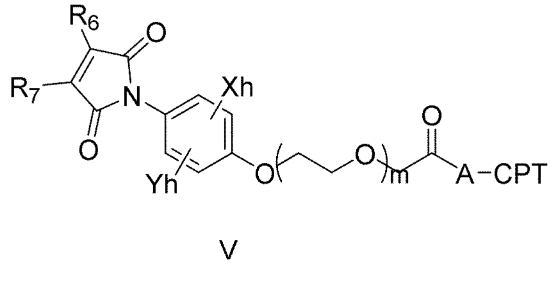

structural formula V,

in structural formula V, $R_6$ and $R_7$ independently of one another are Ar'S in which Ar' is phenyl or phenyl substituted with one or more substituents;
Xh and Yh independently of one another are hydrogen, halogen, haloalkyl, or alkoxy.

**12.** The compound or a pharmaceutically acceptable salt, stereoisomer, solvate or prodrug thereof according to claim 11, characterized that, the compound represented by structural formula V is further a compound represented by structural formula V-A:

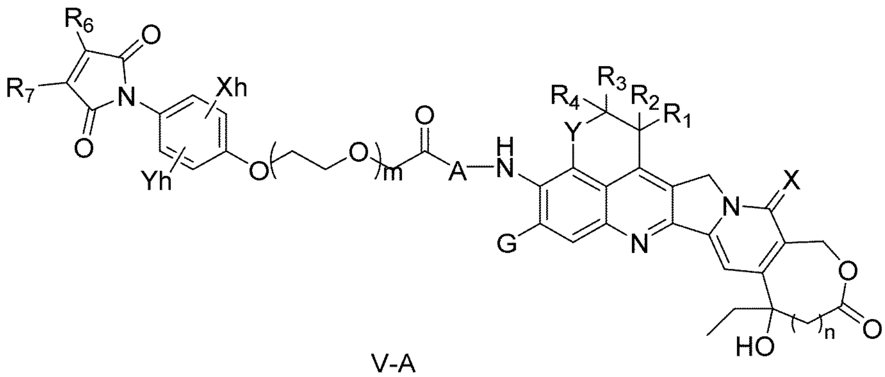

V-A

structural formula V-A;

preferably, the compound represented by structural formula V-A is further a compound represented by structural formula V-A-1:

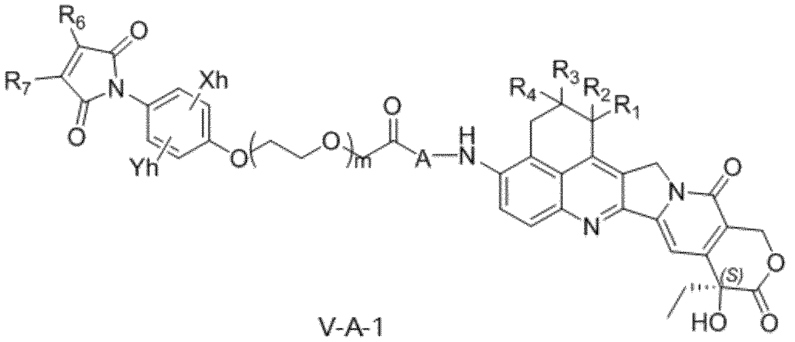

V-A-1

structural formula V-A-1;

alternatively, the compound represented by structural formula V is further a compound represented by structural formula V-B:

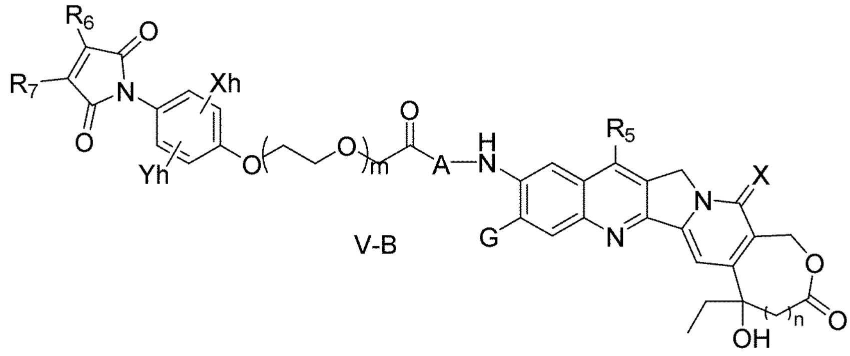

structural formula V-B;

preferably, the compound represented by structural formula V-B is further a compound represented by structural formula V-B-1:

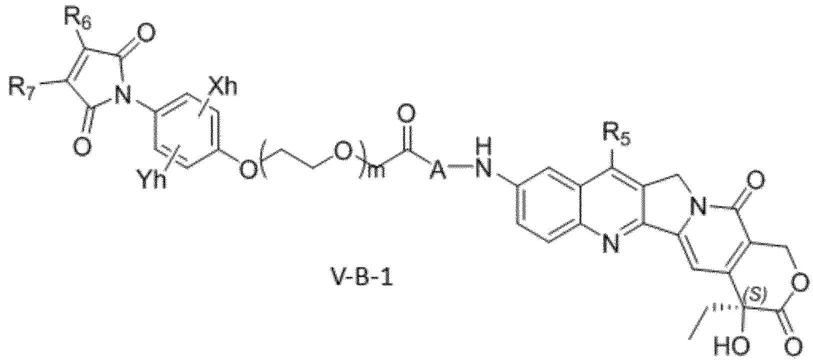

structural formula V-B-1;

alternatively, the compound represented by structural formula V is further a compound represented by structural formula V-C:

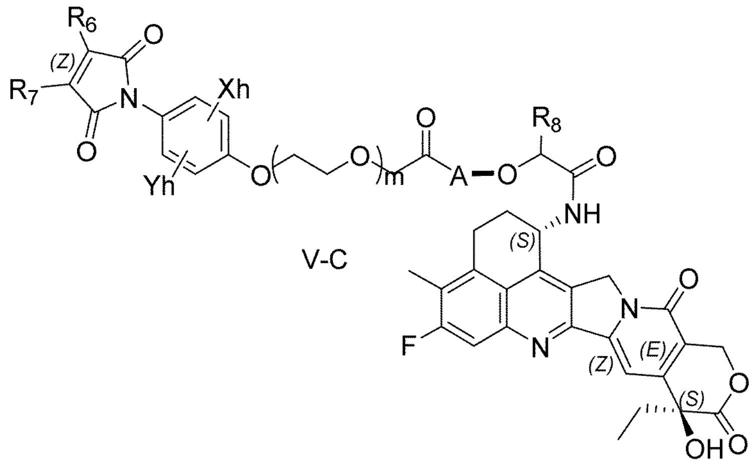

structural formula V-C.

**13.** The compound or a pharmaceutically acceptable salt, stereoisomer, solvate or prodrug thereof according to any one of claims 6 to 12, characterized that, the compound is a compound represented by structural formula VI:

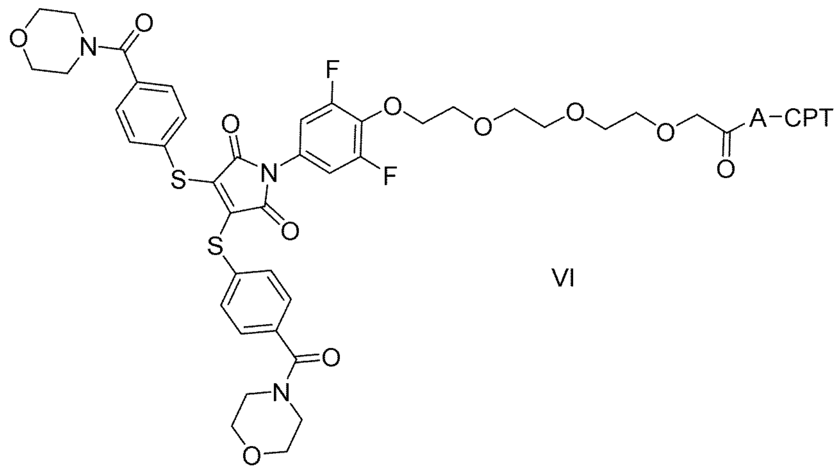

VI

structural formula VI;

preferably, the compound represented by structural formula VI is further a compound represented by structural formula VI-A:

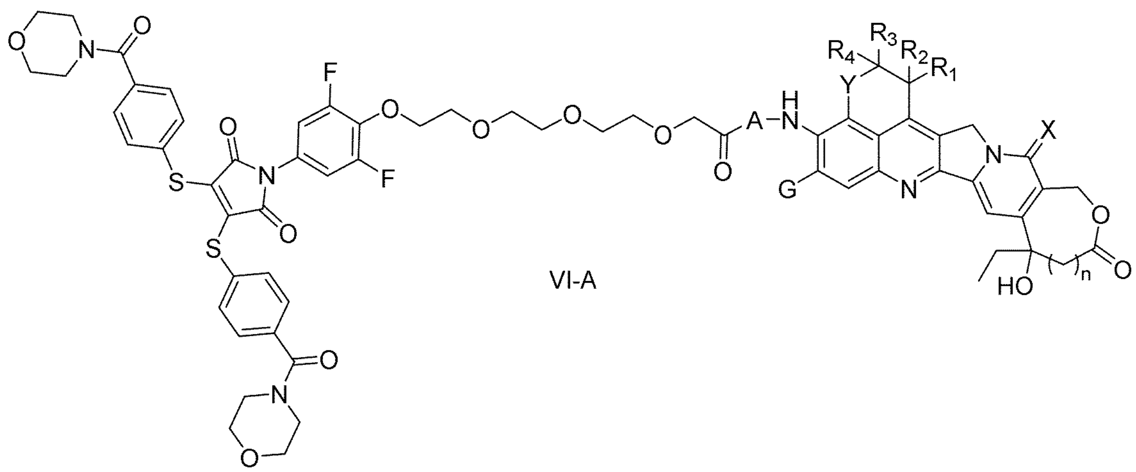

VI-A

structural formula VI-A;

preferably, the compound represented by structural formula VI-A is further a compound represented by structural formula VI-A-1:

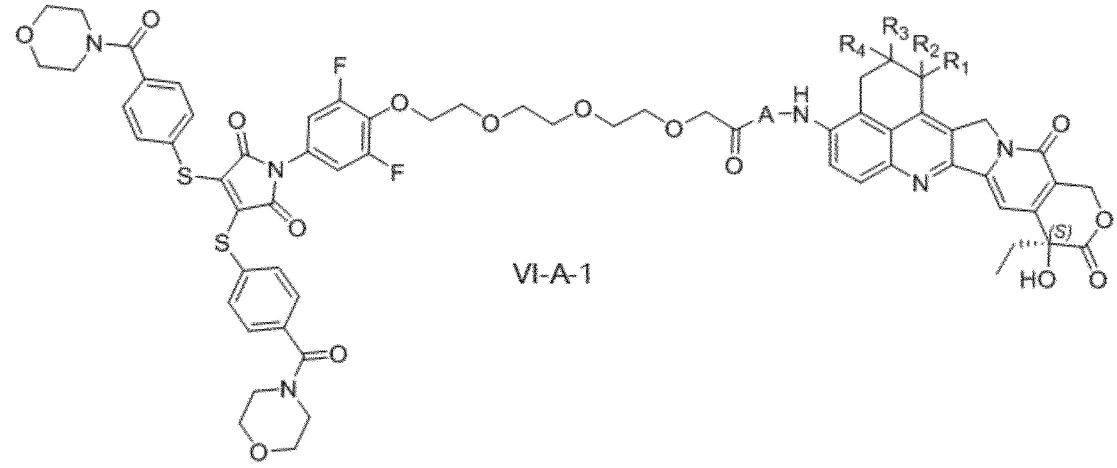

structural formula VI-A-1;

alternatively, the compound represented by structural formula VI is further a compound represented by structural formula VI-B:

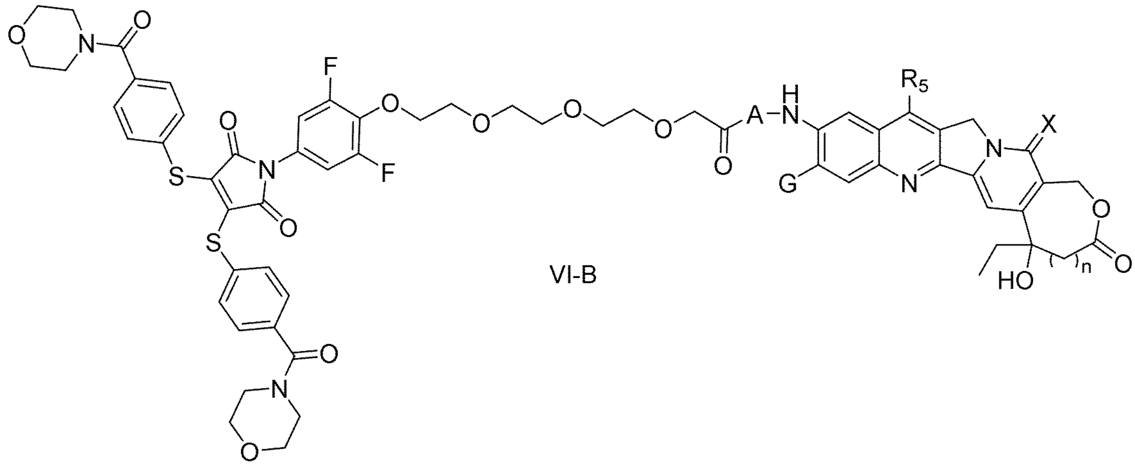

structural formula VI-B;

preferably, the compound represented by structural formula VI-B is further a compound represented by structural formula VI-B-1:

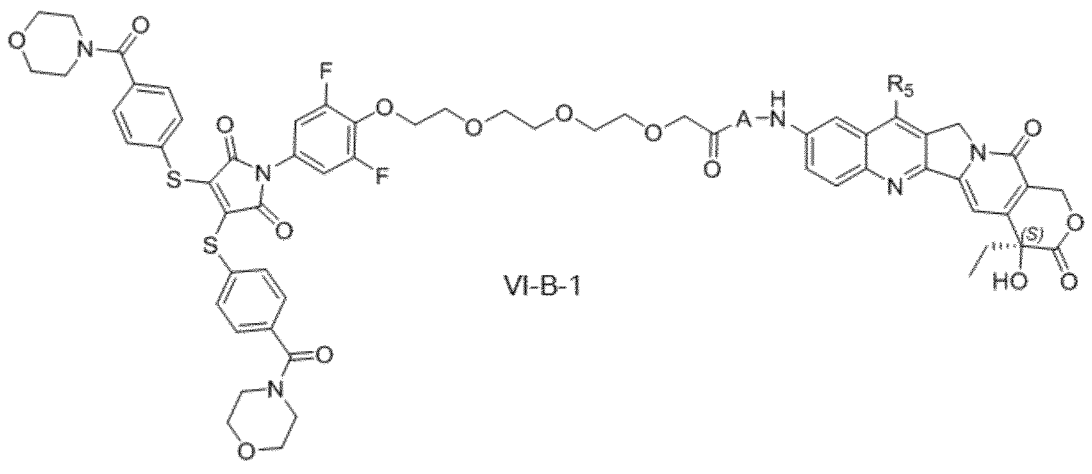

structural formula VI-B-1

alternatively, the compound represented by structural formula VI is further a compound represented by structural formula VI-C:

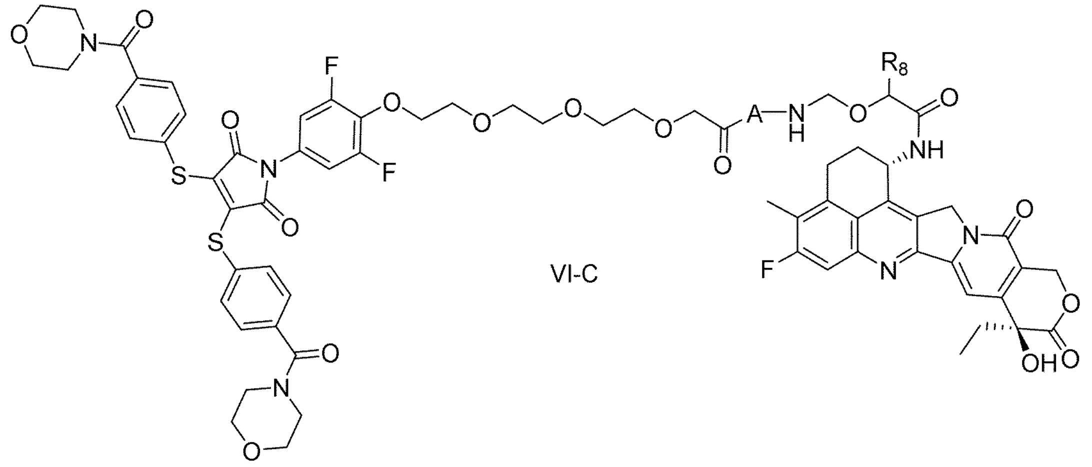

structural formula VI-C.

**14.** The compound or a pharmaceutically acceptable salt, stereoisomer, solvate or prodrug thereof according to any one of claims 6 to 13, characterized that, the compound has one of the following structures:

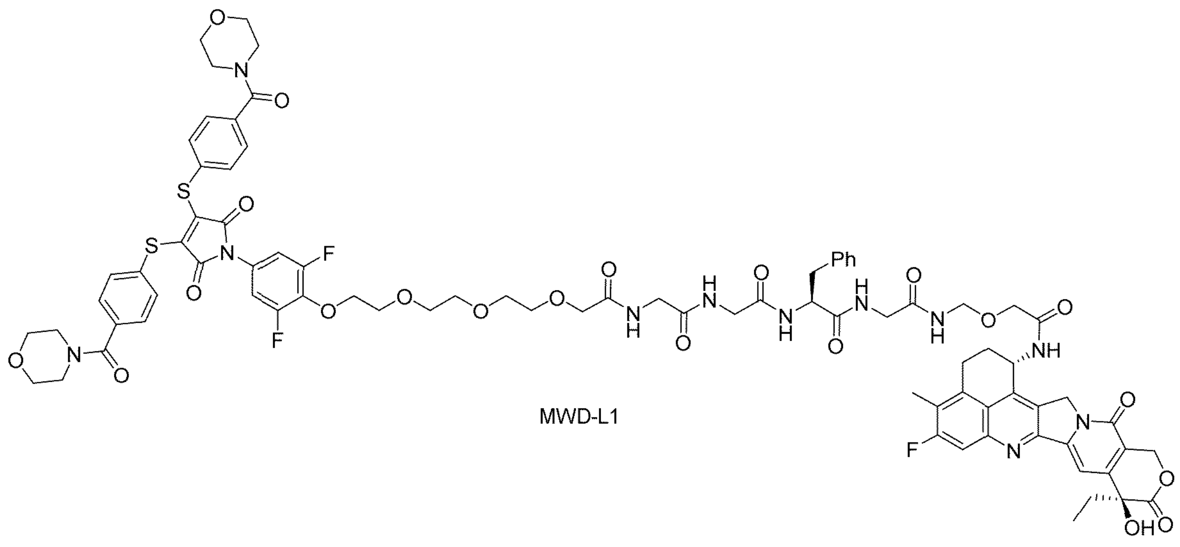

MWD-L1

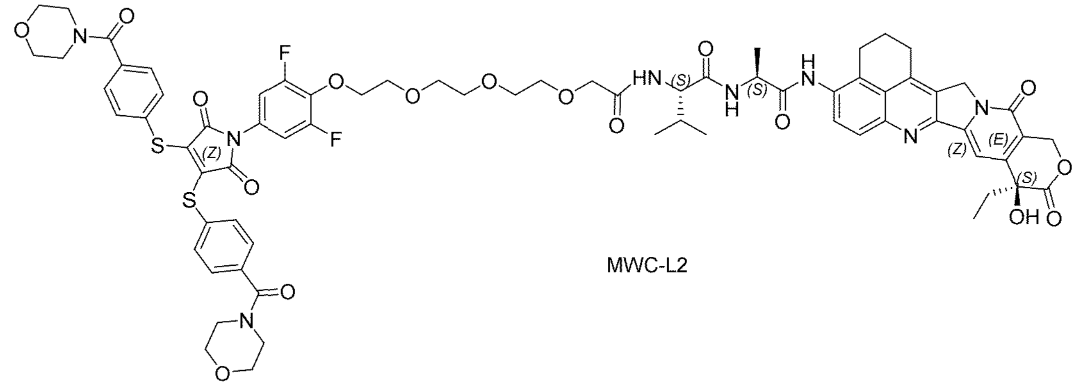

MWC-L2

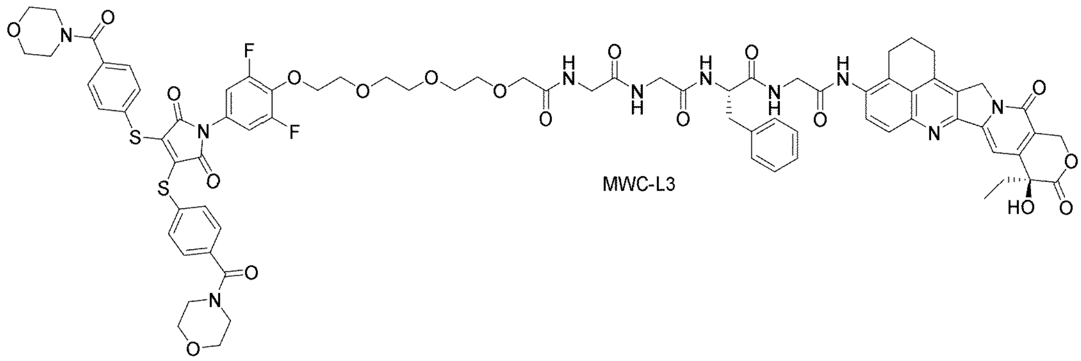

MWC-L3

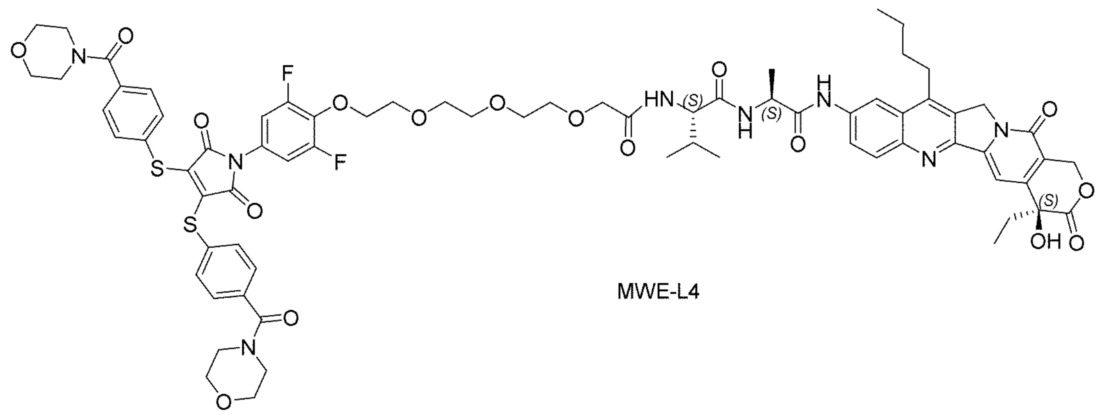

MWE-L4

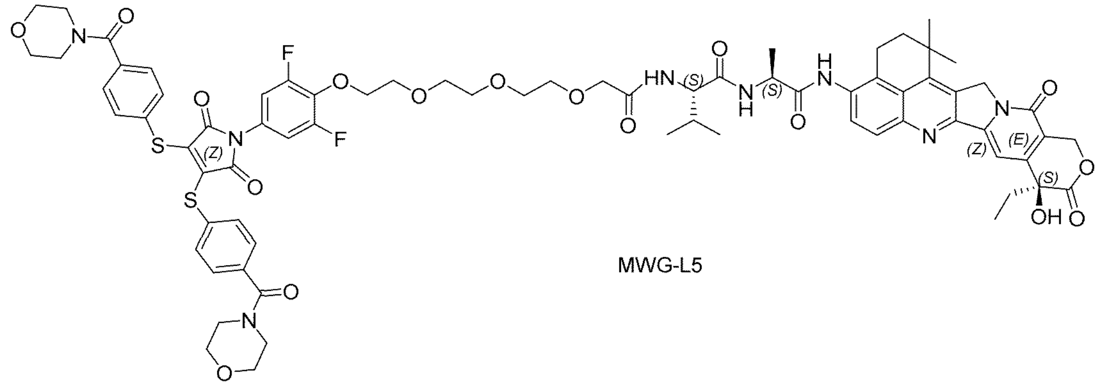

MWG-L5

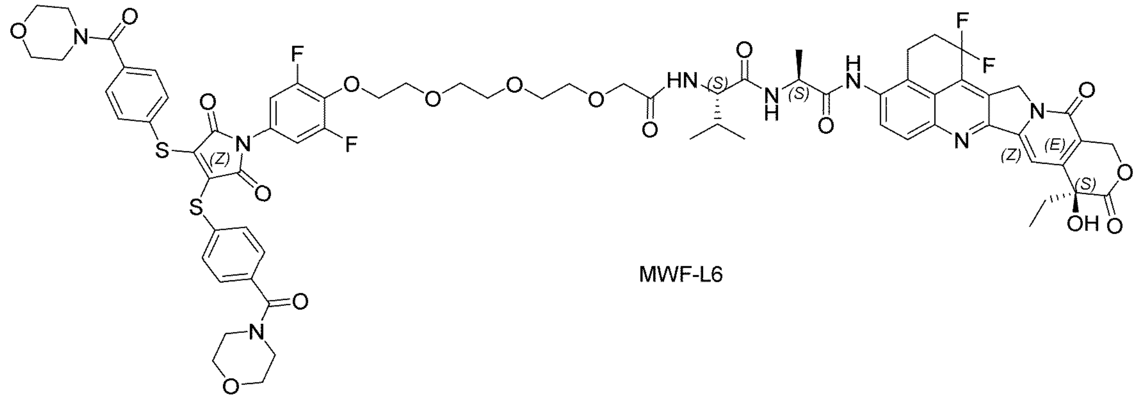

MWF-L6

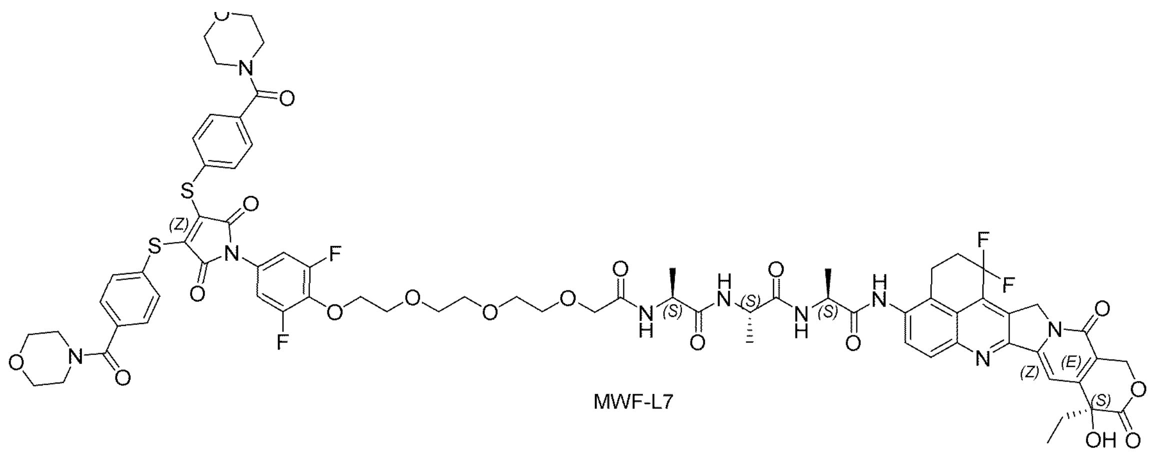

MWF-L7

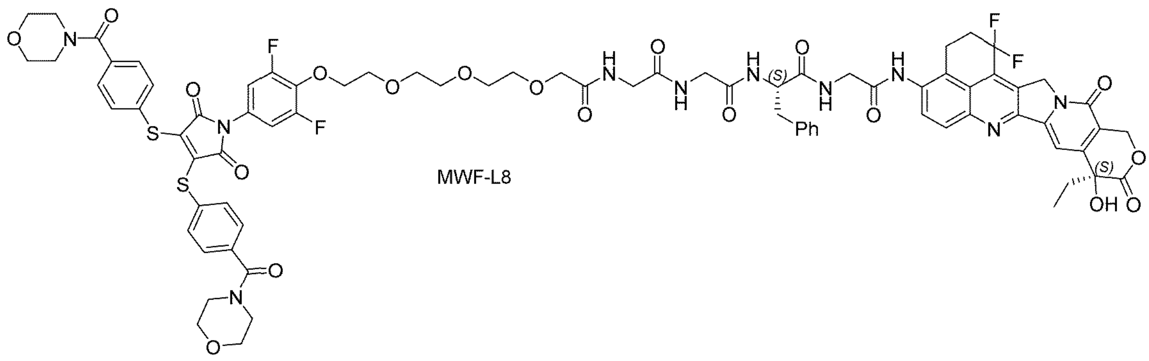

MWF-L8

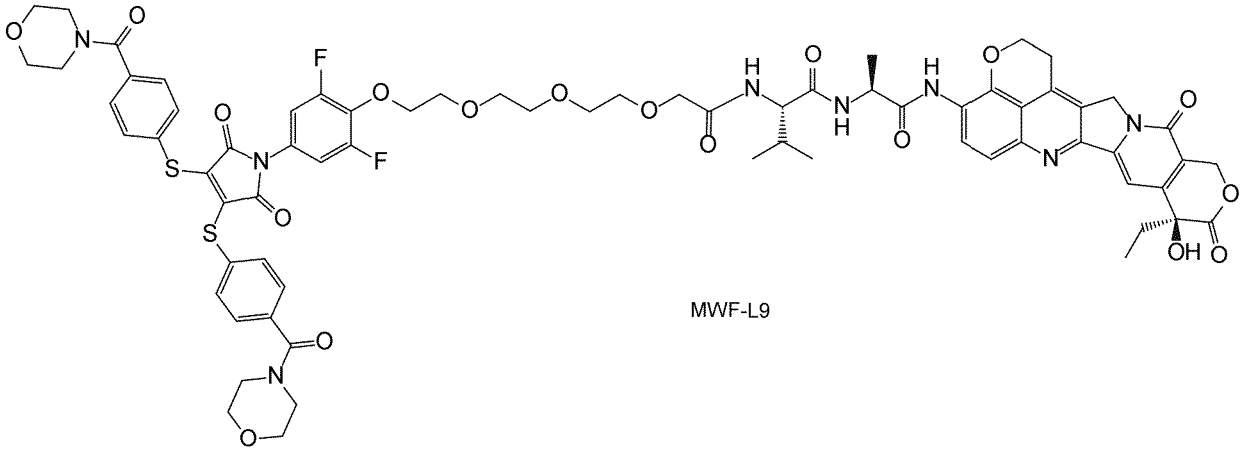

MWF-L9

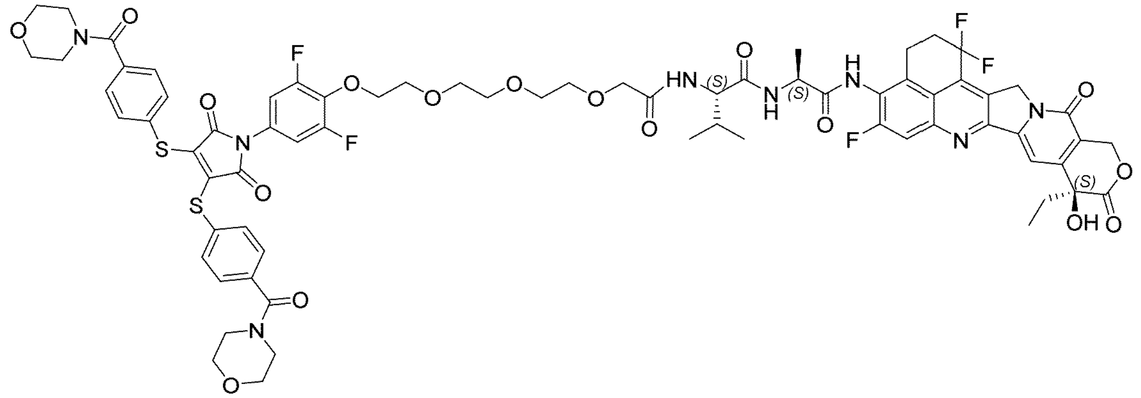

MWF-L10

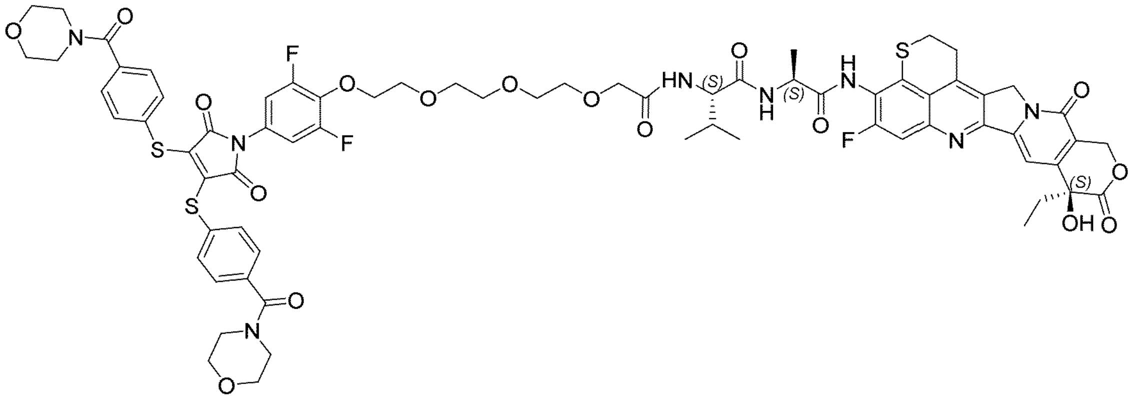

MWF-L11

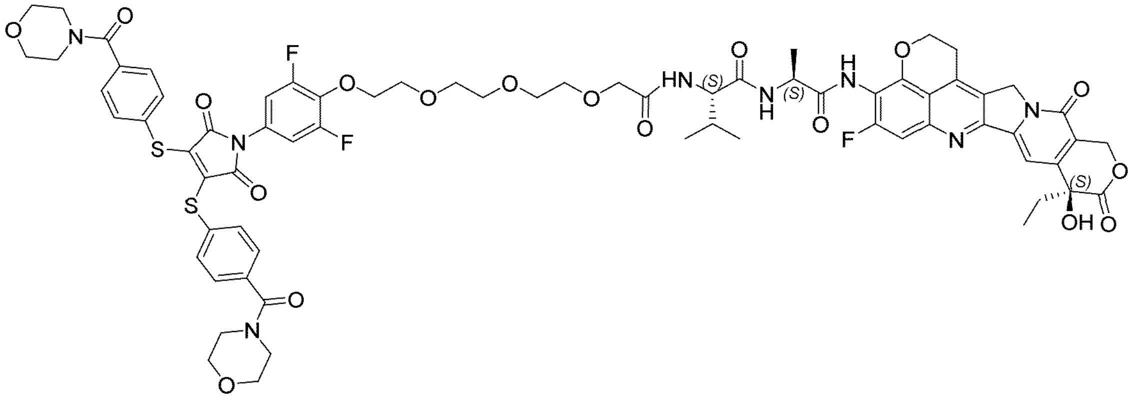

MWF-L12

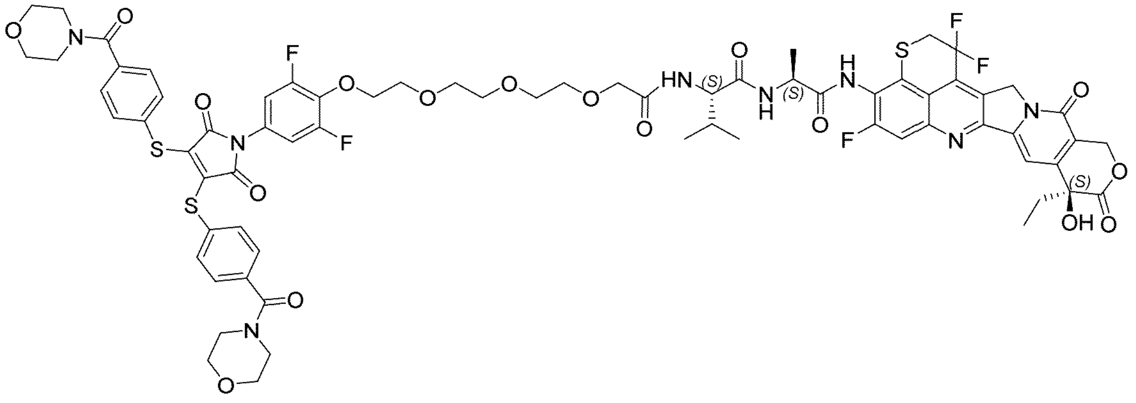

MWF-L13

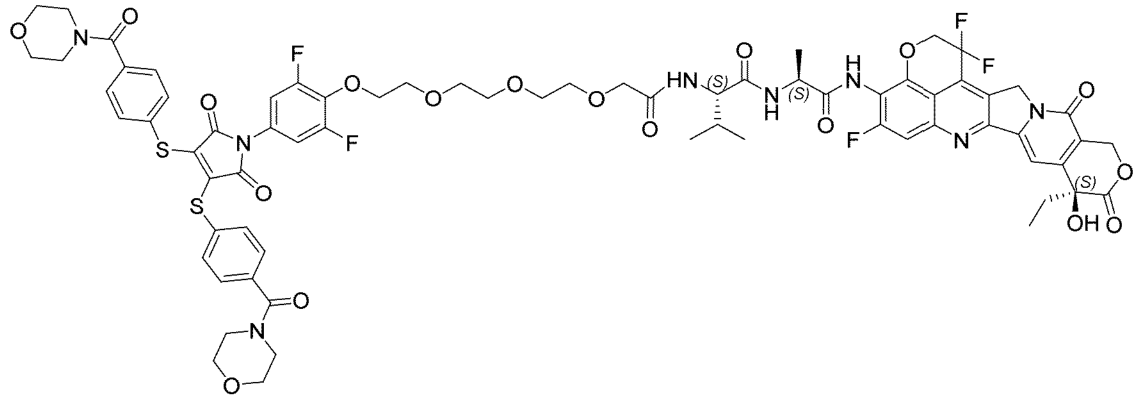

MWF-L14

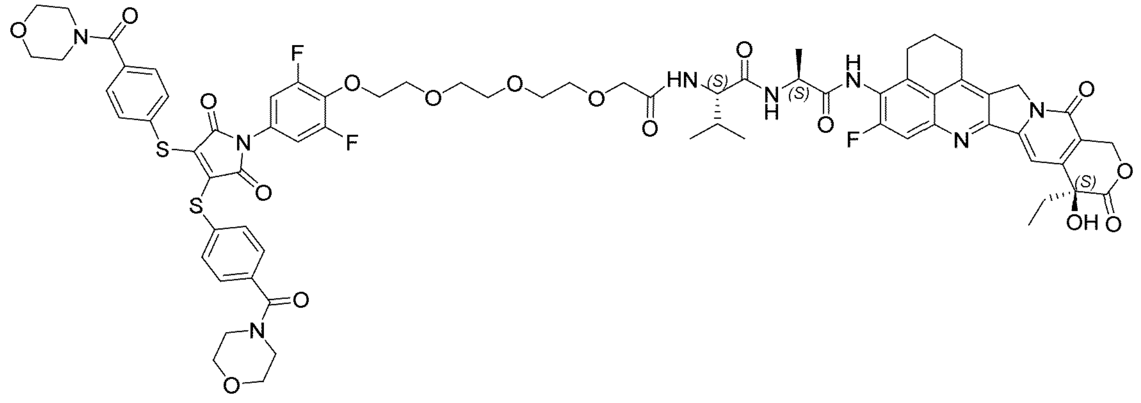

MWF-L15

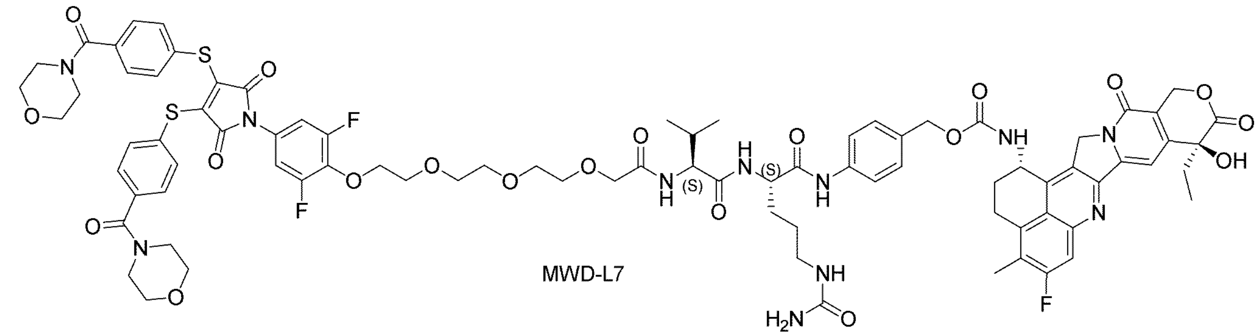

MWD-L7

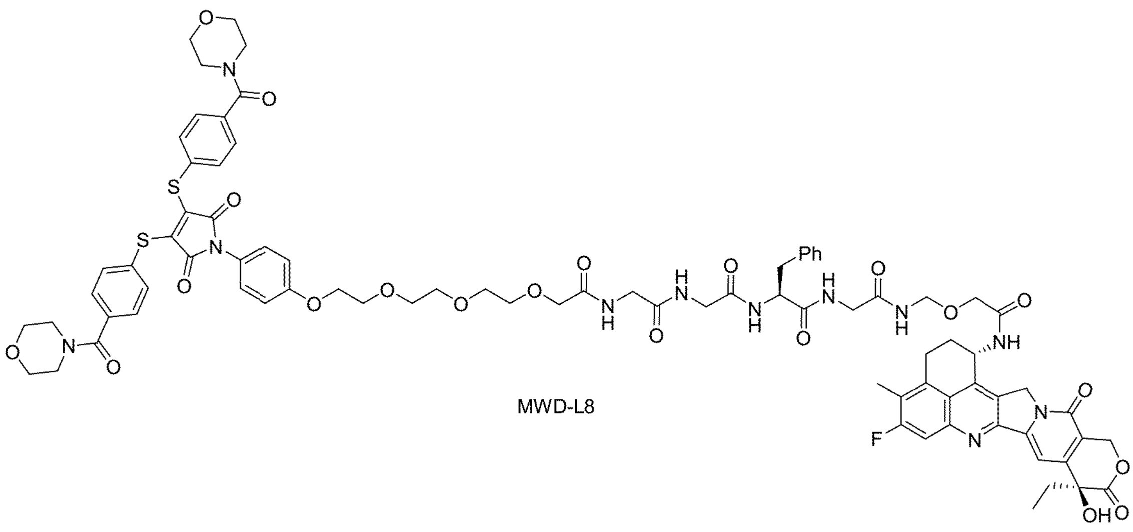

MWD-L8

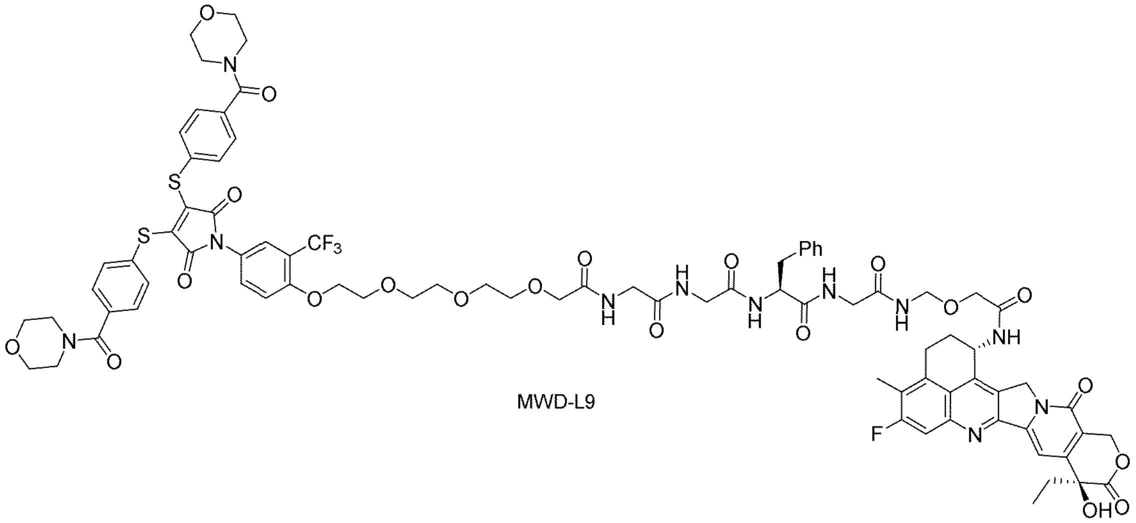

MWD-L9

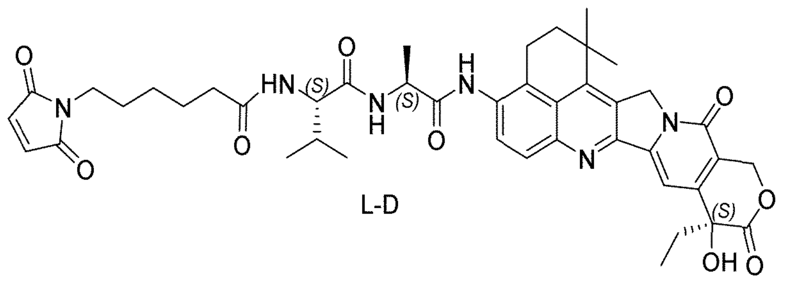

L-D

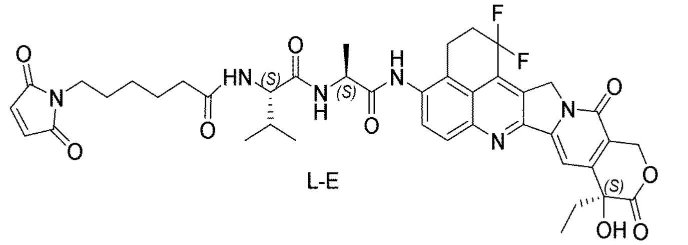

L-E

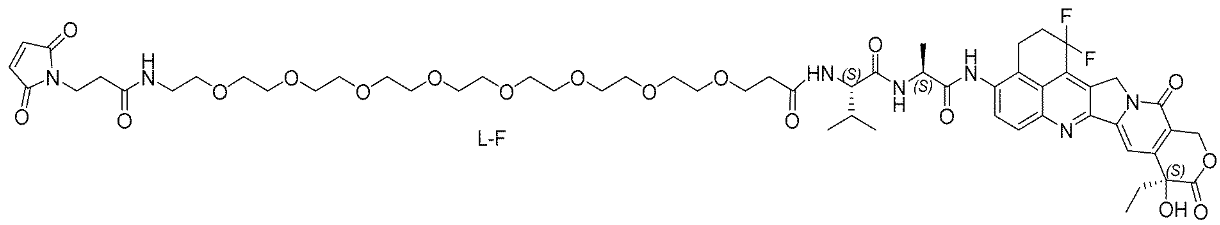

L-F

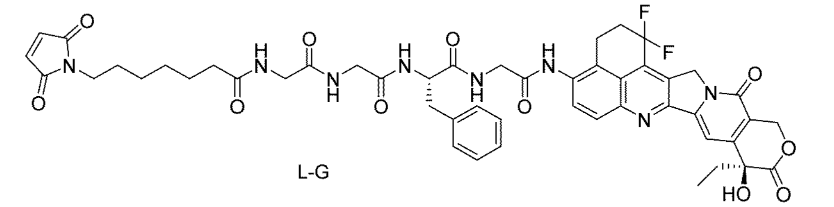

L-G

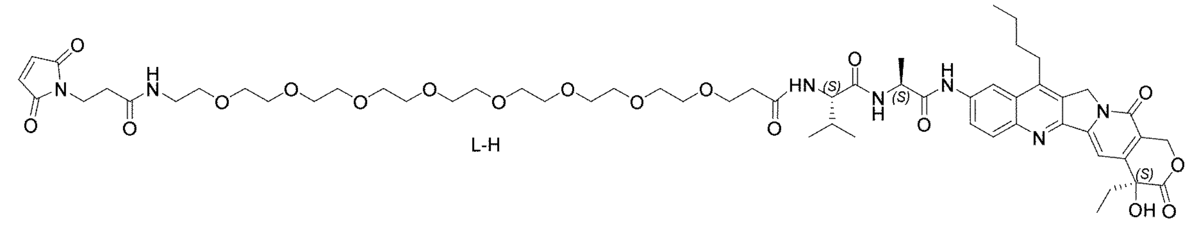

L-H

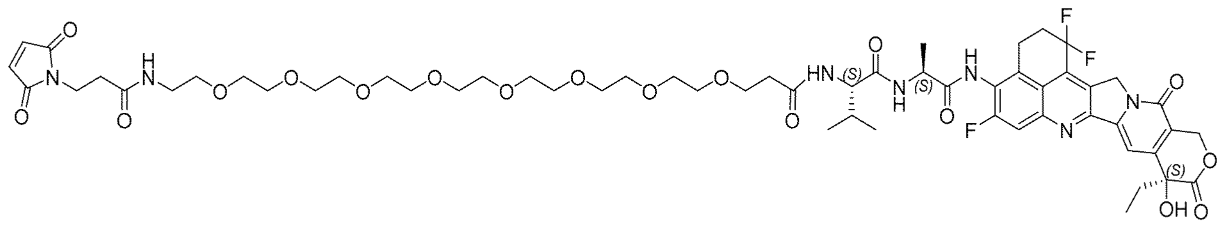

L-I

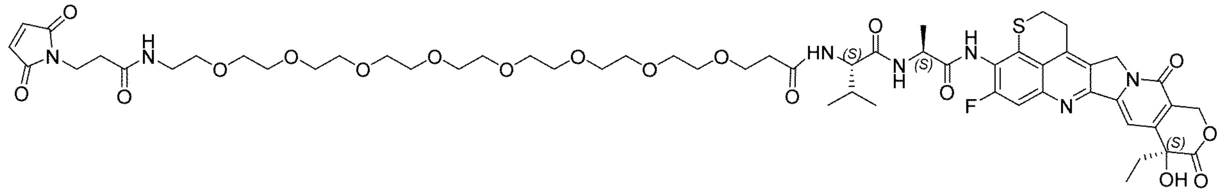

L-J

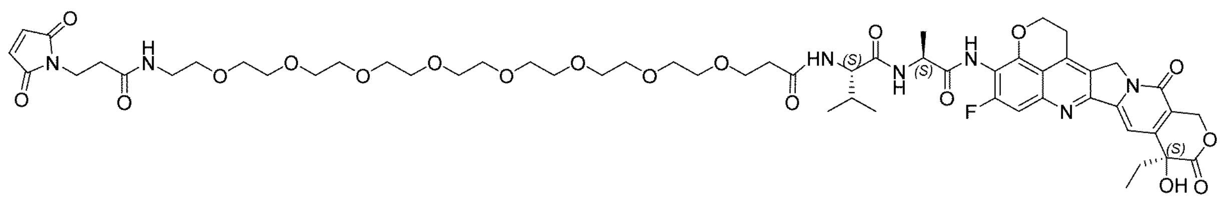

L-K

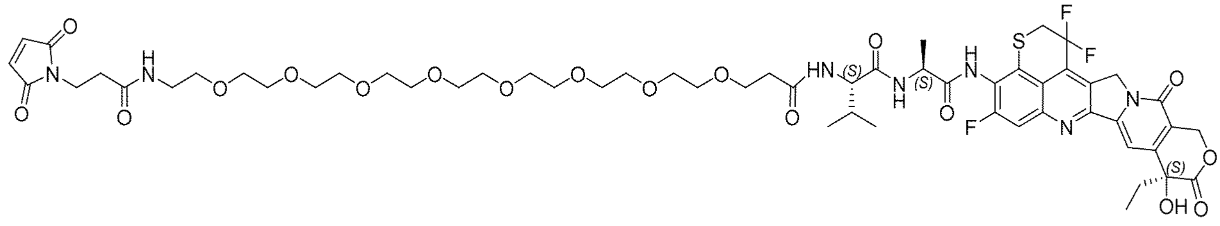

L-L

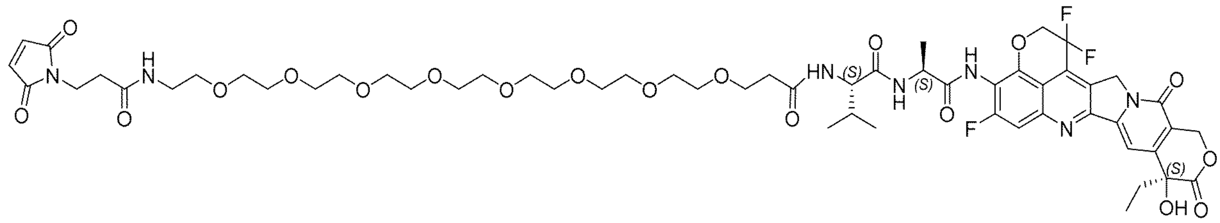

L-M

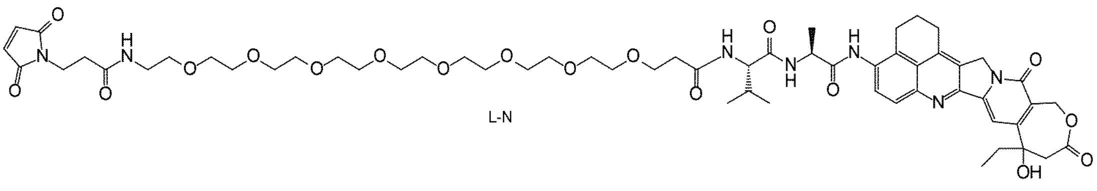

L-N

L-N

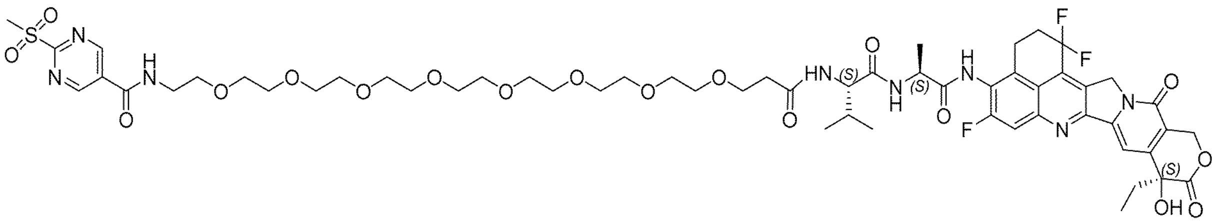

MWS-L1

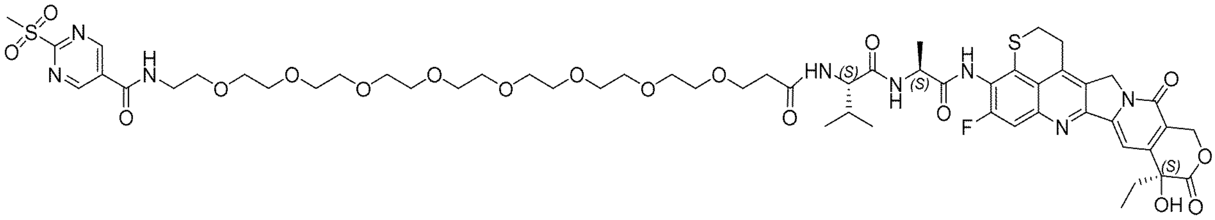

MWS-L2

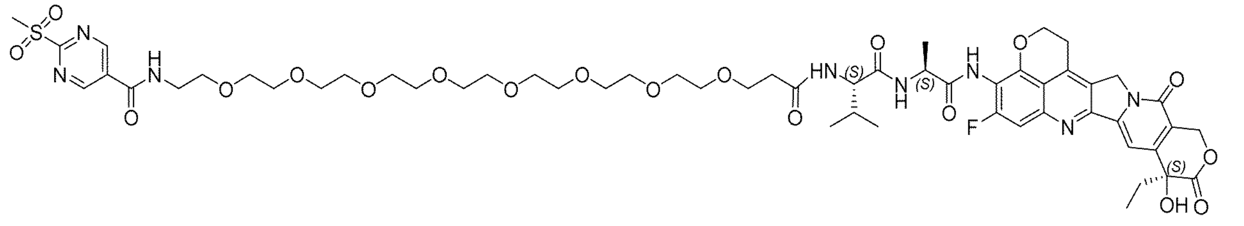

MWS-L3

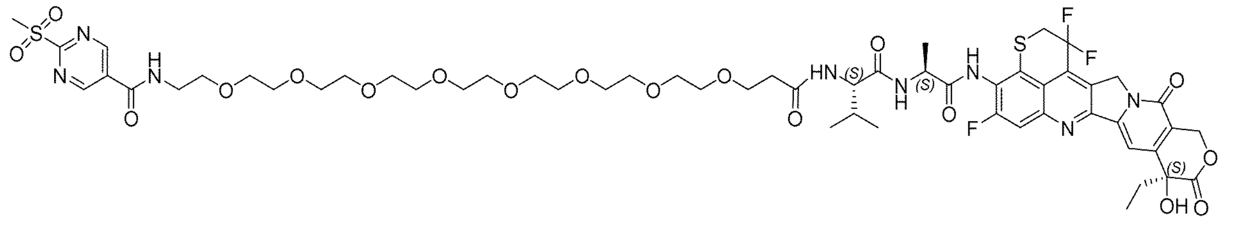

MWS-L4

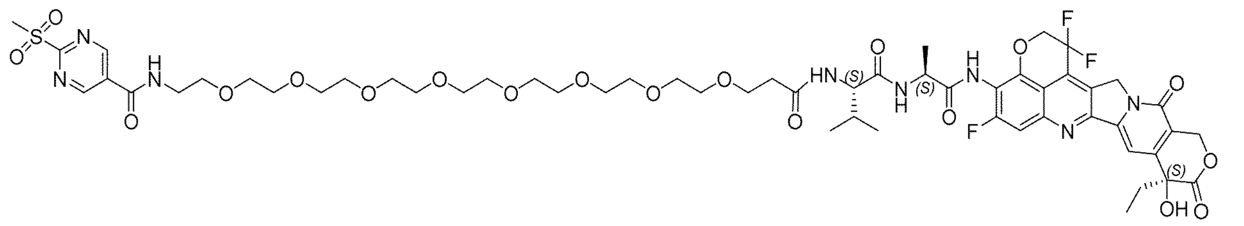

MWS-L5

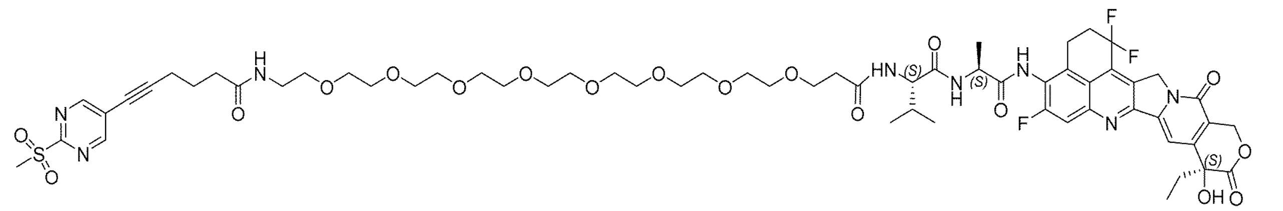

MWS-L6

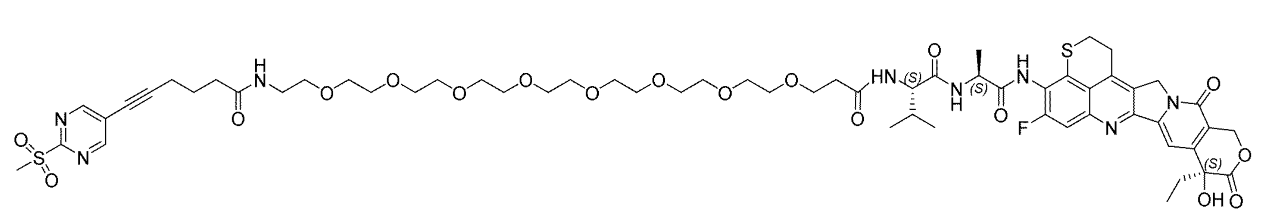

MWS-L7

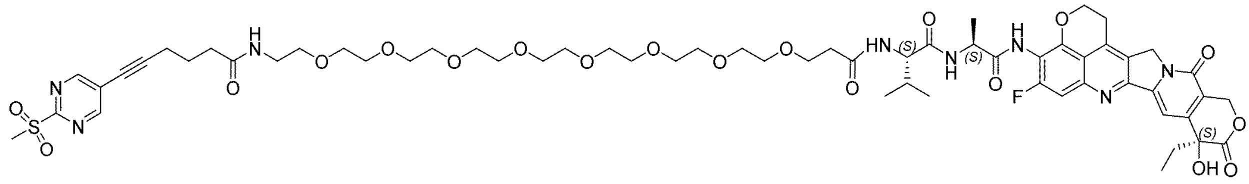

MWS-L8

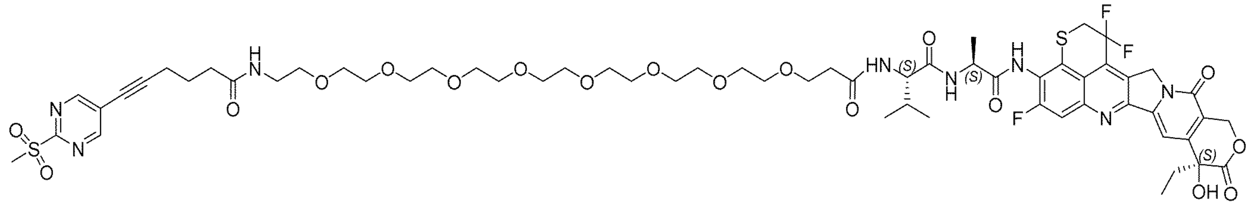

MWS-L9

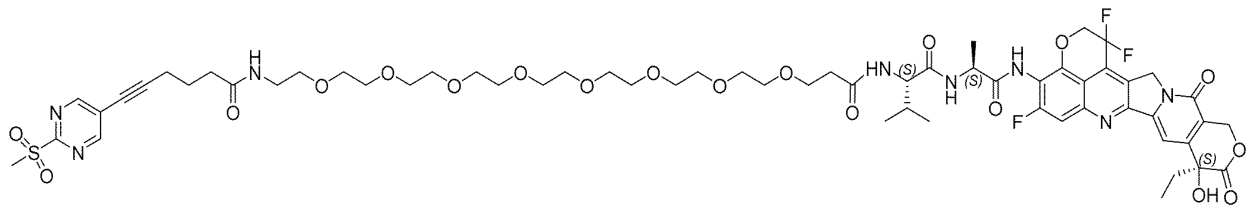

MWS-L10.

**15.** An antibody-drug conjugate prepared using the compound or a pharmaceutically acceptable salt, stereoisomer, solvate or prodrug thereof according to any one of claims 1 to 14 and an antibody or fragment thereof;

preferably, the antibody-drug conjugate has a structure represented by general formula

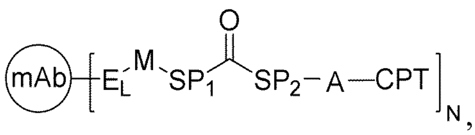

in which mAb means an antibody or fragment thereof, and groups M, $SP_1$, $SP_2$, A and CPT have the same meaning as defined in any one of claims 6 to 14 for the groups M, $SP_1$, $SP_2$, A and CPT; N is in the range of 1 to 10, preferably 1 to 8, more preferably 3 to 8; and
$E_L$ is selected from the group consisting of the following groups, in which

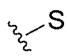

means bonding to the cysteine in mAb,  means bonding to M:
$E_L$-1a and/or $E_L$-1b:

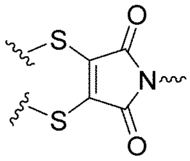 and/or 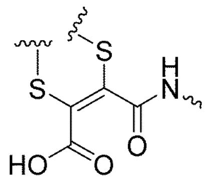 ; $E_L$-2: 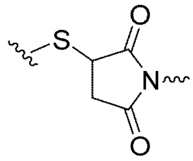 ; $E_L$-3:

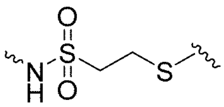 ; $E_L$-4: 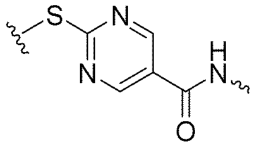 ; $E_L$-5: 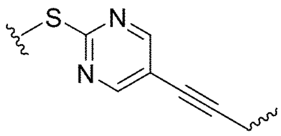 ; $E_L$-6:

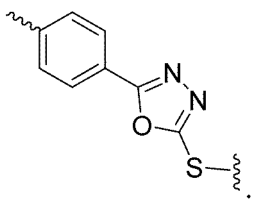.

**16.** The antibody-drug conjugate according to claim 15, characterized that, the antibody-drug conjugate has a structure represented by general formula VII or general formula VIII as follows:

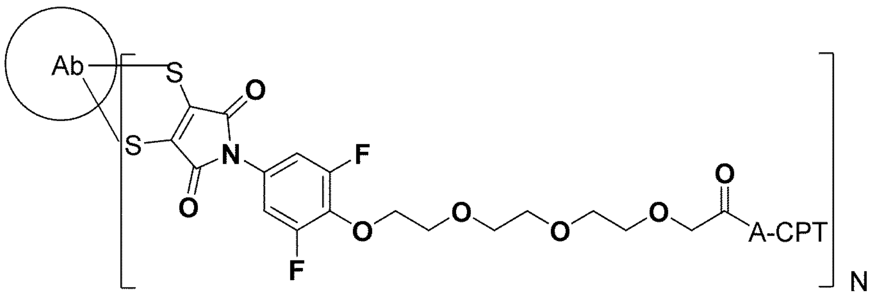

general formula VII

and/or

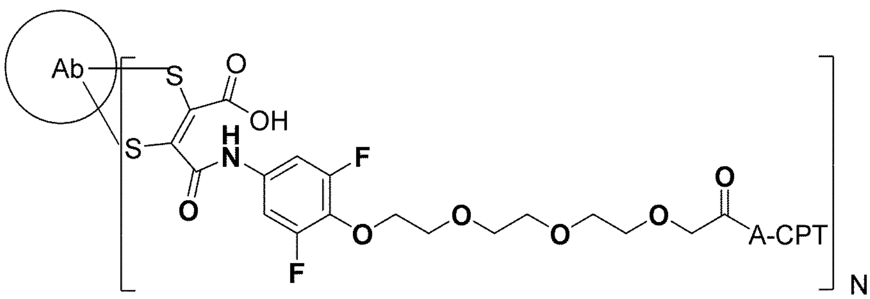

general formula VIII,

in general formula VII and general formula VIII, N is in the range of 1 to 10, preferably 1 to 8, more preferably 3 to 8.

**17.** A prodrug metabolite produced by the antibody-drug conjugate according to claim 15 or 16, characterized that, the prodrug metabolite is a compound represented by structural formula IX:

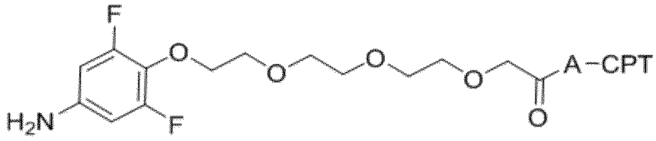

structural formula IX,

in structural formula IX, groups A and CPT have the same meaning as defined in any one of claims 6 to 14 for the groups A and CPT.

**18.** The prodrug metabolite according to claim 17, characterized that, the compound is a compound represented by structural formula IX-A:

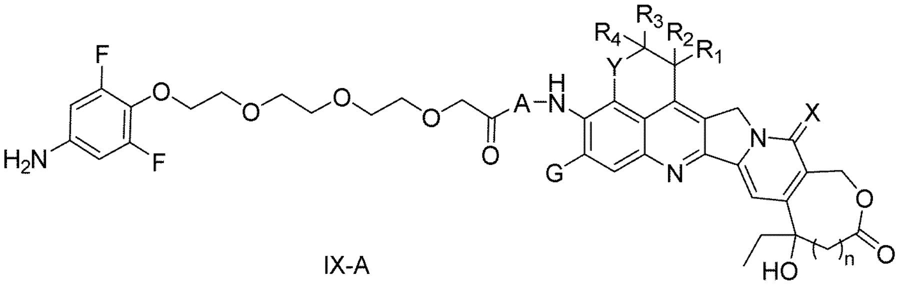

IX-A

structural formula IX-A,

in structural formula IX-A, groups A, G, Y, $R_1$, $R_2$, $R_3$, $R_4$, X, and n have the same meaning as defined in any one of claims 6 to 14 for the groups A, G, Y, $R_1$, $R_2$, $R_3$, $R_4$, X, and n;
preferably, the compound represented by structural formula IX-A is further a compound represented by structural formula IX-A-1:

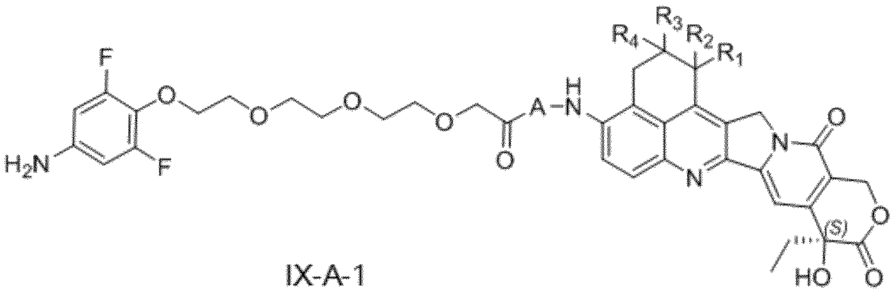

IX-A-1

structural formula IX-A-1;

alternatively, the compound is a compound represented by structural formula IX-B:

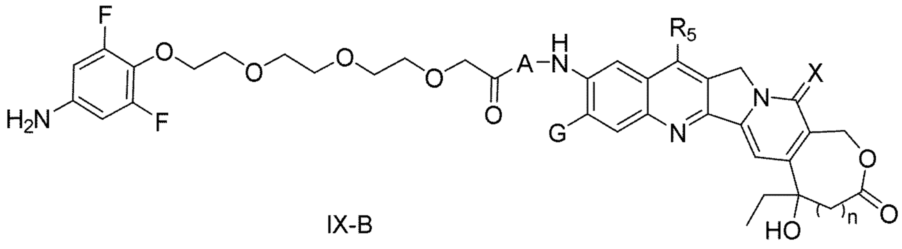

IX-B

structural formula IX-B,

in structural formula IX-B, groups A, G, $R_5$, X, and n have the same meaning as defined in any one of claims 6 to 14 for the groups A, G, $R_5$, X, and n;

alternatively, the compound is a compound represented by structural formula IX-C:

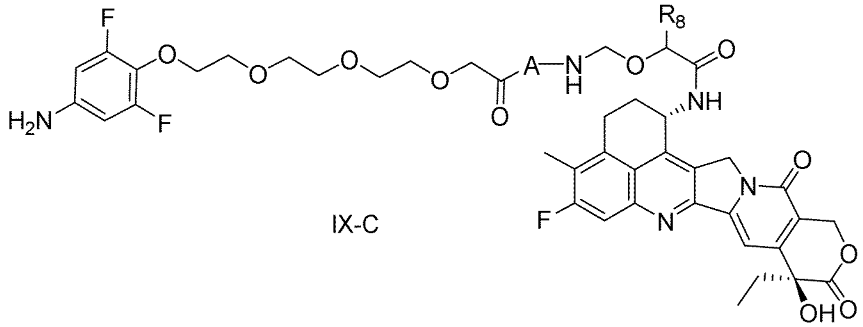

IX-C

structural formula IX-C,

in structural formula IX-C, groups A and $R_8$ have the same meaning as defined in any one of claims 6 to 14 for the groups A and $R_8$.

**19.** Use of the compound or a pharmaceutically acceptable salt, stereoisomer, solvate or prodrug thereof or the antibody-drug conjugate or the prodrug metabolite according to any one of claims 1 to 18 in the manufacture of a medicament for the treatment of a tumor.

**20.** A method for treating a tumor with the compound or a pharmaceutically acceptable salt, stereoisomer, solvate or prodrug thereof or the antibody-drug conjugate according to any one of claims 1 to 16, comprising administering to a subject in need thereof the compound or a pharmaceutically acceptable salt, stereoisomer, solvate or prodrug thereof or the antibody-drug conjugate.

**FIG. 1**

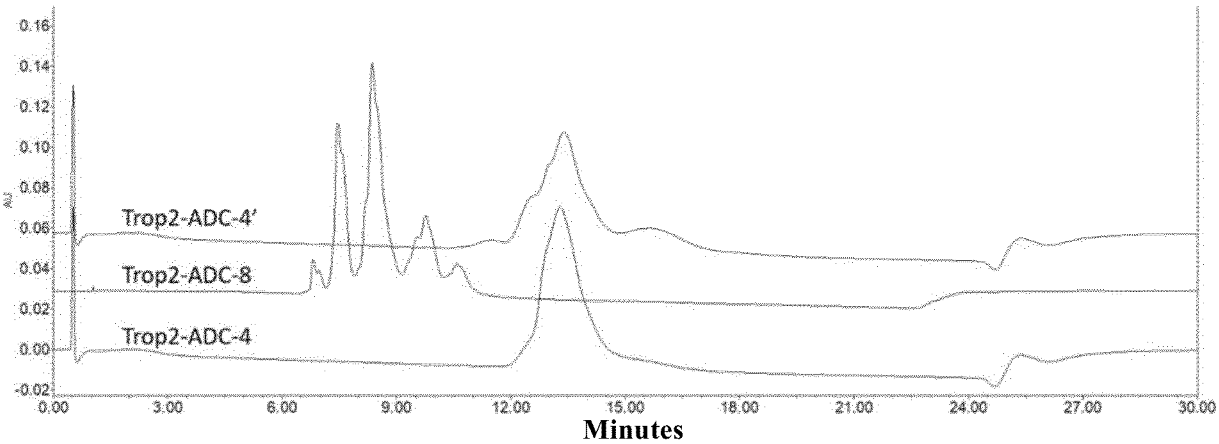
0.16
0.14
0.12
0.10
0.08
0.06
0.04
0.02
0.00
-0.02
AU
Trop2-ADC-4'
Trop2-ADC-8
Trop2-ADC-4
0.00
3.00
6.00
9.00
12.00
15.00
18.00
21.00
24.00
27.00
30.00
Minutes

**FIG. 2**

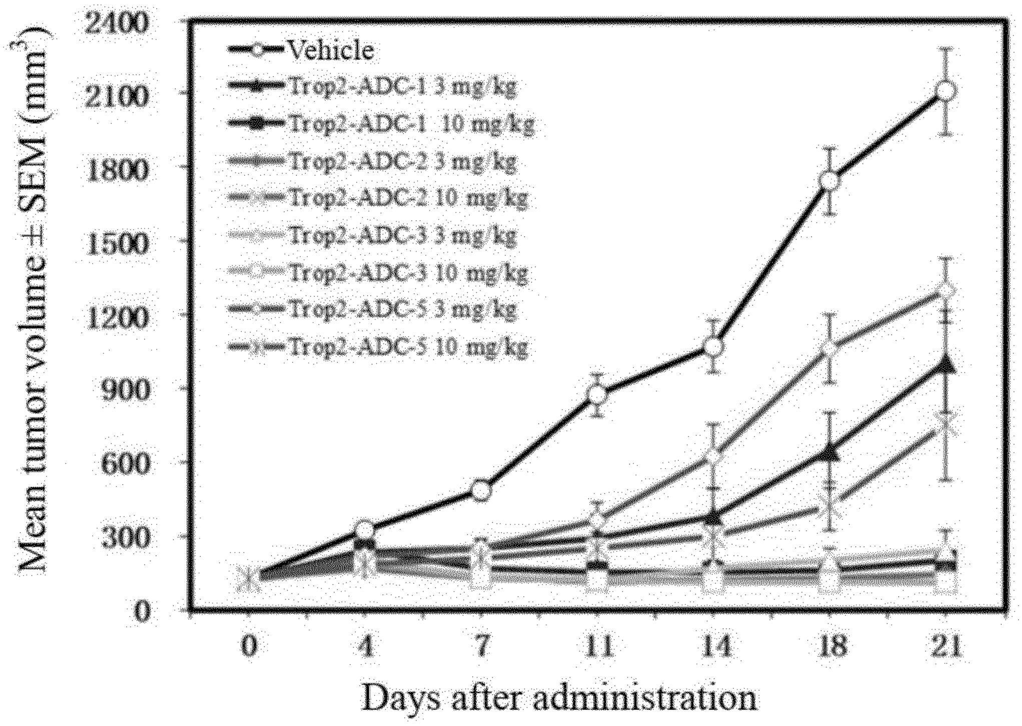
Mean tumor volume ± SEM (mm3)
2400
2100
1800
1500
1200
900
600
300
0
Vehicle
Trop2-ADC-1 3 mg/kg
Trop2-ADC-1 10 mg/kg
Trop2-ADC-2 3 mg/kg
Trop2-ADC-2 10 mg/kg
Trop2-ADC-3 3 mg/kg
Trop2-ADC-3 10 mg/kg
Trop2-ADC-5 3 mg/kg
Trop2-ADC-5 10 mg/kg
0
4
7
11
14
18
21
Days after administration

FIG. 3

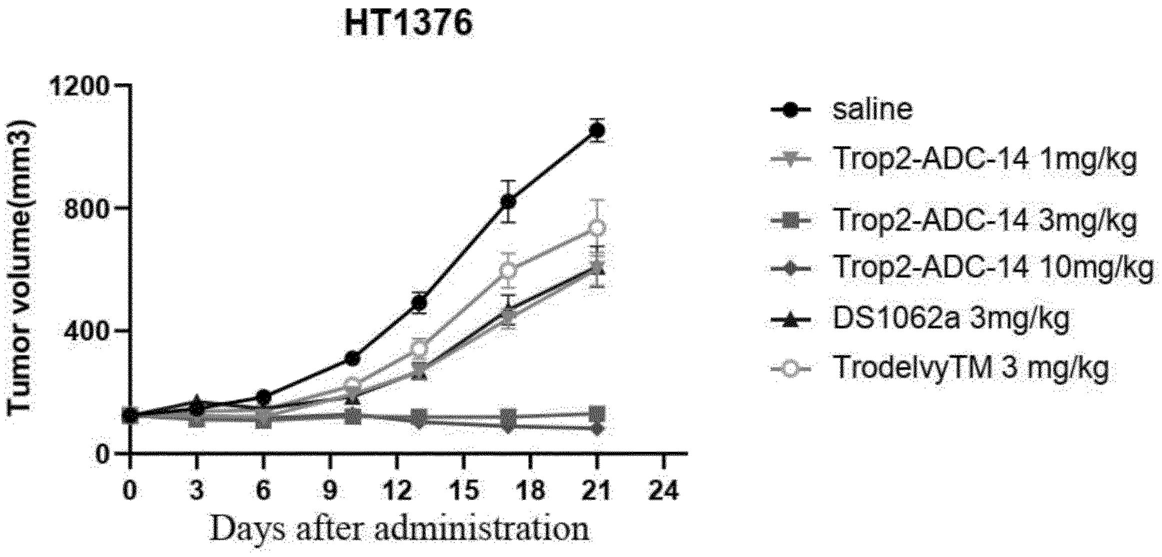
HT1376
Tumor volume(mm3)
1200
800
400
0
0 3 6 9 12 15 18 21 24
Days after administration
saline
Trop2-ADC-14 1mg/kg
Trop2-ADC-14 3mg/kg
Trop2-ADC-14 10mg/kg
DS1062a 3mg/kg
TrodelvyTM 3 mg/kg

FIG. 4

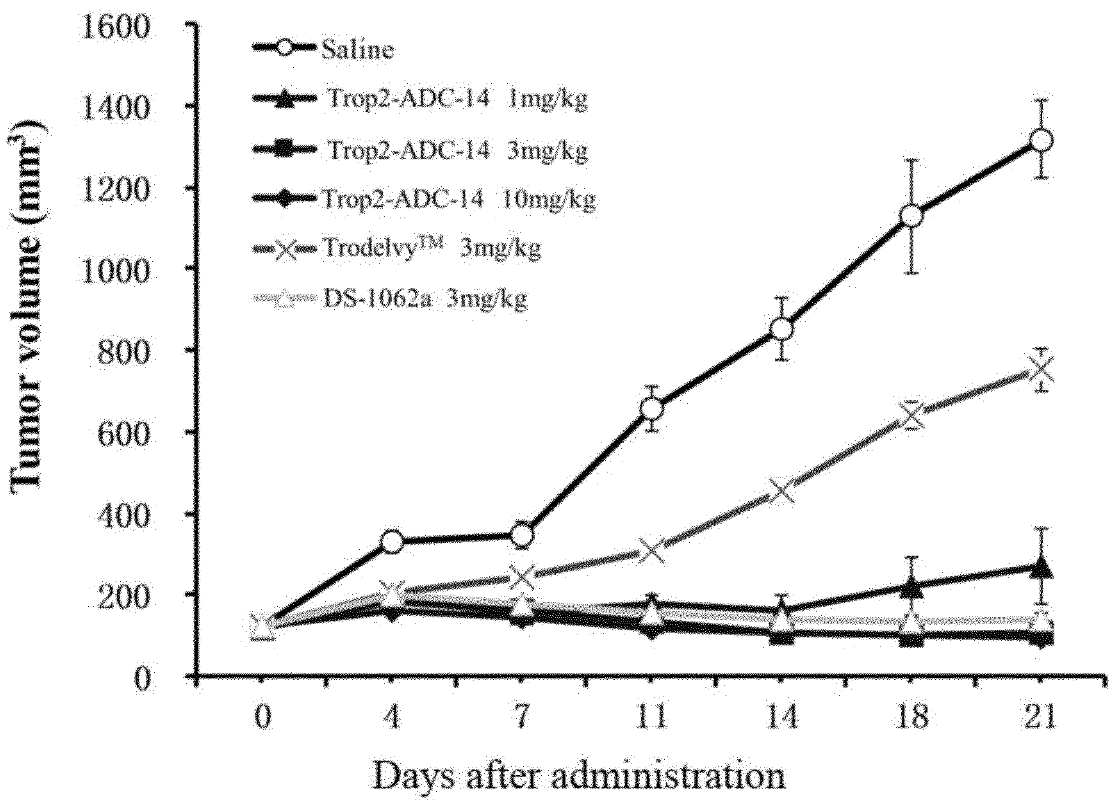
Tumor volume (mm3)
1600
1400
1200
1000
800
600
400
200
0
0 4 7 11 14 18 21
Days after administration
Saline
Trop2-ADC-14 1mg/kg
Trop2-ADC-14 3mg/kg
Trop2-ADC-14 10mg/kg
TrodelvyTM 3mg/kg
DS-1062a 3mg/kg

FIG. 5

5A

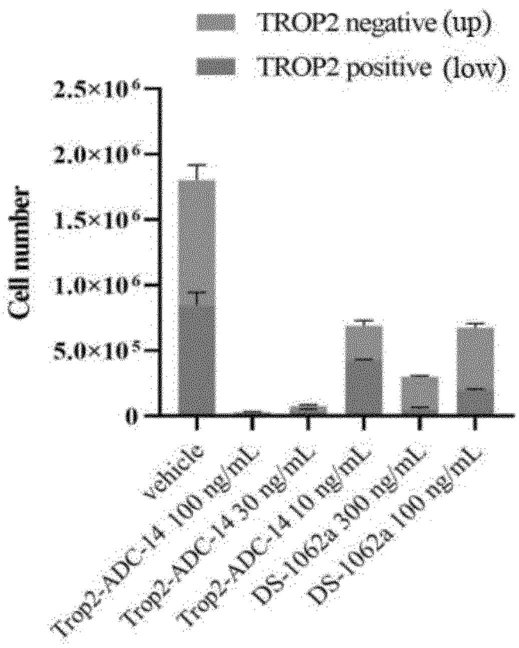


5B

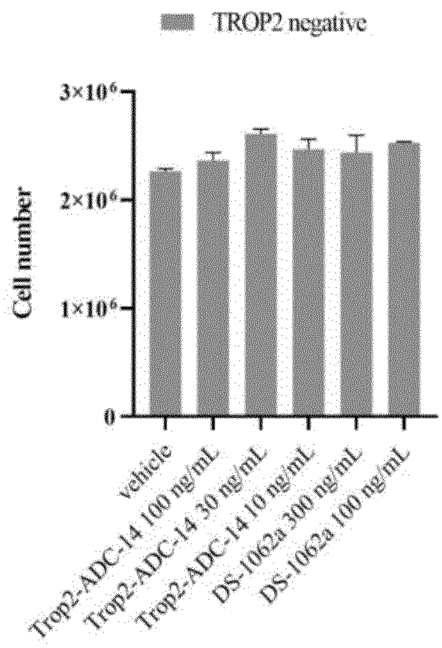


FIG. 6

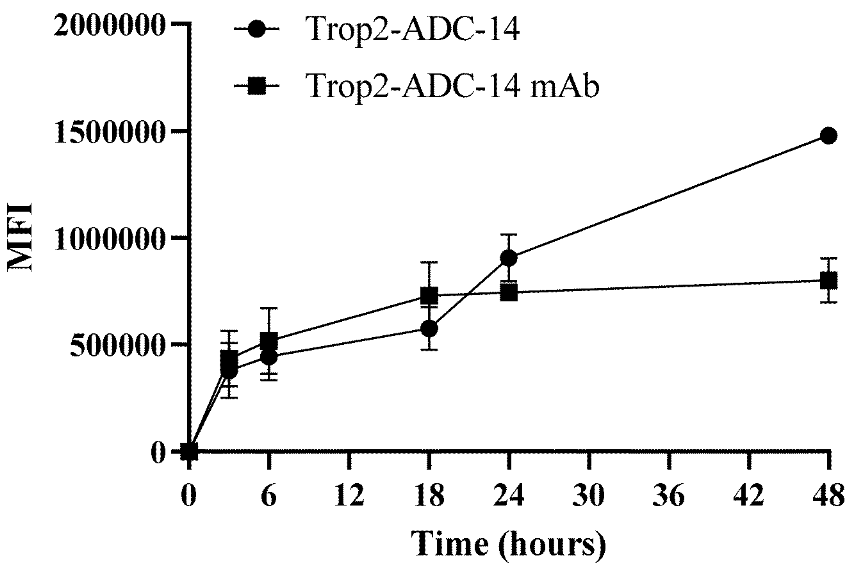

FIG. 7

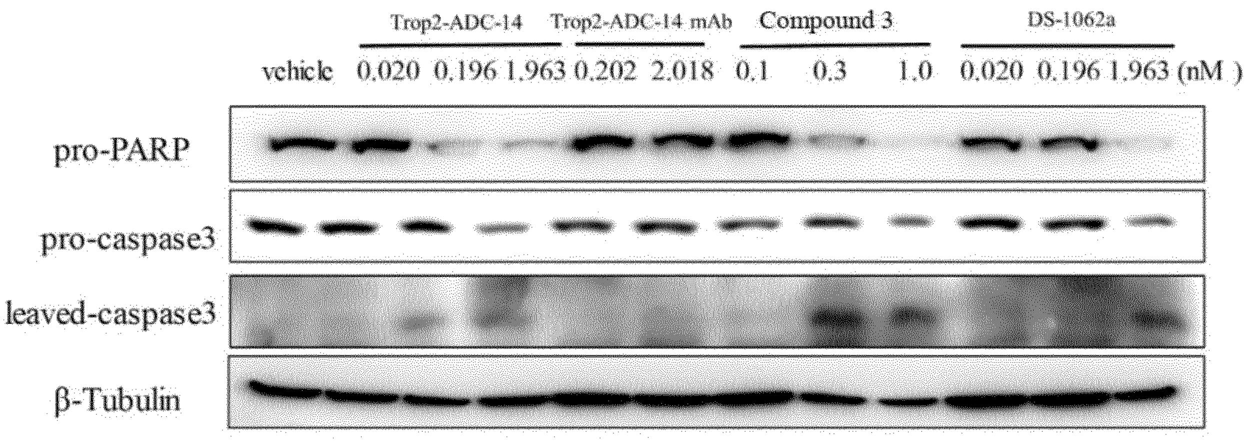
Trop2-ADC-14
Trop2-ADC-14 mAb
Compound 3
DS-1062a
vehicle 0.020 0.196 1.963 0.202 2.018 0.1 0.3 1.0 0.020 0.196 1.963 (nM )
pro-PARP
pro-caspase3
leaved-caspase3
β-Tubulin

FIG. 8

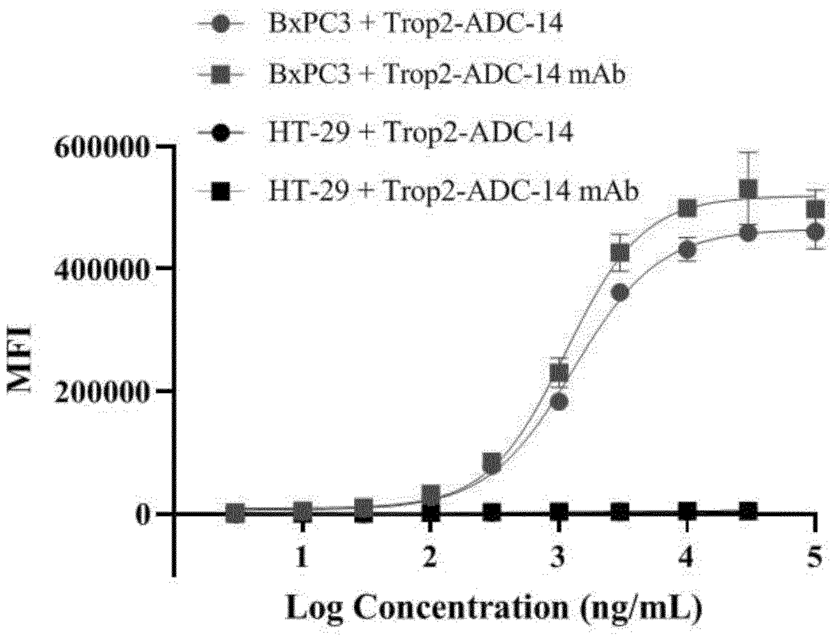
BxPC3 + Trop2-ADC-14
BxPC3 + Trop2-ADC-14 mAb
HT-29 + Trop2-ADC-14
HT-29 + Trop2-ADC-14 mAb
600000
400000
200000
0
MFI
1
2
3
4
5
Log Concentration (ng/mL)

## INTERNATIONAL SEARCH REPORT

International application No.
**PCT/CN2022/139765**

**A. CLASSIFICATION OF SUBJECT MATTER**

C07D491/22(2006.01)i;C07D495/22(2006.01)i;A61K47/68(2017.01)i;A61P35/00(2006.01)i

According to International Patent Classification (IPC) or to both national classification and IPC

**B. FIELDS SEARCHED**

Minimum documentation searched (classification system followed by classification symbols)

C07D,A61K,A61P

Documentation searched other than minimum documentation to the extent that such documents are included in the fields searched

Electronic data base consulted during the international search (name of data base and, where practicable, search terms used)

WPI, CNPAT, CAPLUS(STN), REGISTRY(STN), MARPAT(STN), CNKI, 万方, WANFANG: 喜树碱, 类似物, 衍生物, 偶联物, 缀合物, 连洁子, 拓扑异构酶, 抑制剂, 肿瘤, 癌, camptothecin, analog+, derivative?, tumor?, antitumor?, cancer?, conjugate , topoisomerase inhibitor?, 结构式检索, search for structural formula

**C. DOCUMENTS CONSIDERED TO BE RELEVANT**

| Category* | Citation of document, with indication, where appropriate, of the relevant passages | Relevant to claim No. |
|---|---|---|
| X | EP 0495432 A1 (DAIICHI PHARMACEUTICAL CO., LTD. et al.) 22 July 1992 (1992-07-22)<br>embodiments 26 and 30-31, claim 1, and description, page 1 | 1-5, 19-20 |
| X | CN 113631196 A (MEDIMMUNE LIMITED) 09 November 2021 (2021-11-09)<br>claims 1, 10 and 20-25, and example 1 | 1, 6-10, 12, 14-20 |
| Y | CN 113631196 A (MEDIMMUNE LIMITED) 09 November 2021 (2021-11-09)<br>claims 1, 10 and 20-25, and example 1 | 6-20 |
| X | WO 2021148501 A1 (MEDIMMUNE LIMITED) 29 July 2021 (2021-07-29)<br>claims 1 and 21-25, and example 2 | 1, 19-20 |
| X | WO 2021148500 A1 (MEDIMMUNE LTD.) 29 July 2021 (2021-07-29)<br>description, pages 46-48, and claim 1 | 1, 19-20 |
| Y | CN 108101825 A (SHANGHAI QINGRUN PHARMACEUTICAL TECHNOLOGY CO., LTD.) 01 June 2018 (2018-06-01)<br>claims 1-4 | 6-20 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 February 2023** | **20 February 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

INTERNATIONAL SEARCH REPORT

International application No.
PCT/CN2022/139765

C. DOCUMENTS CONSIDERED TO BE RELEVANT

| Category* | Citation of document, with indication, where appropriate, of the relevant passages | Relevant to claim No. |
|---|---|---|
| X | CN 1352646 A (UNIVERSITY OF PITTSBURGH) 05 June 2002 (2002-06-05) embodiment 22, and claims 18 and 56 | 3-4, 19-20 |
| X | SUGIMORI, Masamichi et al. "Synthesis and Antitumor Activity of Ring A- and F-Modified Hexacyclic Camptothecin Analogues" *Journal of Medicinal Chemistry,* Vol. 41, No. (13), 06 March 1998 (1998-03-06), pp. 2308-2318 | 1, 5, 19-20 |
| X | ADAMS, David J. et al. "Camptothecin Analogs with Enhanced Activity against Hyman Breast Cancercells.I.Correlation of Potency with Lipophilicity and Persistence in the Cleavage Complex" *Cancer Chemother Pharmacol,* Vol. vol. 57, 20 August 2005 (2005-08-20), pp. 135-144 | 3-4, 19-20 |

Form PCT/ISA/210 (second sheet) (July 2022)

**INTERNATIONAL SEARCH REPORT**

International application No.
**PCT/CN2022/139765**

**Box No. II** **Observations where certain claims were found unsearchable (Continuation of item 2 of first sheet)**

This international search report has not been established in respect of certain claims under Article 17(2)(a) for the following reasons:

1. ☑ Claims Nos.: **20**
because they relate to subject matter not required to be searched by this Authority, namely:

Claim 20 relates to a method for treating a disease, and therefore does not comply with PCT Rule 39.1(iv). The search is carried out on the basis that the designation of the subject matter of claim 20 is amended to a corresponding pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/139765**

| Patent document cited in search report | | | Publication date (day/month/year) | Patent family member(s) | | | Publication date (day/month/year) |
|---|---|---|---|---|---|---|---|
| EP | 0495432 | A1 | 22 July 1992 | DE | 69211023 | D1 | 04 July 1996 |
| | | | | DE | 69211023 | T2 | 09 January 1997 |
| | | | | CA | 2059305 | A1 | 17 July 1992 |
| | | | | CA | 2059305 | C | 31 July 2001 |
| | | | | NO | 920201 | D0 | 15 January 1992 |
| | | | | NO | 920201 | L | 17 July 1992 |
| | | | | NO | 180448 | B | 13 January 1997 |
| | | | | NO | 180448 | C | 23 April 1997 |
| | | | | EP | 0495432 | B1 | 29 May 1996 |
| | | | | ES | 2090366 | T3 | 16 October 1996 |
| | | | | HK | 1001561 | A1 | 26 June 1998 |
| | | | | AU | 1018592 | A | 23 July 1992 |
| | | | | AU | 640549 | B2 | 26 August 1993 |
| | | | | GR | 3020395 | T3 | 30 September 1996 |
| | | | | KR | 920014816 | A | 25 August 1992 |
| | | | | KR | 100191193 | B1 | 15 June 1999 |
| | | | | AT | 138661 | T | 15 June 1996 |
| | | | | FI | 920194 | A0 | 16 January 1992 |
| | | | | FI | 920194 | A | 17 July 1992 |
| | | | | FI | 103047 | B | 15 April 1999 |
| | | | | JPH | 0559061 | A | 09 March 1993 |
| | | | | JP | 3008226 | B2 | 14 February 2000 |
| | | | | IE | 920079 | A1 | 29 July 1992 |
| | | | | RU | 2071476 | C1 | 10 January 1997 |
| | | | | DK | 0495432 | T3 | 21 October 1996 |
| CN | 113631196 | A | 09 November 2021 | RS | 63715 | B1 | 30 November 2022 |
| | | | | JP | 2022528851 | A | 16 June 2022 |
| | | | | US | 2022211863 | A1 | 07 July 2022 |
| | | | | LT | 3946464 | T | 10 November 2022 |
| | | | | PT | 3946464 | T | 16 November 2022 |
| | | | | TW | 202104235 | A | 01 February 2021 |
| | | | | HRP | 20221280 | T1 | 23 December 2022 |
| | | | | IL | 286291 | A | 31 October 2021 |
| | | | | US | 2021322401 | A1 | 21 October 2021 |
| | | | | US | 11446292 | B2 | 20 September 2022 |
| | | | | CA | 3133757 | A1 | 08 October 2020 |
| | | | | AU | 2020252034 | A1 | 11 November 2021 |
| | | | | PL | 3946464 | T3 | 19 December 2022 |
| | | | | DK | 3946464 | T3 | 31 October 2022 |
| | | | | BR | 112021018986 | A2 | 18 January 2022 |
| | | | | EP | 3946464 | A1 | 09 February 2022 |
| | | | | EP | 3946464 | B1 | 31 August 2022 |
| | | | | CR | 20210541 | A | 20 December 2021 |
| | | | | ECSP | 21078204 | A | 30 November 2021 |
| | | | | CO | 2021014566 | A2 | 19 November 2021 |
| | | | | WO | 2020200880 | A1 | 08 October 2020 |
| | | | | SG | 11202110524 | VA | 28 October 2021 |
| | | | | CL | 2021002498 | A1 | 03 June 2022 |
| | | | | ES | 2930295 | T3 | 09 December 2022 |
| | | | | US | 2020306243 | A1 | 01 October 2020 |

Form PCT/ISA/210 (patent family annex) (July 2022)

**INTERNATIONAL SEARCH REPORT**
**Information on patent family members**

International application No.
**PCT/CN2022/139765**

| Patent document cited in search report | | | Publication date (day/month/year) | Patent family member(s) | | | Publication date (day/month/year) |
|---|---|---|---|---|---|---|---|
| | | | | ZA | 202106612 | B | 31 August 2022 |
| | | | | KR | 20210144845 | A | 30 November 2021 |
| WO | 2021148501 | A1 | 29 July 2021 | TW | 202140076 | A | 01 November 2021 |
| | | | | IL | 294645 | A | 01 September 2022 |
| | | | | KR | 20220130191 | A | 26 September 2022 |
| | | | | CO | 2022011178 | A2 | 30 August 2022 |
| | | | | BR | 112022013966 | A2 | 11 October 2022 |
| | | | | AU | 2021211892 | A1 | 08 September 2022 |
| | | | | CA | 3167373 | A1 | 29 July 2021 |
| | | | | ECSP | 22064855 | A | 30 September 2022 |
| | | | | EP | 4093438 | A1 | 30 November 2022 |
| | | | | CR | 20220393 | A | 16 September 2022 |
| WO | 2021148500 | A1 | 29 July 2021 | CA | 3166732 | A1 | 29 July 2021 |
| | | | | AU | 2021210570 | A1 | 01 September 2022 |
| | | | | KR | 20220130749 | A | 27 September 2022 |
| | | | | BR | 112022014391 | A2 | 13 September 2022 |
| | | | | EP | 4093439 | A1 | 30 November 2022 |
| CN | 108101825 | A | 01 June 2018 | | None | | |
| CN | 1352646 | A | 05 June 2002 | US | 6136978 | A | 24 October 2000 |
| | | | | JP | 2002532505 | A | 02 October 2002 |
| | | | | JP | 4638987 | B2 | 23 February 2011 |
| | | | | KR | 20010101250 | A | 14 November 2001 |
| | | | | KR | 100750693 | B1 | 22 August 2007 |
| | | | | NZ | 512210 | A | 24 December 2004 |
| | | | | AU | 3123600 | A | 03 July 2000 |
| | | | | AU | 777786 | B2 | 28 October 2004 |
| | | | | EP | 1140948 | A1 | 10 October 2001 |
| | | | | EP | 1140948 | A4 | 02 January 2008 |
| | | | | WO | 0035924 | A1 | 22 June 2000 |
| | | | | CA | 2353822 | A1 | 22 June 2000 |
| | | | | CA | 2353822 | C | 31 May 2011 |
| | | | | HK | 1044156 | A1 | 11 October 2002 |
| | | | | HK | 1044156 | B | 08 April 2005 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202111544686
- US 20140170063 A1
- WO 2020200880 A1
- CN 106349233 A
- WO 2021148501 A
- US 2004266803 A
- US 20190151328 A1
- WO 2018095422 A1
- WO 2020200880 A
- CN 105849126 A
- US 10227417 B2
- CN 105849126 B
- CN 105829346 B


### Non-patent literature cited in the description


- *Bioorganic & Medicinal Chemistry,* 2010, vol. 18 (9), 3140-3146
- *Journal of Medicinal chemistry,* 1998, vol. 41 (13), 2308-2318
- *Journal of Medicinal Chemistry,* 1989, vol. 32 (6), 1217-1230
- *J. Med. Chem.,* 2008, vol. 51, 3040-3044
- *Clin Cancer Res.,* 15 October 2004, vol. 10 (20), 7063-70
- *CHEMICAL ABSTRACTS,* 180288-69-1